# EUROPEAN PATENT SPECIFICATION

(11) **EP 3 004 396 B1**
(45) Date of publication and mention of the grant of the patent: **16.10.2019**
(21) Application number: 14808136.7
(22) Date of filing: 06.06.2014
(51) Int. Cl.: A61K 38/06, A61K 38/17, A61K 31/436, A61K 31/282, A61K 31/4412, A61K 31/444, A61K 31/513, A61K 31/52, A61K 31/5377, A61K 31/7068, A61K 31/7076, C12Q 1/6886, A61K 45/06, A61P 35/00

(54) **COMPOSITIONS FOR THE TREATMENT OF CANCER**
ZUSAMMENSETZUNGEN ZUR KREBSBEHANDLUNG
COMPOSITIONS POUR LE TRAITEMENT DU CANCER

(30) Priority: 06.06.2013 US 201361831758 P
(43) Date of publication of application: 13.04.2016
(73) Proprietor: The General Hospital Corporation, Boston, MA 02114 (US)
(72) Inventor: WHETSTINE, Johnathan R., Winchester, Massachusetts 01890 (US); ATABAKHSH, Elnaz, Charlestown, MA 02129 (US)
(74) Representative: CMS Cameron McKenna Nabarro Olswang LLP
(86) International application number: PCT/US2014/041359
(87) International publication number: WO 2014/197835

(56) References cited:
- WO-A1-2012/037212
- WO-A1-2014/139326
- US-A1- 2011 172 107
- US-A1- 2012 214 757
- DING XIANGMING ET AL: "Epigenetic Activation of AP1 Promotes Squamous Cell Carcinoma Metastasis", SCIENCE SIGNALING, vol. 6, no. 273, April 2013 (2013-04), XP055333810,
- BEI-XU LI ET AL: "Effects of RNA interference-mediated gene silencing of JMJD2A on human breast cancer cell line MDA-MB-231 in vitro", JOURNAL OF EXPERIMENTAL & CLINICAL CANCER RESEARCH, BIOMED CENTRAL LTD, LONDON UK, vol. 30, no. 1, 3 October 2011 (2011-10-03), page 90, XP021114761, ISSN: 1756-9966, DOI: 10.1186/1756-9966-30-90
- W. L. BERRY ET AL: "KDM4/JMJD2 Histone Demethylases: Epigenetic Regulators in Cancer Cells", CANCER RESEARCH, vol. 73, no. 10, 3 May 2013 (2013-05-03), pages 2936-2942, XP055140245, ISSN: 0008-5472, DOI: 10.1158/0008-5472.CAN-12-4300
- SHANNON ET AL.: 'Tumour hypoxia, chemotherapeutic resistance and hypoxia-related therapies.' CANCER TREATMENT REVIEWS vol. 29, no. 4, 01 August 2003, pages 297 - 307, XP055300461 DOI: 10.1016/S0305-7372(03)00003-3
- BLACK ET AL.: 'Conserved Antagonism between JMJD2A/KDM4A and HP1.gamma. during Cell Cycle Progression.' MOLECULAR CELL vol. 40, no. 5, 10 December 2010, pages 736 - 748, XP055300468 DOI: 10.1016/J.MOLCEL.2010.11.008
- COLLINS ET AL.: 'Transcriptional Consequences of Topoisomerase Inhibition.' MOL CELL BIOL vol. 21, no. 24, 01 December 2001, pages 8437 - 8451, XP055300470 DOI: 10.1128/MCB.21.24.8437-8451.2001

### SEQUENCE LISTING

<110> THE GENERAL HOSPITAL CORPORATION
<120> METHODS AND COMPOSITIONS FOR THE TREATMENT OF CANCER
<130> 030258-076041-PCT
<140>
   <141>
<150> 61/831,758
   <151> 2013-06-06
<160> 63
<170> PatentIn version 3.5
<210> 1
   <211> 4526
   <212> DNA
   <213> Homo sapiens
<400> 1
   acggctgcgc agatgccgac tttagaggag gcggagtttc ggccttcgcc tgctggaaaa      60
   gcagtaggat cggccagtgg cgacagcagg agctgagcct aagccctggc ggggctttgg     120
   gctgtagatt cctgtctgac taaagggacc tcaaaaagga gggaaaatgg cttctgagtc     180
   tgaaactctg aatcccagtg ctaggataat gaccttttat ccaactatgg aagagttccg     240
   aaacttcagt agatacattg cctacattga atcccaagga gctcatcggg cagggctagc     300
   caaggttgtt cctccaaaag agtggaagcc acgagcatcc tatgatgaca ttgatgattt     360
   ggtcattcct gcccccattc aacagctggt gacggggcag tctggcctct ttactcagta     420
   caacatacag aagaaagcca tgactgttcg agagttccgc aagatagcca atagcgataa     480
   gtactgtacc ccacgctata gtgagtttga agagctcgag cggaaatact ggaaaaatct     540
   tacattcaat cctccaatct atggtgcaga tgtgaatggt accctctatg aaaagcatgt     600
   tgatgagtgg aatattggcc ggctgagaac aatcctggac ttggtggaaa aggagagtgg     660
   gatcaccatt gagggtgtga acaccccata cctgtacttt ggcatgtgga agacatcctt     720
   tgcttggcac actgaagaca tggacctcta cagcatcaac tacctgcact ttggagaacc     780
   aaagtcctgg tactctgttc cacctgagca tggaaagcgg ttggaacgcc tcgccaaagg     840
   ctttttccca ggaagtgctc aaagctgtga ggcatttctc cgccacaaga tgaccctgat     900
   ttccccgtta atgctgaaga aatatggaat tccctttgac aaggtgactc aagaggctgg     960
   agagtttatg atcactttcc cttatggtta ccatgccggc tttaaccatg gttttaactg    1020
   tgcggagtct accaattttg ctacccgtcg gtggattgag tacggcaagc aagctgtgct    1080
   gtgctcctgt agaaaggaca tggtgaagat ctccatggat gtgtttgtga gaaagttcca    1140
   gccagaaagg tacaaacttt ggaaagctgg gaaggacaac acagttattg accatactct    1200
   gcccacgcca gaagcagctg agtttcttaa ggagagtgaa ctgcctccaa gagctggcaa 1260
   cgaggaggag tgcccagagg aggacatgga aggggtggag gatggagagg aaggagacct 1320
   gaagacaagc ctggccaagc accgaatagg gacaaagagg caccgagttt gtcttgaaat 1380
   accacaggag gtgagtcaga gtgagctctt ccccaaggag gatctgagtt ctgagcagta 1440
   tgagatgacg gagtgcccgg cagccctcgc ccctgtgagg cccacccata gctctgtgcg 1500
   gcaagttgag gatggtctta ccttcccaga ttattctgac tccactgaag tcaaatttga 1560
   agagcttaaa aatgtcaaac tagaagagga ggatgaggag gaagaacaag cagcagctgc 1620
   cttggatctt tctgtgaatc ctgcgtctgt agggggacgc cttgtcttct caggctccaa 1680
   aaagaaatca tcttctagcc tgggctctgg ctcttcacgg gattctatct cttctgattc 1740
   agaaactagt gagcctctct cctgccgagc ccaagggcaa acgggagttc tcactgtgca 1800
   cagttatgcc aaaggggatg gcagggtcac tgtgggagag ccatgcacga ggaagaaagg 1860
   aagcgccgct agaagtttca gtgagcggga gctggcagag gttgcagatg aatacatgtt 1920
   ttccctagaa gagaataaga agtccaaggg acgccgtcag cctttaagca agctcccccg 1980
   ccatcaccca cttgtgctgc aggagtgtgt cagtgatgat gagacatctg aacagctgac 2040
   ccctgaggaa gaggctgagg agacagaggc ctgggccaag cctctgagcc aactgtggca 2100
   gaaccgacct ccaaactttg aggctgagaa ggaattcaat gagaccatgg cccaacaggc 2160
   ccctcactgc gctgtctgta tgatcttcca gacttatcat caggttgaat ttggaggctt 2220
   taatcagaac tgtggaaatg cttcagattt agccccccag aagcagagga ccaagccatt 2280
   gattccagaa atgtgcttca cttcgactgg ctgcagcacg gacatcaacc tttctactcc 2340
   ttatcttgag gaggatggca ccagcatact cgtttcctgc aagaagtgca gcgtccgggt 2400
   ccatgccagt tgctatgggg tcccccctgc aaaggcttct gaagactgga tgtgttctcg 2460
   gtgttcagcc aatgccctag aggaggactg ctgtttatgc tcattacgag gaggggccct 2520
   gcagagagca aatgatgaca ggtgggtcca cgtttcatgt gctgtggcaa ttctggaagc 2580
   aaggtttgtc aacattgcag aaagaagtcc ggtggatgtg agcaaaatcc ccctgccccg 2640
   cttcaaactg aaatgtatct tctgtaagaa gcggaggaaa agaactgctg gctgctgtgt 2700
   gcagtgttct cacggccgct gcccaactgc cttccatgtg agctgcgccc aggctgccgg 2760
   tgtgatgatg cagcctgacg actggccttt tgtggtcttc attacctgct ttcggcacaa 2820
   gattcctaat ttggagcgtg ccaagggggc cttgcaaagc atcactgcag gccagaaagt 2880
   cattagcaag cataagaacg ggcgcttcta ccagtgtgaa gtggtcaggc tcaccaccga 2940
   gaccttctat gaagtcaact ttgatgatgg ctccttcagc gacaatcttt atcctgagga 3000
   catagtgagc caggactgtc tccagtttgg tcctcctgct gaaggggaag tggtccaagt 3060
   gagatggaca gacggccaag tctatggagc caagtttgtg gcctcccacc ctatccaaat 3120
   gtaccaggtg gagtttgagg atggctcaca acttgtggtt aagagagatg atgtatacac 3180
   actggatgaa gagcttccca agagagtcaa atctagactg tcagtagcct cagacatgcg 3240
   cttcaatgag attttcacag agaaagaggt taagcaagaa aagaaacggc aacgagttat 3300
   caactcaaga taccgggaag attatattga gcctgcacta taccgggcca tcatggagta 3360
   ggtgcttcca gggtccaagg gattctcagc catccaggca agagcactct gggttccaca 3420
   gcacagcaga catggaacgc tgaagtctct gaaagtgaag ttgtaaaaag aaaaggaatg 3480
   aaataaccga cccatcatct tctcacccac cctcattgca ttccgctgta gtgaaaggac 3540
   gagccatttc tgggcacgtg gcagcagtcg ctgatctccc agctgagggg ctgagcactg 3600
   gaatgctgtg gctgcactgg ccccagtcca tagaggggtc aactatgctg gctggactgg 3660
   ctgccttgtt cctggcctag gacttagctt cataactatc acctgcaccg actaggctga 3720
   ggtgctggta cttgccccaa cccctacttt tgtatttata tgtgtgtgtg tgtgtgcgtg 3780
   cgtgcgtgcg tgcgtgtatg tttggtctgg accagcttct gccagcccct ggcctttact 3840
   ttcttccttg cctatgcagg gcaaacaaaa tgtgaaattc tgccctcagc tgagctgagt 3900
   aagggctcct gggggttggc tggagatggg tgtggcatct gtccaggcct ggaaccgtct 3960
   caagacagtg ctggcaaagc tgcagtattg agatgctaag gagctgatgc cacctctttg 4020
   tcttccccta aaggagaaca tggggataac atgggtgtgt gcccacaaca ctctaggtgc 4080
   agagcccctg tggcaaagta ttacagggtg tgggtgggga ttaccctgaa tcggggattt 4140
   taatgatgga agcaggcaga gcctggtggg tgattctgtc aacagaaaat tgcaatcatg 4200
   caggggctgg gagggttagg atgaaaaaac tggggccatt ggaggcccac tgtaggtggg 4260
   agggagctga ttttggggtg gggggtggga ctagagggca atactgaagg ggttaaacag 4320
   gtttttgctc ctcaagaatt tgtttgcctg ggcccaggat tggagggctt cacaccaata 4380
   ccctgtgtat acaagaatca gatttataat acttcccctt ttttgttacg tatgaacact 4440
   ataaaccaaa ttattttgaa aactggtgca tcaccttgtc cttagcaata aaatgtgttg 4500
   agcagaggaa aaaaaaaaaa aaaaaa 4526
<210> 2
   <211> 1064
   <212> PRT
   <213> Homo sapiens
<400> 2
   Met Ala Ser Glu Ser Glu Thr Leu Asn Pro Ser Ala Arg Ile Met Thr
   1               5                   10                  15
   Phe Tyr Pro Thr Met Glu Glu Phe Arg Asn Phe Ser Arg Tyr Ile Ala
               20                  25                  30
   Tyr Ile Glu Ser Gln Gly Ala His Arg Ala Gly Leu Ala Lys Val Val
           35                  40                  45
   Pro Pro Lys Glu Trp Lys Pro Arg Ala Ser Tyr Asp Asp Ile Asp Asp
       50                  55                  60
   Leu Val Ile Pro Ala Pro Ile Gln Gln Leu Val Thr Gly Gln Ser Gly
   65                  70                  75                  80
   Leu Phe Thr Gln Tyr Asn Ile Gln Lys Lys Ala Met Thr Val Arg Glu
                   85                  90                  95
   Phe Arg Lys Ile Ala Asn Ser Asp Lys Tyr Cys Thr Pro Arg Tyr Ser
               100                 105                 110
   Glu Phe Glu Glu Leu Glu Arg Lys Tyr Trp Lys Asn Leu Thr Phe Asn
           115                 120                 125
   Pro Pro Ile Tyr Gly Ala Asp Val Asn Gly Thr Leu Tyr Glu Lys His
       130                 135                 140
   Val Asp Glu Trp Asn Ile Gly Arg Leu Arg Thr Ile Leu Asp Leu Val
   145                 150                 155                 160
   Glu Lys Glu Ser Gly Ile Thr Ile Glu Gly Val Asn Thr Pro Tyr Leu
                   165                 170                 175
   Tyr Phe Gly Met Trp Lys Thr Ser Phe Ala Trp His Thr Glu Asp Met
               180                 185                 190
   Asp Leu Tyr Ser Ile Asn Tyr Leu His Phe Gly Glu Pro Lys Ser Trp
           195                 200                 205
   Tyr Ser Val Pro Pro Glu His Gly Lys Arg Leu Glu Arg Leu Ala Lys
       210                 215                 220
   Gly Phe Phe Pro Gly Ser Ala Gln Ser Cys Glu Ala Phe Leu Arg His
   225                 230                 235                 240
   Lys Met Thr Leu Ile Ser Pro Leu Met Leu Lys Lys Tyr Gly Ile Pro
                   245                 250                 255
   Phe Asp Lys Val Thr Gln Glu Ala Gly Glu Phe Met Ile Thr Phe Pro
               260                 265                 270
   Tyr Gly Tyr His Ala Gly Phe Asn His Gly Phe Asn Cys Ala Glu Ser
   275 280 285
   Thr Asn Phe Ala Thr Arg Arg Trp Ile Glu Tyr Gly Lys Gln Ala Val
   290 295 300
   Leu Cys Ser Cys Arg Lys Asp Met Val Lys Ile Ser Met Asp Val Phe
   305 310 315 320
   Val Arg Lys Phe Gln Pro Glu Arg Tyr Lys Leu Trp Lys Ala Gly Lys
   325 330 335
   Asp Asn Thr Val Ile Asp His Thr Leu Pro Thr Pro Glu Ala Ala Glu
   340 345 350
   Phe Leu Lys Glu Ser Glu Leu Pro Pro Arg Ala Gly Asn Glu Glu Glu
   355 360 365
   Cys Pro Glu Glu Asp Met Glu Gly Val Glu Asp Gly Glu Glu Gly Asp
   370 375 380
   Leu Lys Thr Ser Leu Ala Lys His Arg Ile Gly Thr Lys Arg His Arg
   385 390 395 400
   Val Cys Leu Glu Ile Pro Gln Glu Val Ser Gln Ser Glu Leu Phe Pro
   405 410 415
   Lys Glu Asp Leu Ser Ser Glu Gln Tyr Glu Met Thr Glu Cys Pro Ala
   420 425 430
   Ala Leu Ala Pro Val Arg Pro Thr His Ser Ser Val Arg Gln Val Glu
   435 440 445
   Asp Gly Leu Thr Phe Pro Asp Tyr Ser Asp Ser Thr Glu Val Lys Phe
   450 455 460
   Glu Glu Leu Lys Asn Val Lys Leu Glu Glu Glu Asp Glu Glu Glu Glu
   465 470 475 480
   Gln Ala Ala Ala Ala Leu Asp Leu Ser Val Asn Pro Ala Ser Val Gly
   485 490 495
   Gly Arg Leu Val Phe Ser Gly Ser Lys Lys Lys Ser Ser Ser Ser Leu
   500 505 510
   Gly Ser Gly Ser Ser Arg Asp Ser Ile Ser Ser Asp Ser Glu Thr Ser
   515 520 525
   Glu Pro Leu Ser Cys Arg Ala Gln Gly Gln Thr Gly Val Leu Thr Val
   530 535 540
   His Ser Tyr Ala Lys Gly Asp Gly Arg Val Thr Val Gly Glu Pro Cys
   545 550 555 560
   Thr Arg Lys Lys Gly Ser Ala Ala Arg Ser Phe Ser Glu Arg Glu Leu
   565 570 575
   Ala Glu Val Ala Asp Glu Tyr Met Phe Ser Leu Glu Glu Asn Lys Lys
   580 585 590
   Ser Lys Gly Arg Arg Gln Pro Leu Ser Lys Leu Pro Arg His His Pro
   595 600 605
   Leu Val Leu Gln Glu Cys Val Ser Asp Asp Glu Thr Ser Glu Gln Leu
   610 615 620
   Thr Pro Glu Glu Glu Ala Glu Glu Thr Glu Ala Trp Ala Lys Pro Leu
   625 630 635 640
   Ser Gln Leu Trp Gln Asn Arg Pro Pro Asn Phe Glu Ala Glu Lys Glu
   645 650 655
   Phe Asn Glu Thr Met Ala Gln Gln Ala Pro His Cys Ala Val Cys Met
   660 665 670
   Ile Phe Gln Thr Tyr His Gln Val Glu Phe Gly Gly Phe Asn Gln Asn
   675 680 685
   Cys Gly Asn Ala Ser Asp Leu Ala Pro Gln Lys Gln Arg Thr Lys Pro
   690 695 700
   Leu Ile Pro Glu Met Cys Phe Thr Ser Thr Gly Cys Ser Thr Asp Ile
   705 710 715 720
   Asn Leu Ser Thr Pro Tyr Leu Glu Glu Asp Gly Thr Ser Ile Leu Val
   725 730 735
   Ser Cys Lys Lys Cys Ser Val Arg Val His Ala Ser Cys Tyr Gly Val
   740 745 750
   Pro Pro Ala Lys Ala Ser Glu Asp Trp Met Cys Ser Arg Cys Ser Ala
   755 760 765
   Asn Ala Leu Glu Glu Asp Cys Cys Leu Cys Ser Leu Arg Gly Gly Ala
   770 775 780
   Leu Gln Arg Ala Asn Asp Asp Arg Trp Val His Val Ser Cys Ala Val
   785                 790                 795                 800
   Ala Ile Leu Glu Ala Arg Phe Val Asn Ile Ala Glu Arg Ser Pro Val
                   805                 810                 815
   Asp Val Ser Lys Ile Pro Leu Pro Arg Phe Lys Leu Lys Cys Ile Phe
               820                 825                 830
   Cys Lys Lys Arg Arg Lys Arg Thr Ala Gly Cys Cys Val Gln Cys Ser
           835                 840                 845
   His Gly Arg Cys Pro Thr Ala Phe His Val Ser Cys Ala Gln Ala Ala
       850                 855                 860
   Gly Val Met Met Gln Pro Asp Asp Trp Pro Phe Val Val Phe Ile Thr
   865                 870                 875                 880
   Cys Phe Arg His Lys Ile Pro Asn Leu Glu Arg Ala Lys Gly Ala Leu
                   885                 890                 895
   Gln Ser Ile Thr Ala Gly Gln Lys Val Ile Ser Lys His Lys Asn Gly
               900                 905                 910
   Arg Phe Tyr Gln Cys Glu Val Val Arg Leu Thr Thr Glu Thr Phe Tyr
           915                 920                 925
   Glu Val Asn Phe Asp Asp Gly Ser Phe Ser Asp Asn Leu Tyr Pro Glu
       930                 935                 940
   Asp Ile Val Ser Gln Asp Cys Leu Gln Phe Gly Pro Pro Ala Glu Gly
   945                 950                 955                 960
   Glu Val Val Gln Val Arg Trp Thr Asp Gly Gln Val Tyr Gly Ala Lys
                   965                 970                 975
   Phe Val Ala Ser His Pro Ile Gln Met Tyr Gln Val Glu Phe Glu Asp
               980                 985                 990
   Gly Ser Gln Leu Val Val Lys Arg  Asp Asp Val Tyr Thr  Leu Asp Glu
           995                 1000                1005
   Glu Leu  Pro Lys Arg Val Lys  Ser Arg Leu Ser Val  Ala Ser Asp
       1010                1015                1020
   Met Arg  Phe Asn Glu Ile Phe  Thr Glu Lys Glu Val  Lys Gln Glu
       1025                1030                1035
   Lys Lys  Arg Gln Arg Val Ile  Asn Ser Arg Tyr Arg  Glu Asp Tyr
       1040                 1045                 1050
   Ile Glu  Pro Ala Leu Tyr Arg  Ala Ile Met Glu
       1055                 1060
<210> 3
   <211> 2339
   <212> DNA
   <213> Homo sapiens
<400> 3
   cctgtggtcc cgggtttctg cagagtctac ttcagaagcg gaggcactgg gagtccggtt 60
   tgggattgcc aggctgtggt tgtgagtctg agcttgtgag cggctgtggc gccccaactc 120
   ttcgccagca tatcatcccg gcaggcgata aactacattc agttgagtct gcaagactgg 180
   gaggaactgg ggtgataaga aatctattca ctgtcaaggt ttattgaagt caaaatgtcc 240
   aaaaaaatca gtggcggttc tgtggtagag atgcaaggag atgaaatgac acgaatcatt 300
   tgggaattga ttaaagagaa actcattttt ccctacgtgg aattggatct acatagctat 360
   gatttaggca tagagaatcg tgatgccacc aacgaccaag tcaccaagga tgctgcagaa 420
   gctataaaga agcataatgt tggcgtcaaa tgtgccacta tcactcctga tgagaagagg 480
   gttgaggagt tcaagttgaa acaaatgtgg aaatcaccaa atggcaccat acgaaatatt 540
   ctgggtggca cggtcttcag agaagccatt atctgcaaaa atatcccccg gcttgtgagt 600
   ggatgggtaa aacctatcat cataggtcgt catgcttatg gggatcaata cagagcaact 660
   gattttgttg ttcctgggcc tggaaaagta gagataacct acacaccaag tgacggaacc 720
   caaaaggtga catacctggt acataacttt gaagaaggtg gtggtgttgc catggggatg 780
   tataatcaag ataagtcaat tgaagatttt gcacacagtt ccttccaaat ggctctgtct 840
   aagggttggc ctttgtatct gagcaccaaa aacactattc tgaagaaata tgatgggcgt 900
   tttaaagaca tctttcagga gatatatgac aagcagtaca agtcccagtt tgaagctcaa 960
   aagatctggt atgagcatag gctcatcgac gacatggtgg cccaagctat gaaatcagag 1020
   ggaggcttca tctgggcctg taaaaactat gatggtgacg tgcagtcgga ctctgtggcc 1080
   caagggtatg gctctctcgg catgatgacc agcgtgctgg tttgtccaga tggcaagaca 1140
   gtagaagcag aggctgccca cgggactgta acccgtcact accgcatgta ccagaaagga 1200
   caggagacgt ccaccaatcc cattgcttcc atttttgcct ggaccagagg gttagcccac 1260
   agagcaaagc ttgataacaa taaagagctt gccttctttg caaatgcttt ggaagaagtc 1320
   tctattgaga caattgaggc tggcttcatg accaaggact tggctgcttg cattaaaggt 1380
   ttacccaatg tgcaacgttc tgactacttg aatacatttg agttcatgga taaacttgga 1440
   gaaaacttga agatcaaact agctcaggcc aaactttaag ttcatacctg agctaagaag 1500
   gataattgtc ttttggtaac taggtctaca ggtttacatt tttctgtgtt acactcaagg 1560
   ataaaggcaa aatcaatttt gtaatttgtt tagaagccag agtttatctt ttctataagt 1620
   ttacagcctt tttcttatat atacagttat tgccaccttt gtgaacatgg caagggactt 1680
   ttttacaatt tttattttat tttctagtac cagcctagga attcggttag tactcatttg 1740
   tattcactgt cactttttct catgttctaa ttataaatga ccaaaatcaa gattgctcaa 1800
   aagggtaaat gatagccaca gtattgctcc ctaaaatatg cataaagtag aaattcactg 1860
   ccttcccctc ctgtccatga ccttgggcac agggaagttc tggtgtcata gatatcccgt 1920
   tttgtgaggt agagctgtgc attaaacttg cacatgactg gaacgaagta tgagtgcaac 1980
   tcaaatgtgt tgaagatact gcagtcattt ttgtaaagac cttgctgaat gtttccaata 2040
   gactaaatac tgtttaggcc gcaggagagt ttggaatccg gaataaatac tacctggagg 2100
   tttgtcctct ccatttttct ctttctcctc ctggcctggc ctgaatatta tactactcta 2160
   aatagcatat ttcatccaag tgcaataatg taagctgaat cttttttgga cttctgctgg 2220
   cctgttttat ttcttttata taaatgtgat ttctcagaaa ttgatattaa acactatctt 2280
   atcttctcct gaactgttga ttttaattaa aattaagtgc taattaccaa aaaaaaaaa 2339
<210> 4
   <211> 414
   <212> PRT
   <213> Homo sapiens
<400> 4
<210> 5
   <211> 5675
   <212> DNA
   <213> Homo sapiens
<400> 5
   agggctcggt cgccagcaac cgagcggggc ccggcccgag cggggcctgg gggtgcgacg 60
   ccgagggcgg gggagagcgc gccgctgctc ccggaccggg ccgcgcacgc cgcctcagga 120
   accatcactg ttgctggagg cacctgacaa atcctagcga atttttggag catctccacc 180
   caggaacctc gccatccaga agtgtgcttc ccgcacagct gcagccatgg ggtctgagga 240
   ccacggcgcc cagaacccca gctgtaaaat catgacgttt cgcccaacca tggaagaatt 300
   taaagacttc aacaaatacg tggcctacat agagtcgcag ggagcccacc gggcgggcct 360
   ggccaagatc atccccccga aggagtggaa gccgcggcag acgtatgatg acatcgacga 420
   cgtggtgatc ccggcgccca tccagcaggt ggtgacgggc cagtcgggcc tcttcacgca 480
   gtacaatatc cagaagaagg ccatgacagt gggcgagtac cgccgcctgg ccaacagcga 540
   gaagtactgt accccgcggc accaggactt tgatgacctt gaacgcaaat actggaagaa 600
   cctcaccttt gtctccccga tctacggggc tgacatcagc ggctctttgt atgatgacga 660
   cgtggcccag tggaacatcg ggagcctccg gaccatcctg gacatggtgg agcgcgagtg 720
   cggcaccatc atcgagggcg tgaacacgcc ctacctgtac ttcggcatgt ggaagaccac 780
   cttcgcctgg cacaccgagg acatggacct gtacagcatc aactacctgc actttgggga 840
   gcctaagtcc tggtacgcca tcccaccaga gcacggcaag cgcctggagc ggctggccat 900
   cggcttcttc cccgggagct cgcagggctg cgacgccttc ctgcggcata agatgaccct 960
   catctcgccc atcatcctga agaagtacgg gatccccttc agccggatca cgcaggaggc 1020
   cggggaattc atgatcacat ttccctacgg ctaccacgcc ggcttcaatc acgggttcaa 1080
   ctgcgcagaa tctaccaact tcgccaccct gcggtggatt gactacggca aagtggccac 1140
   tcagtgcacg tgccggaagg acatggtcaa gatctccatg gacgtgttcg tgcgcatcct 1200
   gcagcccgag cgctacgagc tgtggaagca gggcaaggac ctcacggtgc tggaccacac 1260
   gcggcccacg gcgctcacca gccccgagct gagctcctgg agtgcatccc gggcctcgct 1320
   gaaggccaag ctcctccgca ggtctcaccg gaaacggagc cagcccaaga agccgaagcc 1380
   cgaagacccc aagttccctg gggagggtac ggctggggca gcgctcctag aggaggctgg 1440
   gggcagcgtg aaggaggagg ctgggccgga ggttgacccc gaggaggagg aggaggagcc 1500
   gcagccactg ccacacggcc gggaggccga gggcgcagaa gaggacggga ggggcaagct 1560
   gcggccaacc aaggccaaga gcgagcggaa gaagaagagc ttcggcctgc tgcccccaca 1620
   gctgccgccc ccgcctgctc acttcccctc agaggaggcg ctgtggctgc catccccact 1680
   ggagcccccg gtgctgggcc caggccctgc agccatggag gagagccccc tgccggcacc 1740
   ccttaatgtc gtgccccctg aggtgcccag tgaggagcta gaggccaagc ctcggcccat 1800
   catccccatg ctgtacgtgg tgccgcggcc gggcaaggca gccttcaacc aggagcacgt 1860
   gtcctgccag caggcctttg agcactttgc ccagaagggt ccgacctgga aggaaccagt 1920
   ttcccccatg gagctgacgg ggccagagga cggtgcagcc agcagtgggg caggtcgcat 1980
   ggagaccaaa gcccgggccg gagaggggca ggcaccgtcc acattttcca aattgaagat 2040
   ggagatcaag aagagccggc gccatcccct gggccggccg cccacccggt ccccactgtc 2100
   ggtggtgaag caggaggcct caagtgacga ggaggcatcc cctttctccg gggaggaaga 2160
   tgtgagtgac ccggacgcct tgaggccgct gctgtctctg cagtggaaga acagggcggc 2220
   cagcttccag gccgagagga agttcaacgc agcggctgcg cgcacggagc cctactgcgc 2280
   catctgcacg ctcttctacc cctactgcca ggccctacag actgagaagg aggcacccat 2340
   agcctccctc ggagagggct gcccggccac attaccctcc aaaagccgtc agaagacccg 2400
   accgctcatc cctgagatgt gcttcacctc tggcggtgag aacacggagc cgctgcctgc 2460
   caactcctac atcggcgacg acgggaccag ccccctgatc gcctgcggca agtgctgcct 2520
   gcaggtccat gccagttgct atggcatccg tcccgagctg gtcaatgaag gctggacgtg 2580
   ttcccggtgc gcggcccacg cctggactgc ggagtgctgc ctgtgcaacc tgcgaggagg 2640
   tgcgctgcag atgaccaccg ataggaggtg gatccacgtg atctgtgcca tcgcagtccc 2700
   cgaggcgcgc ttcctgaacg tgattgagcg ccaccctgtg gacatcagcg ccatccccga 2760
   gcagcggtgg aagctgaaat gcgtgtactg ccggaagcgg atgaagaagg tgtcaggtgc 2820
   ctgtatccag tgctcctacg agcactgctc cacgtccttc cacgtgacct gcgcccacgc 2880
   cgcaggcgtg ctcatggagc cggacgactg gccctatgtg gtctccatca cctgcctcaa 2940
   gcacaagtcg gggggtcacg ctgtccaact cctgagggcc gtgtccctag gccaggtggt 3000
   catcaccaag aaccgcaacg ggctgtacta ccgctgtcgc gtcatcggtg ccgcctcgca 3060
   gacctgctac gaagtgaact tcgacgatgg ctcctacagc gacaacctgt accctgagag 3120
   catcacgagt agggactgtg tccagctggg acccccttcc gagggggagc tggtggagct 3180
   ccggtggact gacggcaacc tctacaaggc caagttcatc tcctccgtca ccagccacat 3240
   ctaccaggtg gagtttgagg acgggtccca gctgacggtg aagcgtgggg acatcttcac 3300
   cctggaggag gagctgccca agagggtccg ctctcggctg tcactgagca cgggggcacc 3360
   gcaggagccc gccttctcgg gggaggaggc caaggccgcc aagcgcccgc gtgtgggcac 3420
   cccgcttgcc acggaggact ccgggcggag ccaggactac gtggccttcg tggagagcct 3480
   cctgcaggtg cagggccggc ccggagcccc cttctaggac agctggccgc tcaggcgacc 3540
   ctcagcccgg cggggaggcc atggcatgcc ccgggcgttc gcttgctgtg aattcctgtc 3600
   ctcgtgtccc cgacccccga gaggccacct ccaagccgcg ggtgccccct agggcgacag 3660
   gagccagcgg gacgccgcac gcggccccag actcagggag cagggccagg cgggctcggg 3720
   ggccggccag gggagcaccc cactcaacta ctcagaattt taaaccatgt aagctctctt 3780
   cttctcgaaa aggtgctact gcaatgccct actgagcaac ctttgagatt gtcacttctg 3840
   tacataaacc acctttgtga ggctctttct ataaatacat attgtttaaa aaaaagcaag 3900
   aaaaaaagga aaacaaagga aaatatcccc aaagttgttt tctagatttg tggctttaag 3960
   aaaaacaaaa caaaacaaac acattgtttt tctcagaacc aggattctct gagaggtcag 4020
   agcatctcgc tgtttttttg ttgttgtttt aaaatattat gatttggcta cagaccaggc 4080
   agggaaagag acccggtaat tggagggtga gcctcggggg gggggcagga cgccccggtt 4140
   tcggcacagc ccggtcactc acggcctcgc tctcgcctca ccccggctcc tgggctttga 4200
   tggtctggtg ccagtgcctg tgcccactct gtgcctgctg ggaggaggcc caggctctct 4260
   ggtggccgcc cctgtgcacc tggccagggg aagcccgggg gtctggggcc tccctccgtc 4320
   tgcgcccacc tttgcagaat aaactctctc ctggggtttg tctatctttg tttctctcac 4380
   ctgagagaaa cgcaggtgtt ccagaggctt ccttgcagac aaagcacccc tgcacctcct 4440
   atggctcagg atgagggagg cccccaggcc cttctggttg gtagtgagtg tggacagctt 4500
   cccagctctt cgggtacaac cctgagcagg tcgggggaca cagggccgag gcaggccttc 4560
   ggggcccctt tcgcctgctt ccgggcaggg acgaggcctg gtgtcctcgc tccacccacc 4620
   cacgctgctg tcacctgagg ggaatctgct tcttaggagt gggttgagct gatagagaaa 4680
   aaacggcctt cagcccaggc tgggaagcgc cttctccagg tgcctctccc tcaccagctc 4740
   tgcacccctc tggggagcct tccccacctt agctgtctcc tgccccaggg agggatggag 4800
   gagataattt gcttatatta aaaacaaaaa atggctgagg caggagtttg ggaccagcct 4860
   gggctatata gcaagacccc atcactacaa attttttaca aattagctag gtgtggtggt 4920
   gcgcacctgt ggtcccagct actcgggagg ctgtggtggg aggattgctt gagtccagga 4980
   ggttgaggct gcagtcagct cagattgcac cactgcactc cagcctgggc aacagagcga 5040
   gaccctgtct ccaaaaaaaa aaaaaagcaa tgtttatatt ataaaagagt gtcctaacag      5100
   tccccgggct agagaggact aaggaaaaca gagagagtgt tacgcaggag caagcctttc      5160
   atttccttgg tgggggaggg gggcggttgc cctggagagg gccggggtcg gggaggttgg      5220
   ggggtgtcag ccaaaacgtg gaggtgtccc tctgcacgca gccctcgccc ggcgtggcgc      5280
   tgacactgta ttcttatgtt gtttgaaaat gctatttata ttgtaaagaa gcgggcgggt      5340
   gcccctgctg cccttgtccc ttgggggtca cacccatccc ctggtgggct cctgggcggc      5400
   ctgcgcagat gggccacaga agggcaggcc ggagctgcac actctcccca cgaaggtatc      5460
   tctgtgtctt actctgtgca aagacgcggc aaaacccagt gccctggttt ttccccaccc      5520
   gagatgaagg atacgctgta ttttttgcct aatgtccctg cctctaggtt cataatgaat      5580
   taaaggttca tgaacgctgc gaaaaaaaaa aaaaaaaaaa aaaaaaaaaa aaaaaaaaaa      5640
   aaaaaaaaaa aaaaaaaaaa aaaaaaaaaa aaaaa                                 5675
<210> 6
   <211> 1096
   <212> PRT
   <213> Homo sapiens
<400> 6
<210> 7
   <211> 4687
   <212> DNA
   <213> Homo sapiens
<400> 7
   gccataggtg cgcgtcggcg cccaggagga cgtgtggcgc gtggactaca tcaggtccag        60
   ccctgcggga ccccagccag cgcttccggg caaggttctg tgcacctgtt ttctccttct       120
   acgcgagtat ctttcccctc cggaaagaat gggatatgcc tgtgtccaaa ggacaagaag 180
   atgcgcgcca gcaagcctaa gttaaccaca gcgcggaagt tgagcccaaa gcaagagcgt 240
   gccgggcacc tttaagctgt ttgtaagccc acgtgactca ccaagtgcgg gccccagcgg 300
   tcacgtgacg gcgcgcgcgc cctcgcgcag ggagagccgg cggtgcgcgc gccttcgccg 360
   ctgcctccca cccaccccct cgacgggagg gtgaggcgcg gcgcagtgat cgggcggccg 420
   gggtcctgtg cgcgtgcgca gcgaacagct gtcacctagt gcggaacaag tctcccaaat 480
   ttcccaaatc tccctgggcc ggaggccact gtcttctctt cctcctccac cgagtcgtgc 540
   tctcgcccca acccgcgcgc cagacactgc cctaaccatc atggaggtgg ccgaggtgga 600
   aagtcctctg aaccccagct gtaagataat gaccttcaga ccctccatgg aggagttccg 660
   ggagttcaac aaataccttg catacatgga gtctaaagga gcccatcgtg cgggtcttgc 720
   aaaggtgatt cctcctaagg agtggaagcc aagacagtgc tatgatgaca ttgataattt 780
   gctcattcca gcaccaattc agcagatggt cacagggcag tcaggactgt tcactcagta 840
   caacatccag aaaaaagcga tgactgtgaa ggagttcagg cagctggcca acagtggcaa 900
   atattgtact ccaagatact tggattacga agatttggag cgcaagtact ggaagaactt 960
   aacttttgtg gcacctatct atggtgcaga tattaatggg agcatatatg atgagggtgt 1020
   ggatgaatgg aacatagctc gcctcaatac agtcttggat gtggttgaag aagagtgtgg 1080
   catttctatt gagggtgtaa ataccccata tctctatttt ggcatgtgga agaccacgtt 1140
   tgcatggcac accgaagaca tggacctcta tagcattaat tatctccact ttggagagcc 1200
   caagtcttgg tatgctatac ctccggagca tggaaaacga cttgaaagac tagctcaagg 1260
   ttttttccca agcagctccc aagggtgtga tgcatttctt cgccacaaga tgacattgat 1320
   ttctccatca gtattgaaga aatatggtat tccctttgac aagataaccc aggaggctgg 1380
   agaattcatg atcactttcc catatggcta ccatgctggt tttaatcatg gtttcaactg 1440
   tgcagaatct acaaattttg ctactgtcag atggattgac tatggaaaag ttgccaaatt 1500
   gtgcacttgc aggaaagaca tggtgaagat ttcaatggat atctttgtga ggaaatttca 1560
   gccagacaga tatcagcttt ggaaacaagg aaaggatata tacaccattg atcacacgaa 1620
   gcctactcca gcatccaccc ctgaagtaaa agcatggctg cagaggagga ggaaagtaag 1680
   aaaagcatcc cgaagcttcc agtgtgctag gtctacctct aaaaggccta aggctgatga 1740
   ggaagaggaa gtgtcagatg aagtcgatgg ggcagaggtc cctaaccccg actcagtcac 1800
   agatgacctc aaggtcagtg aaaagtcaga agcagcagtg aagctgagga acacagaagc 1860
   atcttcagaa gaagagtcat ctgctagcag gatgcaggtg gagcagaatt tatcagatca 1920
   tatcaaactc tcaggaaaca gctgcttaag tacatctgta acagaagaca taaaaactga 1980
   ggatgacaaa gcttatgcat atagaagtgt accttctata tccagtgagg ctgatgattc 2040
   cattccattg tctagtggct atgagaagcc cgagaaatca gacccatccg agctttcatg 2100
   gccaaagtca cctgagtcat gctcatcagt ggcagagagt aatggtgtgt taacagaggg 2160
   agaagagagt gatgtggaga gccatgggaa tggccttgaa cctggggaaa tcccagcggt 2220
   ccccagtgga gagagaaata gcttcaaagt ccccagtata gcagagggag agaacaaaac 2280
   ctctaagagt tggcgccatc cacttagcag gcctccagca agatctccga tgactcttgt 2340
   gaagcagcag gcgccaagtg atgaagaatt gcctgaggtt ctgtccattg aggaggaagt 2400
   ggaagaaaca gagtcttggg cgaaacctct catccacctt tggcagacga agtcccctaa 2460
   cttcgcagct gagcaagagt ataatgcaac agtggccagg atgaagccac actgtgccat 2520
   ctgcactctg ctcatgccgt accacaagcc agatagcagc aatgaagaaa atgatgctag 2580
   atgggagaca aaattagatg aagtcgttac atcggaggga aagactaagc ccctcatacc 2640
   agagatgtgt tttatttata gtgaagaaaa tatagaatat tctccaccca atgccttcct 2700
   tgaagaggat ggaacaagtc tccttatttc ctgtgcaaag tgctgcgtac gggttcatgc 2760
   aagttgttat ggtattcctt ctcatgagat ctgtgatgga tggctgtgtg cccggtgcaa 2820
   aagaaatgcg tggacagcag aatgctgtct ctgcaatttg agaggaggtg ctcttaagca 2880
   aacgaagaac aataagtggg cccatgtcat gtgcgccgtt gcggtcccag aagttcgatt 2940
   cactaatgtc ccagaaagga cacaaataga tgtaggcaga atacctttac agaggttaaa 3000
   attgaaatgc atcttctgca gacaccgggt taagagggtc tctggagcct gcatccagtg 3060
   ttcctacggt cgctgcccgg cctccttcca tgtcacttgt gcccatgctg ctggggtact 3120
   gatggagcct gatgactggc cttatgtggt gaacattaca tgctttcgac ataaggtcaa 3180
   ccccaacgtg aagtccaagg cttgcgagaa ggtcatttcc gtgggtcaaa cggtcatcac 3240
   gaagcatcgg aacacccggt attacagttg cagagtgatg gctgtgacat cgcagacctt 3300
   ctatgaggtc atgtttgatg atggctcctt tagcagagac acatttcctg aggatatcgt 3360
   gagccgagac tgtctgaagc tgggcccacc tgctgaggga gaagtcgtcc aagtcaagtg 3420
   gcccgatggc aaactctatg gagcaaaata ttttggatca aatattgccc acatgtacca 3480
   ggttgagttt gaagatggat cccagatagc aatgaagaga gaggacatct acactttaga 3540
   tgaagagtta cccaagagag tgaaagctcg attttccaca gcctctgaca tgcgatttga 3600
   agacacgttt tatggagcag acattatcca aggggagaga aagagacaaa gagtgctgag 3660
   ctccaggttt aagaatgaat atgtggccga ccctgtatac cgcacttttt tgaagagctc 3720
   tttccagaag aagtgccaga agagacagta gtctgcatac atcgctgcag gccacagagc 3780
   agcttgggtt ggaagagaga agatgaaggg acatccttgg ggctgtgccg tgagttttgc 3840
   tggcataggt gacagggtgt gtctctgaca gtggtaaatc gggtttccag agtttggtca 3900
   ccaaaaatac aaaatacacc caatgaattg gacgcagcaa tctgaaatca tctctagtct 3960
   tgctttcact tgtgagcagt tgtcttctat gatcccaaag aagttttcta agtgaaagga 4020
   aatactagtg aatcacccac aaggaaaagc cactgccaca gaggaggcgg gtccccttgt 4080
   gcggcttagg gccctgtcag gaaacacacg gggacctctc tctctagctc cagcaggtgg 4140
   cacctcggta cccagcgggt agggcgataa tttatatatt ttccacagtc agggaaggac 4200
   tctcacttat ttgtttcaaa ttgcagtttt tataaaacat ttttaaaaca caaatggcat 4260
   gtatgctaat gagatttacc cgtgtgctat ctgtatttcc cttgtacaga acttttacat 4320
   ttttgaatat tcctattact tttgattgtg tctgatggga actgagttgt tggcctttgt 4380
   gaaatgaaat ttttggctct tgagaaagaa ttcttatgaa ttgttatgcg aattttatat 4440
   atttaaagag ggagatctgg ggctgttatt tttaaacact ttttttcata atacatattc 4500
   cgagtagata tttataaaat atatgtttct ttcattatgt gtttgtaaaa ttagagttta 4560
   aataaatatg ctttgatgca tagttttgaa ctaatgtaac atgatttttc ttttttaaaa 4620
   cagcctgaaa atgtactagt gtttaaaaat aaagatttcc attttctcca aaaaaaaaaa 4680
   aaaaaaa 4687
<210> 8
   <211> 1056
   <212> PRT
   <213> Homo sapiens
<400> 8
   Met Glu Val Ala Glu Val Glu Ser Pro Leu Asn Pro Ser Cys Lys Ile
   1               5                   10                  15
   Met Thr Phe Arg Pro Ser Met Glu Glu Phe Arg Glu Phe Asn Lys Tyr
               20                  25                  30
   Leu Ala Tyr Met Glu Ser Lys Gly Ala His Arg Ala Gly Leu Ala Lys
           35                  40                  45
   Val Ile Pro Pro Lys Glu Trp Lys Pro Arg Gln Cys Tyr Asp Asp Ile
       50                  55                  60
   Asp Asn Leu Leu Ile Pro Ala Pro Ile Gln Gln Met Val Thr Gly Gln
   65                  70                  75                  80
   Ser Gly Leu Phe Thr Gln Tyr Asn Ile Gln Lys Lys Ala Met Thr Val
                   85                  90                  95
   Lys Glu Phe Arg Gln Leu Ala Asn Ser Gly Lys Tyr Cys Thr Pro Arg
               100                 105                 110
   Tyr Leu Asp Tyr Glu Asp Leu Glu Arg Lys Tyr Trp Lys Asn Leu Thr
           115                 120                 125
   Phe Val Ala Pro Ile Tyr Gly Ala Asp Ile Asn Gly Ser Ile Tyr Asp
       130                 135                 140
   Glu Gly Val Asp Glu Trp Asn Ile Ala Arg Leu Asn Thr Val Leu Asp
   145                 150                 155                 160
   Val Val Glu Glu Glu Cys Gly Ile Ser Ile Glu Gly Val Asn Thr Pro
                   165                 170                 175
   Tyr Leu Tyr Phe Gly Met Trp Lys Thr Thr Phe Ala Trp His Thr Glu
               180                 185                 190
   Asp Met Asp Leu Tyr Ser Ile Asn Tyr Leu His Phe Gly Glu Pro Lys
           195                 200                 205
   Ser Trp Tyr Ala Ile Pro Pro Glu His Gly Lys Arg Leu Glu Arg Leu
       210                 215                 220
   Ala Gln Gly Phe Phe Pro Ser Ser Ser Gln Gly Cys Asp Ala Phe Leu
   225                 230                 235                 240
   Arg His Lys Met Thr Leu Ile Ser Pro Ser Val Leu Lys Lys Tyr Gly
                   245                 250                 255
   Ile Pro Phe Asp Lys Ile Thr Gln Glu Ala Gly Glu Phe Met Ile Thr
               260                 265                 270
   Phe Pro Tyr Gly Tyr His Ala Gly Phe Asn His Gly Phe Asn Cys Ala
           275                 280                 285
   Glu Ser Thr Asn Phe Ala Thr Val Arg Trp Ile Asp Tyr Gly Lys Val
       290                 295                 300
   Ala Lys Leu Cys Thr Cys Arg Lys Asp Met Val Lys Ile Ser Met Asp
   305                 310                 315                 320
   Ile Phe Val Arg Lys Phe Gln Pro Asp Arg Tyr Gln Leu Trp Lys Gln
                   325                 330                 335
   Gly Lys Asp Ile Tyr Thr Ile Asp His Thr Lys Pro Thr Pro Ala Ser
               340                 345                 350
   Thr Pro Glu Val Lys Ala Trp Leu Gln Arg Arg Arg Lys Val Arg Lys
           355                 360                 365
   Ala Ser Arg Ser Phe Gln Cys Ala Arg Ser Thr Ser Lys Arg Pro Lys
   370 375 380
   Ala Asp Glu Glu Glu Glu Val Ser Asp Glu Val Asp Gly Ala Glu Val
   385 390 395 400
   Pro Asn Pro Asp Ser Val Thr Asp Asp Leu Lys Val Ser Glu Lys Ser
   405 410 415
   Glu Ala Ala Val Lys Leu Arg Asn Thr Glu Ala Ser Ser Glu Glu Glu
   420 425 430
   Ser Ser Ala Ser Arg Met Gln Val Glu Gln Asn Leu Ser Asp His Ile
   435 440 445
   Lys Leu Ser Gly Asn Ser Cys Leu Ser Thr Ser Val Thr Glu Asp Ile
   450 455 460
   Lys Thr Glu Asp Asp Lys Ala Tyr Ala Tyr Arg Ser Val Pro Ser Ile
   465 470 475 480
   Ser Ser Glu Ala Asp Asp Ser Ile Pro Leu Ser Ser Gly Tyr Glu Lys
   485 490 495
   Pro Glu Lys Ser Asp Pro Ser Glu Leu Ser Trp Pro Lys Ser Pro Glu
   500 505 510
   Ser Cys Ser Ser Val Ala Glu Ser Asn Gly Val Leu Thr Glu Gly Glu
   515 520 525
   Glu Ser Asp Val Glu Ser His Gly Asn Gly Leu Glu Pro Gly Glu Ile
   530 535 540
   Pro Ala Val Pro Ser Gly Glu Arg Asn Ser Phe Lys Val Pro Ser Ile
   545 550 555 560
   Ala Glu Gly Glu Asn Lys Thr Ser Lys Ser Trp Arg His Pro Leu Ser
   565 570 575
   Arg Pro Pro Ala Arg Ser Pro Met Thr Leu Val Lys Gln Gln Ala Pro
   580 585 590
   Ser Asp Glu Glu Leu Pro Glu Val Leu Ser Ile Glu Glu Glu Val Glu
   595 600 605
   Glu Thr Glu Ser Trp Ala Lys Pro Leu Ile His Leu Trp Gln Thr Lys
   610 615 620
   Ser Pro Asn Phe Ala Ala Glu Gln Glu Tyr Asn Ala Thr Val Ala Arg
   625 630 635 640
   Met Lys Pro His Cys Ala Ile Cys Thr Leu Leu Met Pro Tyr His Lys
   645 650 655
   Pro Asp Ser Ser Asn Glu Glu Asn Asp Ala Arg Trp Glu Thr Lys Leu
   660 665 670
   Asp Glu Val Val Thr Ser Glu Gly Lys Thr Lys Pro Leu Ile Pro Glu
   675 680 685
   Met Cys Phe Ile Tyr Ser Glu Glu Asn Ile Glu Tyr Ser Pro Pro Asn
   690 695 700
   Ala Phe Leu Glu Glu Asp Gly Thr Ser Leu Leu Ile Ser Cys Ala Lys
   705 710 715 720
   Cys Cys Val Arg Val His Ala Ser Cys Tyr Gly Ile Pro Ser His Glu
   725 730 735
   Ile Cys Asp Gly Trp Leu Cys Ala Arg Cys Lys Arg Asn Ala Trp Thr
   740 745 750
   Ala Glu Cys Cys Leu Cys Asn Leu Arg Gly Gly Ala Leu Lys Gln Thr
   755 760 765
   Lys Asn Asn Lys Trp Ala His Val Met Cys Ala Val Ala Val Pro Glu
   770 775 780
   Val Arg Phe Thr Asn Val Pro Glu Arg Thr Gln Ile Asp Val Gly Arg
   785 790 795 800
   Ile Pro Leu Gln Arg Leu Lys Leu Lys Cys Ile Phe Cys Arg His Arg
   805 810 815
   Val Lys Arg Val Ser Gly Ala Cys Ile Gln Cys Ser Tyr Gly Arg Cys
   820 825 830
   Pro Ala Ser Phe His Val Thr Cys Ala His Ala Ala Gly Val Leu Met
   835 840 845
   Glu Pro Asp Asp Trp Pro Tyr Val Val Asn Ile Thr Cys Phe Arg His
   850 855 860
   Lys Val Asn Pro Asn Val Lys Ser Lys Ala Cys Glu Lys Val Ile Ser
   865                 870                 875                 880
   Val Gly Gln Thr Val Ile Thr Lys His Arg Asn Thr Arg Tyr Tyr Ser
                   885                 890                 895
   Cys Arg Val Met Ala Val Thr Ser Gln Thr Phe Tyr Glu Val Met Phe
               900                 905                 910
   Asp Asp Gly Ser Phe Ser Arg Asp Thr Phe Pro Glu Asp Ile Val Ser
           915                 920                 925
   Arg Asp Cys Leu Lys Leu Gly Pro Pro Ala Glu Gly Glu Val Val Gln
       930                 935                 940
   Val Lys Trp Pro Asp Gly Lys Leu Tyr Gly Ala Lys Tyr Phe Gly Ser
   945                 950                 955                 960
   Asn Ile Ala His Met Tyr Gln Val Glu Phe Glu Asp Gly Ser Gln Ile
                   965                 970                 975
   Ala Met Lys Arg Glu Asp Ile Tyr Thr Leu Asp Glu Glu Leu Pro Lys
               980                 985                 990
   Arg Val Lys Ala Arg Phe Ser Thr  Ala Ser Asp Met Arg  Phe Glu Asp
           995                 1000                1005
   Thr Phe  Tyr Gly Ala Asp Ile  Ile Gln Gly Glu Arg  Lys Arg Gln
       1010                1015                1020
   Arg Val  Leu Ser Ser Arg Phe  Lys Asn Glu Tyr Val  Ala Asp Pro
       1025                1030                1035
   Val Tyr  Arg Thr Phe Leu Lys  Ser Ser Phe Gln Lys  Lys Cys Gln
       1040                1045                1050
   Lys Arg  Gln
       1055
<210> 9
   <211> 2988
   <212> DNA
   <213> Homo sapiens
<400> 9
   aaggggcggg gccgaagcgg cccagggggc gggcgtttga aatcagtgcc ttagagtaga 60
   ccctaaacct cattttatac cttcaagaac caattactta atgtctcttc cgtcttttcc 120
   gtccccgacc ccctcccaga ctccttcatt ccggtactgc gtggacggaa agccccgggt 180
   agccgacacc acgtccccgg ctagcgggag agagcgtgga aaaggattac accaaactgt 240
   ttaaatccaa cgactcctgc ttccatcctt tctcctgagc tagaaccaac aaacctagag 300
   agttgggctt cggaaaaact agtgttttca tttaattgga tatgaagaaa gaacaaatat 360
   gtacggggca accacgatct ttacaaagaa cataagttcc aggaaagcag gaaccttgtc 420
   tctcttgttc actgggtgta tcctctgcat atagaacagt gcctggcaca taataggtgc 480
   tgaattttgt tctaaacact gaggacattc tctgctacat ttgggtcgta cccccaggtc 540
   tgagtaattc aatagactta agaagacaga gcccagcagc aaccgaaaca taacagagtt 600
   gcaggatcag ctaacgtcaa tgcctgggca aagctgctgc ccagagtgga atctcactag 660
   tgaataaaca agcccaagaa agattatcat ctcatttgca aaaaaaaaag tacgctggta 720
   gatcctgcta cctcatagat aacaccagtc aaattttttt ttaaagtagc attttcctac 780
   attgtcaact atctagaaca tacctaaaaa ctaagagttt actgcttatt aaatggaaac 840
   tatgaagtct aaggccaact gtgcccagaa tccaaattgt aacataatga tatttcatcc 900
   aaccaaagaa gagtttaatg attttgataa atatattgct tacatggaat cccaaggtgc 960
   acacagagct ggcttggcta agataattcc acccaaagaa tggaaagcca gagagaccta 1020
   tgataatatc agtgaaatct taatagccac tcccctccag caggtggcct ctgggcgggc 1080
   aggggtgttt actcaatacc ataaaaaaaa gaaagccatg actgtggggg agtatcgcca 1140
   tttggcaaac agtaaaaaat atcagactcc accacaccag aatttcgaag atttggagcg 1200
   aaaatactgg aagaaccgca tctataattc accgatttat ggtgctgaca tcagtggctc 1260
   cttgtttgat gaaaacacta aacaatggaa tcttgggcac ctgggaacaa ttcaggacct 1320
   gctggaaaag gaatgtgggg ttgtcataga aggcgtcaat acaccctact tgtactttgg 1380
   catgtggaaa accacgtttg cttggcatac agaggacatg gacctttaca gcatcaacta 1440
   cctgcacctt ggggagccca aaacttggta tgtggtgccc ccagaacatg gccagcgcct 1500
   ggaacgcctg gccagggagc tcttcccagg cagttcccgg ggttgtgggg ccttcctgcg 1560
   gcacaaggtg gccctcatct cgcctacagt tctcaaggaa aatgggattc ccttcaatcg 1620
   cataactcag gaggctggag agttcatggt gacctttccc tatggctacc atgctggctt 1680
   caaccatggt ttcaactgcg cagaggccat caattttgcc actccgcgat ggattgatta 1740
   tggcaaaatg gcctcccagt gtagctgtgg ggaggcaagg gtgacctttt ccatggatgc 1800
   cttcgtgcgc atcctgcaac ctgaacgcta tgacctgtgg aaacgtgggc aagaccgggc 1860
   agttgtggac cacatggagc ccagggtacc agccagccaa gagctgagca cccagaagga 1920
   agtccagtta cccaggagag cagcgctggg cctgagacaa ctcccttccc actgggcccg 1980
   gcattcccct tggcctatgg ctgcccgcag tgggacacgg tgccacaccc ttgtgtgctc 2040
   ttcactccca cgccgatctg cagttagtgg cactgctacg cagccccggg ctgctgctgt 2100
   ccacagctct aagaagccca gctcaactcc atcatccacc cctggtccat ctgcacagat 2160
   tatccacccg tcaaatggca gacgtggtcg tggtcgccct cctcagaaac tgagagctca 2220
   ggagctgacc ctccagactc cagccaagag gcccctcttg gcgggcacaa catgcacagc 2280
   ttcgggccca gaacctgagc ccctacctga ggatggggct ttgatggaca agcctgtacc 2340
   actgagccca gggctccagc atcctgtcaa ggcttctggg tgcagctggg cccctgtgcc 2400
   ctaagtccac gggctgtctt tatatcccac tgccctgctg tgtgacagtt tgatgaaact 2460
   ggttacattt acatcccaaa actttggttg agtttgcagg actctaggca tgcatgaaag 2520
   agcccccctg gtgatgccct tggatgctgc caagtccatg gtagttttca attttgccat 2580
   acttttgttc ttcctaccgg accctggaat gtctttggat attgctaaaa tctatttctg 2640
   cagctgaggt tttatccact ggacacattt gtgtgtgaga actaggtctt gttgaggtta 2700
   gcgtaacctg gtatatgcaa ctaccatcct ctgggccaac tgtggaagct gctgcacttg 2760
   tgaagaatcc tgagctttga ttcctcttca gtctacgcat ttctctcttc ccctccctca 2820
   cccccttttt cttataaaac taggttcttt atacagataa ggtcagtaga gttccagaat 2880
   aaaagatatg acttttctga gttatttatg tacttaaaat atgttgtcac agtatttgtt 2940
   cccaaatata ttaaaggtaa ccaaaatgtt aaaaaaaaaa aaaaaaaa 2988
<210> 10
   <211> 523
   <212> PRT
   <213> Homo sapiens
<400> 10
   Met Glu Thr Met Lys Ser Lys Ala Asn Cys Ala Gln Asn Pro Asn Cys
   1               5                   10                  15
   Asn Ile Met Ile Phe His Pro Thr Lys Glu Glu Phe Asn Asp Phe Asp
               20                  25                  30
   Lys Tyr Ile Ala Tyr Met Glu Ser Gln Gly Ala His Arg Ala Gly Leu
           35                  40                  45
   Ala Lys Ile Ile Pro Pro Lys Glu Trp Lys Ala Arg Glu Thr Tyr Asp
       50                  55                  60
   Asn Ile Ser Glu Ile Leu Ile Ala Thr Pro Leu Gln Gln Val Ala Ser
   65                  70                  75                  80
   Gly Arg Ala Gly Val Phe Thr Gln Tyr His Lys Lys Lys Lys Ala Met
                   85                  90                  95
   Thr Val Gly Glu Tyr Arg His Leu Ala Asn Ser Lys Lys Tyr Gln Thr
               100                 105                 110
   Pro Pro His Gln Asn Phe Glu Asp Leu Glu Arg Lys Tyr Trp Lys Asn
           115                 120                 125
   Arg Ile Tyr Asn Ser Pro Ile Tyr Gly Ala Asp Ile Ser Gly Ser Leu
       130                 135                 140
   Phe Asp Glu Asn Thr Lys Gln Trp Asn Leu Gly His Leu Gly Thr Ile
   145                 150                 155                 160
   Gln Asp Leu Leu Glu Lys Glu Cys Gly Val Val Ile Glu Gly Val Asn
                   165                 170                 175
   Thr Pro Tyr Leu Tyr Phe Gly Met Trp Lys Thr Thr Phe Ala Trp His
               180                 185                 190
   Thr Glu Asp Met Asp Leu Tyr Ser Ile Asn Tyr Leu His Leu Gly Glu
           195                 200                 205
   Pro Lys Thr Trp Tyr Val Val Pro Pro Glu His Gly Gln Arg Leu Glu
       210                 215                 220
   Arg Leu Ala Arg Glu Leu Phe Pro Gly Ser Ser Arg Gly Cys Gly Ala
   225                 230                 235                 240
   Phe Leu Arg His Lys Val Ala Leu Ile Ser Pro Thr Val Leu Lys Glu
                   245                 250                 255
   Asn Gly Ile Pro Phe Asn Arg Ile Thr Gln Glu Ala Gly Glu Phe Met
               260                 265                 270
   Val Thr Phe Pro Tyr Gly Tyr His Ala Gly Phe Asn His Gly Phe Asn
           275                 280                 285
   Cys Ala Glu Ala Ile Asn Phe Ala Thr Pro Arg Trp Ile Asp Tyr Gly
       290                 295                 300
   Lys Met Ala Ser Gln Cys Ser Cys Gly Glu Ala Arg Val Thr Phe Ser
   305                 310                 315                 320
   Met Asp Ala Phe Val Arg Ile Leu Gln Pro Glu Arg Tyr Asp Leu Trp
                   325                 330                 335
   Lys Arg Gly Gln Asp Arg Ala Val Val Asp His Met Glu Pro Arg Val
               340                 345                 350
   Pro Ala Ser Gln Glu Leu Ser Thr Gln Lys Glu Val Gln Leu Pro Arg
   355 360 365
   Arg Ala Ala Leu Gly Leu Arg Gln Leu Pro Ser His Trp Ala Arg His
   370 375 380
   Ser Pro Trp Pro Met Ala Ala Arg Ser Gly Thr Arg Cys His Thr Leu
   385 390 395 400
   Val Cys Ser Ser Leu Pro Arg Arg Ser Ala Val Ser Gly Thr Ala Thr
   405 410 415
   Gln Pro Arg Ala Ala Ala Val His Ser Ser Lys Lys Pro Ser Ser Thr
   420 425 430
   Pro Ser Ser Thr Pro Gly Pro Ser Ala Gln Ile Ile His Pro Ser Asn
   435 440 445
   Gly Arg Arg Gly Arg Gly Arg Pro Pro Gln Lys Leu Arg Ala Gln Glu
   450 455 460
   Leu Thr Leu Gln Thr Pro Ala Lys Arg Pro Leu Leu Ala Gly Thr Thr
   465 470 475 480
   Cys Thr Ala Ser Gly Pro Glu Pro Glu Pro Leu Pro Glu Asp Gly Ala
   485 490 495
   Leu Met Asp Lys Pro Val Pro Leu Ser Pro Gly Leu Gln His Pro Val
   500 505 510
   Lys Ala Ser Gly Cys Ser Trp Ala Pro Val Pro
   515 520
<210> 11
   <211> 2339
   <212> DNA
   <213> Homo sapiens
<400> 11
   ccaagcctga gaatcaggag aagcctcaca gtgacacccc caactgagga aactcacaga      60
   gctgggacat actctacttc ttcagaaaaa agtatactga ctagagtgga gtccccctgg     120
   ggagtcagaa agcctgtgaa agatctcact tgttcaaaag tccaagtgtg aattactgtc     180
   tcacagataa accaaagtat tttgaaaaac aaaggggaga aaagaaatta ctccccagaa     240
   ctctcaggca tctagaggac acccaagaac gtgggagtca gctgcttctt gtgtgcagcc     300
   atgaagtctg tgcactccag tccccagaac acgagtcata ccatcatgac gttttaccca     360
   accatggaag aatttgcaga tttcaacaca tatgttgctt acatggagtc ccaaggcgca 420
   catcaagctg gccttgccaa ggtaattcca cccaaggaat ggaaagccag acagatgtat 480
   gatgatatcg aagacatctt aatagccact cccctccagc aggtgacctc tgggcaggga 540
   ggtgtgttta ctcaatacca taaaaagaag aaagccatga gggtggggca gtatcgccgc 600
   ttggcaaaca gtaaaaaata tcagactccg ccacaccaga attttgcaga tttggagcaa 660
   cgatactgga agagccaccc cggtaatcca ccaatttatg gtgctgatat cagcggctcc 720
   ttatttgaag aaagcactaa acaatggaac ctaggacacc tgggaacaat tctggacctg 780
   ttggagcagg aatgtggggt tgtcatcgag ggtgtcaaca caccctacct gtactttggc 840
   atgtggaaga ccacgtttgc ctggcacaca gaggacatgg acctttacag catcaactac 900
   ctgcactttg gggagcccaa aacttggtac gtggtgcccc cagaacatgg tcagcacctg 960
   gaacgcctgg ccagggagct cttcccagac atttctcggg gctgtgaggc cttcctgcgg 1020
   cacaaagtgg ccctcatctc gcctacagtt ctcaaggaaa atgggattcc cttcaattgc 1080
   atgactcagg aggctgggga gttcatggtg acctttccct atggctacca tgctggcttc 1140
   aatcacggct tcaactgcgc agaagccatt aattttgcca ctccacgatg gattgattat 1200
   ggcaaaatgg cctctcagtg tagctgtggg gagtcgacag tgaccttttc catggacccc 1260
   tttgtgcgca ttgtgcaacc cgagagttat gagctctgga aacacaggca agacttggcc 1320
   attgtggaac acacagagcc cagggttgca gaaagccaag agctgagcaa ctggagagat 1380
   gatatagtac ttagaagagc tgctctgggc ctgaggcttc tcccaaacct cacagcccag 1440
   tgtcccacac agcctgtgtc ctcagggcac tgttacaacc caaaaggctg tggcactgat 1500
   gctgtgcctg gatccgcatt ccaaagctct gcatatcata cccagaccca gtcacttacc 1560
   ctggggatgt cagccagggt tcttctccct tccactggaa gctggggttc tggtcgtggt 1620
   cgtggtcgtg gtcaaggtca aggtcgaggt tgcagtcgtg gtcgtggtca tggttgttgt 1680
   actcgagaac tggggactga ggagccaact gttcagcctg catccaagag gcgcctttta 1740
   atgggtacaa ggagtagagc tcaaggccac aggcctcagc tcccgcttgc caatgatttg 1800
   atgacaaatc tgtccctttg agtggtggcc ttcagcatct tgccaaggct tctggctgct 1860
   gctgtgtccc tgatcttcaa ctcctggggc ccccactgga tcgtgatgaa accatgcacc 1920
   ctggcctgtg cctgctatcc ctcaacagca ctactagtaa tctccctgat gttgtctgca 1980
   tgactcctcc caatgtcatt gtgcctttga ttaagttttc cagggacact ggtggggact 2040
   ggaactgatt aagttcacca gggacacttg cctggtgaac atgggcaagg ctgtagcaat 2100
   ggaccacttt tacggctcta gggttctgac tccaactaag ttttccagaa tctcctgggc 2160
   tcctgactca tctgctgggt ctaaagacac tgagtttagg gatattttcc tccaatacat 2220
   gatcaatcct ctggatccac ggctatggaa tatggtgaca aatgtcagtg tctctcttat 2280
   tccaacccca ggatcagaga agattcttta cctgcagtaa ctgacacatt tccaaggcc 2339
<210> 12
   <211> 506
   <212> PRT
   <213> Homo sapiens
<400> 12
   Met Lys Ser Val His Ser Ser Pro Gln Asn Thr Ser His Thr Ile Met
   1               5                   10                  15
   Thr Phe Tyr Pro Thr Met Glu Glu Phe Ala Asp Phe Asn Thr Tyr Val
               20                  25                  30
   Ala Tyr Met Glu Ser Gln Gly Ala His Gln Ala Gly Leu Ala Lys Val
           35                  40                  45
   Ile Pro Pro Lys Glu Trp Lys Ala Arg Gln Met Tyr Asp Asp Ile Glu
       50                  55                  60
   Asp Ile Leu Ile Ala Thr Pro Leu Gln Gln Val Thr Ser Gly Gln Gly
   65                  70                  75                  80
   Gly Val Phe Thr Gln Tyr His Lys Lys Lys Lys Ala Met Arg Val Gly
                   85                  90                  95
   Gln Tyr Arg Arg Leu Ala Asn Ser Lys Lys Tyr Gln Thr Pro Pro His
               100                 105                 110
   Gln Asn Phe Ala Asp Leu Glu Gln Arg Tyr Trp Lys Ser His Pro Gly
           115                 120                 125
   Asn Pro Pro Ile Tyr Gly Ala Asp Ile Ser Gly Ser Leu Phe Glu Glu
       130                 135                 140
   Ser Thr Lys Gln Trp Asn Leu Gly His Leu Gly Thr Ile Leu Asp Leu
   145                 150                 155                 160
   Leu Glu Gln Glu Cys Gly Val Val Ile Glu Gly Val Asn Thr Pro Tyr
                   165                 170                 175
   Leu Tyr Phe Gly Met Trp Lys Thr Thr Phe Ala Trp His Thr Glu Asp
               180                 185                 190
   Met Asp Leu Tyr Ser Ile Asn Tyr Leu His Phe Gly Glu Pro Lys Thr
           195                 200                 205
   Trp Tyr Val Val Pro Pro Glu His Gly Gln His Leu Glu Arg Leu Ala
       210                 215                 220
   Arg Glu Leu Phe Pro Asp Ile Ser Arg Gly Cys Glu Ala Phe Leu Arg
   225                 230                 235                 240
   His Lys Val Ala Leu Ile Ser Pro Thr Val Leu Lys Glu Asn Gly Ile
                   245                 250                 255
   Pro Phe Asn Cys Met Thr Gln Glu Ala Gly Glu Phe Met Val Thr Phe
               260                 265                 270
   Pro Tyr Gly Tyr His Ala Gly Phe Asn His Gly Phe Asn Cys Ala Glu
           275                 280                 285
   Ala Ile Asn Phe Ala Thr Pro Arg Trp Ile Asp Tyr Gly Lys Met Ala
       290                 295                 300
   Ser Gln Cys Ser Cys Gly Glu Ser Thr Val Thr Phe Ser Met Asp Pro
   305                 310                 315                 320
   Phe Val Arg Ile Val Gln Pro Glu Ser Tyr Glu Leu Trp Lys His Arg
                   325                 330                 335
   Gln Asp Leu Ala Ile Val Glu His Thr Glu Pro Arg Val Ala Glu Ser
               340                 345                 350
   Gln Glu Leu Ser Asn Trp Arg Asp Asp Ile Val Leu Arg Arg Ala Ala
           355                 360                 365
   Leu Gly Leu Arg Leu Leu Pro Asn Leu Thr Ala Gln Cys Pro Thr Gln
       370                 375                 380
   Pro Val Ser Ser Gly His Cys Tyr Asn Pro Lys Gly Cys Gly Thr Asp
   385                 390                 395                 400
   Ala Val Pro Gly Ser Ala Phe Gln Ser Ser Ala Tyr His Thr Gln Thr
                   405                 410                 415
   Gln Ser Leu Thr Leu Gly Met Ser Ala Arg Val Leu Leu Pro Ser Thr
               420                 425                 430
   Gly Ser Trp Gly Ser Gly Arg Gly Arg Gly Arg Gly Gln Gly Gln Gly
           435                 440                 445
   Arg Gly Cys Ser Arg Gly Arg Gly His Gly Cys Cys Thr Arg Glu Leu
       450                 455                 460
   Gly Thr Glu Glu Pro Thr Val Gln Pro Ala Ser Lys Arg Arg Leu Leu
   465                 470                 475                 480
   Met Gly Thr Arg Ser Arg Ala Gln Gly His Arg Pro Gln Leu Pro Leu
                   485                 490                 495
   Ala Asn Asp Leu Met Thr Asn Leu Ser Leu
               500                 505
<210> 13
   <211> 2405
   <212> DNA
   <213> Homo sapiens
<400> 13
   tccggcgtgg tgcgcaggcg cggtatcccc cctcccccgc cagctcgacc ccggtgtggt 60
   gcgcaggcgc agtctgcgca gggactggcg ggactgcgcg gcggcaacag cagacatgtc 120
   gggggtccgg ggcctgtcgc ggctgctgag cgctcggcgc ctggcgctgg ccaaggcgtg 180
   gccaacagtg ttgcaaacag gaacccgagg ttttcacttc actgttgatg ggaacaagag 240
   ggcatctgct aaagtttcag attccatttc tgctcagtat ccagtagtgg atcatgaatt 300
   tgatgcagtg gtggtaggcg ctggaggggc aggcttgcga gctgcatttg gcctttctga 360
   ggcagggttt aatacagcat gtgttaccaa gctgtttcct accaggtcac acactgttgc 420
   agcacaggga ggaatcaatg ctgctctggg gaacatggag gaggacaact ggaggtggca 480
   tttctacgac accgtgaagg gctccgactg gctgggggac caggatgcca tccactacat 540
   gacggagcag gcccccgccg ccgtggtcga gctagaaaat tatggcatgc cgtttagcag 600
   aactgaagat gggaagattt atcagcgtgc atttggtgga cagagcctca agtttggaaa 660
   gggcgggcag gcccatcggt gctgctgtgt ggctgatcgg actggccact cgctattgca 720
   caccttatat ggaaggtctc tgcgatatga taccagctat tttgtggagt attttgcctt 780
   ggatctcctg atggagaatg gggagtgccg tggtgtcatc gcactgtgca tagaggacgg 840
   gtccatccat cgcataagag caaagaacac tgttgttgcc acaggaggct acgggcgcac 900
   ctacttcagc tgcacgtctg cccacaccag cactggcgac ggcacggcca tgatcaccag 960
   ggcaggcctt ccttgccagg acctagagtt tgttcagttc caccctacag gcatatatgg 1020
   tgctggttgt ctcattacgg aaggatgtcg tggagaggga ggcattctca ttaacagtca 1080
   aggcgaaagg tttatggagc gatacgcccc tgtcgcgaag gacctggcgt ctagagatgt 1140
   ggtgtctcgg tccatgactc tggagatccg agaaggaaga ggctgtggcc ctgagaaaga 1200
   tcacgtctac ctgcagctgc accacctacc tccagagcag ctggccacgc gcctgcctgg 1260
   catttcagag acagccatga tcttcgctgg cgtggacgtc acgaaggagc cgatccctgt 1320
   cctccccacc gtgcattata acatgggcgg cattcccacc aactacaagg ggcaggtcct 1380
   gaggcacgtg aatggccagg atcagattgt gcccggcctg tacgcctgtg gggaggccgc     1440
   ctgtgcctcg gtacatggtg ccaaccgcct cggggcaaac tcgctcttgg acctggttgt     1500
   ctttggtcgg gcatgtgccc tgagcatcga agagtcatgc aggcctggag ataaagtccc     1560
   tccaattaaa ccaaacgctg gggaagaatc tgtcatgaat cttgacaaat tgagatttgc     1620
   tgatggaagc ataagaacat cggaactgcg actcagcatg cagaagtcaa tgcaaaatca     1680
   tgctgccgtg ttccgtgtgg gaagcgtgtt gcaagaaggt tgtgggaaaa tcagcaagct     1740
   ctatggagac ctaaagcacc tgaagacgtt cgaccgggga atggtctgga acacggacct     1800
   ggtggagacc ctggagctgc agaacctgat gctgtgtgcg ctgcagacca tctacggagc     1860
   agaggcacgg aaggagtcac ggggcgcgca tgccagggaa gactacaagg tgcggattga     1920
   tgagtacgat tactccaagc ccatccaggg gcaacagaag aagccctttg aggagcactg     1980
   gaggaagcac accctgtcct atgtggacgt tggcactggg aaggtcactc tggaatatag     2040
   acccgtgatc gacaaaactt tgaacgaggc tgactgtgcc accgtcccgc cagccattcg     2100
   ctcctactga tgagacaaga tgtggtgatg acagaatcag cttttgtaat tatgtataat     2160
   agctcatgca tgtgtccatg tcataactgt cttcatacgc ttctgcactc tggggaagaa     2220
   ggagtacatt gaagggagat tggcacctag tggctgggag cttgccagga acccagtggc     2280
   cagggagcgt ggcacttacc tttgtccctt gcttcattct tgtgagatga taaaactggg     2340
   cacagctctt aaataaaata taaatgaaca aactttcttt tatttccaaa aaaaaaaaaa     2400
   aaaaa                                                                 2405
<210> 14
   <211> 664
   <212> PRT
   <213> Homo sapiens
<400> 14
<210> 15
   <211> 1161
   <212> DNA
   <213> Homo sapiens
<400> 15
   acccgggaaa ccggaagccg cctcccactt ggttgctcgt acgcggctag tgggtcctca      60
   gtggatgtag gctgggcgcc gcgatgttcg acgggacacc ggcggagagc gacctcgggg     120
   ttaaggggtg gggctgacgt caggagccaa gatggcggcg gtggtcgccc tctccttgag     180
   gcgccggttg ccggccacaa cccttggcgg agcctgcctg caggcctccc gaggagccca     240
   gacagctgca gccacagctc cccgtatcaa gaaatttgcc atctatcgat gggacccaga     300
   caaggctgga gacaaacctc atatgcagac ttatgaagtt gaccttaata aatgtggccc     360
   catggtattg gatgctttaa tcaagattaa gaatgaagtt gactctactt tgaccttccg     420
   aagatcatgc agagaaggca tctgtggctc ttgtgcaatg aacatcaatg gaggcaacac     480
   tctagcttgc acccgaagga ttgacaccaa cctcaataag gtctcaaaaa tctaccctct     540
   tccacacatg tatgtgataa aggatcttgt tcccgatttg agcaacttct atgcacagta     600
   caaatccatt gagccttatt tgaagaagaa ggatgaatct caggaaggca agcagcagta     660
   tctgcagtcc atagaagagc gtgagaaact ggacgggctc tacgagtgca ttctctgtgc     720
   ctgctgtagc accagctgcc ccagctactg gtggaacgga gacaaatatc tggggcctgc     780
   agttcttatg caggcctatc gctggatgat tgactccaga gatgacttca cagaggagcg     840
   cctggccaag ctgcaggacc cattctctct ataccgctgc cacaccatca tgaactgcac     900
   aaggacctgt cctaagggtc tgaatccagg gaaagctatt gcagagatca agaaaatgat     960
   ggcaacctat aaggagaaga aagcttcagt ttaactgttt ccatgctaaa catgatttat    1020
   aaccagctca gagctgaaca taatttatat ctaatttgag ttcctttaaa gatcttggtt    1080
   ttccatgaat acagcatgta taataaaaat tttaagaaat aaatgttatt ctactttatt    1140
   aacaaaaaaa aaaaaaaaaa a                                              1161
<210> 16
   <211> 280
   <212> PRT
   <213> Homo sapiens
<400> 16
<210> 17
   <211> 2858
   <212> DNA
   <213> Homo sapiens
<400> 17
   gcgtcacttc cgtccagacc ggaacccaag atggctgcgc tgttgctgag acacgttggt 60
   cgtcattgcc tccgagccca ctttagccct cagctctgta tcagaaatgc tgttcctttg 120
   ggaaccacgg ccaaagaaga gatggagcgg ttctggaata agaatatagg ttcaaaccgt 180
   cctctgtctc cccacattac tatctacagt tggtctcttc ccatggcgat gtccatctgc 240
   caccgtggca ctggtattgc tttgagtgca ggggtctctc tttttggcat gtcggccctg 300
   ttactccctg ggaactttga gtcttatttg gaacttgtga agtccctgtg tctggggcca 360
   gcactgatcc acacagctaa gtttgcactt gtcttccctc tcatgtatca tacctggaat 420
   gggatccgac acttgatgtg ggacctagga aaaggcctga agattcccca gctataccag 480
   tctggagtgg ttgtcctggt tcttactgtg ttgtcctcta tggggctggc agccatgtga 540
   agaaaggagg ctcccagcat catcttccta cacattatta cattcaccca tctttctgtt 600
   tgtcattctt atctccagcc tgggaaaagt tctccttatt tgtttagatc cttttgtatt 660
   ttcagatctc cttggagcag tagagtacct ggtagaccat aatagtggaa aagggtctag 720
   ttttcccctt gtttctaaag atgaggtggc tgcaaaaact cccctttttt gcccacagct 780
   tgcctactct cggcctagaa gcagttattc tctctccata ttgggctttg atttgtgctg 840
   agggtcagct tttggctcct tcttcctgag acagtggaaa caatgccagc tctgtggctt 900
   ctgccctggg gatgggccgg gttggggggt gggttggtga ggctttgggt gccactgcct 960
   gtgggttgct ggcttaaagg acaattctct tcattggtga gagcccaggc cattaacacc 1020
   tacacagtgt tattgaaaga agagaggtgg gggtggaggg gaattagtct gtcccagcta 1080
   gagggagata aagagggcta gttagttctt ggagcagctg cttttgagga gaaaatatat 1140
   agctttggac acgaggaaga tctagaaaat tatcattgaa catattaatg gttatttctt 1200
   tttcttggat ttccagaaaa gcctcttaat tttatgcttt ctcatcgaag taatgtaccc 1260
   tttttttctg aaactgaatt aaatactcat tttatctttg actctccttg aaatctagag 1320
   aaaccaagaa aatggctgtt gggaaggaac caatttcctc ctcttccctc gggtctcagg 1380
   catttacatc ctccctctcc ccgcaatctg acctttacca ggagggaaac agttctccta 1440
   catctcatca ttggaaaagt tttcagggaa tcagatagaa cttagccaga gatttaaata 1500
   tcacagaaaa gcctcagaga aggaaggaga aaaagaaaag aagtgacgca tgtagagtgc 1560
   ttttgggtta taggcaccaa aatcccatta aggactgatt ataagcttca tggtacagtt 1620
   cagcaaatta tgattcattg aggggcacag aggaccagtg ttggtgacag ctaggggatg 1680
   atgacctgag gttataggct tggggtgaat gaagcataga gttttttttt aaaaaagaag 1740
   ggattgttga aaacctggca aaaatgtata atttaatgag aaaacttgct gcttttaaaa 1800
   tccatatagg ccaggcttta gcaggcatgc tgttttgata gtttttggga actctggaat 1860
   aagagactta tctcatctgt cacttcgagt tgttggccag ccagttaaag ctgtgggtcg 1920
   aagaggggaa atgttaactg gctggtgtca attggatagg aagaccttag ttaaggtggg 1980
   gacccgctgt tttaacagtc ttcattcagg gtcccaaata gtatttggct ttaagtaatg 2040
   attggttttc cctttttact agaggggccc tgggaagtcc ttgtaccttt cctccatttt 2100
   aaaccagctg ttctctactt tgtccttgga atgagggaca agtgatcatg acagaacact 2160
   ttgtcattgg gactgggaaa ggtttggcaa gggcattaaa tttaacagcc agtgccagga 2220
   aaatataaaa tggggtcaat gataaacagc tgttagaggc tggaaaatgg gtagggcagt 2280
   tgaatttttt ggtggtttcg taccatttgg gtgattgaag catggtagtg ggtgggtggg 2340
   gggtgtgaca gagcatcatg tttgttattc tgccttaaat ttctacttac tgtctttttc 2400
   ttctctgtct tccaatcact ttaatatctt caattttcaa actatatttg tacttgggct 2460
   tagatagaaa gtcttacaca agcatagtat cttctacttt ggttttccct accttttctt 2520
   ccccaccttc tccaaacaca catatacata ctctactcaa ttctatttct gattttgtag 2580
   ttgttagttg tccatgctca ggataaaaaa tgagtgggta gggttgaggg actggttctt 2640
   tgaggttctg ccctttttcc atgattcaga ccaactttct cttggtcatt tctggagtat 2700
   aactgactca attcttgtaa aaatgtgttc cacccaaacc actgtatgtt cttttcccta 2760
   ctttattttc tcctaccttc cttctcctaa ttgtgttaca agaggcagcc atagcaagaa 2820
   tggaaaatcc agatcagtaa aagattcaac aaaaaaaa 2858
<210> 18
   <211> 169
   <212> **PRT**
   <213> Homo sapiens
<400> 18
<210> 19
   <211> 1395
   <212> **DNA**
   <213> Homo sapiens
<400> 19
   cctgttttct ttcgtcgtcg tgggtgggaa ttgtcgccta agtggttccg ggttggtgga       60
   tgaccttgag ccctcaggaa cgagatggcg gttctctgga ggctgagtgc cgtttgcggt      120
   gccctaggag gccgagctct gttgcttcga actccagtgg tcagacctgc tcatatctca      180
   gcatttcttc aggaccgacc tatcccagaa tggtgtggag tgcagcacat acacttgtca      240
   ccgagccacc attctggctc caaggctgca tctctccact ggactagcga gagggttgtc      300
   agtgttttgc tcctgggtct gcttccggct gcttatttga atccttgctc tgcgatggac      360
   tattccctgg ctgcagccct cactcttcat ggtcactggg gccttggaca agttgttact      420
   gactatgttc atggggatgc cttgcagaaa gctgccaagg cagggctttt ggcactttca      480
   gctttaacct ttgctgggct ttgctatttc aactatcacg atgtgggcat ctgcaaagct      540
   gttgccatgc tgtggaagct ctgacctttt tgacttcata ctttgaagaa ttgatgtatg      600
   cctctttgcc tctgctttgt catgccatta agctcacaat aaggaagaaa taacagataa      660
   gtccattggt ggacagcctt cttctcttaa tcacaagatt attttcagaa tttaatcttt      720
   gaggaaaagg tttgagagga attatatcta agttgtgaga ctgagttcta tattctggtg      780
   agttaatggg gttgcctccc agcttcttat aagactcaca gtataactaa acatgatata      840
   tcagcttttg cctttcaatt tatcaatctc ttaaagagaa tccaacttta ttacgattag      900
   tatatgatca aacttccata tttgccttgg gaataatgga caaagggaaa tactcttaat      960
   tcatgaataa aaactttgca gaaaattaga cagtgtttaa ttttcgaaaa cttccctctc     1020
   tagacagtag ataccaccta ctgatggtta catatactag ggaaatttta aaattaggaa     1080
   atgctgatag ctcatattat aaatttctaa atcctaggaa gaaacgcttg gagtgcttct     1140
   gaatatacag aagttccatt taagggcaag tttccctgta gatgtatcaa aatactacca     1200
   actgtaaatt gagatttaat tcccaaatgt attctacttg ttctaaaaca atctgtccac     1260
   aaatataaaa ctataagtaa taaattgtta ttttcgcaca atgggaatct ctaatgtgaa     1320
   aatgtattct atgaaaataa tttttttaaa taaaatgtta tataataaaa gtgtcttcta      1380
   tgcttttata tatta                                                      1395
<210> 20
   <211> 159
   <212> PRT
   <213> Homo sapiens
<400> 20
<210> 21
   <211> 73
   <212> DNA
   <213> Homo sapiens
<400> 21
   ggccggctgg ggttcctggg gatgggattt gcttcctgtc acaaatcaca ttgccaggga    60
   tttccaaccg acc                                                        73
<210> 22
   <211> 97
   <212> DNA
   <213> Homo sapiens
<400> 22
   ctcaggtgct ctggctgctt gggttcctgg catgctgatt tgtgacttaa gattaaaatc 60
   acattgccag ggattaccac gcaaccacga ccttggc 97
<210> 23
   <211> 90
   <212> DNA
   <213> Homo sapiens
<400> 23
   ccgggcccct gtgagcatct taccggacag tgctggattt cccagcttga ctctaacact 60
   gtctggtaac gatgttcaaa ggtgacccgc 90
<210> 24
   <211> 95
   <212> DNA
   <213> Homo sapiens
<400> 24
   ccagctcggg cagccgtggc catcttactg ggcagcattg gatggagtca ggtctctaat 60
   actgcctggt aatgatgacg gcggagccct gcacg 95
<210> 25
   <211> 68
   <212> DNA
   <213> Homo sapiens
<400> 25
   ccctcgtctt acccagcagt gtttgggtgc ggttgggagt ctctaatact gccgggtaat 60
   gatggagg 68
<210> 26
   <211> 102
   <212> DNA
   <213> Homo sapiens
<400> 26
   ggtcctctga ctctcttcgg tgacgggtat tcttgggtgg ataatacgga ttacgttgtt 60
   attgcttaag aatacgcgta gtcgaggaga gtaccagcgg ca 102
<210> 27
   <211> 14
   <212> PRT
   <213> Homo sapiens
<220>
   <221> MOD_RES
   <222> (8)..(8)
   <223> Glu or Ala
<400> 27
   Glu Asp Glu Glu Glu Glu Gln Xaa Ala Ala Ala Leu Asp Leu
   1               5                   10
<210> 28
   <211> 14
   <212> **PRT**
   <213> Pan sp.
<400> 28
   Glu Asp Glu Glu Glu Glu Glu Glu Ala Ala Ala Leu Asp Leu
   1               5                   10
<210> 29
   <211> 14
   <212> PRT
   <213> Pongo sp.
<400> 29
   Glu Asp Glu Glu Glu Glu Gln Glu Ala Ala Ala Leu Asp Leu
   1               5                   10
<210> 30
   <211> 14
   <212> PRT
   <213> Macaca sp.
<400> 30
   Glu Asp Glu Glu Glu Glu Gln Glu Ala Ala Ala Leu Asp Leu
   1               5                   10
<210> 31
   <211> 14
   <212> PRT
   <213> Papio sp.
<400> 31
   Glu Asp Glu Glu Glu Glu Gln Glu Ala Ala Ala Leu Asp Leu
   1               5                   10
<210> 32
   <211> 14
   <212> PRT
   <213> Unknown
<220>
   <223> Description of Unknown: Marmoset peptide
<400> 32
   Glu Asp Glu Glu Glu Glu Gln Glu Ala Ala Ala Leu Asp Leu
   1 5 10
<210> 33
   <211> 14
   <212> PRT
   <213> Tarsius sp.
<400> 33
   Glu Asp Glu Glu Glu Glu Gln Glu Ala Ala Ala Leu Asp Leu
   1 5 10
<210> 34
   <211> 14
   <212> PRT
   <213> Microcebus sp.
<400> 34
   Glu Asp Glu Glu Glu Glu Gln Glu Ala Ala Ala Leu Asp Leu
   1 5 10
<210> 35
   <211> 14
   <212> PRT
   <213> Galago sp.
<400> 35
   Glu Asp Glu Glu Glu Glu Gln Glu Ala Ser Ala Leu Asp Leu
   1 5 10
<210> 36
   <211> 14
   <212> PRT
   <213> Tupaia sp.
<400> 36
   Glu Asp Glu Glu Glu Glu Gln Glu Ala Ala Ala Leu Asp Leu
   1 5 10
<210> 37
   <211> 14
   <212> PRT
   <213> Mus sp.
<400> 37
   Glu Asp Glu Glu Asp Glu Pro Glu Ala Ala Ala Leu Asp Leu
   1 5 10
<210> 38
   <211> 14
   <212> PRT
   <213> Rattus sp.
<400> 38
   Glu Asp Glu Asp Glu Glu Pro Glu Ala Ala Ala Leu Asp Leu
   1 5 10
<210> 39
   <211> 14
   <212> PRT
   <213> Dipodomys sp.
<400> 39
   Glu Asp Glu Glu Glu Glu Gln Glu Ala Ala Ala Leu Asp Leu
   1 5 10
<210> 40
   <211> 14
   <212> PRT
   <213> Cavia sp.
<400> 40
   Glu Asp Glu Glu Glu Glu Gln Glu Ala Ala Ala Leu Asp Leu
   1 5 10
<210> 41
   <211> 14
   <212> PRT
   <213> Unknown
<220>
   <223> Description of Unknown: Squirrel peptide
<400> 41
   Glu Glu Asp Glu Glu Glu Gln Glu Ala Ala Ala Leu Asp Leu
   1 5 10
<210> 42
   <211> 14
   <212> PRT
   <213> Oryctolagus cuniculus
<400> 42
   Glu Asp Glu Asp Glu Asp Gln Glu Ala Ala Ala Leu Asp Leu
   1 5 10
<210> 43
   <211> 14
   <212> PRT
   <213> Ochotona sp.
<400> 43
   Glu Asp Glu Glu Glu Glu Gln Glu Ala Ala Ala Leu Asp Leu
   1 5 10
<210> 44
   <211> 14
   <212> PRT
   <213> Vicugna pacos
<400> 44
   Glu Glu Glu Glu Glu Glu Gln Glu Ala Ala Ala Leu Asp Leu
   1               5                   10
<210> 45
   <211> 14
   <212> PRT
   <213> Unknown
<220>
   <223> Description of Unknown: Dolphin peptide
<400> 45
   Glu Glu Glu Glu Glu Asp Gln Glu Ala Ala Ala Leu Asp Leu
   1               5                   10
<210> 46
   <211> 14
   <212> PRT
   <213> Bos sp.
<400> 46
   Asp Asp Glu Glu Glu Glu His Glu Ala Ala Val Leu Asp Leu
   1               5                   10
<210> 47
   <211> 14
   <212> PRT
   <213> Equus caballus
<400> 47
   Glu Glu Asp Glu Glu Glu Pro Glu Ala Ala Val Leu Asp Leu
           1               5                   10
<210> 48
   <211> 14
   <212> PRT
   <213> Felis catus
<400> 48
   Glu Glu Glu Glu Glu Glu Gln Glu Ala Ala Ala Trp Asp Leu
   1               5                   10
<210> 49
   <211> 14
   <212> PRT
   <213> Canis lupus
<400> 49
   Glu Glu Glu Glu Glu Glu Gln Glu Ala Ala Ala Leu Asp Leu
   1               5                   10
<210> 50
   <211> 14
   <212> PRT
   <213> Unknown
<220>
   <223> Description of Unknown: Micro-bat peptide
<400> 50
   Glu Glu Glu Glu Glu Glu Glu Ala Ala Ala Ala Leu Asp Leu
   1               5                   10
<210> 51
   <211> 14
   <212> PRT
   <213> Unknown
<220>
   <223> Description of Unknown: Mega-bat peptide
<400> 51
   Glu Glu Glu Glu Glu Glu Gln Glu Ala Ala Val Leu Asp Leu
   1               5                   10
<210> 52
   <211> 14
   <212> PRT
   <213> Unknown
<220>
   <223> Description of Unknown: Hedgehog peptide
<400> 52
   Glu Glu Glu Glu Glu Glu Gln Glu Ala Ala Ala Leu Asp Leu
   1               5                   10
<210> 53
   <211> 14
   <212> PRT
   <213> Unknown
<220>
   <223> Description of Unknown: Elephant peptide
<400> 53
   Glu Glu Asp Glu Glu Glu Gln Glu Ala Ala Ala Leu Asp Leu
   1 5 10
<210> 54
   <211> 14
   <212> PRT
   <213> Procavia capensis
<400> 54
   Glu Glu Glu Glu Glu Glu Gln Glu Ala Ala Ala Leu Asp Leu
   1 5 10
<210> 55
   <211> 13
   <212> PRT
   <213> Tenrec ecaudatus
<400> 55
   Glu Glu Glu Glu Glu Gln Glu Ala Ala Ala Leu Asp Leu
   1 5 10
<210> 56
   <211> 14
   <212> PRT
   <213> Unknown
<220>
   <223> Description of Unknown: Armadillo peptide
<400> 56
   Asn Asn Asn Asn Asn Asn Asn Asn Asn Asn Asn Asn Asn Asn
   1 5 10
<210> 57
   <211> 14
   <212> PRT
   <213> Unknown
<220>
   <223> Description of Unknown: Sloth peptide
<400> 57
   Glu Glu Asp Asp Glu Glu Gln Glu Ser Ala Ala Leu Asp Leu
   1 5 10
<210> 58
   <211> 11
   <212> PRT
   <213> Unknown
<220>
   <223> Description of Unknown: Opossum peptide
<400> 58
   Glu Asp Glu Glu Glu Ala Ala Ala Leu Asp Leu
   1 5 10
<210> 59
   <211> 14
   <212> PRT
   <213> Ornithorhynchus anatinus
<400> 59
   Glu Glu Glu Glu Glu Glu Gln Glu Ala Ala Ala Leu Asp Leu
   1 5 10
<210> 60
   <211> 13
   <212> PRT
   <213> Gallus gallus
<400> 60
   Glu Glu Glu Glu Glu Gln Glu Ala Ala Val Leu Asp Leu
   1 5 10
<210> 61
   <211> 10
   <212> PRT
   <213> Taeniopygia guttata
<400> 61
   Glu Glu Gln Glu Ala Ala Ala Leu Asp Leu
   1 5 10
<210> 62
   <211> 12
   <212> PRT
   <213> Unknown
<220>
   <223> Description of Unknown: Lizard peptide
<400> 62
   Glu Glu Glu Glu Pro Glu Ala Ala Val Leu Asp Leu
   1 5 10
<210> 63
   <211> 12
   <212> PRT
   <213> Tetraodon sp.
<400> 63
   Leu Lys Lys Gly Gln Gly Ala Ala Ala Arg Arg Leu
   1 5 10

## Description

### CROSS-REFERENCE TO RELATED APPLICATIONS

This application claims benefit under 35 U.S.C. § 119(e) of U.S. Provisional Application No. 61/831,758 filed June 6, 2013.

### GOVERNMENT SUPPORT

This invention was made with federal funding under Grant Nos. U24CA143845, R01CA155202, CA059267, and GM097360 awarded by the National Institutes of Health. The U.S. government has certain rights in the invention.

### SEQUENCE LISTING

The instant application contains a Sequence Listing which has been submitted electronically in ASCII format. Said ASCII copy, created on June 6, 2014, is named 030258-076041-PCT_SL.txt and is 100,694 bytes in size

### TECHNICAL FIELD

The technology described herein relates to treatment of cancer and other conditions, e.g. graft vs. host and neurological diseases such as autism and schizophrenia.

### BACKGROUND

Genomic instability is a major contributing factor to the development and onset of age-related diseases such as cancer (Maslov and Vijg, 2009; Negrini et al., 2010). Cancer cells are often characterized by copy number alterations: copy gains or losses of chromosome arms and/or whole chromosomes as well as amplifications of smaller genomic fragments (Beroukhim et al., 2010; Hook et al., 2007; Stratton et al., 2009). Even though cancer genomes frequently have altered chromosomal regions, there is little knowledge about the regulatory mechanisms or factors that are involved in promoting copy number alterations at specific regions of the genome.

### SUMMARY

The invention is as defined in the claims. KDM4A is a demethylase, controlling the accessibility of the chromosomal DNA in a cell. As demonstrated herein, increased levels of KDM4A in cancer cells leads to certain segments of the genome being inappropriately amplified (replicated) and predicts a worse outcome for patients having such an increase in KDM4A.

The inventor, as demonstrated herein, has further discovered that the level of activity of KDM4A (and/or certain mutations that affect the activity of KDM4A, e.g., SNPs) can determine whether a tumor is senstitive or resistant to a given chemotherapeutic. Described herein are methods of modulating the level and/or activity of KDM4A, e.g., by using small molecules and/or nucleic acids. Accordingly, provided herein are methods of treatment relating to subjects having such KDM4A mutations and/or altered activity levels as well as methods of treatment that comprise modulating the activity level of KDM4A.

In one aspect, described herein is a method of treating cancer, the method comprising administering an S-phase chemotherapeutic to a subject determined to have a level of KDM4A gene expression which is not higher than a reference level or determined not to have KDM4A gene amplification and not administering an S-phase chemotherapeutic to a subject determined to have a level of KDM4A gene expression which is higher than a reference level or determined to have KDM4A gene amplification. In some embodiments, the S-phase chemotherapeutic is selected from the group consisting of cisplatin; 5-flurouracil; 6-mercaptopurine; capecitabine; cladribine; clorfarabine; cytarabine; doxorubicin; fludarabine; floxuridine; gemcitabine; hydroxyurea; methotrexate; pemetrexed; pentostatin; prednisone; procarbazine; and thioguanine.

In one aspect, described herein is a method of treating cancer, the method comprising administering a chemotherapeutic selected from the group consisting of mTOR inhibitors; protein synthesis inhibitors; Braf inhibitors; PI3K inhibitors; Cdk inhibitors; Aurora B inhibitors; FLT3 inhibitors; PLK1/2/3 inhibitors; Eg5 inhibitors; β-tubulin inhibitors; BMP inhibitors; HDAC inhibitors; Akt inhibitors; IGF1R inhibitors; p53 inhibitors; hdm2 inhibitors; STAT3 inhibitors; and VEGFR inhibitors and not administering a chemotherapeutic selected from the group consisting of EGFR inhibitors; ErbB2 inhibitors; transcription inhibitors; and MEK1/2 inhibitors to a subject determined to have a KDM4A dampening mutation. In some embodiments, the KDM4A dampening mutation is present in the tumor but not the non-tumor cells of the subject. In one aspect, described herein is a method of treating cancer, the method comprising administering a reduced dose of a chemotherapeutic agent selected from the group consisting of mTOR inhibitors; protein synthesis inhibitors; Braf inhibitors; PI3K inhibitors; Cdk inhibitors; Aurora B inhibitors; FLT3 inhibitors; PLK1/2/3 inhibitors; Eg5 inhibitors; β-tubulin inhibitors; BMP inhibitors; HDAC inhibitors; Akt inhibitors; IGF1R inhibitors; p53 inhibitors; hdm2 inhibitors; STAT3 inhibitors; and VEGFR inhibitors to a subject determined to have a KDM4A dampening mutation in non-tumor cells.

In some embodiments, the KDM4A dampening mutation is a mutation that decreases KDM4A enzymatic activity; a mutation that increases the proportion or level of KDM4A that is located in the cytoplasm; or a mutation that increases the turnover rate of KDM4A polypeptide. In some embodiments, the KDM4A dampening mutation comprises a mutation of KDM4A selected from the group consisting of E23K; S28N; 187V; E113K; K123I; N128S; R152W; R218W; G225C; A235V; R239H; G278S; T289I; V319M; P326T; P348L; E368K; G376V; R400Q; E426K; V490M; R498H; D524V; E558Q; R597H; A662S; S713L; V743I; R765Q; G783FS; L803GS; R825C; R825H; V919M; L941F; S948T; V1003A; D1023Y; R1025C; and E1032K. In some embodiments, the KDM4A dampening mutation comprises a mutation selected from Table 7. In some embodiments, the KDM4A dampening mutation comprises a mutation of IDH resulting in increased 2-HG production. In some embodiments, the mutation is present in a cancer selected from the group consisting of chondrosarcoma; glioblastoma multiforme (GBM); and acute myeloid leukemia (AML). In some embodiments, the KDM4A dampening mutation comprises a mutation of SDH resulting in increased levels of succinate.

In some embodiments, the presence of the mutation is determined using an assay selected from the group consisting of hybridization; sequencing; exome capture; PCR; RFLP; high-throughput sequencing; and KDM4A immunochemical detection methods. In some embodiments, the mutation is present in the genomic DNA of the tumor cell. In some embodiments, the mutation is present in the mRNA transcripts of the tumor cell.

In one aspect, described herein is a method of treating cancer, the method comprising administering an inhibitor of KDM4A; and administering a chemotherapeutic agent selected from the group consisting of S-phase chemotherapeutics; mTOR inhibitors; protein synthesis inhibitors; Braf inhibitors; PI3K inhibitors; Cdk inhibitors; Aurora B inhibitors; FLT3 inhibitors; PLK1/2/3 inhibitors; Eg5 inhibitors; β-tubulin inhibitors; BMP inhibitors; HDAC inhibitors; Akt inhibitors; IGF1R inhibitors; p53 inhibitors; hdm2 inhibitors; STAT3 inhibitors; and VEGFR inhibitors. In some embodiments, the inhibitor of KDM4A is selected from the group consisting of an inhibitory nucleic acid; an aptamer; a miRNA; Suv39H1; HP1; increased oxygen levels; and succinate.

In some embodiments, the method can further comprise administering an ubiquitination inhibitor or proteasomal inhibitor. In some embodiments, the method can further comprise the step of generating a report based on the detection of a KDM4A dampening mutation.

In one aspect, described herein is a method of treating cancer, the method comprising administering an agonist of KDM4A to a subject determined to have a level of KDM4A gene expression which is higher than a reference level or determined to have KDM4A gene amplification.

In some embodiments, the cancer is selected from the group consisting of ovarian cancer; non-small cell lung cancer; multiple myeloma; breast cancer; pancreatic cancer; head and neck cancer; lung cancer; adenocarcinoma; lung adenocarcinoma; lung squamous cell carcinoma; renal cancer; stomach cancer; melanoma; colorectal cancer; AML; and uterine and endometrial cancer.

In one aspect, described herein is an assay for determining the likelihood of a subject experiencing a positive outcome following treatment for cancer, the assay comprising determining the level or mutational status of KDM4A; KDM4C; KDM4B; KDM4E; and/or KDM4D in a tumor cell sample obtained from the subject; wherein the subject has a decreased likelihood of experiencing a positive outcome following treatment for cancer if:
a. the subject is determined to have a deletion or decreased level of expression of KDM4C as compared to a reference level;
b. the subject is determined to have a deletion, amplification, or increased or decreased level of KDM4D, KDM4C, KDM4E, or KDM4B as compared to a reference level;
c. the subject is determined to have an amplification or increased level of expression of KDM4A as compared to a reference level;
d. the subject is determined to have a KDM4A dampening mutation;
e. the subject is determined to have a mutation of a KDM4 family member selected from any of Tables 2-6.

In some embodiments, the subject determined to have an amplification or increased level of expression of KDM4A as compared to a reference level is a subject having ovarian cancer. In some embodiments, the subject determined to have a KDM4A dampening mutation is a subject having non-small cell lung cancer.

In one aspect, described herein is a method of treating graft versus host disease, the method comprising administering an inhibitor of KDM4A; and administering an mTOR inhibitor. In some embodiments, the mTOR inhibitor is rapamycin. In some embodiments, the inhibitor of KDM4A is selected from the group consisting of an inhibitory nucleic acid; an aptamer; a miRNA; Suv39H1; HP1; increased oxygen levels; and succinate. In some embodiments, the method further comprises administering a ubiquitination inhibitor or proteasomal inhibitor.

### BRIEF DESCRIPTION OF THE DRAWINGS

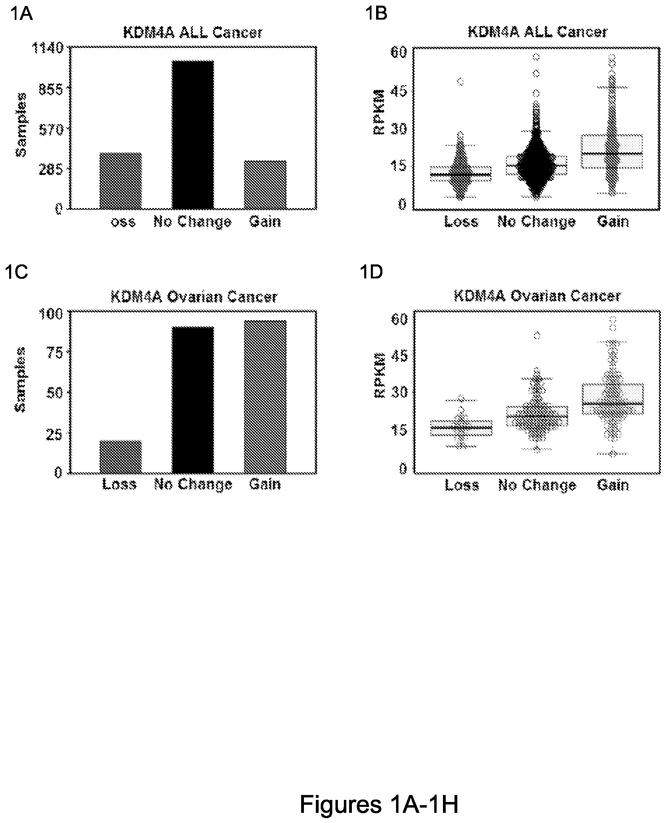

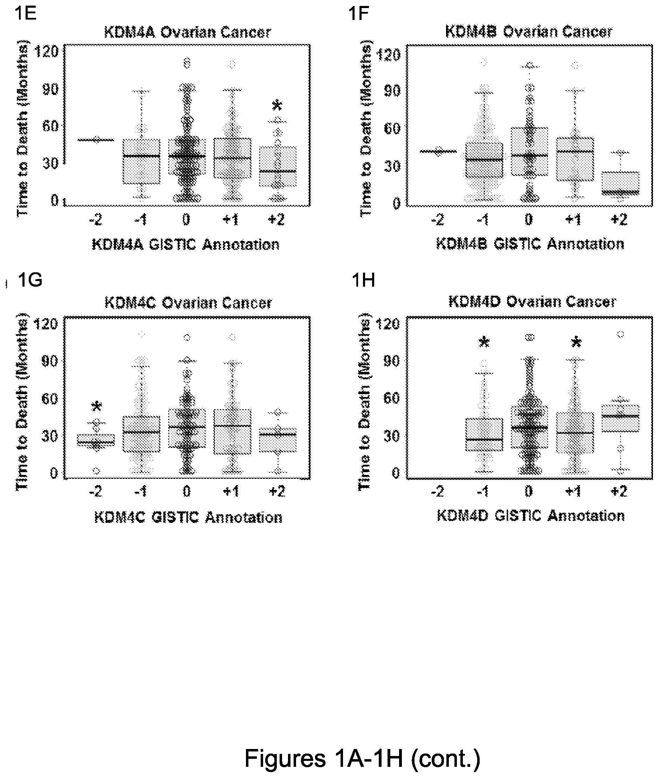

Figs. 1A-1H demonstrate that KDM4A is amplified and overexpressed in cancer and correlates with poor outcome in ovarian cancer. Fig. 1A depicts the distribution of gain (GISTIC annotation +1 or +2) or loss (GISTIC annotation -1 or -2) of copy of KDM4A in 1770 samples, which had both SNP array and RNAseq data across all cancers of the TCGA dataset. Fig. 1B demonstrates that the amplification of KDM4A correlates with increased expression of KDM4A in the TCGA data set. Analysis of expression from RNAseq data compared to copy number for KDM4A in 1770 samples from the TCGA data set (P=1.6x10-22 for Gain vs No change and P=9.2x10-32 for Loss vs No change by one-tailed Student's t-test). Fig. 1C demonstrates that KDM4A is frequently amplified in ovarian cancer (P=1.4x10-21 for Gain vs No change or Loss by Fisher's exact test). Distribution of gain or loss of copy of KDM4A in 204 ovarian cancer samples from the TCGA data set. Fig. 1D demonstrates that the amplification of KDM4A in ovarian cancer correlates with increased expression of KDM4A. Analysis of expression from RNAseq data compared to copy number for KDM4A in 204 ovarian cancer samples from the TCGA data set (P=23x10-6 for Gain vs No Change and P=5.3x10-5 for Loss vs No Change by one-tailed Student's t-test). Fig. 1E demonstrates that focal amplification of KDM4A in ovarian cancer correlates with poor outcome in 285 deceased ovarian cancer samples (P = 0.02 by one-tailed Student's t-test and 0.048 by one-tailed, Wilcoxon rank sum test for +2 vs 0). Fig. 1F demonstrates that copy number of KDM4B does not correlate with outcome in ovarian cancer. Fig. 1G demonstrates that deletion of KDM4C in ovarian cancer correlates with outcome (P = 0.014 for Loss vs None). Fig. 1H demonstrates that copy number loss and gain of KDM4D correlate with outcome in ovarian cancer (P = 0.018 for Gain vs None and 0.013 for Loss vs None by Student's t-test). * indicates significant difference from No Change samples (P < 0.05). RPKM denotes Reads per kilobase exon model per million reads of RNA seq data

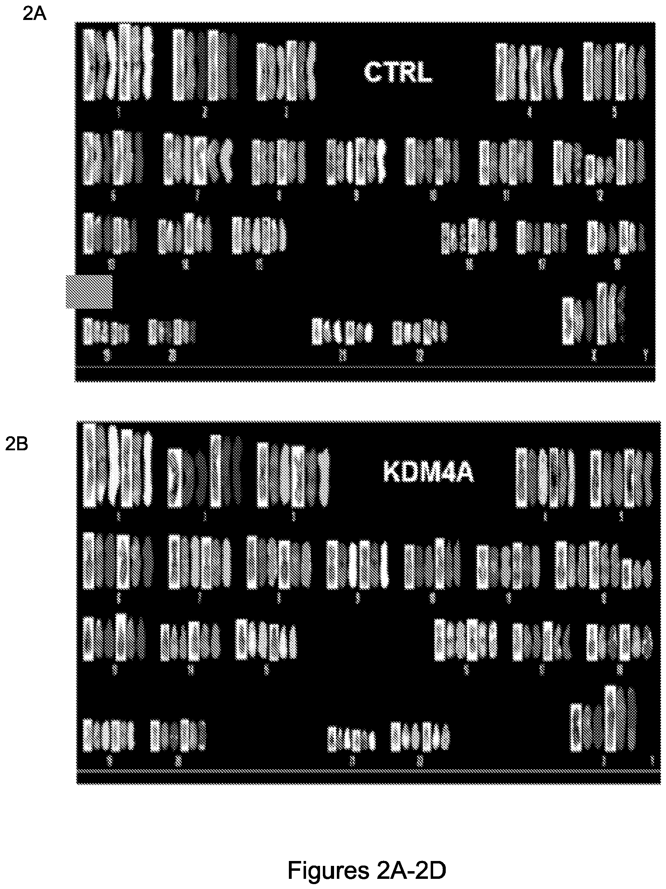

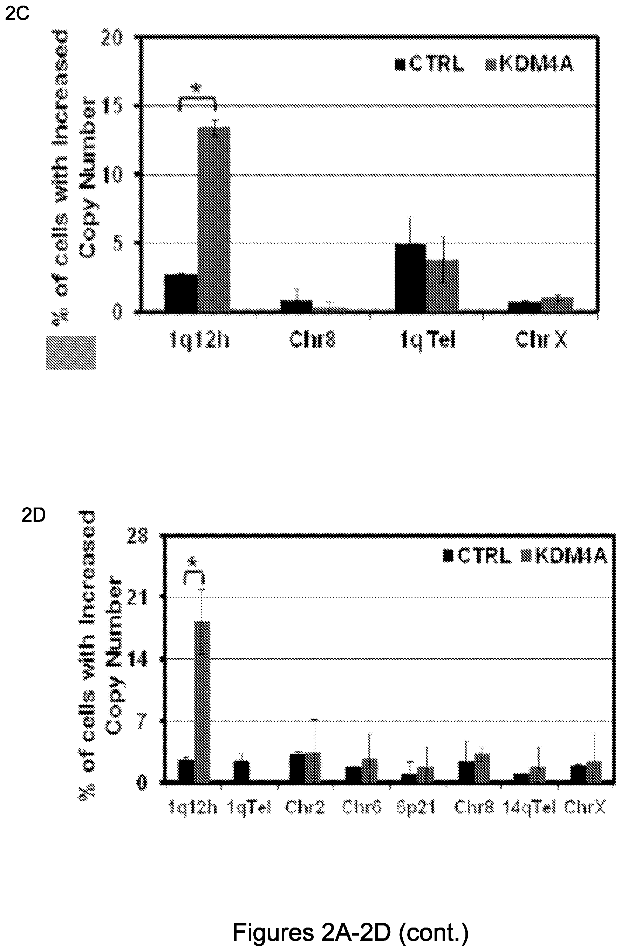

Figs. 2A-2D demonstrate that overexpression of KDM4A results in low level amplification of 1q12h, but does not induce widespread chromosome instability. Fig. 2A depicts an image of spectral karyotyping (SKY) analysis of RPE GFP-CTRL cells. Fig. 2B depicts the results of SKY analysis of RPE GFP-KDM4A cells. Fig. 2C depicts a graph of the quantification of FISH experiments in stable 293T GFP-CTRL and stable 293T GFP-KDM4A cells with the indicated FISH probes. Error bars represent the S.E.M. FISH was performed on stable RPE cells overexpressing GFP-CTRL (DAPI, 2L) for detection of 1q12h (Green, 2M) and a control probe at the Chr 8 centromere (Red, 2N). Fig. 2D depicts a graph of the quantitation of FISH experiments depicting altered copy number of 1q12h, but not other genomic regions. Arrowheads indicate each foci in FISH images. Error bars represent the S.E.M. * indicates significant difference from GFP-CTRL (P<0.05) by two-tailed students t-test. Scale bars represent 2µm.

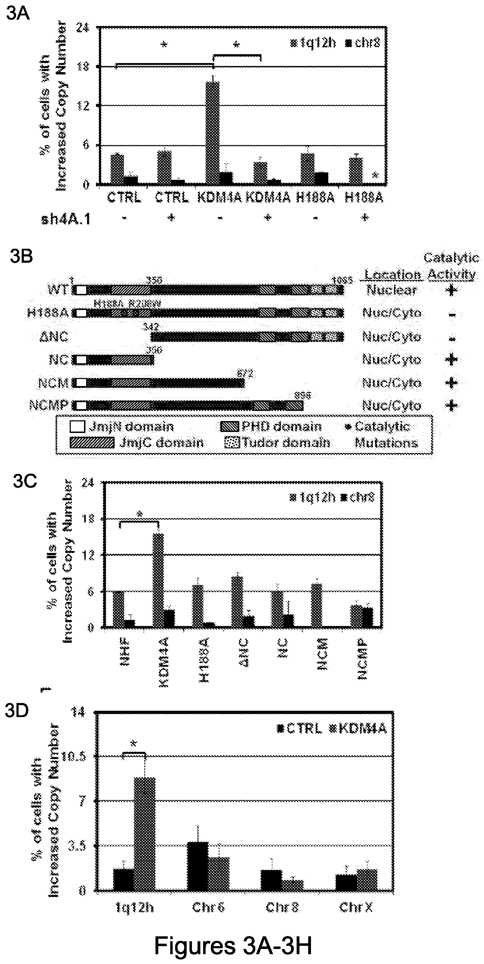

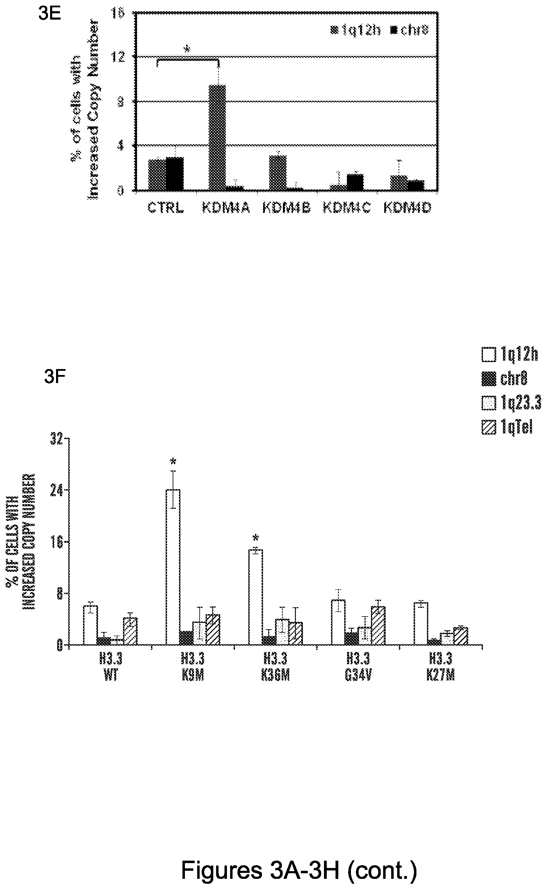

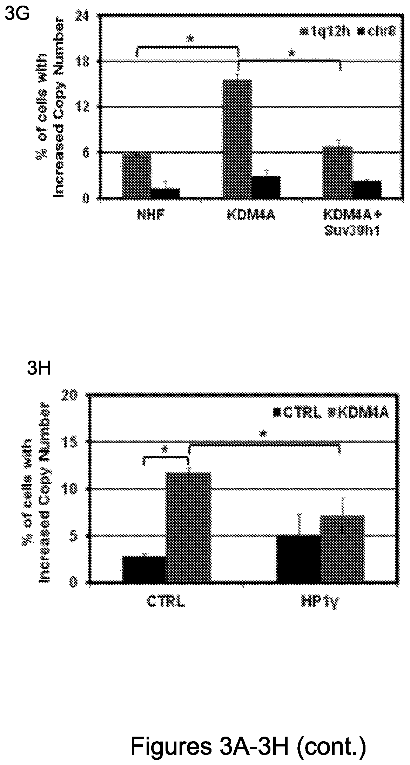

Figs. 3A-3H demonstrate that increased copy number of 1q12h can be induced transiently, is dependent on catalytically active KDM4A, and can be abrogated by co-expression of Suv39h1 and HP1γ. Fig. 3A depicts the quantification of FISH experiments in 293T cells overexpressing CTRL, KDM4A or catalytically inactive KDM4A (H188A) with and without depletion of endogenous KDM4A (sh4A.1) with the indicated FISH probes. Fig. 3B depicts a schematic of NHF-tagged KDM4A constructs used in 3C. Subcellular localization ("Location") was assessed by immunofluorescence (IF) and the indicated compartment represents the primary localization of greater than 80% of assayed cells. Catalytic activity was assessed by IF with H3K36me3 in transfected cells. "+" indicates strong reduction in total nuclear H3K36me3 staining relative to adjacent untransfected cells. Fig. 3C depicts the quantification of FISH experiments in RPE cells transfected for 24 hours with the indicated NHF-KDM4A constructs and corresponding FISH probes. Fig. 3D depicts a graph of the quantification of FISH experiments in RPE cells transfected for 24 hours with GFP-CTRL or GFP-KDM4A with the indicated FISH probes. Fig. 3E is a graph demonstrating that increased copy number of 1q12h is specific to KDM4A overexpression and not other KDM4 family members. Quantification of FISH experiments in RPE cells transfected for 24 hours with GFP-CTRL, GFP-KDM4A, GFP-KDM4B, GFP-KDM4C, or GFP-KDM4D and analyzed for copy number of 1q12h or Chr 8 centromere by FISH. Fig. 3F demonstrates that expression of H3.3 histone variants for H3K9 or H3K36 promotes increased copy of 1q12h. Quantification of FISH experiments in RPE cells 24 hours post infection with the indicated H3.3 histone variants analyzed for the indicated FISH probes. Fig. 3G demonstrates that co-expression of Suv39h1 abrogates KDM4A-dependent formation of extra copies of 1q12h. RPE cells were transiently transfected with NHF-CTRL, NHF-KDM4A, or NHF-KDM4A with Halo-Suv39h1 for 24 hours and analyzed for changes in 1q12h by FISH. Fig. 3H is a graph demonstrating that co-expression of HP1γ abrogates formation of extra copies of 1q12h. RPE cells were transiently transfected with GFP-CTRL or GFP-KDM4A with addition of RFP-Ctrl or RFP-HP1γ for 24 hours and analyzed for changes in 1q12h by FISH. Error bars represent the S.E.M. * indicates significant difference from GFP-CTRL or NHF-CTRL (P<0.05) by two-tailed students t-test.

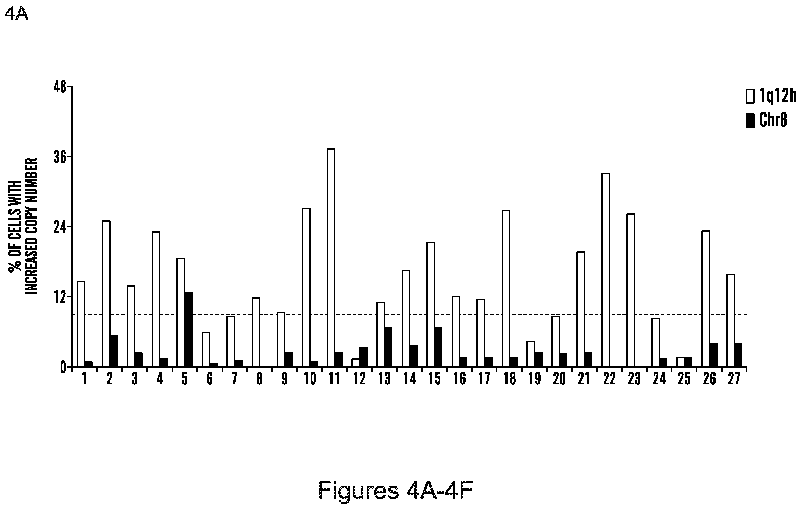

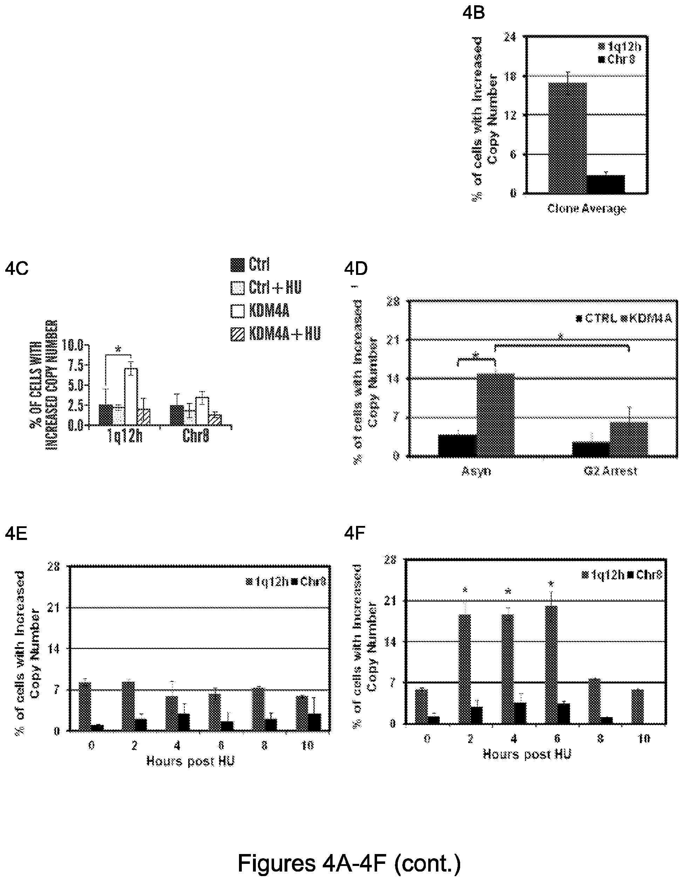

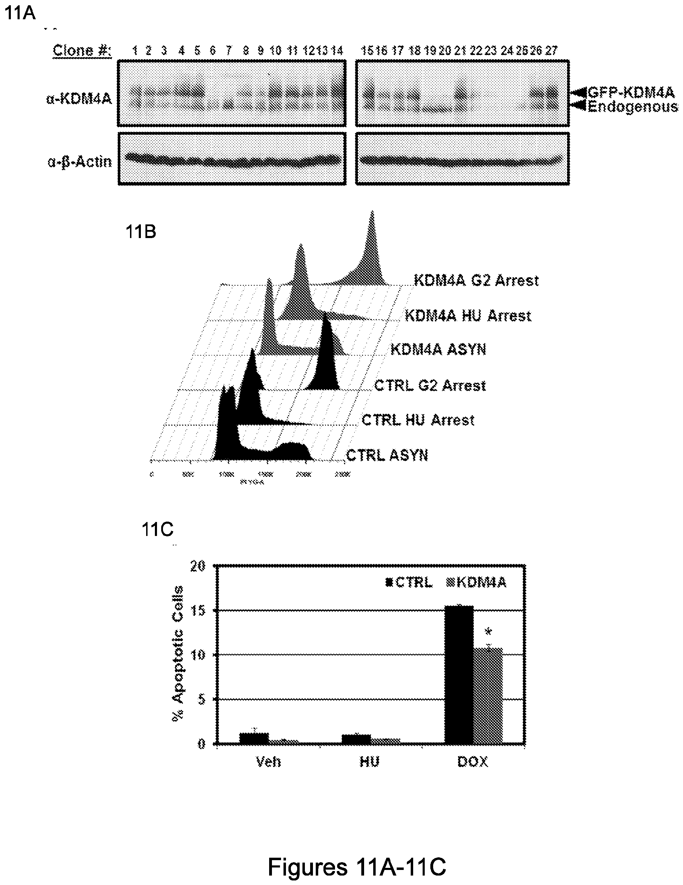

Figs. 4A-4F demonstrate that increased copy number of 1q12h is not stably inherited and requires replication each cell cycle for maintenance. Fig. 4A demonstrates that increased 1q12h copy number in GFP-KDM4A RPE cells is not inherited. Single cell clones were isolated from stable GFP-KDM4A RPE cells and analyzed by FISH for 1q12h and Chr 8 centromere. Clones with 1q12h copy gain above the dashed black line all overexpress GFP-KDM4A (Fig. 11A). Fig. 4B is a graph of average copy gain for 27 single cell clones from 4A. Fig. 4C demonstrates that increased copy number of 1q12h requires replication. Stable GFP-CTRL and GFP-KDM4A cells were arrested in hydroxyurea (HU) for 20 hours and analyzed for copy number of 1q12h or Chr 8 centromere by FISH. Fig. 4D demonsrates that increased copy number of 1q12 is lost by the end of G2. Stable GFP-CTRL and GFP-KDM4A cells were arrested in G2 using the CDK1 inhibitor RO-3306 (G2 Arrest) for 20 hours and analyzed for copy number of 1q12h or Chr 8 centromere by FISH. Figs. 4E-4F depict graphs that demonsrate that ddditional copies of 1q12h are generated in S-phase. Stable GFP-CTRL (Fig. 4E) and GFP-KDM4A (Fig. 4F) cells were arrested in hydroxyurea (HU) for 20 hours and released for the time indicated prior to analysis for copy number of 1q12h or Chr 8 centromere by FISH. Error bars represent the S.E.M. * indicates significant difference from GFP-CTRL (P<0.05) by two-tailed students t-test. For the HU release, P values are based on the comparison of KDM4A to CTRL at each individual time point (Figs. 4F and 4E, respectively).

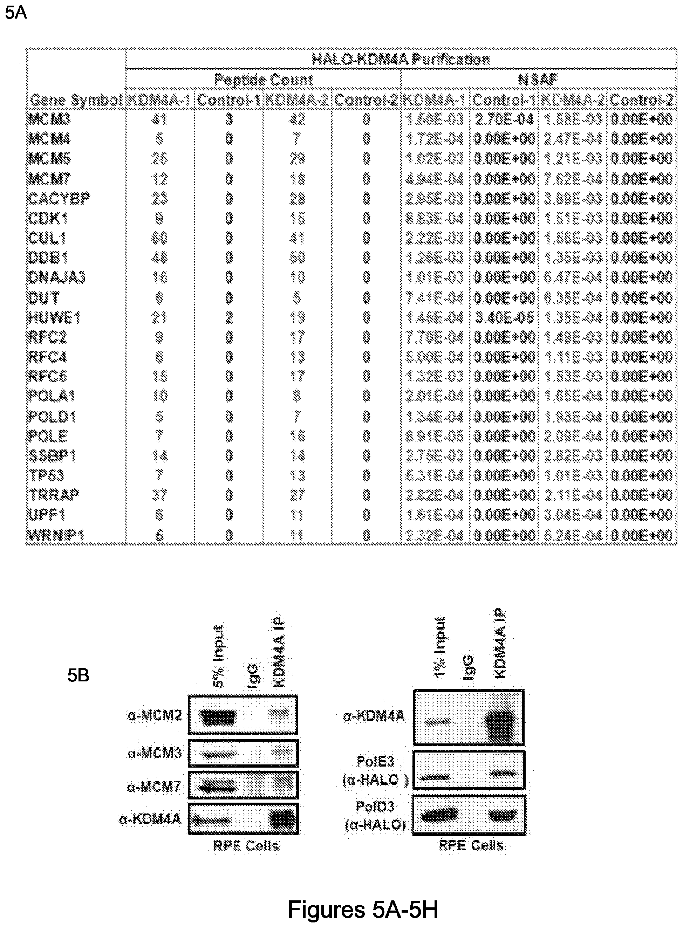

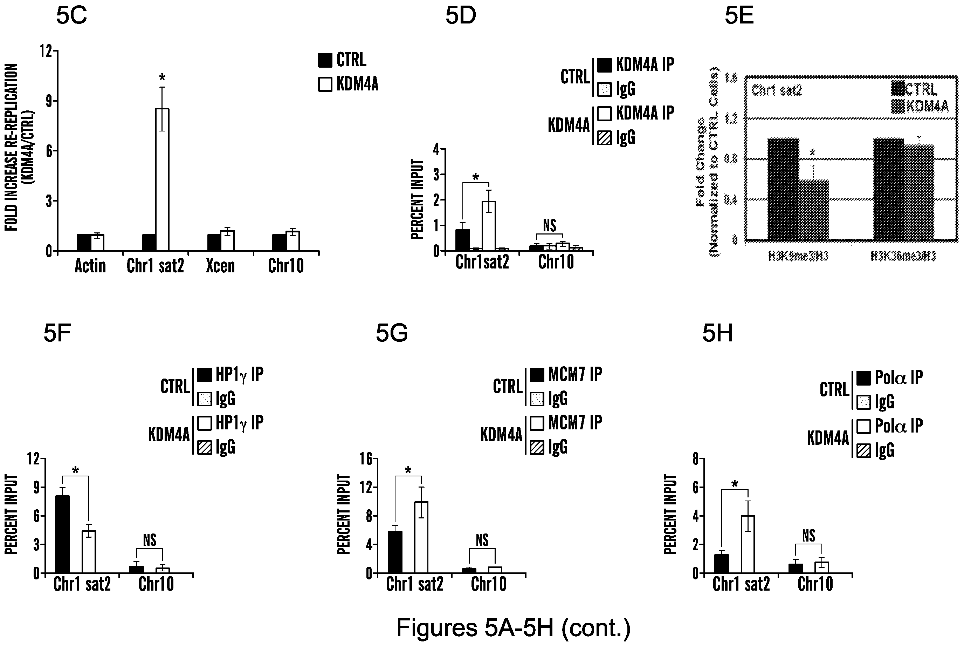

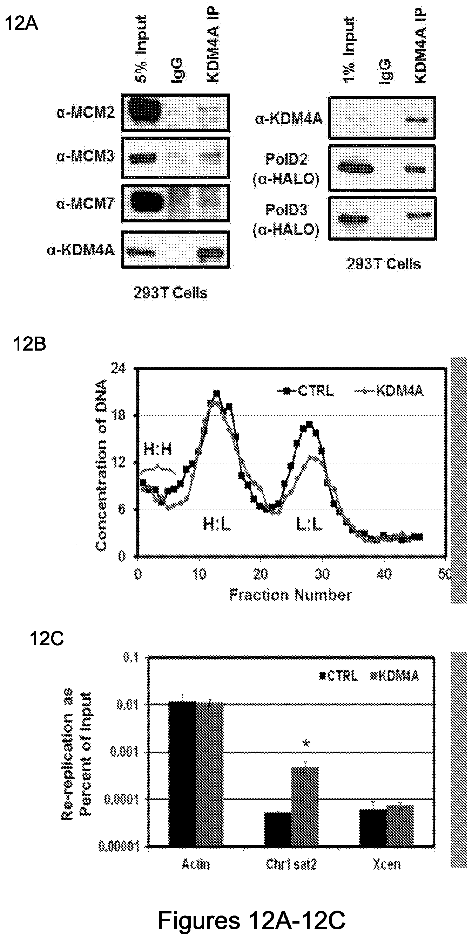

Figs. 5A-5H demonstrate that KDM4A interacts with replication machinery and overexpression of KDM4A promotes re-replication. Fig. 5A depicts a table of mass spectrometry analysis of KDM4A interacting proteins related to replication. The total number of peptides as well as the NSAF values from replicate purifications is indicated for each of the KDM4A-associated proteins with a known role in replication. Fig. 5B depicts images of Western blots of co-immunoprecipitation of endogenous KDM4A and the indicated licensing and replication machinery in RPE Cells. Fig. 5C depicts a graph of overexpression of KDM4A in RPE cells leads to re-replication of Chrl sat2. Graph depicts qPCR analysis of the indicated regions from the heavy:heavy fraction of a CsCl gradient (Fig. 12B). Fig. 5D demonstrates that KDM4A is enriched at Chrl sat2 (1q12) but not a negative region chromosome 10 (Chr10) in KDM4A-overexpressing RPE cells. KDM4A ChIP was conducted in HU arrested CTRL or GFP-KDM4A overexpressing RPE cells. Fig. 5E demonstrates that H3K9me3 but not H3K36me3 decreases at Chrl sat2. H3K9me3 or H3K36me3 ChIP was conducted in HU arrested CTRL or GFP-KDM4A overexpressing RPE cells. Fig. 5F demonstrates that HP1γ enrichment decreases at Chrl sat2 (1q12), but not at chromosome 10 (Chr10) in KDM4A-overexpressing cells. HP1γ ChIP was conducted in HU arrested GFP-CTRL or GFP-KDM4A overexpressing RPE cells. Fig. 5G depicts MCM7 enrichment at Chrl sat2 (1q12), but not at chromosome 10 (Chr10) in KDM4A-overexpressing cells. MCM7 ChIP was conducted in HU arrested GFP-CTRL or GFP-KDM4A overexpressing RPE cells. Fig. 5H depicts DNA polymerase α (Pol α) enrichment at Chrl sat2 (1q12), but not at chromosome 10 (Chr10) in KDM4A-overexpressing cells. Pol α ChIP was conducted in HU arrested GFP-CTRL or GFP-KDM4A overexpressing RPE cells. Error bars represent the S.E.M. * indicates significant difference from GFP-CTRL (P<0.05) by two-tailed students t-test.

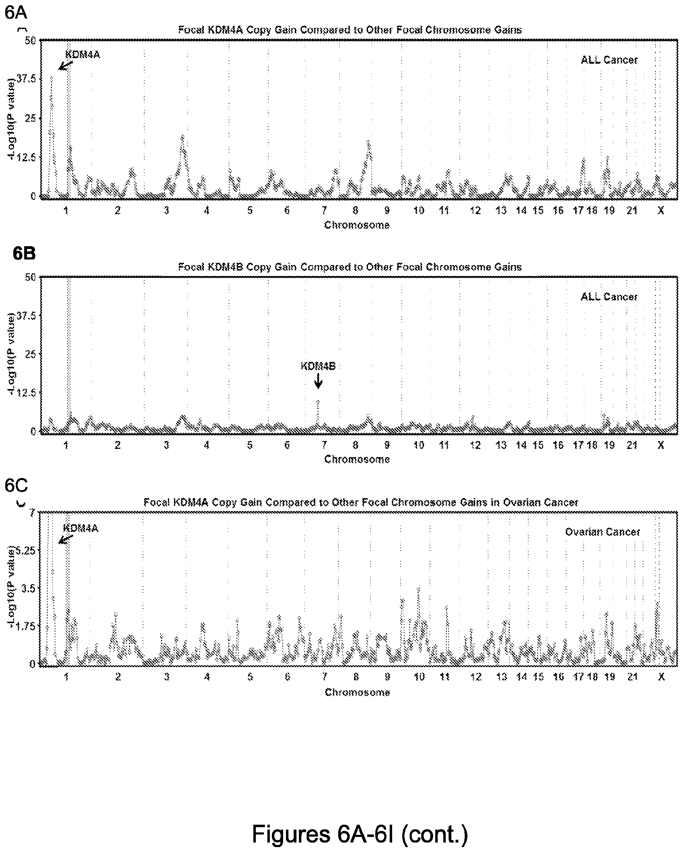

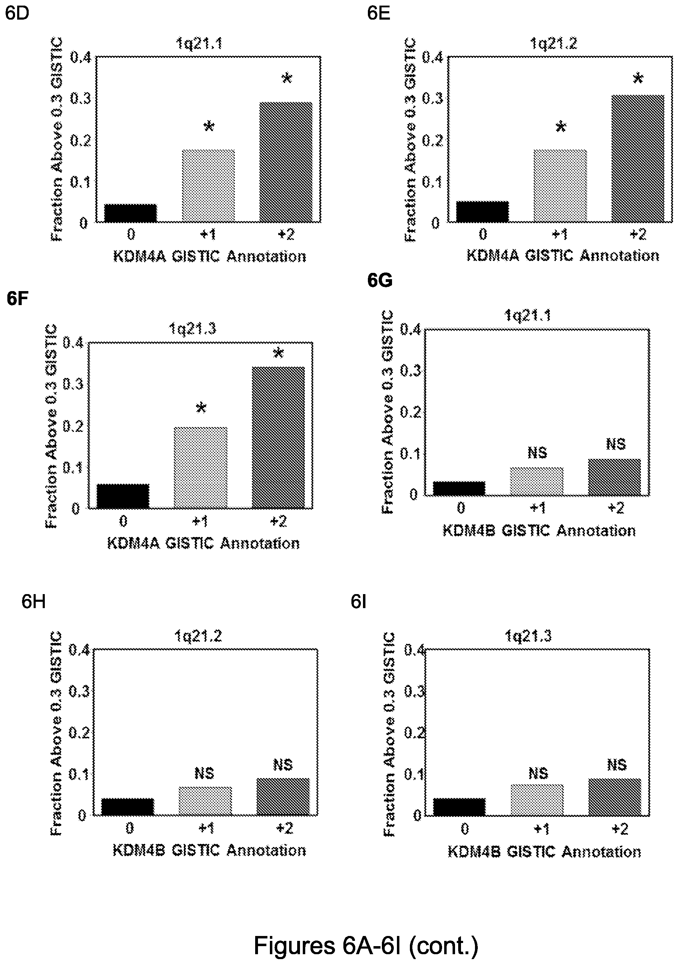

Figs. 6A-6I demonstrate the identification of cytogenetic bands co-amplified with KDM4A in cancer. Fig. 6A depicts a graph demonstrating focal amplification of specific cytogenetic bands correlates with amplification of KDM4A in 4,420 samples across all cancers from TCGA data set. Graph depicts the -log(10) P-value of the one-tailed Student's t-test for correlation of the copy number of each cytogenetic band with the copy number of KDM4A. The line represents the locus of KDM4A and its gene-specific significance is P=1.5x10-37. Fig. 6B depicts a graph of focal amplification of specific cytogenetic bands with amplification of KDM4B across all cancers from TCGA data set. Graph depicts the -log(10) P-value for correlation of the copy number of each cytogenetic band with the copy number of KDM4B. The blue dot represents the gene-specific significance of KDM4B. Fig. 6C depicts a plot of focal amplification of specific cytogenetic bands correlates with amplification of KDM4A in 547 ovarian cancer samples. The line represents the locus of KDM4A and its gene-specific significance is P=1.1x10-19. For each co-amplification plot, shaded regions indicate 1p11.2 through 1q21.3 and dashed lines indicates Xp11.2 through Xq13.2. Fig. 6D demonstrates that increased copy of KDM4A is associated with increased mean focal copy of 1q21.1. The fraction of samples possessing a mean focal copy number exceeding 0.3 in 1q21.1 stratified by the KDM4A copy number status; unaltered (0), Amplified (+1), or focally Amplified (+2). P=2x10-9 for +2 vs 0 and P= 2.04x10-25 for +1 vs 0 by Fisher's exact test. Fig. 6E demonstrates that increased copy of KDM4A is associated with increased copy of 1q21.2 (P = 1.9x10-9 for +2 vs 0 and P=6.28x10-22 for +1 vs 0). Fig. 6F demonstrates that increased copy of KDM4A is associated with increased copy of 1q21.3 (P=1.02x10-10 for +2 vs 0 and 3x10-24 for +1 vs 0). Fig. 6G demonstrates that increased copy number of KDM4B is not associated with increased copy of 1q21.1 (P = 0.18 for +2 vs 0). Fig. 6H demonstrates that increased copy number of KDM4B is not associated with increased copy of 1q21.2 (P=0.22 for +2 vs 0). Fig. 6I demonstrates that increased copy number of KDM4B is not associated with increased copy number of 1q21.3 (P=0.24 for +2 vs 0). * indicates significant difference of +1 or +2 vs 0 by Fisher's exact test. NS indicates not significantly different from 0.

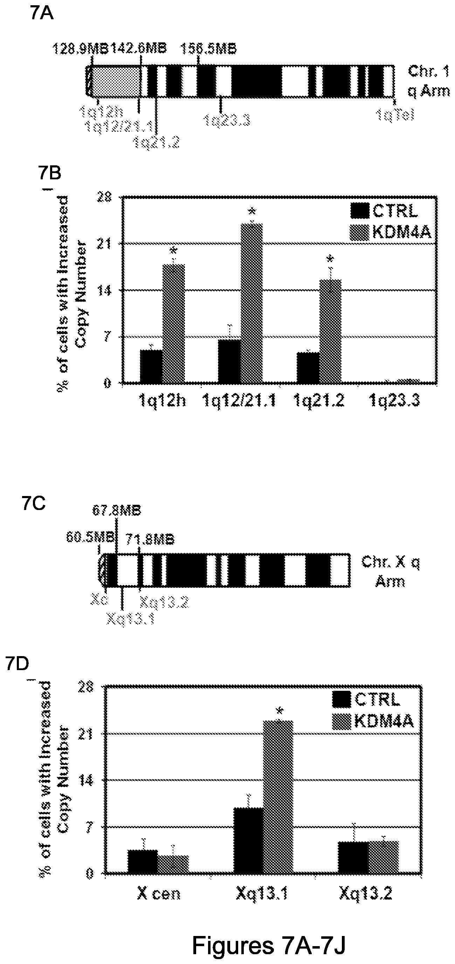

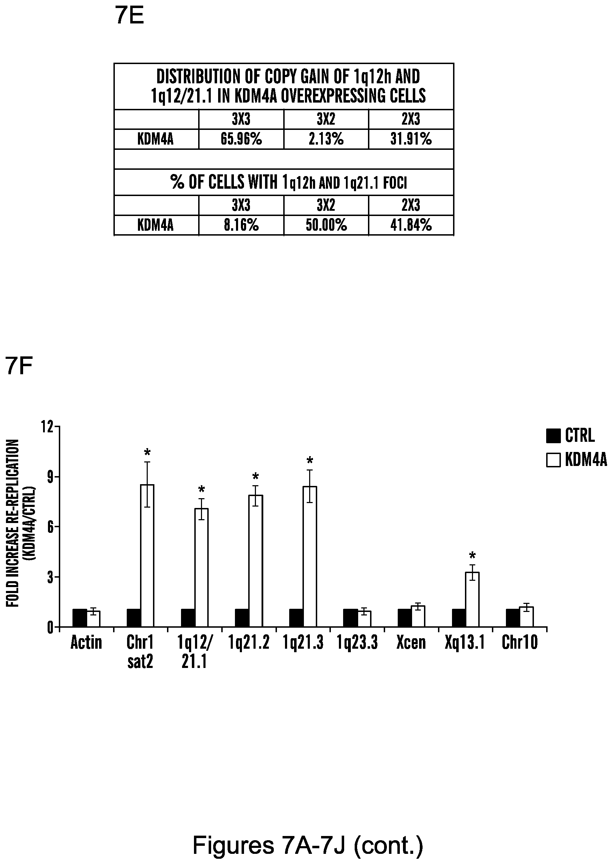

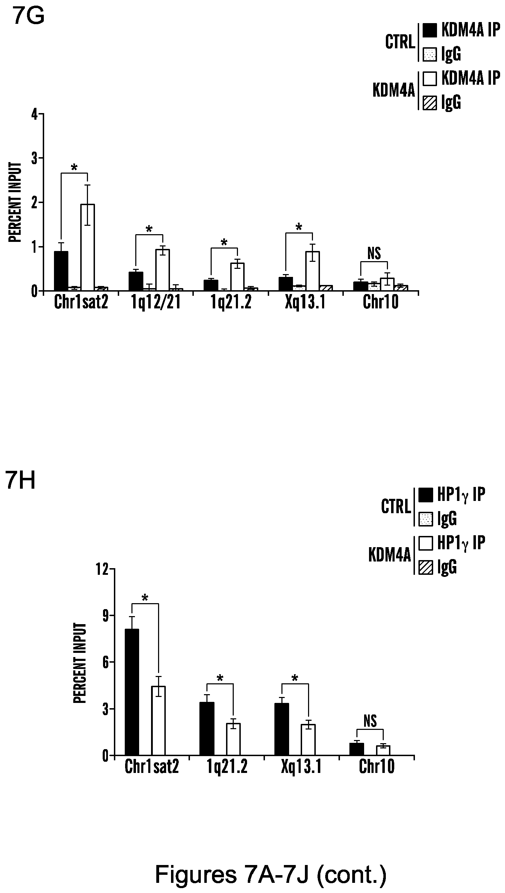

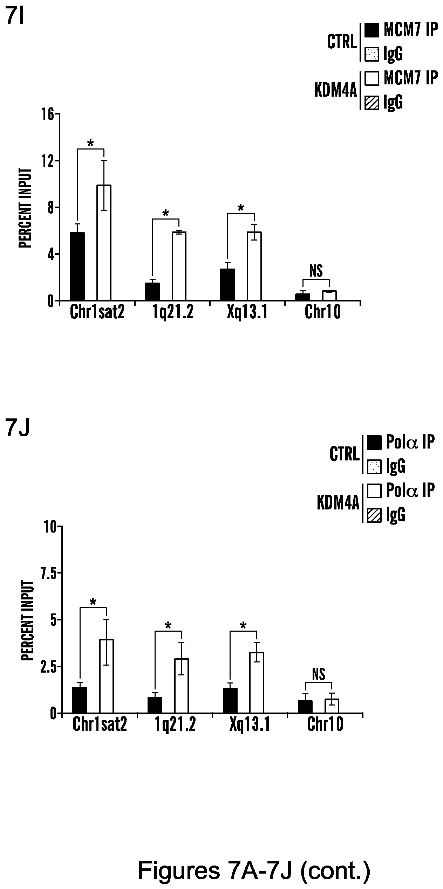

Figs. 7A-7J demonstrate that overexpression of KDM4A leads to increased copy number and re-replication of regions correlated with KDM4A amplification in cancer. Fig. 7A is a chromosome arm schematic depicting location of FISH probes used on chromosome 1. Fig. 7B demonstrates that KDM4A overexpression increased copy number of 1q12h, 1q12/21.1 and 1q21.2 but not 1q23.3. Fig. 7C is a chromosome arm schematic depicting location of FISH probes used on chromosome X. Fig. 7D demonstrates that KDM4A overexpression increases copy number of Xq13.1 but not X cen or Xq13.2 in RPE cells Fig. 7E depicts a table summarizing co-amplification of 1q12h, 1q12/21.1 and 1q21.2. Data are presented as % of amplified cells having 2 or 3 copies of the indicated FISH probes. Fig. 7F depicts the te-replication of chromosomal domains in 1q12 (Chr1 sat2) 1q12/21, 1q21.2 (BCL9), 1q21.3, and Xq13.1, but not 1q23.3, or Xcen in KDM4A overexpressing cells. CsCl density gradient centrifugation was used to isolate heavy-heavy DNA (Fig. 12B). Purified DNA was analyzed by qPCR and compared to input DNA to determine if regions were re-replicated. Fig. 7G demonstrates that KDM4A is enriched at Chrl sat2 (1q12), 1q12/21, 1q21.2 (BCL9), and Xq13.1, but not 1q23.3 in KDM4A-overexpressing RPE cells. KDM4A ChIP in HU arrested GFP-CTRL or GFP-KDM4A overexpressing RPE cells. Fig. 7H demonstrats that HPly enrichment decreases at Chrl sat2 (1q12), 1q21.2 (BCL9), and Xq13.1, but not Chr10 in KDM4A-overexpressing cells. HPly ChIP in GFP-CTRL or GFP-KDM4A overexpressing RPE cells following 1 hour release from HU arrest. Fig. 7I demonstrates that MCM7 enrichment decreases at Chrl sat2 (1q12), 1q21.2 (BCL9), and Xq13.1, but not ChrlO in KDM4A-overexpressing cells. MCM7 ChIP was conducted in HU arrested GFP-CTRL or GFP-KDM4A overexpressing RPE cells. Fig. 7J demonstrates that DNA polymerase α (Pol α) enrichment decreases at Chrl sat2 (1q12), 1q21.2 (BCL9), and Xq13.1, but not ChrlO in KDM4A-overexpressing cells. Pol α ChIP was conducted in HU arrested GFP-CTRL or GFP-KDM4A overexpressing RPE cells. Error bars represent the S.E.M. * indicates significant difference from GFP-CTRL (P<0.05) by two-tailed students t-test. For Re-replication and ChIP experiments (V, W, Y, Z) Chrl sat2, and Chr10 are the data presented in Figs. 5A=5H for reference. Scale bars represent 2pm.

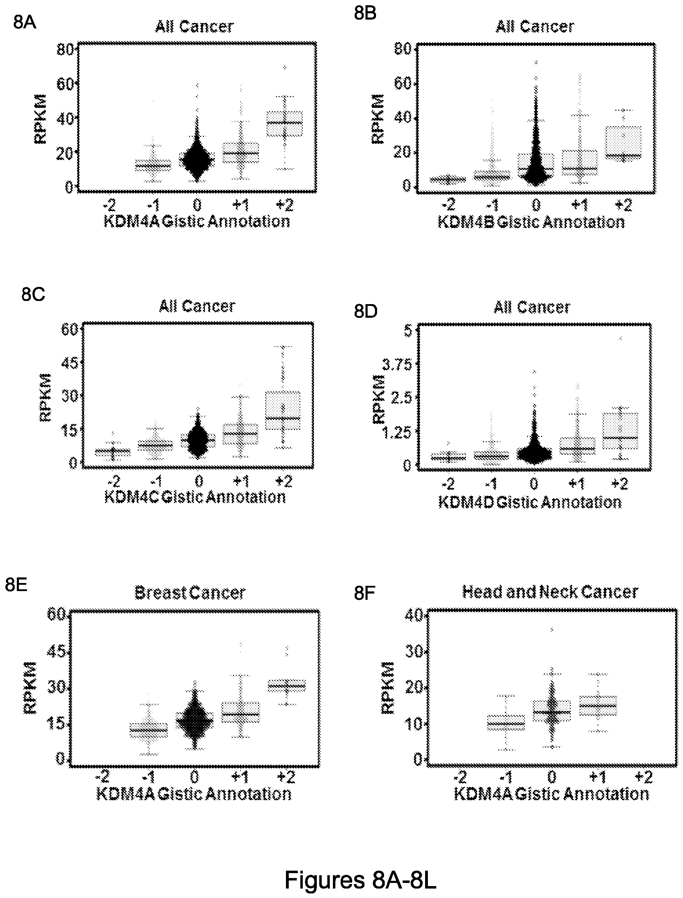

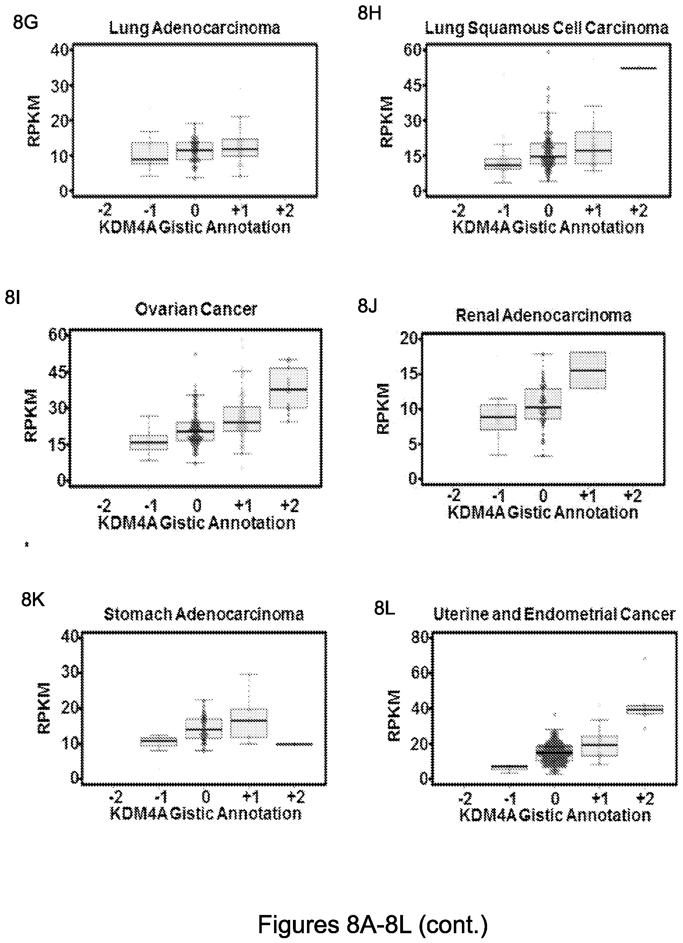

Figs. 8A-8L demonstrate the amplification and overexpression of KDM4A in cancer. Fig. 8A depicts analysis of RNAseq data from all cancer types indicating expression level of KDM4A relative to KDM4A copy number binned by GISTIC annotation. Fig. 8B depicts analysis of RNAseq data from all cancer types indicating expression level of KDM4B relative to KDM4B copy number binned by GISTIC annotation. Fig. 8C depicts analysis of RNAseq data from all cancer types indicating expression level of KDM4C relative to KDM4C copy number binned by GISTIC annotation. Fig. 8D depicts analysis of RNAseq data from all cancer types indicating expression level of KDM4D relative to KDM4D copy number binned by GISTIC annotation. Fig. 8E depicts analysis of RNAseq data from Breast Cancer indicating expression level of KDM4A relative to KDM4A copy number binned by GISTIC annotation. Fig. 8F depicts analysis of RNAseq data from Head and Neck squamous cell carcinoma indicating expression level of KDM4A relative to KDM4A copy number binned by GISTIC annotation. Fig. 8G depicts analysis of RNAseq data from lung adenocarcinoma indicating expression level of KDM4A relative to KDM4A copy number binned by GISTIC annotation. Fig. 8H depicts analysis of RNAseq data from lung squamous cell carcinoma indicating expression level of KDM4A relative to KDM4A copy number binned by GISTIC annotation. Fig. 8I depicts analysis of RNAseq data from ovarian cancer indicating expression level of KDM4A relative to KDM4A copy number binned by GISTIC annotation. Fig. 8J depicts analysis of RNAseq data from renal adenocarcinoma indicating expression level of KDM4A relative to KDM4A copy number binned by GISTIC annotation. Fig. 8K depicts analysis of RNAseq data from stomach adenocarcinoma indicating expression level of KDM4A relative to KDM4A copy number binned by GISTIC annotation. Fig. 8L depicts analysis of RNAseq data from uterine and endometrial cancer indicating expression level of KDM4A relative to KDM4A copy number binned by GISTIC annotation.

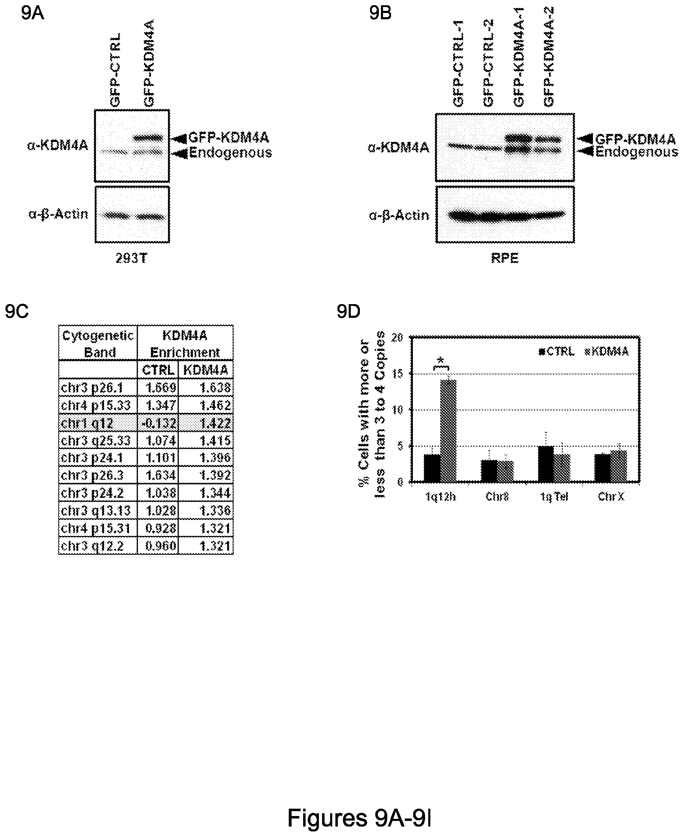

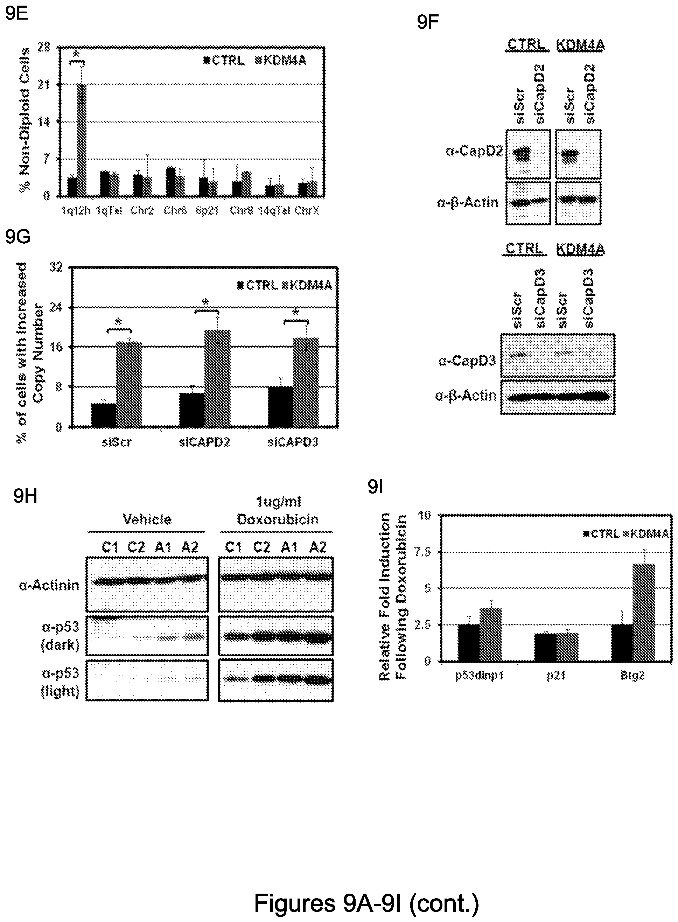

Figs. 9A-9I demonstrate that enrichment of KDM4A in a specific cytogenetic band that has altered copy number. Fig. 9A depicts an image of Western blot depicting expression of GFP-KDM4A in a stable 293T cell line. Fig. 9B depicts an image of Western blot depicting expression of GFP-KDM4A in two different polyclonal stable RPE cells. Fig. 9C is a table depicting increased binding of KDM4A in chr1 q12 cytogenetic band in 293T cells overexpressing KDM4A. Enrichment in ChIP-chip data is depicted as the Z-score for the average KDM4A/Input level for each probe in the cytogenetic band. Fig. 9D depicts a graph of FISH analysis of 293T cells stably overexpressing GFP-CTRL or GFP-KDM4A. Data are presented as percent of cells with foci number different from the mean (not 3 or 4 foci in 293T cells). Fig. 9E depicts a graph of FISH analysis of RPE cells stably overexpressing GFP-CTRL or GFP-KDM4A. Data are presented as percent of cells with foci number different from the mean (not 2 foci in RPE cells). Fig. 9F depicts an image of Western blot demonstrating siRNA depletion of CapD2 and CapD3 in CTRL and KDM4A cells. GFP-CTRL and GFP-KDM4A panels for CapD2 are the same exposure from a different, non-adjacent section of the same western blot. Fig. 9G depicts FISH analysis for 1q12h in RPE cells stably overexpressing GFP-CTRL or GFP-KDM4A treated with siRNA against condensin 1 (CAPD2) and condensing 2 (CAPD3). Fig. 9H depicts Western analysis of p53 induction following DNA damage by Doxorubicin (1uM for 16 hrs) in GFP-CTRL (C1 and C2) or GFP-KDM4A cells (A1 and A2). Fig. 9I depicts a graph of induction of p53 target genes analyzed by quantitative PCR after reverse transcription from doxorubicin damaged GFP-CTRL or GFP-KDM4A cells. Data are presented as fold induction relative to its own uninduced stable cell and normalized to expression of β-actin. Error bars represent the S.E.M. * indicates P<0.05 using two-tailed students T-test.

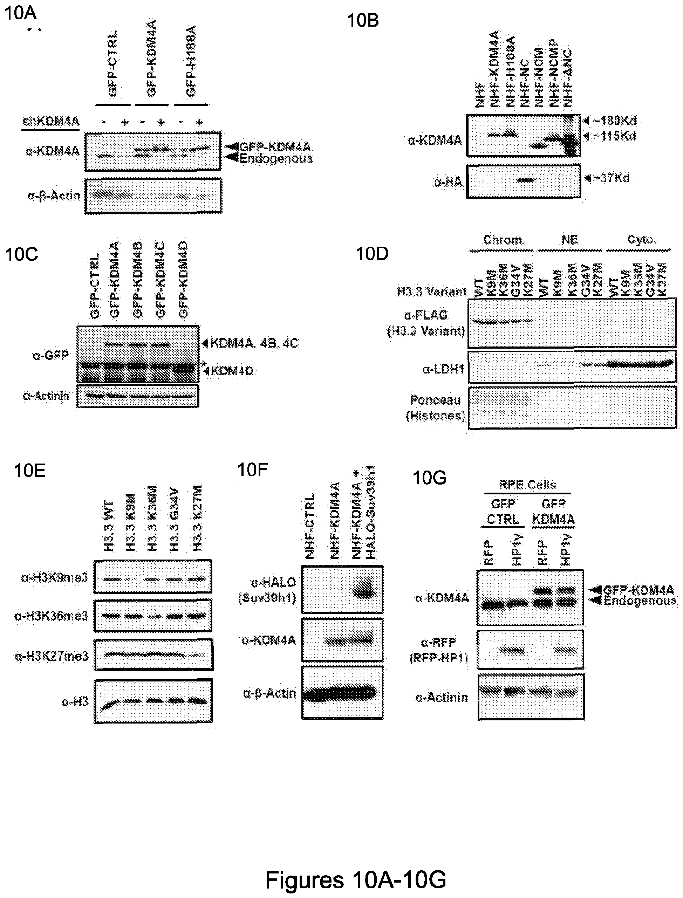

Figs. 10A-10G relate to Figs. 3A-3H. Fig. 10A depicts the expression of KDM4A following depletion and overexpression of GFP-CTRL or GFP-KDM4A in 293T cells. Fig. 10B depicts the expression of NHF-tagged KDM4A deletion constructs in RPE cells. Since the NC constructs lacks the KDM4A antibody epitope the HA western blot is shown for this fragment. Fig. 10C demonstrates the expression of GFP-KDM4A, GFP-KDM4B, GFP-KDM4C, and GFP-KDM4D in transiently transfected RPE cells. * indicates a non-specific band. Fig. 10D depicts the expression and incorporation into chromatin of FLAG-tagged H3.3 variants. Fig. 10E depicts the expression of HA-FLAG-tagged H3.3 K-to-M variants reduced the corresponding tri-methylation. Fig. 10F depicts the expression of KDM4A and Halo-Suv39h1 in transiently transfected RPE cells. Fig. 10G depicts the expression of KDM4A and RFP-HP1γ in transiently transfected RPE cells. Panels were assembled from the same exposure from non-adjacent lanes on different sections of the same blot.
Figs. 11A-11C are related to Figs. 4A-4F. Fig. 11A depicts the expression of KDM4A and GFP-KDM4A in RPE stable clones. Fig. 11B depicts the results of FACS analysis demonstrating HU and G2 arrest of CTRL and KDM4A RPE stable cells. Total DNA content as measured using propidium iodide is depicted on the x-axis. Fig. 11C demonstreates that KDM4A overexpressing RPE cells are not more apoptotic following treatment with hydroxyurea (HU) as measured by percent Annexin V positive cells. However, 12 hours of doxorubicin treatment induces apoptosis. * indicates P<0.05 using two-tailed students T-test.
Figs. 12A-12C are related to Figs. 5A-5H and demonstrate that KDM4A associates with replication machinery and promotes re-replication of KDM4A target regions. Fig. 12A depicts images of Western blots of co-immunoprecipitation of endogenous KDM4A with the indicated licensing and replication machinery in 293T Cells. Fig. 12B depicts a representative CsCl density gradient curve used for determining re-replication of KDM4A target regions. Data are presented as the DNA concentration of the indicated fraction (X-axis) taken from the bottom of the CsCl gradient. The positions of the light:light (L:L) and heavy:light (H:L) peaks and the heavy:heavy (H:H) region taken for analysis are indicated. Fig. 12C depicts a graph depicting the KDM4A-dependent Chrl sat2 re-replication plotted as a percent of input DNA loaded onto the CsCl gradient.

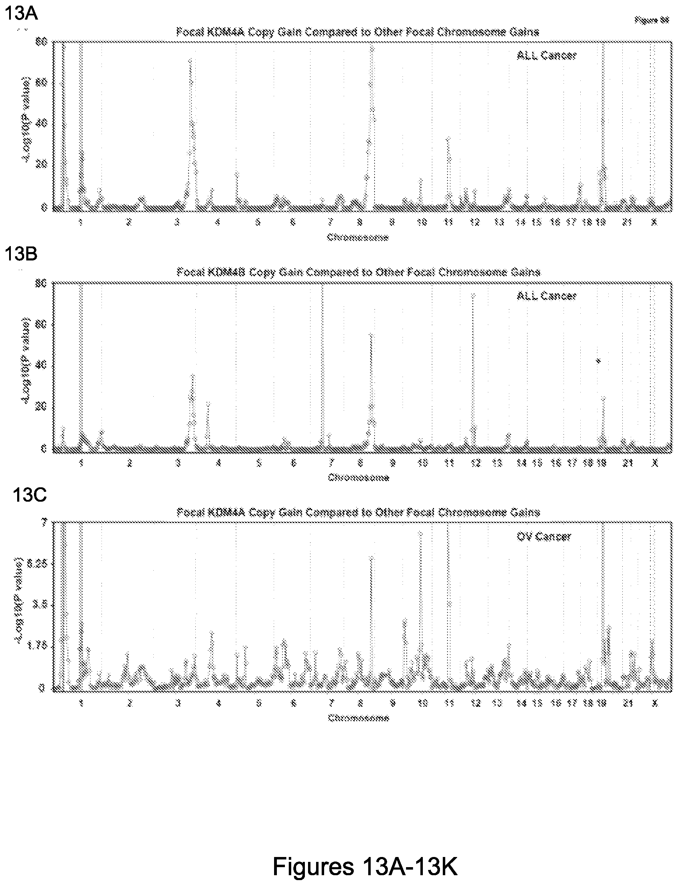

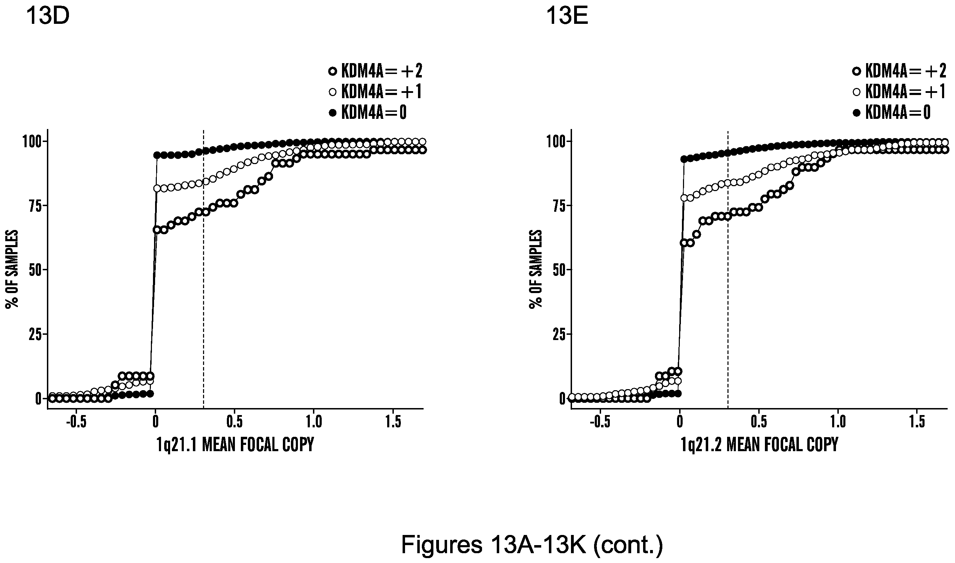

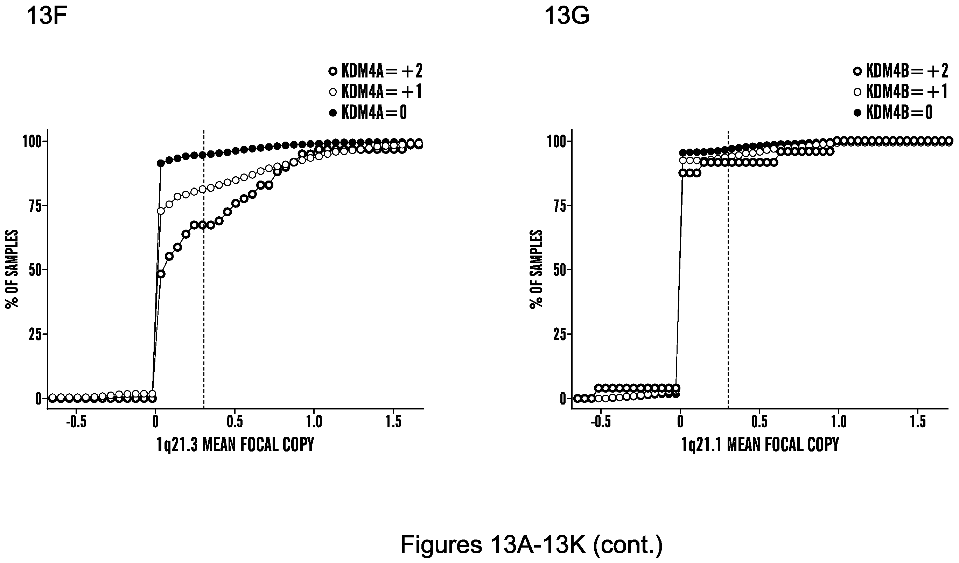

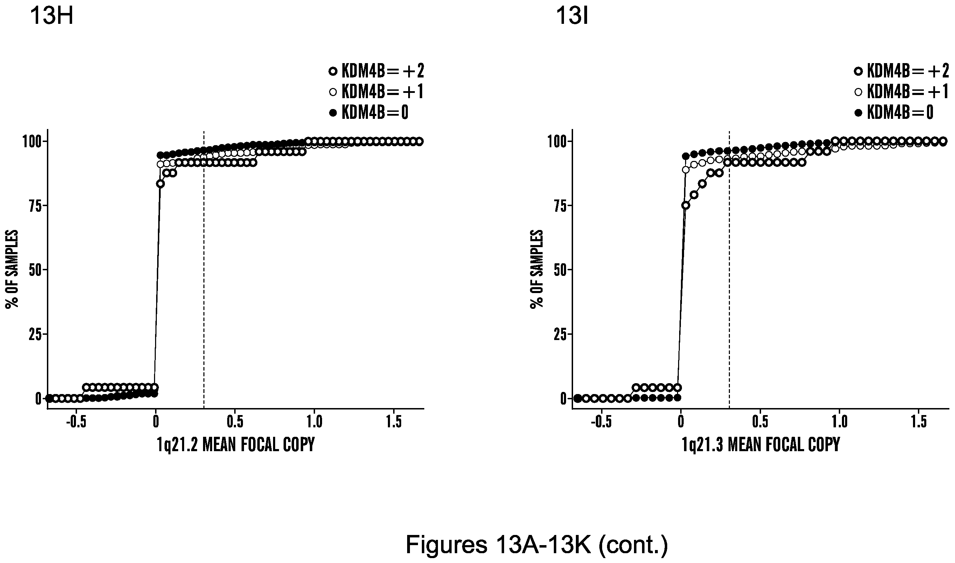

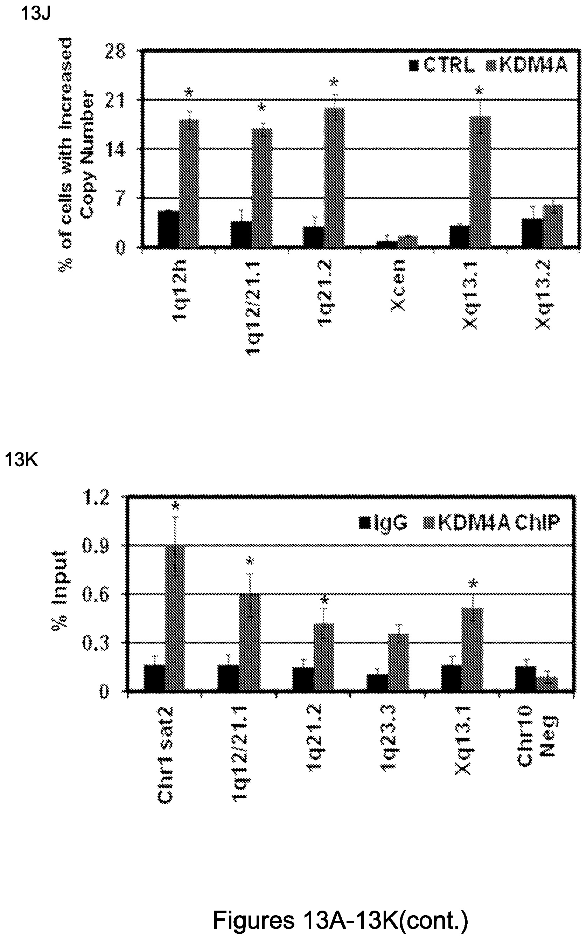

Figs. 13A-13K are related to Figs. 6A-6I and 7A-7J. Fig. 13A depicts a graph of focal amplification of specific cytogenetic bands determined by the statistical test based on the null distribution of mean cytoband copy differences correlated with amplification of KDM4A in 4,420 samples across all cancers from the TCGA data set. Graph depicts the -log(10) P-value for correlation of the copy number of each cytogenetic band with the copy number of KDM4A. The blue line represents the locus of KDM4A (gene-specific significance of P=2x10-142). Fig. 13B depicts a graph of focal amplification of specific cytogenetic bands determined by the statistical test based on the null distribution of mean cytoband copy differences correlated with amplification of KDM4B in 4,420 samples across all cancers from the TCGA data set. The blue dot represents the gene-specific significance of KDM4B. Fig. 13C depicts a graph of focal amplification of specific cytogenetic bands determined by the statistical test based on the null distribution of mean cytoband copy differences correlated with amplification of KDM4A in 547 ovarian cancer samples. The line represents the locus of KDM4A (gene-specific significance of P=1.4x10-42). For each co-amplification plot, blue shaded regions indicate 1p11.2 through 1q21.3 and dashed lines indicate Xp11.2 through Xq13.2. Figs. 13D-13F depict graphs of empirical cumulative distribution function (% of samples possessing mean focal copy number less than or equal to the value on the x-axis) in 1q21.1-1q21.3 with unaltered (0) copy number of KDM4A, copy Gain of KDM4A (+1), or focally Amplified KDM4A (+2). The dashed lines at 0.3 is a cutoff to define the fraction of co-amplified samples with KDM4A amplification in Fig. 6D-6I. Figs. 13G-13I depict the same data as in 13D-13F but for KDM4B. Fig. 13J demonstrates that KDM4A overexpression increased copy number of 1q12h, 1q12/21.1 and 1q21.2 and Xq13.1 but not X cen or Xq13.2 in 293T cells. Copy number was assessed using the indicated FISH probes. Fig. 13K depicts a graph demonstrating that KDM4A-dependent re-replicated regions are bound by KDM4A. KDM4A ChIP was conducted in 293T cells at regions of re-replication. Error bars represent the S.E.M. * indicates P<0.05 using two-tailed students T-test.

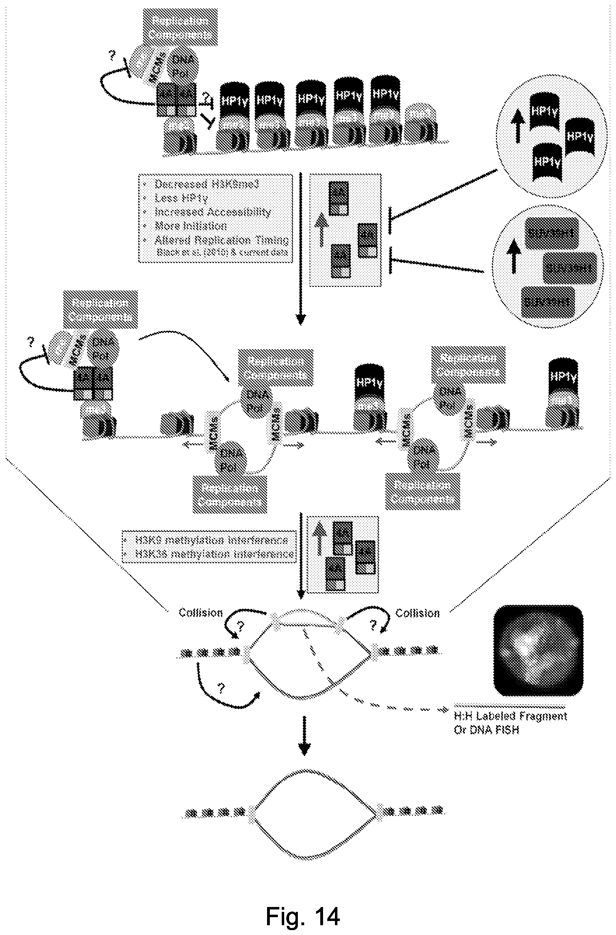

Fig. 14 depicts a schematic depicting the model by which KDM4A could promote copy number gain.

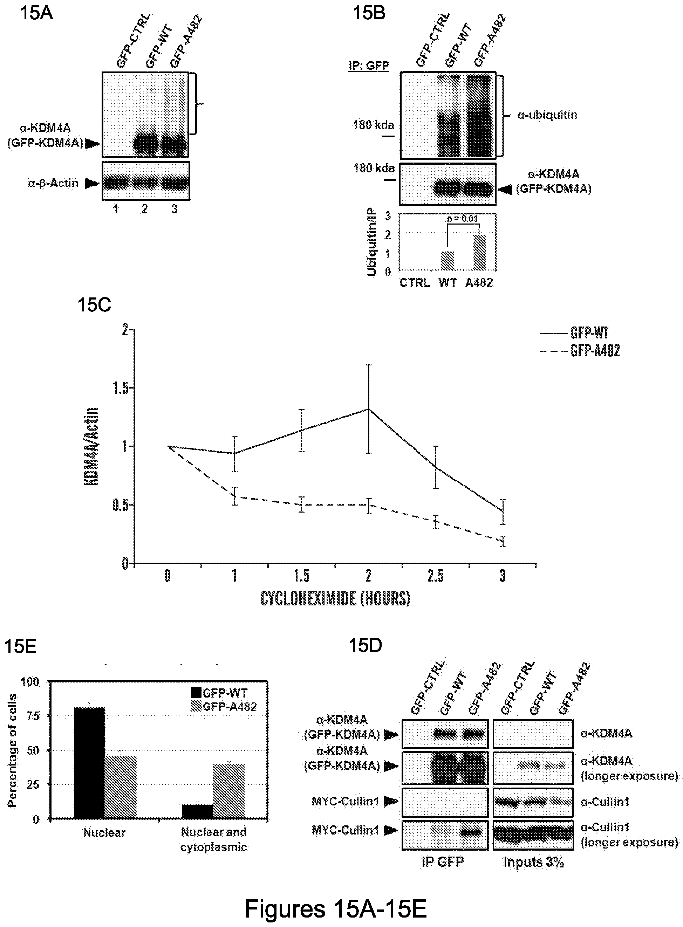

Figs. 15A-15E demonstrate that SNP-A482 impacts KDM4A ubiquitination and turnover. Fig. 15A depicts images of western blots demonstrating that cells overexpressing GFP-KDM4A-SNPA482 have more laddering than cells overexpressing GFP-KDM4A-WT. Fig. 15B depicts a representative immunoblot and summary graph from experiments in which GFP-KDM4A-WT and SNP-A482 were immunoprecipitated under denaturing conditions. SNP-A482 is two-fold more ubiquitinated than GFP-KDM4A-WT. Fig. 15C depicts a graph demonstrating that GFP-KDM4A-SNP-A482 has a shorter half-life than GFP-KDM4A-WT. HEK293T cells overexpressing GFP-KDM4A-WT or SNP-A482 were treated with cycloheximide. The *y* axis represents the ratio of GFP-KDM4A relative to time 0, which was normalized to β-actin. The average of 16 independent experiments is shown. Fig. 15D depicts immunoblots from coimmunoprecipitation experiments which demonstrate GFP-KDM4A-SNP-A482 interacts more strongly with MYC-Cullin1 than GFP-KDM4A-WT. Fig. 15E depicts a graph of cellular localization of KDM4A based on immunofluorescence experiments. GFP-KDM4A-WT is 35% more exclusively nuclear than GFP-KDM4A-SNPA482. The average of 2 independent experiments is represented. All error bars represent the SEM.

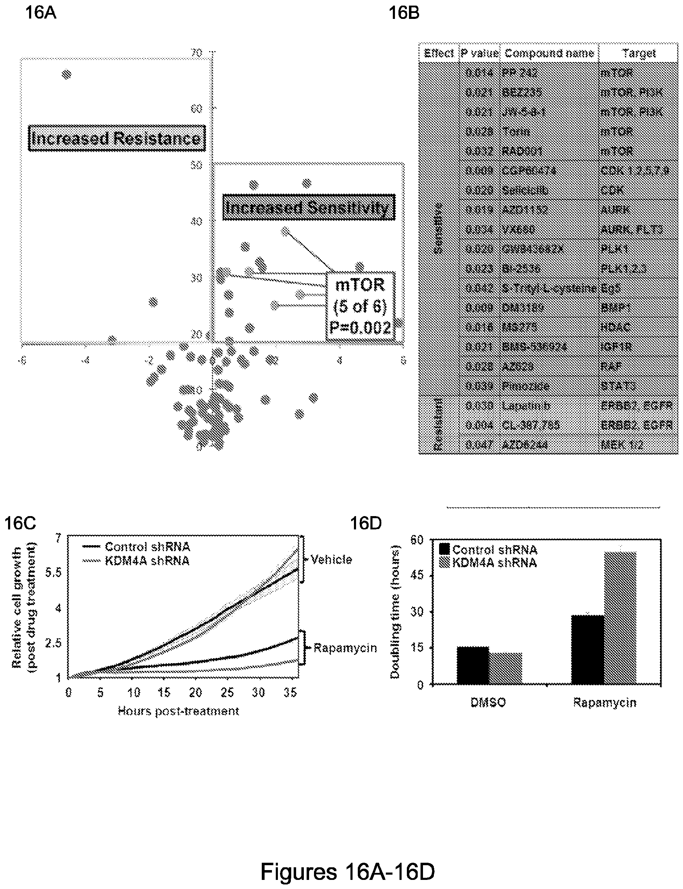

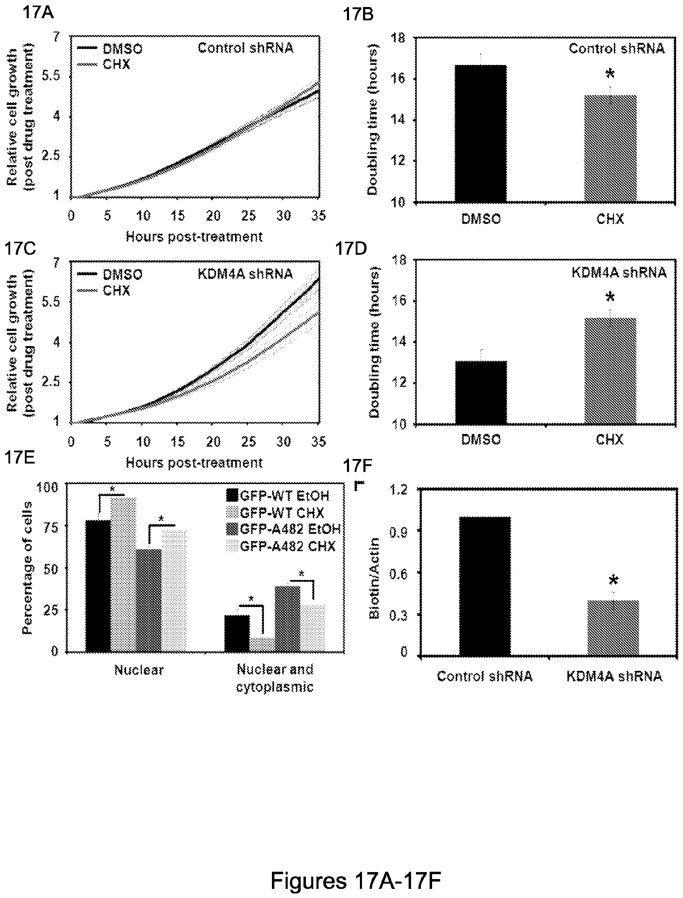

Figs. 16A-16D demonstrate that SNP-A482 impacts cellular sensitivity to compounds targeting the mTOR pathway. Fig. 16A depicts a volcano plot representing statistical significance (inverted *y* axis) versus the effect of SNP-A482 on drug sensitivity. Compounds above the x axis are statistically significant (p<0.05). Fig. 16B depicts a table of compounds with statistically different sensitivity in Fig. 17A and the associated P value. Fig. 16C depicts a graph demonstrating that HEK293T cells transfected with 3 different shRNA directed against KDM4A are more sensitive to Rapamycin than cells transfected with the control vector. Growth curves: the *y* axis represents the relative cell number normalized at the time of treatment. The graph represents the average of 3 independent experiments. Fig. 16D depicts a graph demonstrating that the doubling time between 5h and 35h after Rapamycin treatment. The graph represents an average of 3 independent experiments.
Figs. 17A-17F demonstrate that KDM4A impacts cellular sensitivity to the protein synthesis inhibitor Cycloheximide. Fig. 17A depicts a graph demonstrating that HEK293T cells transfected with the control vector do not slow growth after treatment with 30 nM of Cycloheximide (CHX) compare to DMSO. Growth curves: the *y* axis represents the relative cell number normalized at the time of treatment. The graph represents the average of 3 independent experiments. Fig. 17B depicts a graph demonstrating that doubling time between 5h and 35h after treatment. The graph represents the average of 3 independent experiments. Fig. 17C depicts a graph demonstrating that HEK293T cells transfected with a shRNA directed against KDM4A slow growth after treatment with 30 nM of Cycloheximide compare to DMSO. Growth curves: the *y* axis represents the relative cell number normalized at the time of treatment. The graph represents the average of 3 independent experiments. Fig. 17D depicts a graph of doubling time between 5h and 35h after treatment. The graph represents the average of 3 independent experiments. Fig. 17E depicts a graph of cellular localization of KDM4A based on immunofluorescence experiments. GFP-KDM4A-WT and GFP-KDM4A-SNPA482 are more nuclear after 3h of treatment with Cycloheximide. The average of 4 independent experiments is represented. Fig. 17F depicts a graph of newly synthesized proteins labeled for 2h followed by detection by western blot. The *y* axis represents the ratio of newly synthesized proteins normalized to β-actin relative to control shRNA. An average of 5 independent experiments performed with 2 different KDM4A shRNA is represented. All error bars represent the SEM. * represents P<0.05.

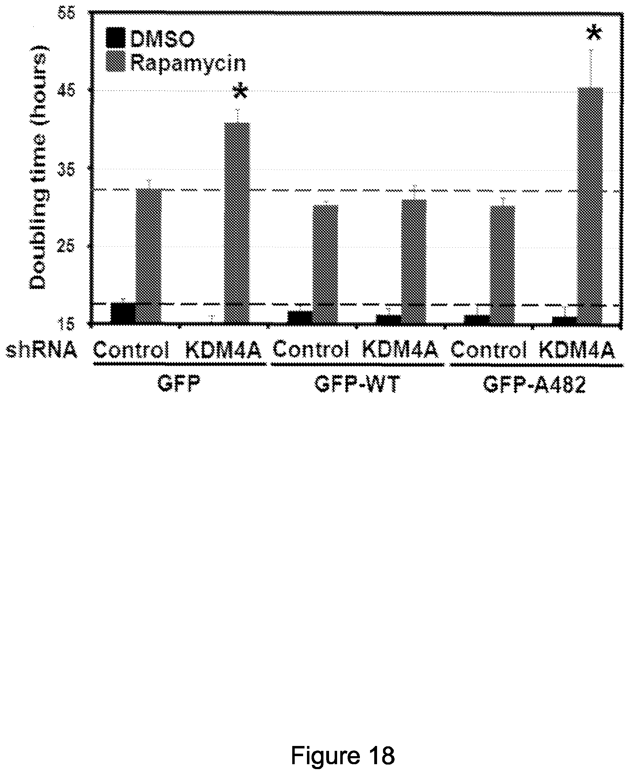

Fig. 18 depicts a graph demonstrating that KDM4A germline and somatic variants influence cellular sensitivity to Rapamycin. Re-expression of KDM4A-WT rescues the increased sensitivity to Rapamycin of KDM4A depleted cells while SNP-A482 does not. Doubling time between 5h and 35h after Rapamycin treatment of cells transfected with shRNA KDM4A and GFP-KDM4A constructs resistant to the shRNA. The graph represents an average of 2 independent experiments. All errors bars represent the SEM. * represents P<0.05.

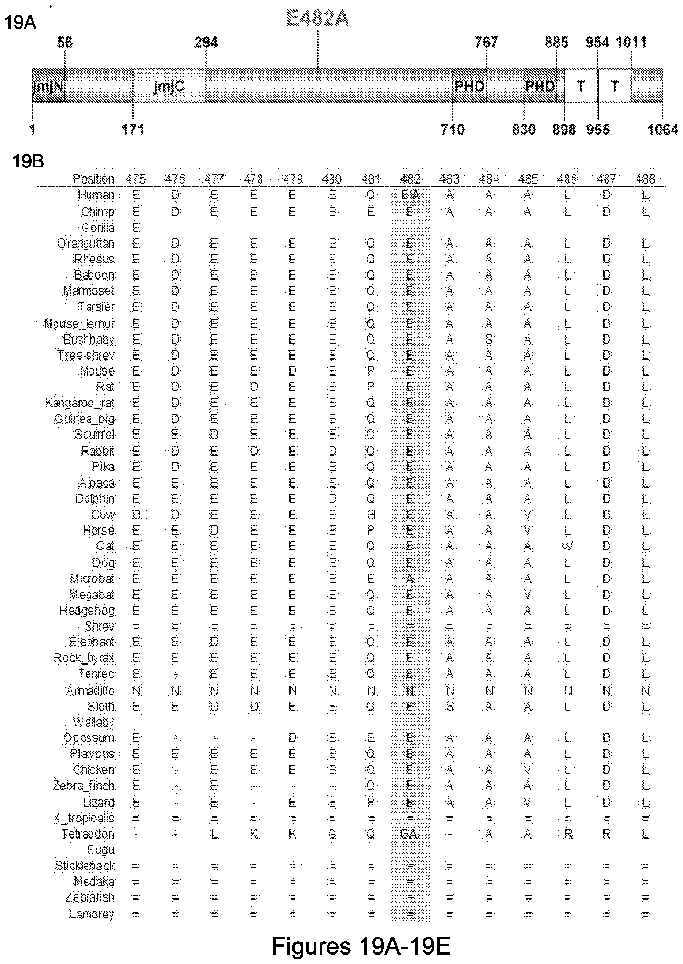

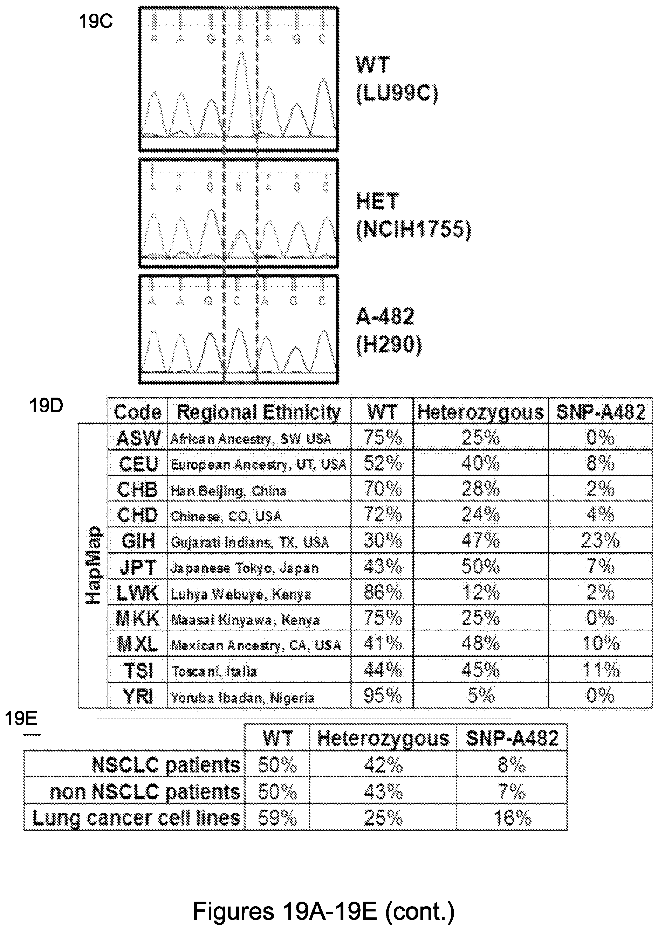

Figs. 19A-19E demonstrate the conservation and frequency of KDM4A SNP E482A (rs586339). Fig. 19A depicts a schematic of KDM4A representing the domains of the protein and the position of the SNP E482A (rs586339). Fig. 19B depicts a table of alignment across species of the sequence surrounding the human E/A482. E482 is the wild type allele (SEQ ID NOS 27-63, respectively, in order of appearance). Fig. 19C depicts a graph of representative sequencing plots of 3 lung cancer cell lines for KDM4A, homozygote wild type (WT), heterozygote (HET) and homozygote SNP (A-482). Fig. 19D depicts a table of HapMap frequency for rs586339 (August 2010 HapMap public release #28). ASW African Ancestry in SW USA (n=57); CEU U.S. Utah residents with ancestry from northern and western Europe (n=113); CHB Han Chinese in Beijing, China (n=135); CHD Chinese in Metropolitan Denver, CO, USA (n=109); GIH Gujarati Indians in Houston, TX, USA (n=99); JPT Japanese in Tokyo, Japan(n=113); LWK Luhya in Webuye, Kenya (n=110); MKK Maasai in Kinyawa, Kenya (n=155); MXL Mexican Ancestry in Los Angeles, CA, USA (n=58); TSI Toscani in Italia (n=102); YRI Yoruba in Ibadan, Nigeria (n=147). Fig. 19E depicts a table of genotype frequency for NSCLC and non NSCLC patients and lung cell lines.

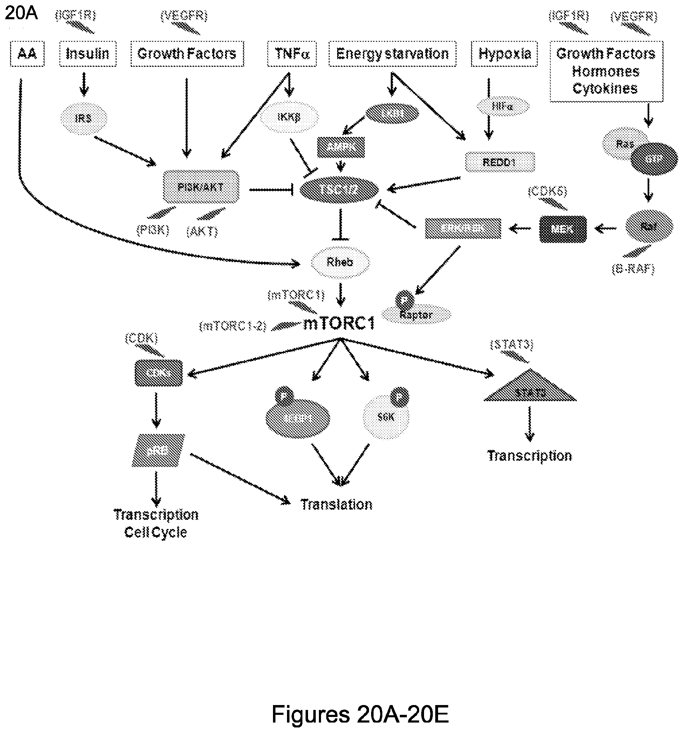

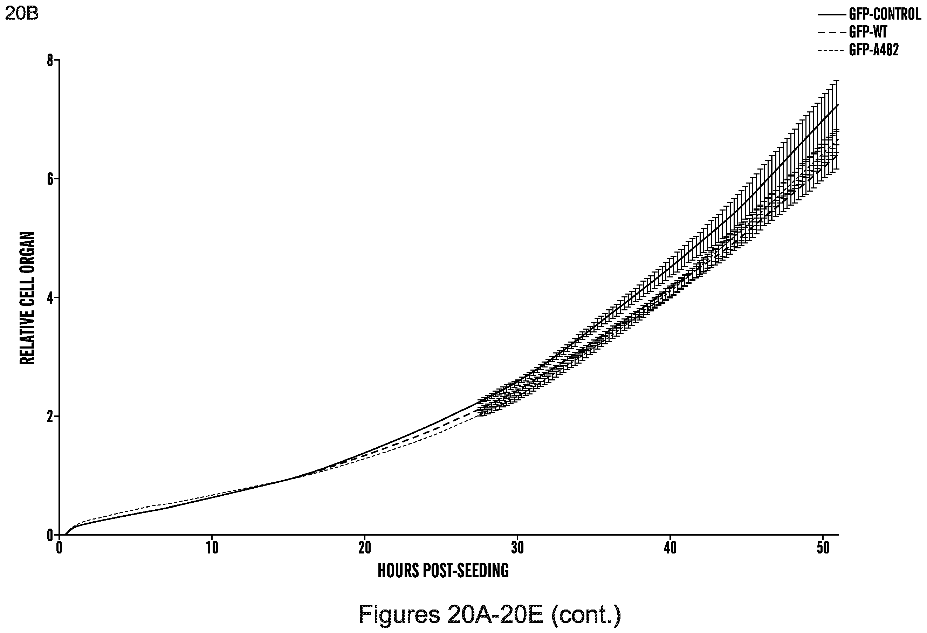

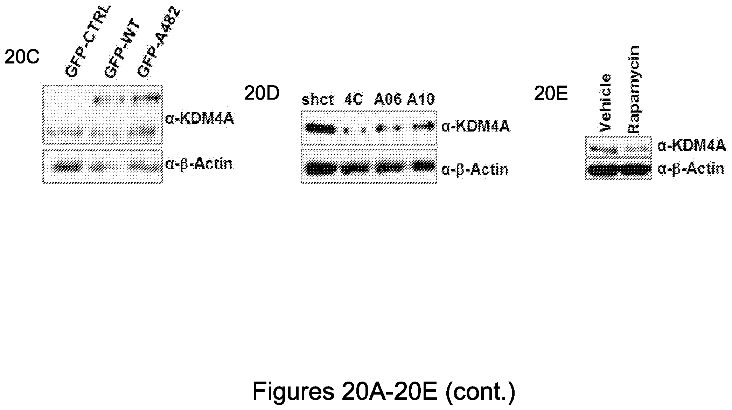

Figs. 20A-20E demonstrate that SNP-A482 impacts cellular sensitivity to drugs targeting the mTOR pathway. Fig. 20A depicts a schematic of the mTOR pathway highlighting the target of the drugs for which homozygote KDM4A-SNP-A482 lung cell lines present increased sensitivity (Fig. 16B). Fig. 20B depicts a graph demonstrating that the overexpression of KDM4A WT or SNP-A482 does not affect cell growth rate. Growth curves: the *y* axis represents the relative cell number normalized at the time of seeding. The graph represents the average of 2 independent experiments. Error bars represent SEM. Fig. 20C depicts images of immunoblots of HEK293T stable cell lines overexpressing GFP-control (GFP), GFP-KDM4A wild type (WT) and GFP-KDM4A-SNP-A482 (A482). Fig. 20D depicts images of Western blots demonstrating the knock-down efficiency of the cells used in Fig. 17C-17D. Fig. 20E depicts images of Western blot showing the decrease in protein level of KDM4A in HEK293T cells after 3h of Rapamycin treatment.

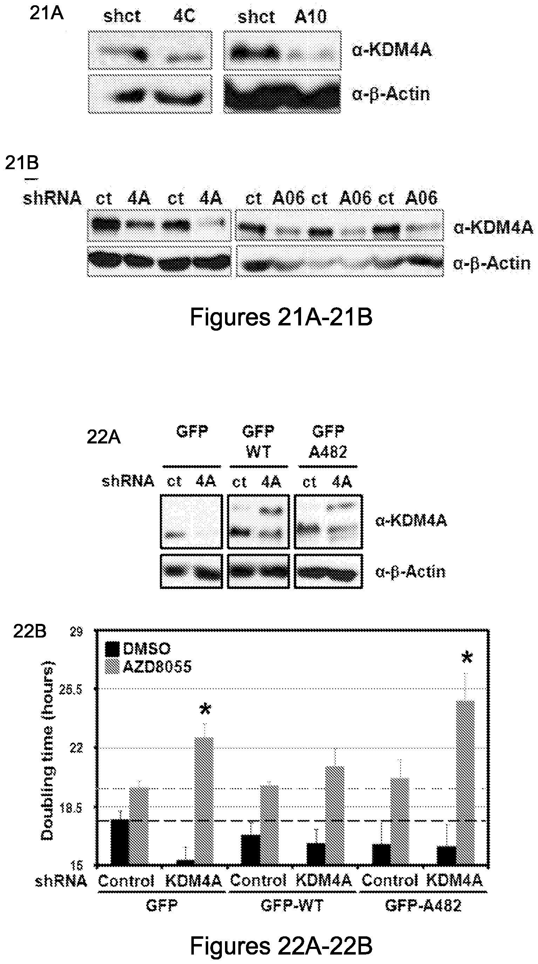

Figs. 21A-21B demonstrate that KDM4A knock-down impacts general translation. Fig. 21A depicts images of immunoblots of Western blot showing the knock-down efficiency of the cells used in Fig. 17A-17D. Fig. 21B depicts images of immunoblots of Western blot showing the knock-down efficiency of the cells used in Fig. 17F.
Figs. 22A-22B demonstrate that KDM4A germline and somatic variants influence cellular sensitivity to AZD8055. Fig. 22A depicts images of Western blot showing the expression of the cells used in Fig. 19A. Fig. 22B depicts a graph demonstrating that KDM4A wild type rescues the increased sensitivity to AZD8055 treatment due to KDM4A depletion while SNP-A482 does not. Doubling time between 5h and 35h after AZD8055 treatment of cells transfected with shRNA KDM4A and GFP-KDM4A constructs non targetable by the shRNA. The graph represents an average of 2 independent experiments. All error bars represent the SEM. * represents P<0.05.

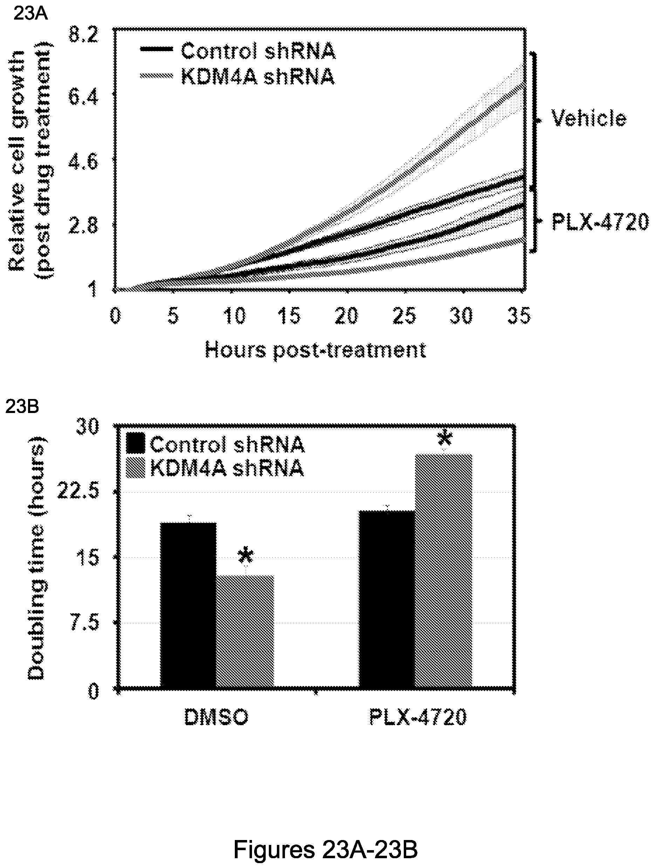

Figs. 23A-23B demonstrate that KDM4A impacts cellular sensitivity to PLX-4720. Fig. 23A depicts a graph demonstrating that HEK293T cells transfected with a shRNA directed against KDM4A are more sensitive to PLX-4720 than cells transfected with the control vector. Growth curves: the *y* axis represents the relative cell number normalized at the time of treatment. Fig. 23B depicts a graph of the doubling time between 5h and 35h after treatment. * represents P<0.05.

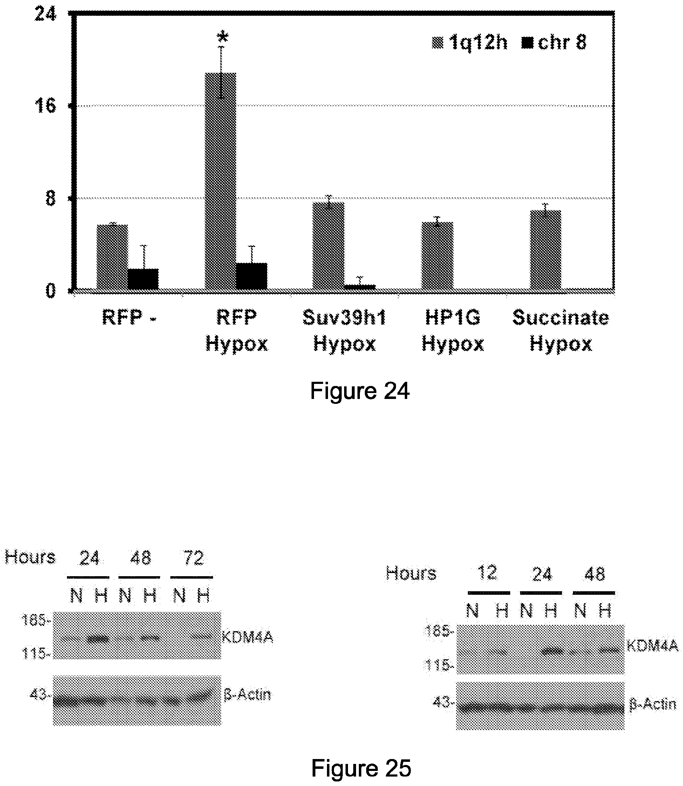

Fig. 24 demonstrates that hypoxia induces 1q12h copy number gains which can be antagonized by Suv39H1, HP1 gamma (G) or succinate. RPE cells were transfected with RFP, Suv39h1 or HP1G or treated with 2mM succinate for 24 hours. Cells were moved to 4% oxygen (hypox) or kept at normoxic conditions (-). The graph depicts copy number for the indicated conditions. * indicates P<0.05 by two-tailed student's t-test.
Fig. 25 depicts images of immunoblots demonstrating that hypoxia increases the protein levels of KDM4A in different cell lines. U2OS (left) and RPE (right) cells were incubated under either normoxic (N, 21% O2) or hypoxic (H, 4% O2) conditions for the indicated periods of time, after which lysates were prepared and analyzed by immunoblotting.

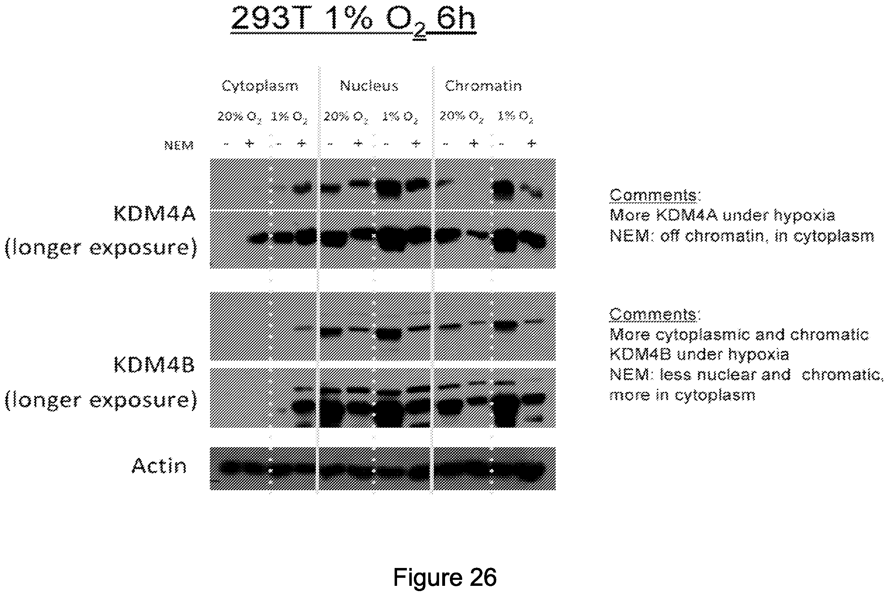

Fig. 26 depicts images of immunoblots demonstrating that KDM4A levels are increased in all compartments under hypoxic conditions, while NEM reduces the level of hypoxia-induced changes in KDM4A expression/localization. 293T cells were cultivated in hypoxic (1% O2) or normoxic (20% O2) conditions for 6h, then harvested and fractionated in presence of NEM which stabilizes SUMOylation and ubiquitination. NEM also altered the chromatin association.

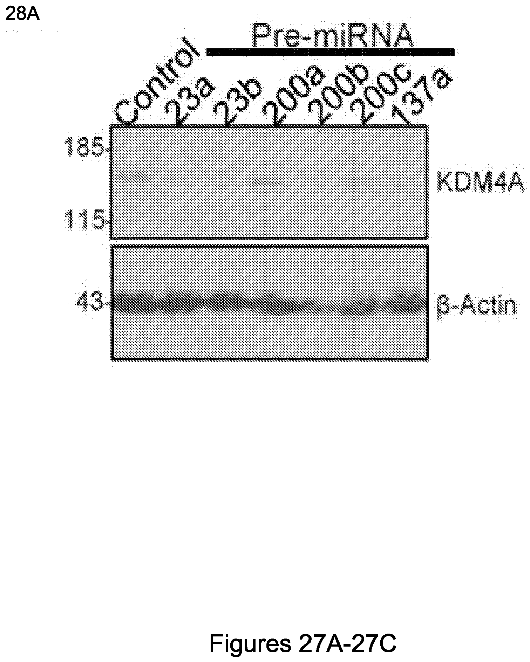

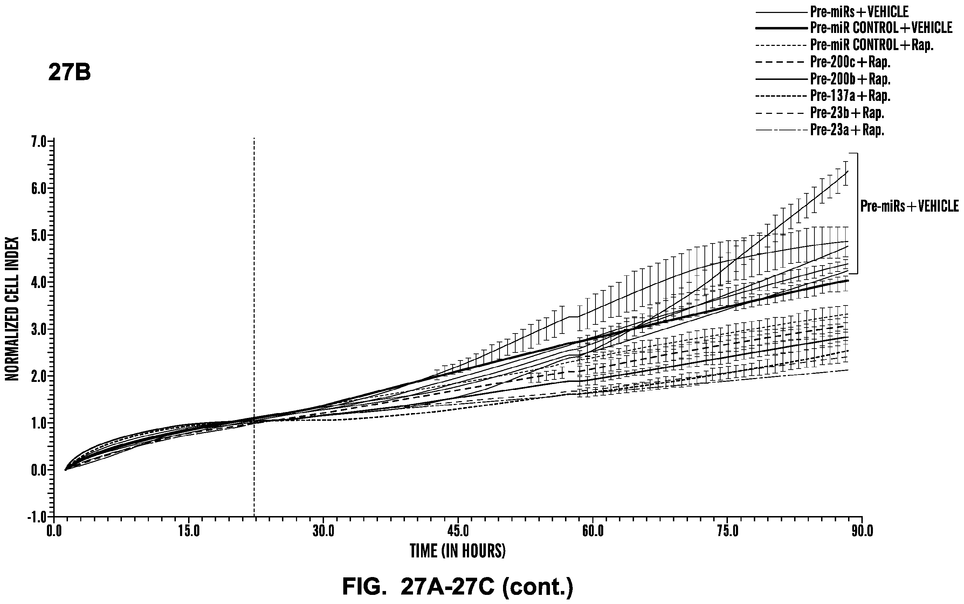

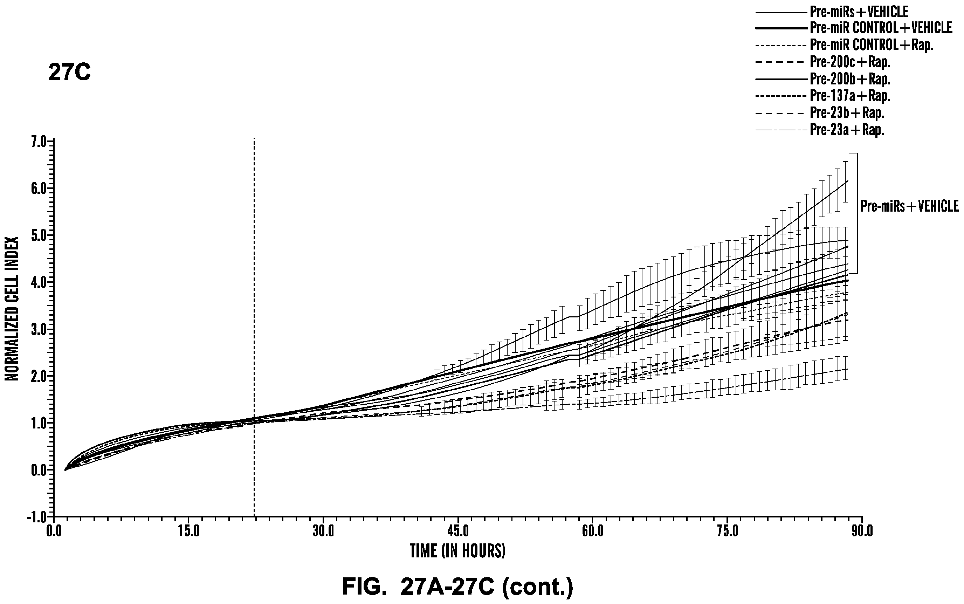

Figs. 27A-27C demonstrate that miRNAs regulate KDM4A expression. Fig. 27A depicts images of immunoblots demonstrating that transfection of pre-miRNAs downregulates KDM4A expression. U2OS cells were transfected with the indicated miRNAs and 48h post-transfection, lysates were prepared and analyzed by immunoblotting. Figs. 27B-27C depicts graphs of cell growth demonstrating that miRNA expression sensitizes U2OS cells to inhibitors of mTOR and BRaf. U2OS cells were transfected with miRNAs indicated in (Fig. 27A), and 48h post-transfection cells were seeded onto 96-well xCelligence plates and cell growth rates were measured upon treatment with (Fig. 27B) DMSO control or 10ng/ml Rapamycin (mTOR inhibitor), and (Fig. 27C) DMSO control or 10µM PLX4720 (BRaf inhibitor).

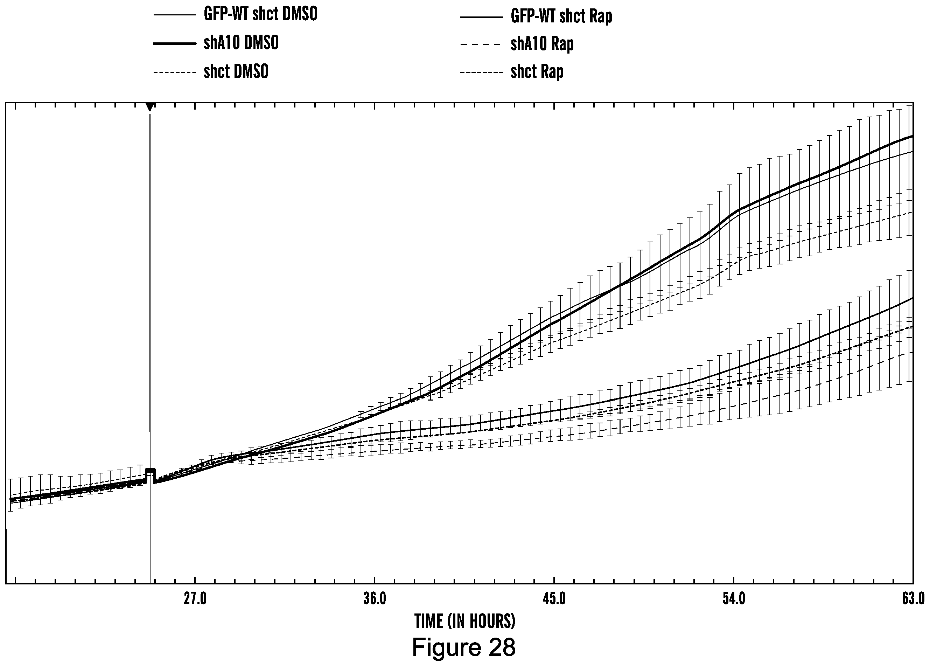

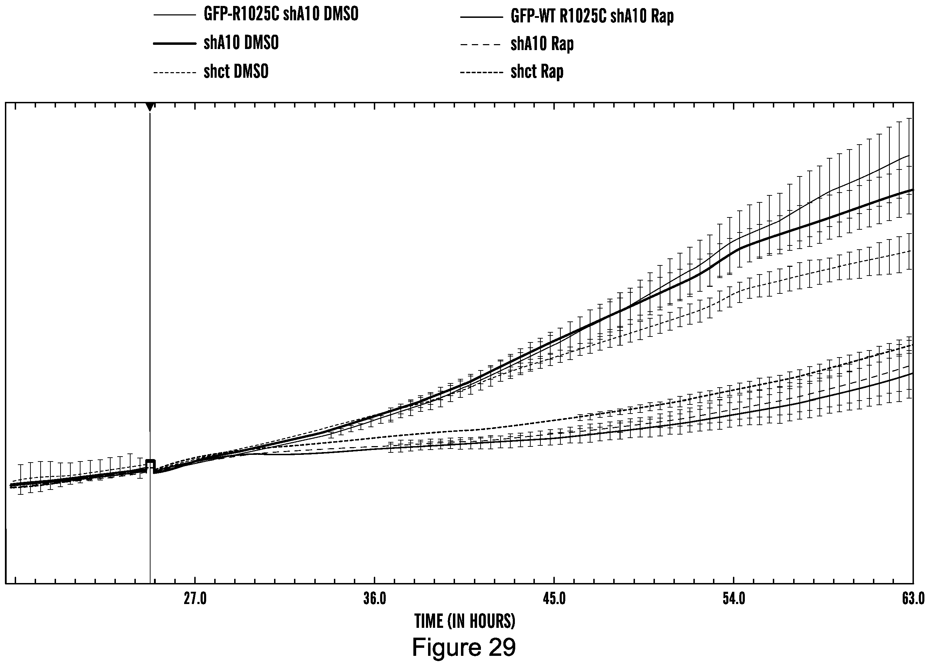

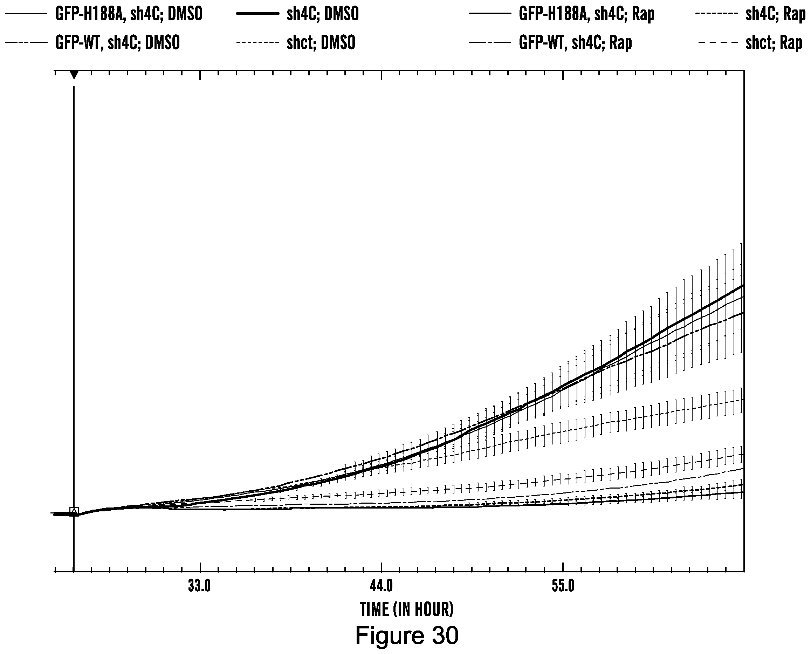

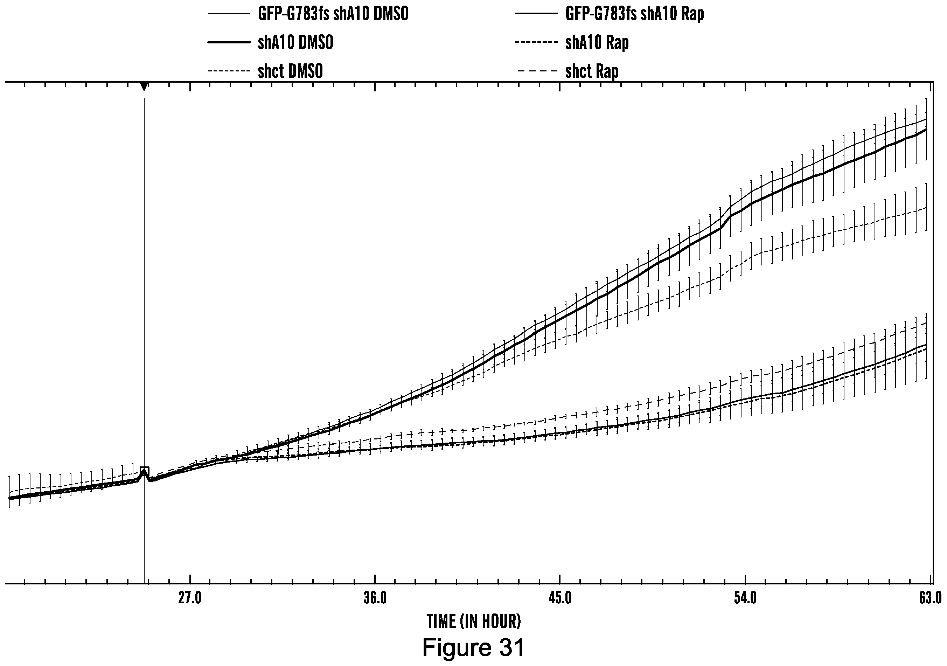

Figs. 28-31 demonstrate the effects of KMD4A mutations on drug senstivity. Cells were depleted of KDM4A (shA10) and transfected with wild-type (WT) or mutant KDM4A. The sensitivity of the cells to rapamycin (Rap) was then determined. Fig. 28 depicts a graph demonstrating that WT expression in the depletion background was less sensitive than shA10 alone. Fig. 29 depicts a graph demonstrating that a SNV mutation that causes a R1025C mutation was equally as sensitive to KDM4A/JMJD2A depletion (shA10) when treated with Rapamycin (Rap). This differed from WT expression in the depletion background, which was less sensitive. Fig. 30 depicts a graph demonstrating that the catalytic mutant H188A cannot reduce sensitivity to rapamycin when compared to WT KDM4A. Fig. 31 depicts a graph demonstrating that a mutation that causes a frameshift G783fs was equally as sensitive to KDM4A/JMJD2A depletion (shA10) when treated with Rapamycin (Rap). This differed from WT expression in the depletion background, which was less sensitive.

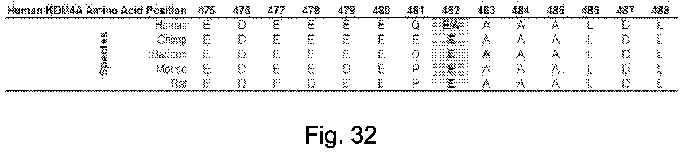

Fig. 32 depicts the conservation and frequency of KDM4A SNP-A482 (rs586339). E482 is the conserved allele. Alignment across species of the sequence surrounding the human E482A (SEQ ID NOS 27, 28, 31, 37, and 38, respectively, in order of appearance).

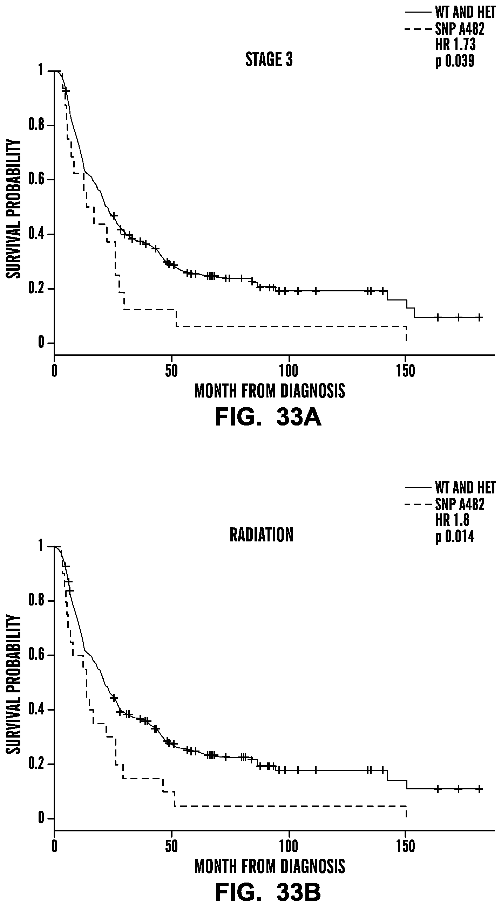

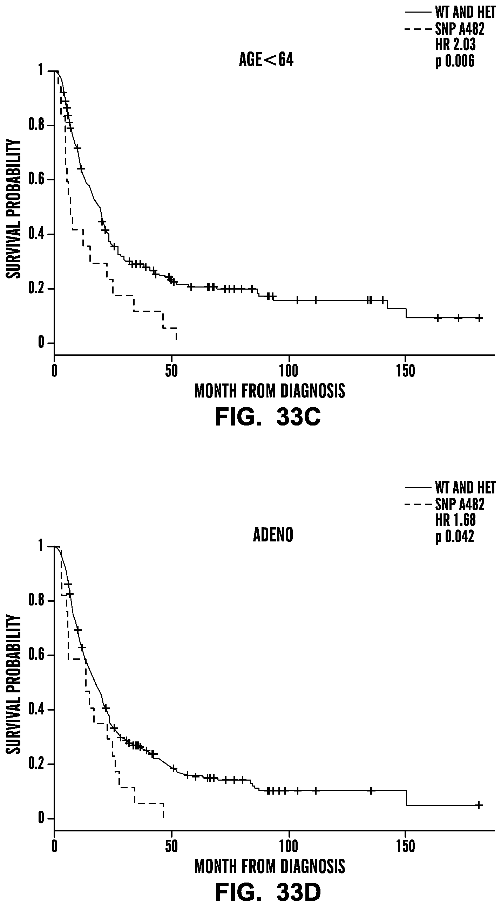

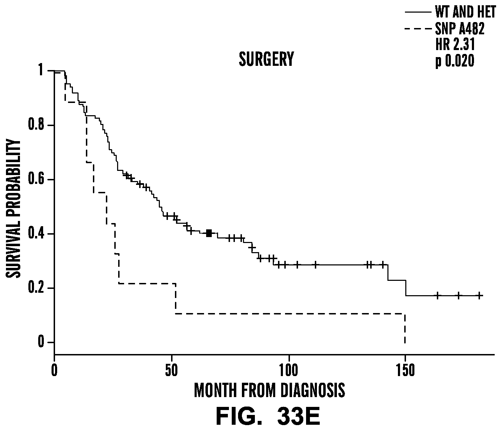

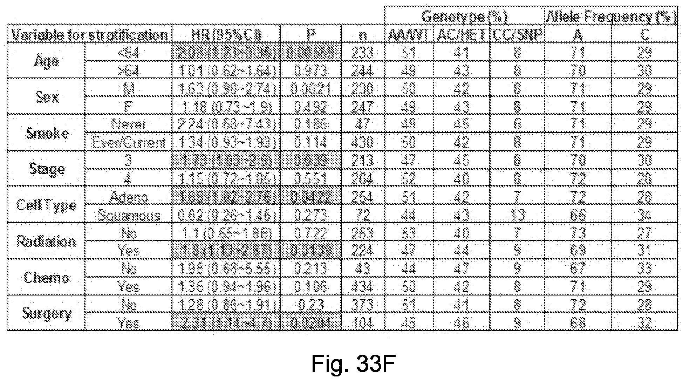

Figs. 33A-33F demonstrate that SNP-A482 correlates with worse outcome for late stage NSCLC patients. Survival curves plotted based on genotype- wild type (WT) and heterozygous (HET) versus homozygous SNP-A482 (SNP-A482). X axis is the months since diagnosis of NSCLC. Y axis is the probability of survival. Fig. 33A depicts patients with late onset stage 3 cancers. Fig. 33 depicts treatment with radiation. Fig. 33C depicts patients under 64 years of age. Fig. 33D depicts patients with an Adenocarcinoma. Fig. 33E depicts treatment by surgery. Fig. 33F depicts KDM4A SNP-A482 stratification for late stage NSCLC patients. HR represents the hazard ratio, 95%CI the 95% confidence interval, P the P value, n the number of patients in each category, AA the genotype for homozygote wild-type (E482), AC for heterozygote, CC for homozygote SNP (A482).

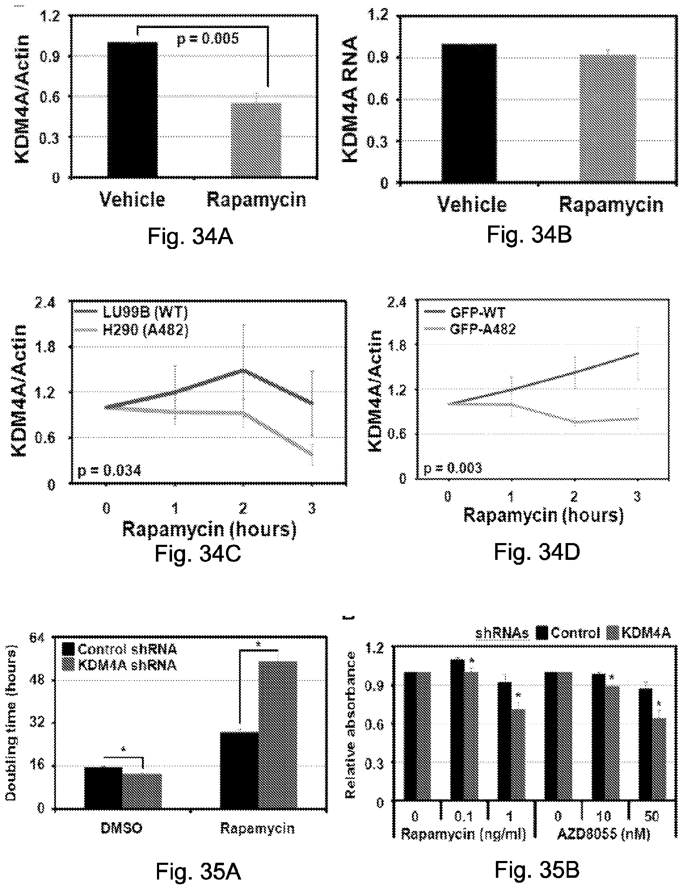

Figs. 34A-34D demonstrate that KDM4A SNP-A482 protein levels are more affected by mTOR inhibition. Fig. 34A depicts a graph of an average of 3 independent experiments demonstrating that KDM4A protein levels decrease upon Rapamycin treatment. HEK 293T cells were treated with 100ng/ml of Rapamycin for 24h. Fig. 34B depicts a graph demonstrating that KDM4A RNA levels are stable upon Rapamycin treatment. HEK 293T cells were treated with 100ng/ml of Rapamycin for 24h before RNA was harvested. An average of 3 independent experiments is represented. Fig. 34C depicts a graph demonstrating that Rapamycin causes a selective decrease of KDM4A SNP-A482 levels versus WT. LU99B (KDM4A homozygote WT) and H290 (KDM4A homozygote SNP-A482) cell lines were treated with 100ng/ml of Rapamycin for the indicated period of time. The *Y* axis represents the ratio of KDM4A relative to time 0, which was normalized to β-actin. The average of three independent experiments is shown. All error bars represent the SEM. Fig. 34D depicts a graph demonstrating that HEK 293T cells transiently overexpressing GFP-KDM4A WT or GFP-KDM4A SNP-A482 were treated with 100ng/ml of Rapamycin for the indicated period of time. The *Y* axis represents the ratio of GFP-KDM4A relative to time 0, which was normalized to β-actin. The average of three independent experiments is shown. *P* value represents the two-tailed student's *t* test for Fig. 34A and 34B and two-way ANOVA (significance for overall difference) for Figs. 34C and 34D.

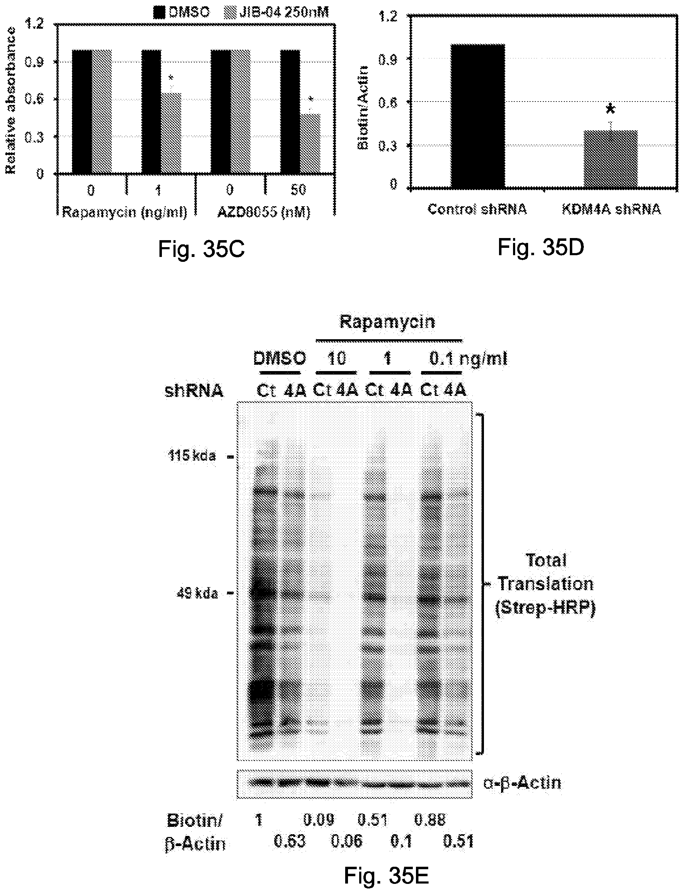

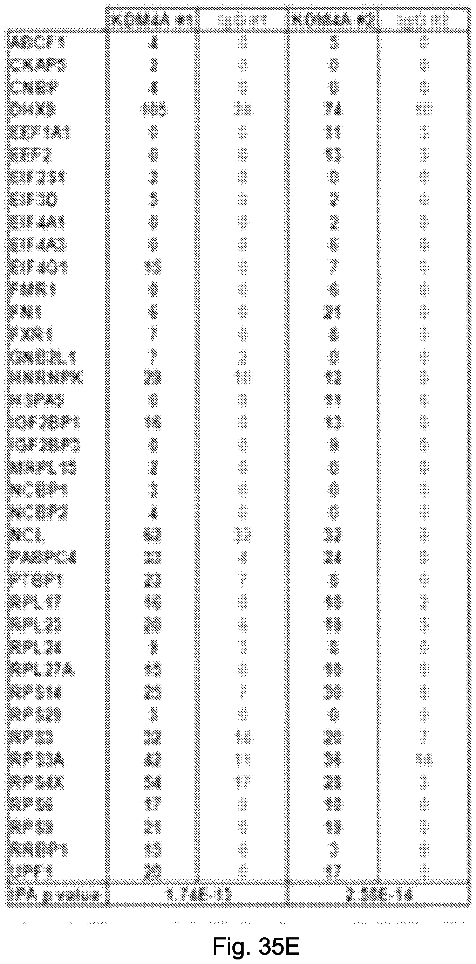

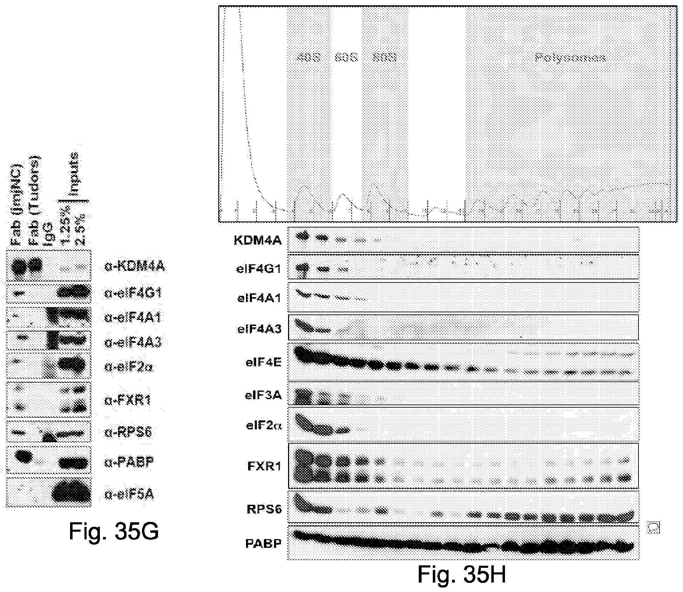

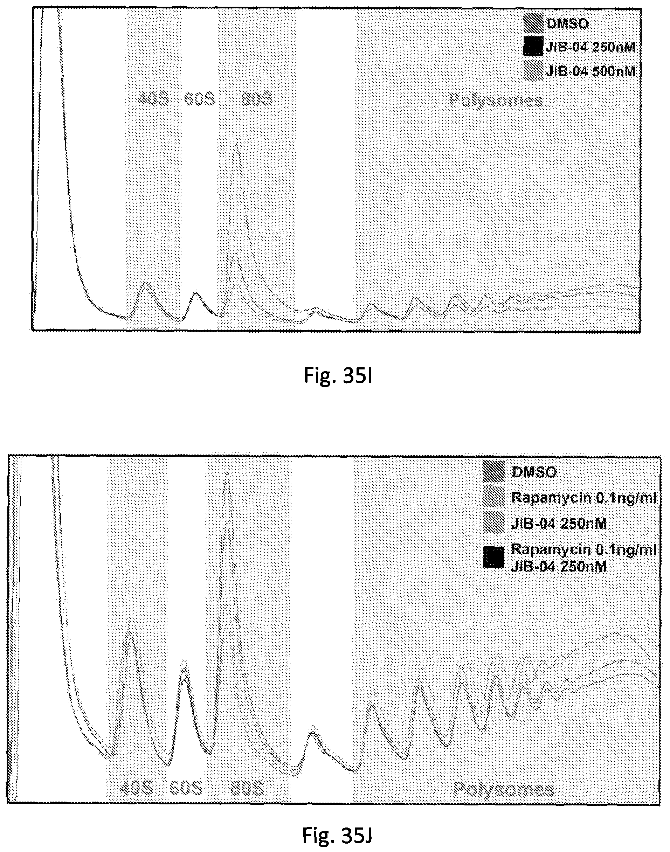

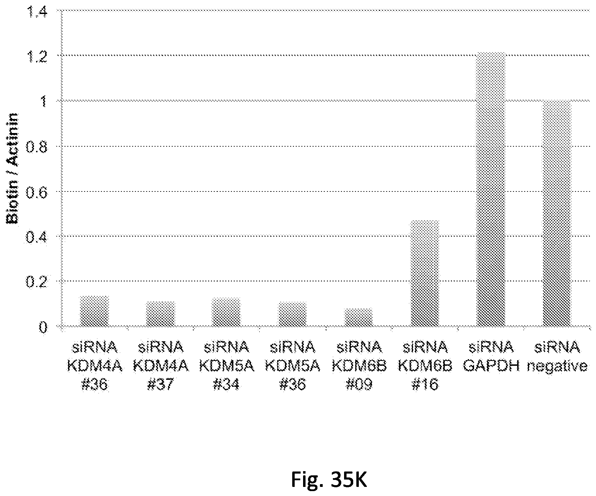

Figs. 35A-35K demonstrate that KDM4A impacts cellular sensitivity to mTOR inhibitors and translation. Fig. 35A depicts a graph demonstrating that HEK 293T cells transfected with three different shRNAs directed against KDM4A are more sensitive to Rapamycin than cells transfected with the control vector. HEK 293T cells were seeded 24h after transfection and treated with 100ng/ml of Rapamycin 24h after seeding. The graphs represent the doubling time between 5h and 35h after Rapamycin treatment. An average of three independent experiments is represented. Fig. 35B depicts a graph demonstrating that HEK 293T cells transfected with shRNA 4A.10 are more sensitive to Rapamycin and AZD8055 than cells transfected with the control vector. Cells were seeded 48h after shRNA transfection, treated with the indicated drugs and concentration after 24h for 48h before being analyzed by colorimetric proliferation and viability MTT assay. The assays were normalized to an assay done at treatment time. The average of four independent experiments is represented. Fig. 35C depicts a graph demonstrating that HEK 293T cells treated with JIB-04 are more sensitive to Rapamycin and AZD8055 than cells treated with vehicle. Cells were treated with the indicated drugs 24h after seeding and for 48h before been analyzed by MTT assay. The average of three independent experiments is represented. All error bars represent the SEM. Fig. 35D depicts a graph demonstrating that HEK 293T cells transfected with KDM4A shRNA present a decrease in overall translation. Forty eight hours after transfection, cells were deprived of Cysteine and Methionine for 1h and grown in the presence of the nucleotide analog AHA (L-Azidohomoalanine) for 2h. The newly synthesized proteins were labeled with biotin and equal total amounts of protein were immunoblotted with streptavidin-HRP. The graph represents an average of five independent experiments done with two different KDM4A shRNA. The *Y* axis represents the ratio of total biotinylated proteins normalized to b-actin. Fig. 35E demonstrates that HEK 293T cells transfected with KDM4A shRNA 4A.2 enhanced the decrease in translation obtained after Rapamycin treatment. Forty eight hours after transfection, cells were treated with the indicated concentration of Rapamycin for 24h, and then treated as in Fig. 35D; the ratio biotin/b-actin has been calculated with ImageJ and represents the average of two independent experiments. All error bars represent the SEM. Fig. 35F depicts the results of mass spectrometry analysis of KDM4A endogenous IPs in 293T cells. For each protein (row) the number of peptides pull downed is annotated. The IPA p value represent the p value for the representation of the translation category on the entire mass spectrometry data after IPA analysis. Fig. 35G demonstrates the endogenous interaction of KDM4A and translation factors in 293T cells. Two different Fab antibodies have been used; JmjNC targeting the catalytic domain and Tudor targeting the Tudor domains of KDM4A. Fig. 35H depicts Western blot of polysome profile fractions. KDM4A is present in the initiation of translation fraction 40S. Fig. 35I depicts polysome profiles from 293T cells treated with JIB-04 at 250 and 500nM. JIB-04 creates a defect in translation initiation. Fig. 35J depicts polysome profiles from 293T cells treated with JIB-04 250nM and Rapamycin 0.1ng/ml. JIB-04 and Rapamycin 0.1ng/ml show an enhancement of translation initiation defect. Fig. 35K depicts a graph demonstrating that HEK 293T cells transfected with KDM4A, KDM5A and KDM6B siRNAs present a decrease in overall translation. Forty eight hours after transfection, cells were treated as in Fig. 35D.

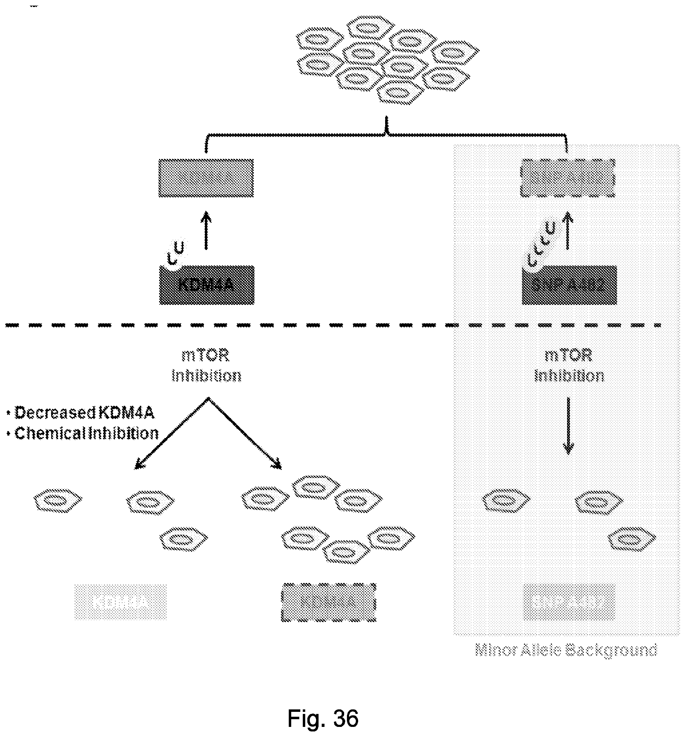

Fig. 36 depicts a model. Top: KDM4A SNP-A482 is more ubiquitinated than KDM4A WT but does not affect KDM4A biological functions. Bottom: KDM4A inhibition or its decreased levels by shRNA (far left) or in the presence of homozygote SNP-A482 background (far right) enhanced the decreased cell proliferation due to mTOR inhibition.

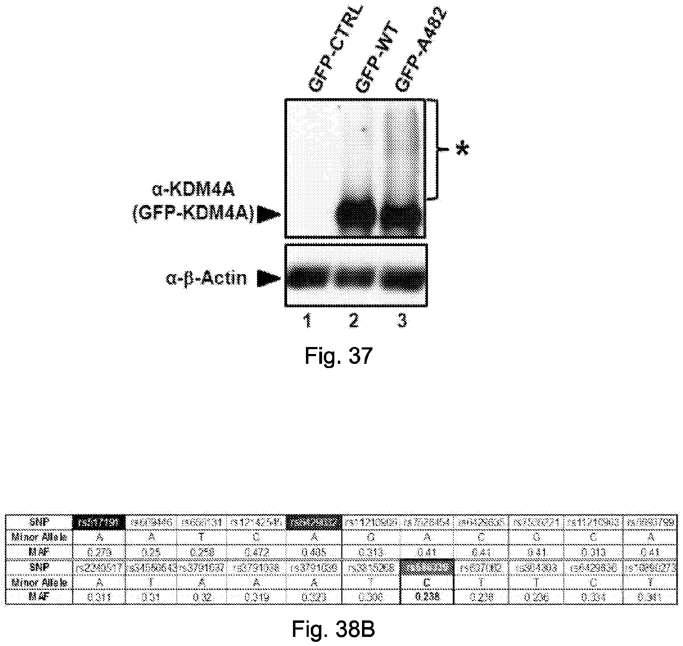

Fig. 37 demonstrates that SNP-A482 impacts KDM4A ubiquitination. Cells overexpressing GFP-KDM4A SNP-A482 present more laddering than cells overexpressing GFP-KDM4A WT. Higher molecular weight products are marked with the *.

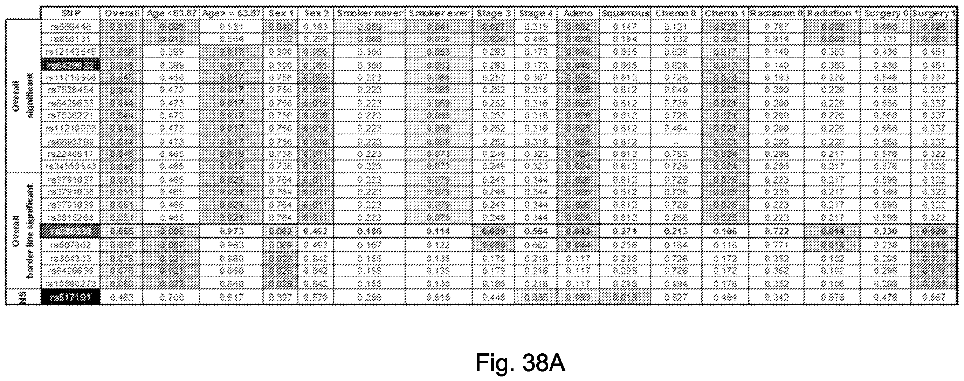

Figs. 38A-38B demonstrate that KDM4A SNPA-482 frequency and outcome in stratified NSCLC patients. Fig. 38A depicts stratification for late stage NSCLC patients based on KDM4A SNPs status. The numbers in the table represent the p value of the relation of each homozygous SNP to outcome versus the other genotypes (heterozygote and homozygote for the major alleles). Cells highlighted in dark gray represent a significant p value (p<0.05); in light gray a borderline significant p value (p<0.1). Fig 38B demonstrates Minor Allele Frequency (MAF) of KDM4A SNPs reported in Fig. 38A from the dbSNP database. rs586339 (SNP-A482) is the only coding SNP.

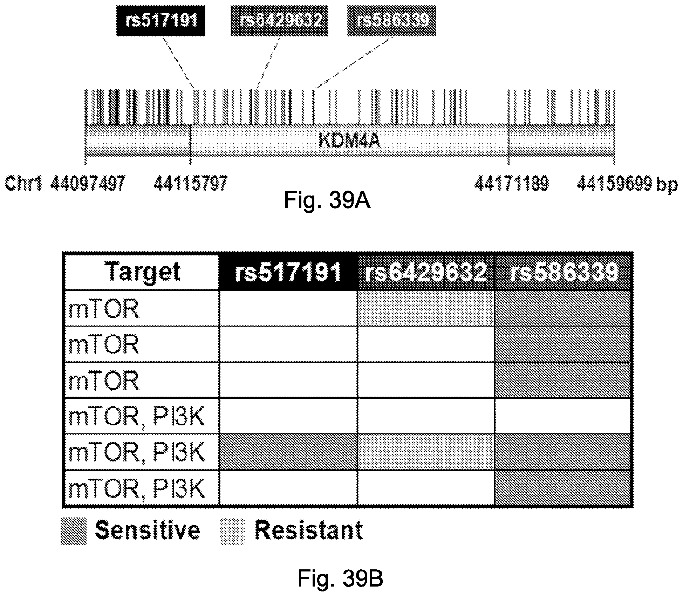

Figs. 39A-39B demonstrate that rs517191 and rs586339 are not associated with increased sensitivity to mTOR inhibitors. Fig. 39A depicts a schematic of human *kdm4a* gene with the evaluated SNPs indicated- rs586339 (SNP-A482), rs517191 and rs6429632. Fig. 39B depicts a comparison of the homozygous SNP status to that of mTOR chemotherapeutic sensitivity. Seventy five NSCLC cell lines and 88 compounds were used for these analyses. Green blocks represent increased sensitivity and orange blocks represent increased resistance to each drug.

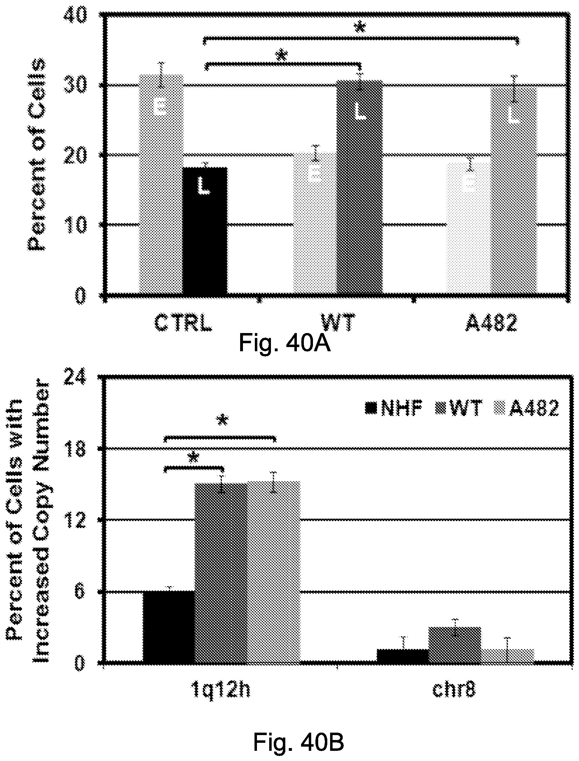

Figs. 40A-40B demonstrate that SNP-A482 does not impact KDM4A catalytic activity, faster S phase and copy gain. Fig. 40A demonstrates that overexpression of KDM4A SNP-A482 promotes faster S phase progression like that of wild type KDM4A. A graphical summary of the average percentage of GFP, KDM4A-WT, or KDM4A-A482 HEK 293T stable cell lines in early (E) and late (L) S phase, 12h after nocodazole release. Fig. 40B demonsrates that overexpression of KDM4A SNP-A482 promotes 1q12h copy gain, similarly to wild type KDM4A overexpression. Quantification of FISH experiments in transfected RPE with NHF, NHF-KDM4A WT and NHF-KDM4A SNP-A482 with the indicated FISH probes. All error bars represent the SEM.

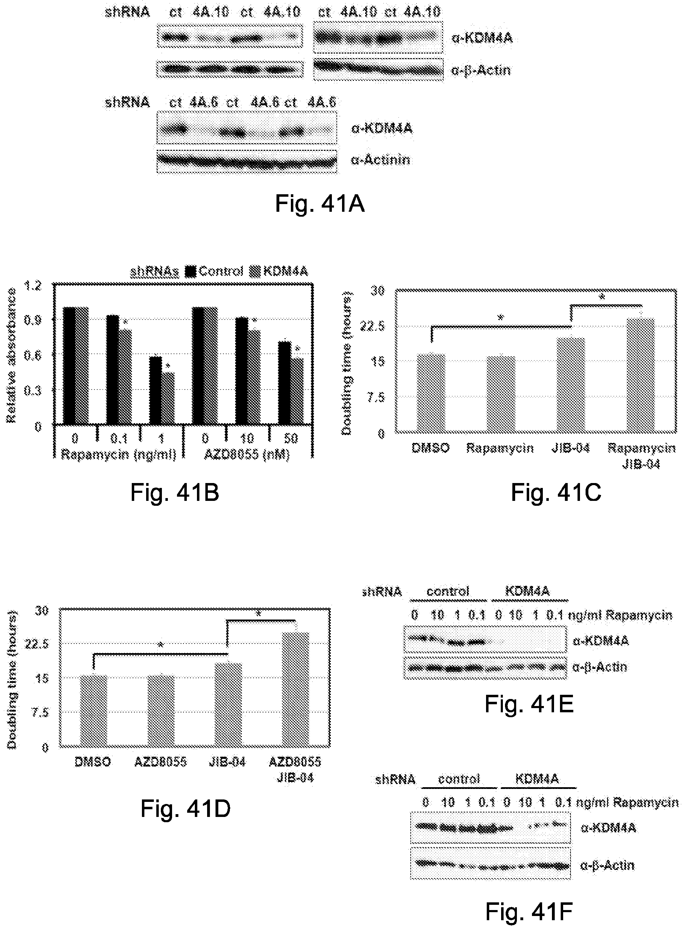

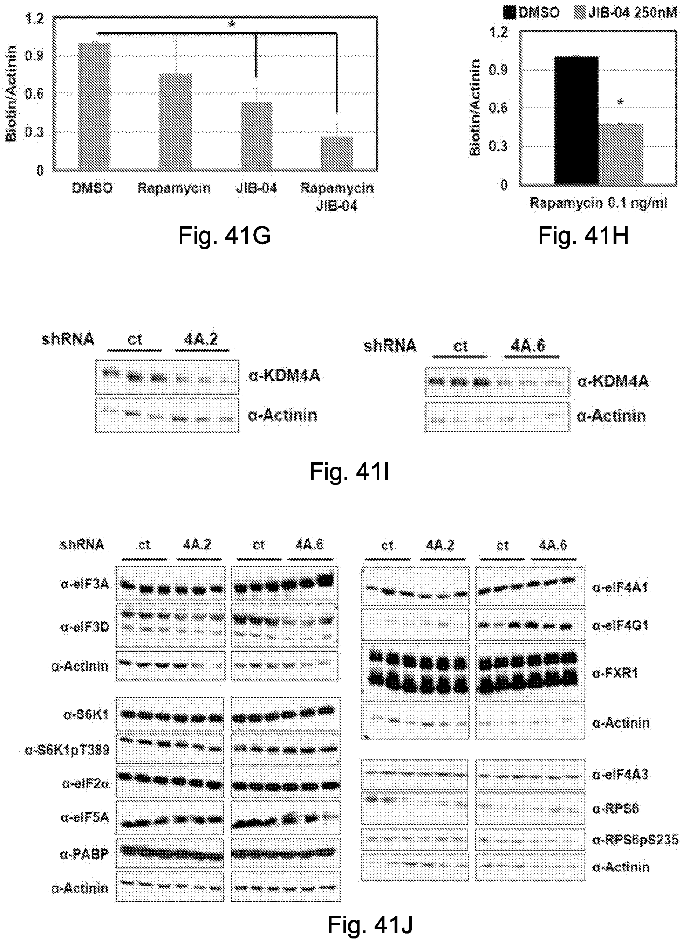

Figs. 41A-41J demonstrate that KDM4A depletion and chemical inhibition increase mTOR inhibitor sensitivity. Fig. 41A depicts a Western blot representing the knock-down efficiency in the cells used in the MTT assay in Figure Fig. 35B and 41B. Fig. 41B demonstrates that HEK 293T cells transfected with shRNA 4A.06 are more sensitive to Rapamycin and AZD8055 than cells transfected with the control vector. Cells were seeded 48h after shRNA transfection, treated with the indicated drugs and concentration after 24h, for 48h, before being analyzed by MTT assay. The assays were normalized to an assay done at treatment time. The average of three independent experiments is represented. Fig. 41C demonstrates that JIB-04 increases cell sensitivity to Rapamycin. HEK293T cells were treated with 250nM JIB-04 and 1ng/ml of Rapamycin 24h after seeding. The graphs represent the doubling time between 5h and 35h after Rapamycin treatment. An average of three independent experiments is represented. Fig. 41D demonstrates that JIB-04 increases cell sensitivity to AZD8055. HEK293T cells were treated with 250nM JIB-04 and 50nM of AZD8055 24h after seeding. The graphs represent the doubling time between 5h and 35h after Rapamycin treatment. An average of three independent experiments is represented. Fig. 41E depicts a Western blot representing the knock-down efficiency in the cells used in Fig. 35E. Fig. 41F depicts a Western blot representing the knock-down efficiency in the cells used in the replicate of Fig. 35E (used for the ratio biotin/b-Actin). All error bars represent the SEM. Fig. 41G demonstrates that JIB-04 enhanced the decrease in translation obtained after Rapamycin treatment.Twenty-four hours after treatment with 250nM of JIB-04 and 0.1ng/ml of Rapamycin, cells were deprived of Cysteine and Methionine for 1h and grown in the presence of the nucleotide analog AHA (L-Azidohomoalanine) for 2h. The newly synthesized proteins were labeled with biotin and equal total amounts of protein were immunoblotted with streptavidin-HRP. The graph represents an average of five independent experiments. The Y axis represents the ratio of total biotinylated proteins normalized to actinin. Fig. 41H depicts a graph representing the data in Fig. 41G relative to Rapamycin treatment. Fig. 41I depicts a Western blot representing the knock-down efficiency in the cells used in Figure 35J. Fig. 35J demonstrates that KDM4A knock-down does not affect the level of most of the translation initiation factors. HEK 293T transfected with KDM4A shRNA 4A.2 and 4A.6 for 48h were blotted for translation initiation factors interacting with KDM4A.

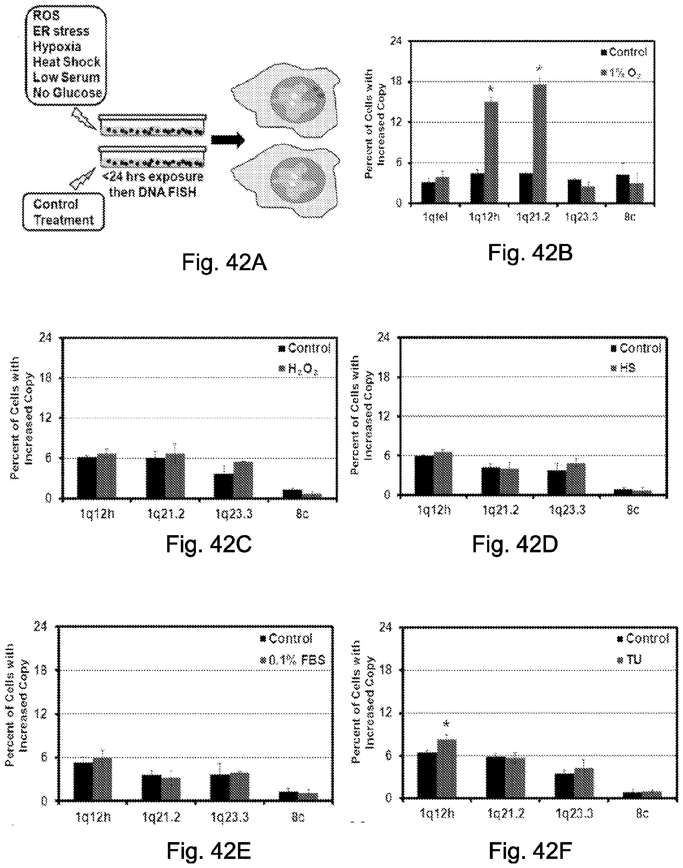

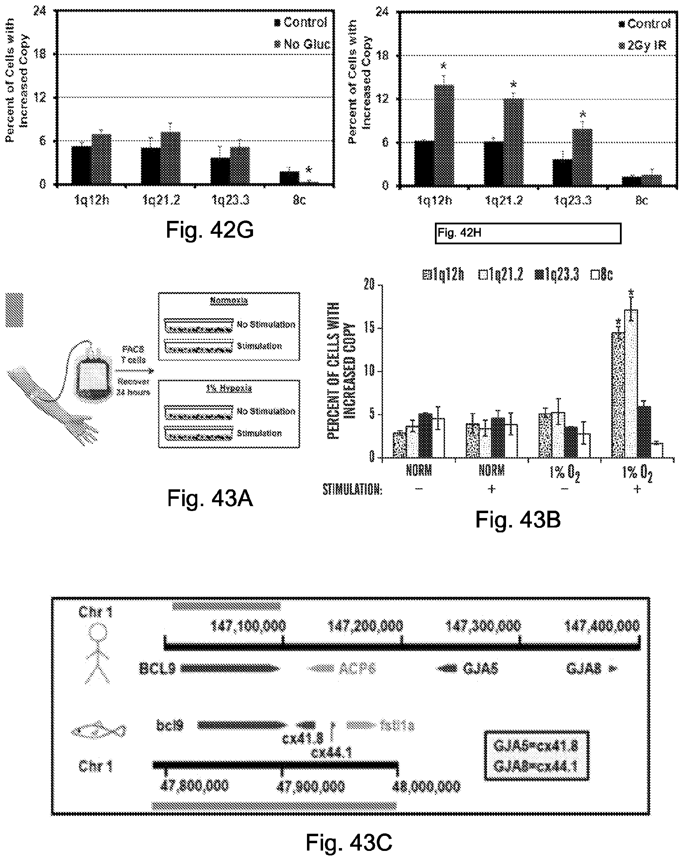

Figs. 42A-42H demonstrate that hypoxia, but not other physiological stresses promotes site-specific copy gain. Fig. 42A depicts a schematic detailing approach for screen of physiological stress. RPE cells were exposed to the indicated stress for 24 hours prior to collection for FISH and FACS analysis. Fig. 42B depicts a graph demonstrating that hypoxia promotes site-specific copy gain. Quantification of FISH for 1q12h, Chr 8, 1q23.3 and 1q21.2 after 24 hours of 21% O₂ or 1% O₂. Figs. 42C-42H demonstrate that treatment with chemical and metabolic stresses does not promote copy gain. Figs. 42C-42D depict quantification of FISH for 1q12h, Chr 8, 1q23.3 and 1q21.2 after 24 hours of ROS (H₂O₂) or 43°C heat shock (HS). Fig. 42E depicts quantification of FISH for 1q12h, Chr 8, 1q23.3 and 1q21.2 after 24 hours of reduced serum (0.1% FBS). Figs. 42F demonstrates that ER Stress does not promote copy gain. Quantification of FISH for 1q12h, Chr 8, 1q23.3 and 1q21.2 after 24 hours of Tunicamycin (TU) treatment. Figs. 42G-42H demonstrate that quantification of FISH for 1q12h, Chr 8, 1q23.3 and 1q21.2 24 hours after glucose deprivation (No Gluc) or 2Gy gamma irradiation (2Gy IR).

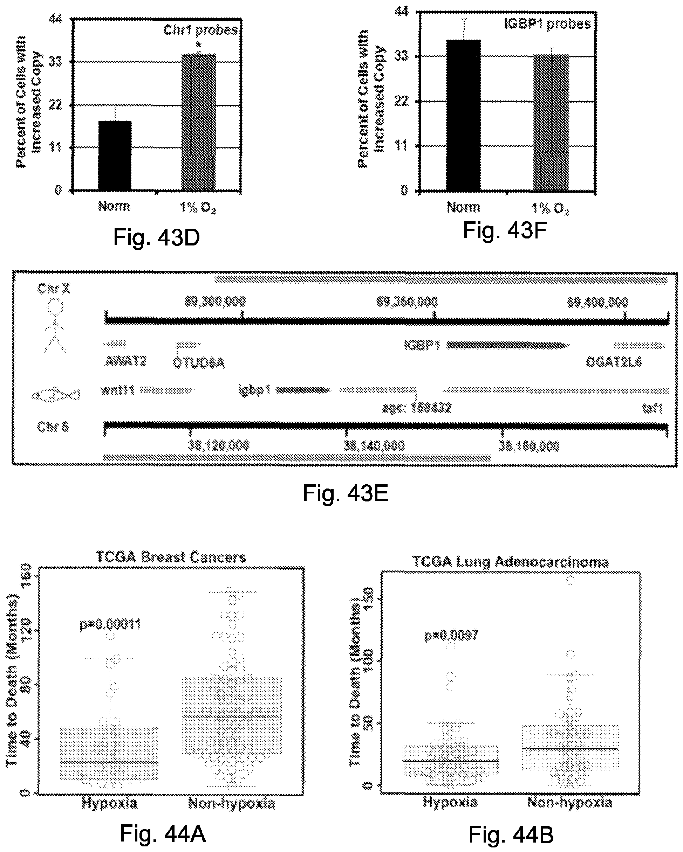

Figs. 43A-43F demonstrate that hypoxia induces site-specific copy gains in primary cells and is conserved in zebrafish. Fig. 43A depicts a schematic illustrating collection, isolation and stimulation of primary human T cells. Fig. 43B demonstrates that hypoxia induces site-specific copy gain only in stimulated primary human T cells. CD4+ T cells were isolated by flow cytometry and allowed to recover for 24 hours in 21% O₂, in the absence or presence of stimulation with IL2 and anti-CD3/CD28 antibodies. Following recovery T cells were exposed to 21% O₂ or 1% O₂ for 24 hours and assayed by FISH for copy gain. Fig. 43C depicts a schematic depicting syntenic region of 1q21.2 in zebrafish used for FISH analysis. Bars indicate the location of the human (stick figure) and zebrafish (fish icon) probes used. Fig. 43D demonstates that hypoxia promotes copy gain of Bc19 in zebrafish AB.9 cells. Quantification of FISH for Bcl9 after 72 hours of 21% O₂ or 1% O₂. Fig. 43E depicts a schematic of IGBP1 homologous region in zebrafish. Bars indicate the location of the human (stick figure) and zebrafish (fish icon) probes used. Fig. 43F deomostrates that hypoxia does not induce copy gain of IGBP1 in zebrafish. Quantification of FISH for IGBP1 after 72 hours of 21% O₂ or 1% O₂. Error bars represent the S.E.M. * indicates significant difference from control samples (P<0.05) by two-tailed students t-test.

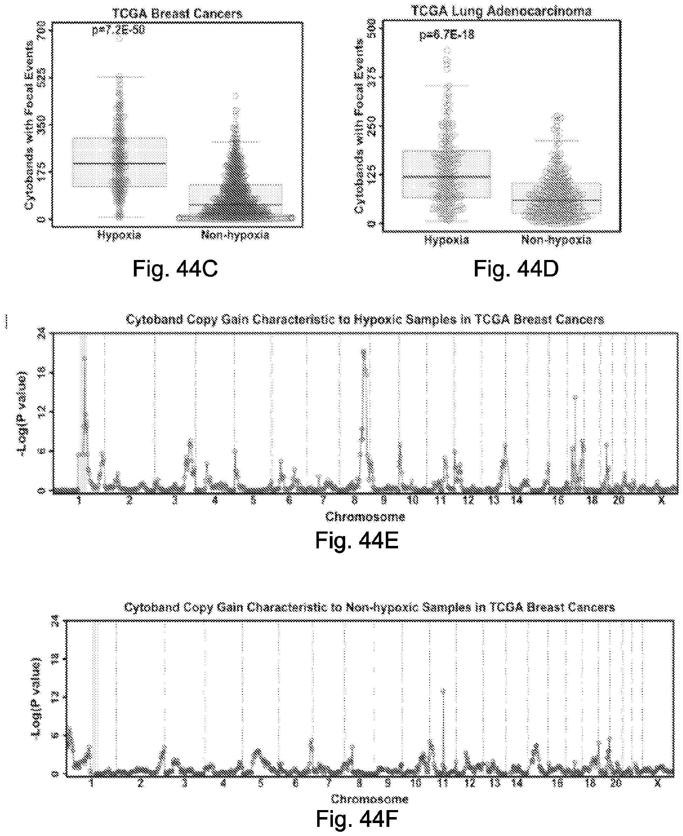

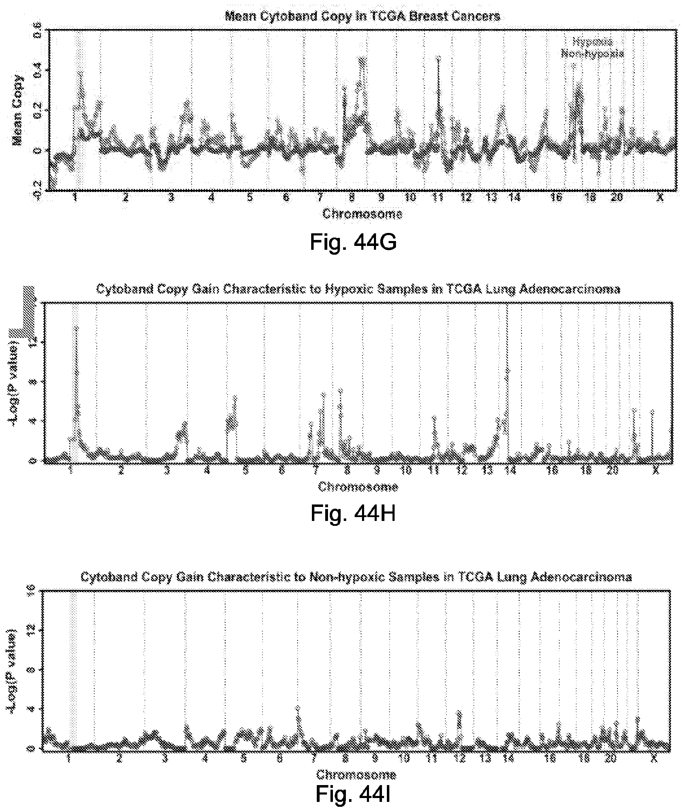

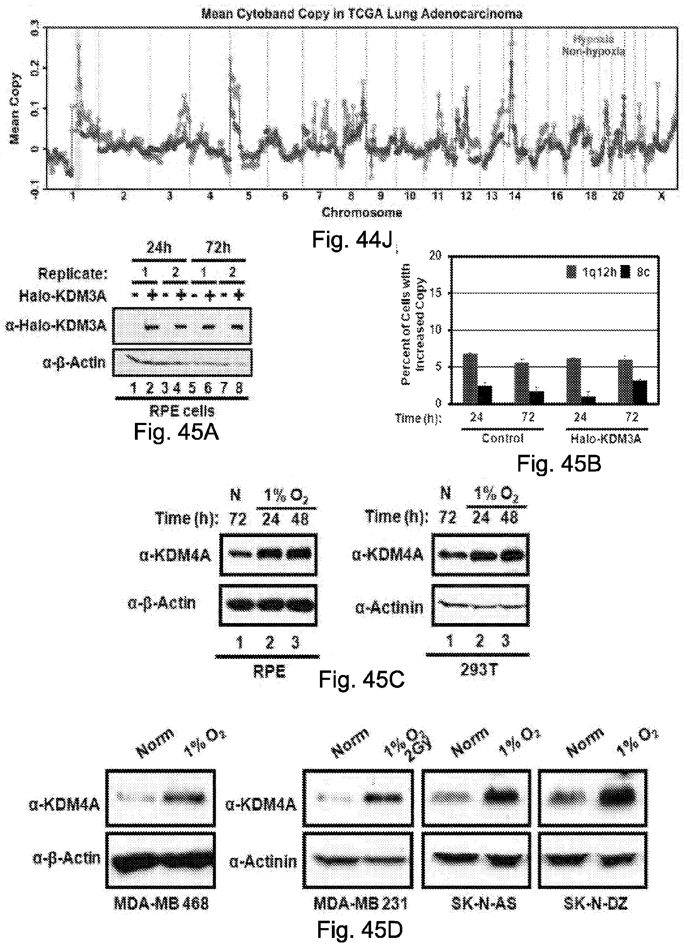

Figs. 44A 44J demonstrate that tumors with a hypoxic signature have copy gains of regions observed in hypoxic cell culture. Fig. 44A demonstrates that TCGA Breast Cancer samples with a hypoxic gene signature have a faster time to death. Fig. 44B demonstrates that TCGA Lung Adenocarcinoma samples with a hypoxic gene signature have a faster time to death. Fig. 44C demonstrates that TCGA Breast Cancer samples with a hypoxic gene signature have increased focal copy number variation. Fig. 44D demonstrates that TCGA Lung Adenocarcinoma samples with a hypoxic gene signature have increased focal copy number variation. Fig. 44E demonstrates that TCGA Breast Cancer samples with a hypoxic gene signature have increased copy of 1p11.2 through 1q23.3. Fig. 44F demonstrates that TCGA Breast Cancer samples without a hypoxic gene signature do not have amplification of 1p11.2 through 1q23.3. Fig. 44G demonstrates that mean copy number of hypoxic and non-hypoxic breast cancer samples. Fig. 44H demonstrates that TCGA Lung Adenocarcinoma samples with a hypoxic gene signature have increased copy of 1p11.2 through 1q23.3. Fig. 44I demonstrates that TCGA Lung Adenocarcinoma samples without a hypoxic gene signature do not have amplification of 1p11.2 through 1q23.3. Fig. 44J demonstrates that mean copy number of hypoxic and non-hypoxic lung adenocarcinoma samples. For each co-amplification plot, blue shaded regions indicate 1p11.2 through 1q23.3.

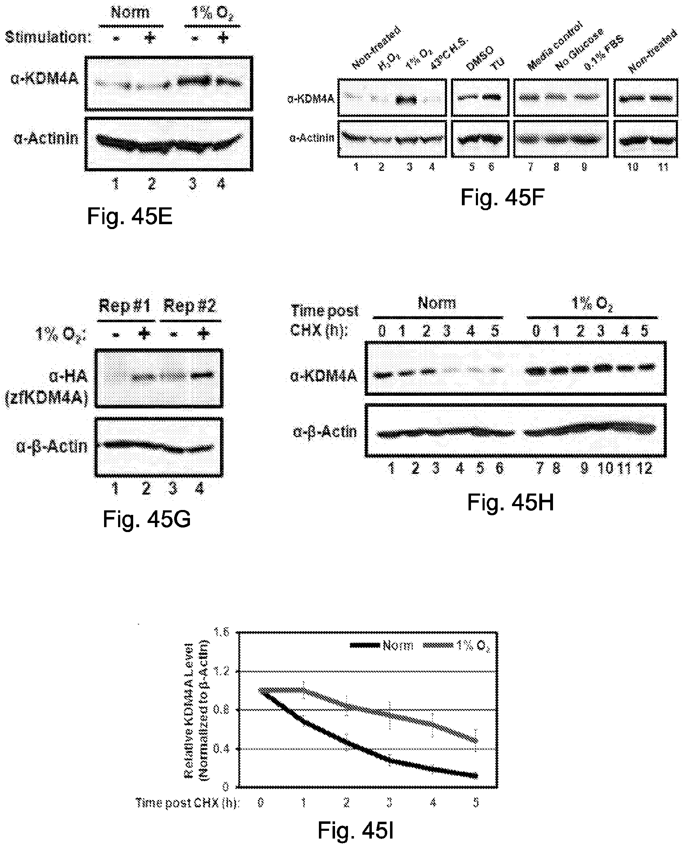

Figs. 45A-45I demonstrate that hypoxia stabilizes KDM4A protein levels. Figs. 45A-45B demonstrate that overexpression of KDM3A does not promote copy gain. Western blot depicting overexpression of Halo-KDM3A for 24 or 72 hours (Fig. 45A), which is insufficient to promote copy gain of 1q12h (Fig. 45B). Fig. 45C demonstrates that hypoxia stabilizes KDM4A protein levels in RPE cells (left) and 293T cells (right). Western blot indicates protein levels after 24 and 48 hours of hypoxic treatment. Fig. 45D demonstrates that hypoxia increases KDM4A protein levels in breast and neuroblastoma cells. Fig. 45E demonstrates that hypoxia induces KDM4A levels in primary human T cells. Fig. 45F demonstrates that hypoxia and TU treated cells exhibit increased KDM4A levels, but not in other stress conditions. Fig. 45G demonstrates that hypoxia increases zebrafish KDM4A levels (zfKDM4A) transfected into RPE cells. Fig. 45H demonstrates that hypoxia promotes stabilization of KDM4A protein levels in RPE cells. Half-life experiment demonstrates that KDM4A is stabilized in hypoxia following cyclohexamide treatment. Fig. 45I depicts a graphical representation of KDM4A half-life in RPE cells. Quantification of half-life indicates a half-life of 1hr 49min ± 3min in normoxia, and 4hr 56min ± 37min in hypoxia. Error bars represent the S.E.M. * indicates significant difference from control samples (P<0.05) by two-tailed students t-test.

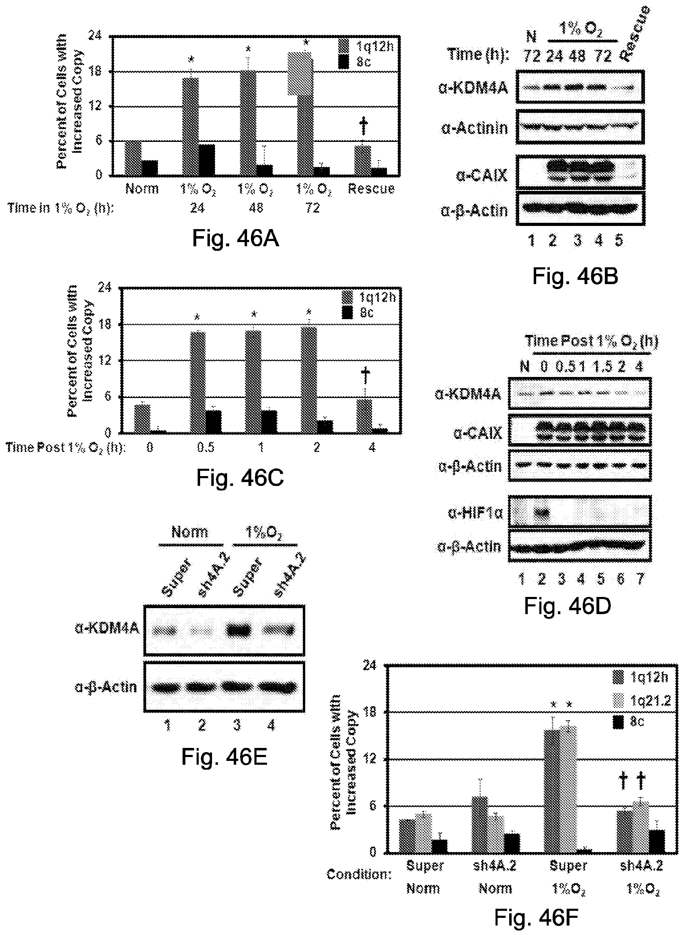

Figs. 46A-46F demonstrate that hypoxia-dependent copy gain is reversible and correlates with KDM4A protein levels. Fig. 46A demonstrates that hypoxia induces reversible copy gain of 1q12h. Quantification of FISH for 1q12h and Chr 8 after 24-72 hours of 21% O₂ (normoxia), 1% O₂ (hypoxia), or return to normoxia from 1% O₂ for 24 hours (Rescue). Fig. 46B demonstrates that KDM4A levels are increased in hypoxia but return to baseline when cells are returned to 21% O₂ (Rescue). Fig. 46C demonstrates that hypoxia-dependent copy gains are removed within 4 hours of return to 21%. Quantification of FISH for the indicated probes after 48 hours of normoxia or hypoxia treatment. Fig. 46D demonstrates that KDM4A levels return to baseline within 4 hours of return to 21% O₂. KDM4A levels were analyzed by western blot at the indicated times after a 48 hour 1% O₂ treatment. Fig. 46E demonstrates that Western blot depicting depletion of KDM4A under normoxic and hypoxic conditions. Fig. 46F demonstrates that hypoxia-induced 1q12h and 1q21.2 copy gains require KDM4A. Quantification of FISH for 1q12h, 1q21.2 and 8C in 293T cells after 24 hours of normoxia (21% O₂) or hypoxia (1% O₂) and with or without depletion of KDM4A. Error bars represent the S.E.M. * indicates significant difference from normoxia (21% O₂) (P<0.05) by two-tailed students t-test. † indicates significant difference from Super (1% O₂) (P<0.05) by two-tailed students t-test.

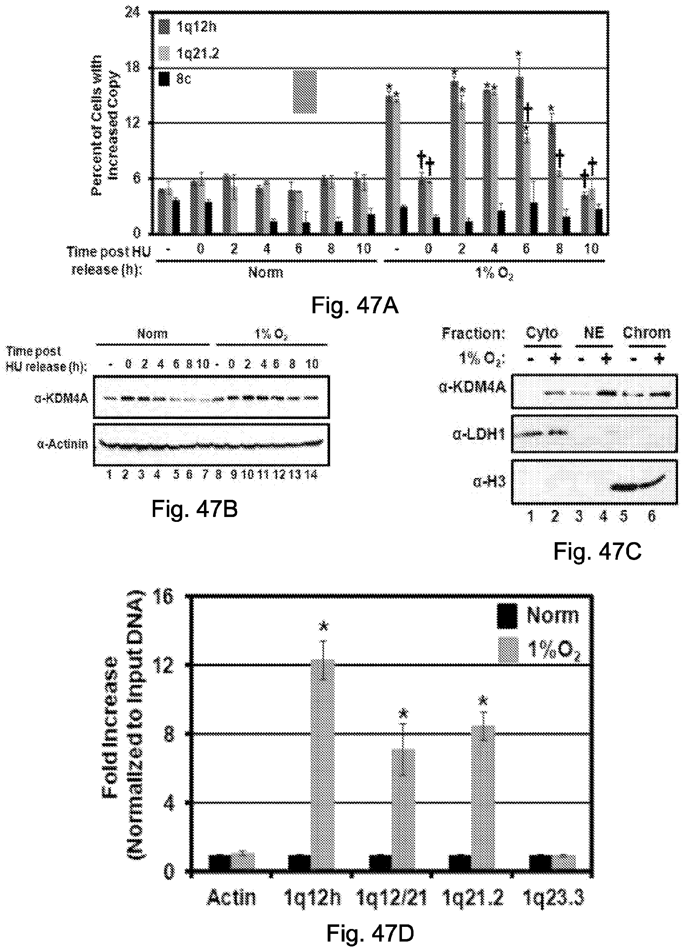

Figs. 47A-47D demonstrate that hypoxia-induced copy gains require S phase and are rereplicated. Fig. 47A demonstrates that hypoxia-induced copy gains occur during S phase. Quantification of FISH for 1q12h, 1q21.2 and 8C in RPE cells following HU arrest in 21% O₂ or 1% O₂ (time 0) or the indicated time after HU release. Fig. 47B depicts a Western blot depicting KDM4A levels in HU arrested and released cells in hypoxic and normoxic conditions. Fig. 47C demonstrates that KDM4A levels are increased on chromatin during hypoxia (1% O₂). Fig. 47D demonstrates that regions with hypoxia-dependent copy gain are rereplicated. CsCl density gradient purification of rereplicated DNA was analyzed by qPCR for regions amplified in hypoxia. Error bars represent the S.E.M. * indicates significant difference from normoxia (P<0.05) by two-tailed students t-test. † indicates significant difference from Asynchronous (-) 1% O₂ (P<0.05) by two-tailed students t-test.

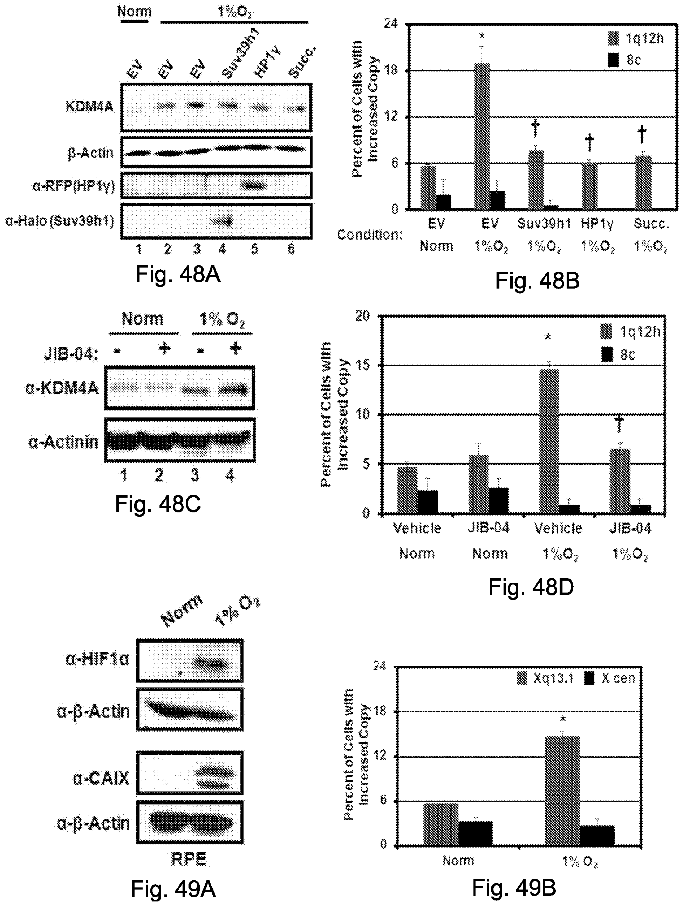

Figs. 48A-48D demonstrate inhibition of hypoxia-dependent copy gains. Fig. 48A depicts a Western blot depicting overexpression of HP1γ or SUV39h1. Fig. 48B demonstrates that hypoxia-dependent copy gains can be suppressed by overexpression of HPly or SUV39h1 or treatment with 2mM succinate. Quantification of FISH for 1q12h and Chr 8 in RPE cells after 24 hours of normoxia or hypoxia and with or without expression of HP1γ or SUV39h1 or treatment with 2mM succinate. Fig. 48C depicts a Western blot depicting KDM4A levels in normoxia and hypoxia, with or without JIB-04 treatment. Fig. 48D demonstrates quantification of FISH for 1q12h and Chr 8 in RPE cells upon JIB-04 treatment. RPE cells were pre-treated with vehicle or JIB-04 for 24 hours in normoxia, and maintained in normoxia or hypoxia for an additional 24 hours. Error bars represent the S.E.M. In all panels, * indicates significant difference from control samples (P<0.05) by two-tailed students t-test. † indicates significant difference from EV (1% O₂) in Fig. 48B and significant difference from Vehicle (1% O₂) in Fig. 48D, (P<0.05) by two-tailed students t-test.

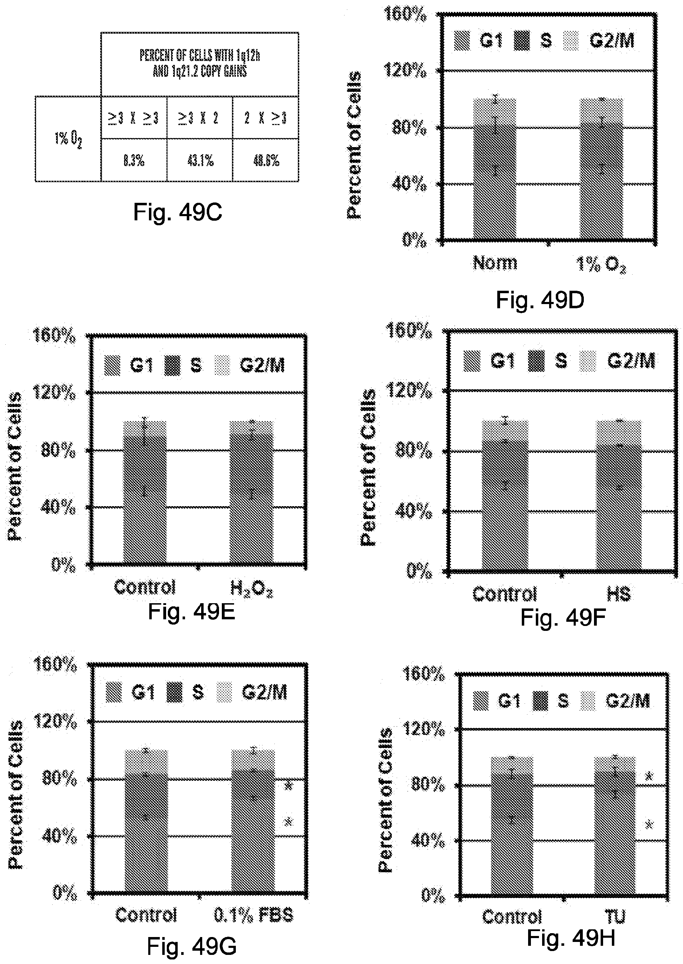

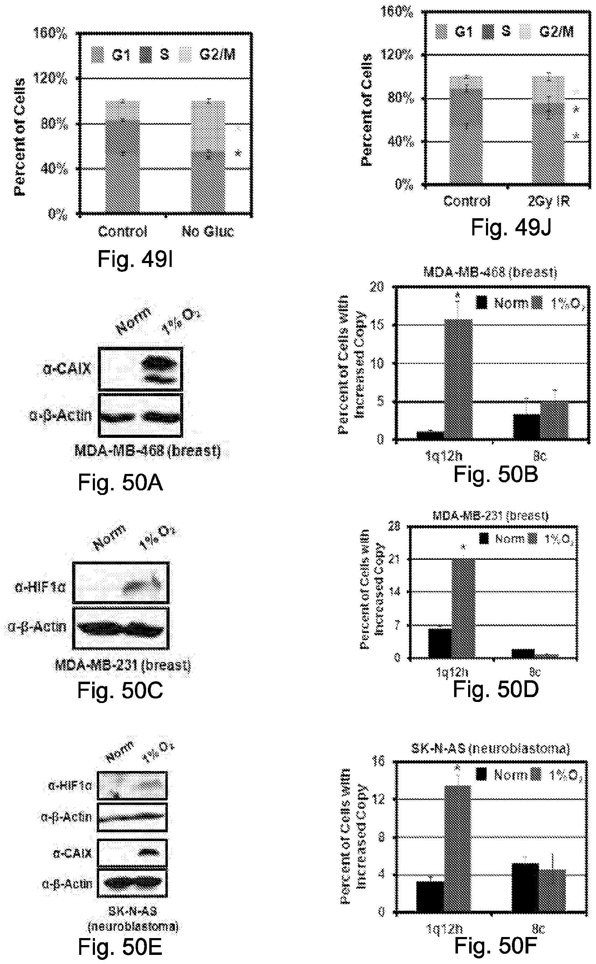

Figs. 49A-49J demonstrate that hypoxia induces mutually exclusive copy gains without changes in cell cycle profile. Fig. 49A demonstrates that hypoxic conditions increase HIF1α and CAIX levels in RPE cells. Western blot indicating protein levels of HIF1α and CAIX in normoxia (Norm, 21% O₂) or following 24 hours in hypoxia (1% O₂). Fig. 49B demonstrates that hypoxia induces copy gain of Xq13.1 but not the X centromere (X cen). FISH analysis of X centromere and Xq13.1 copy number. Fig. 49C demonstrates that hypoxia amplified regions are not contiguous. Table summarizing co-amplification of 1q12h, and 1q21.2. Data are presented as percent of all amplified cells (sum of all replicates) having 2 or 3 or more (3+) copies of the indicated FISH probes. Fig. 49D-49J depict cell cycle analyses following 24 hours exposure to the indicated stresses. Error bars represent the S.E.M. * indicates significant difference from control samples (P<0.05) by two-tailed students t-test. * adjacent to bar graphs for cell cycle distribution indicate P<0.05 compared to control samples for that cell cycle phase.

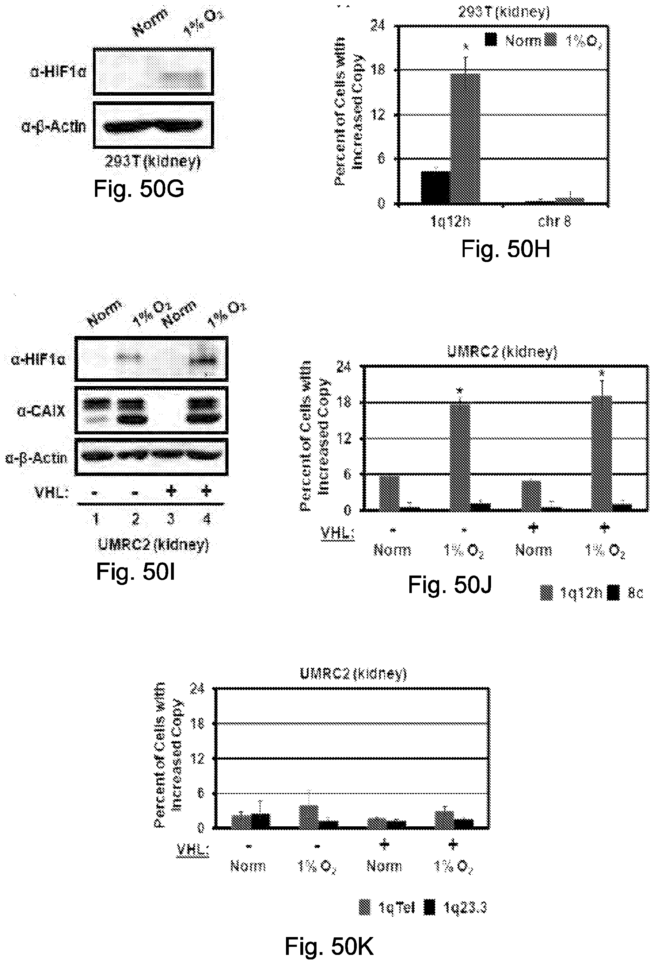

Figs. 50A-50K demonstrate that hypoxia-induced copy gains are observed across disparate cell types. Figs. 50A-50D demonstrate that hypoxia promotes site-specific gains in breast cancer cell lines. Western blots depict the hypoxic response of MDA-MB 468 (Fig. 50A) and MDA-MB 231 (fig. 50C) cells following 24 hours of hypoxic exposure. Quantification of FISH indicates amplification of 1q12h, but not 8C in hypoxic MDA-MB 468 (Fig. 50B) and MDA-MB 231 (Fig. 50D) cells. Figs. 50E-50H demonstrate that SK-N-AS neuroblastoma (Figs. 50E-50F) and 293T kidney (Figs. 50G-50H) cell lines are hypoxic and exhibit copy gain of 1q12h following 24 hours of 1% O₂. Figs. 50I-50K demonstrate that hypoxia promotes site-specific gain in renal cancer cells independent of activated HIF (UMRC2 - lack VHL and have constitutively active HIF). Quantification of FISH for 1q12h and 8c (Fig. 50J) and 1q23.3 and 1qtel (Fig. 50K), after 24 hours of 21% O₂ or 1% O₂. Error bars represent the S.E.M. * indicates significant difference from control samples (P<0.05) by two-tailed students t-test.

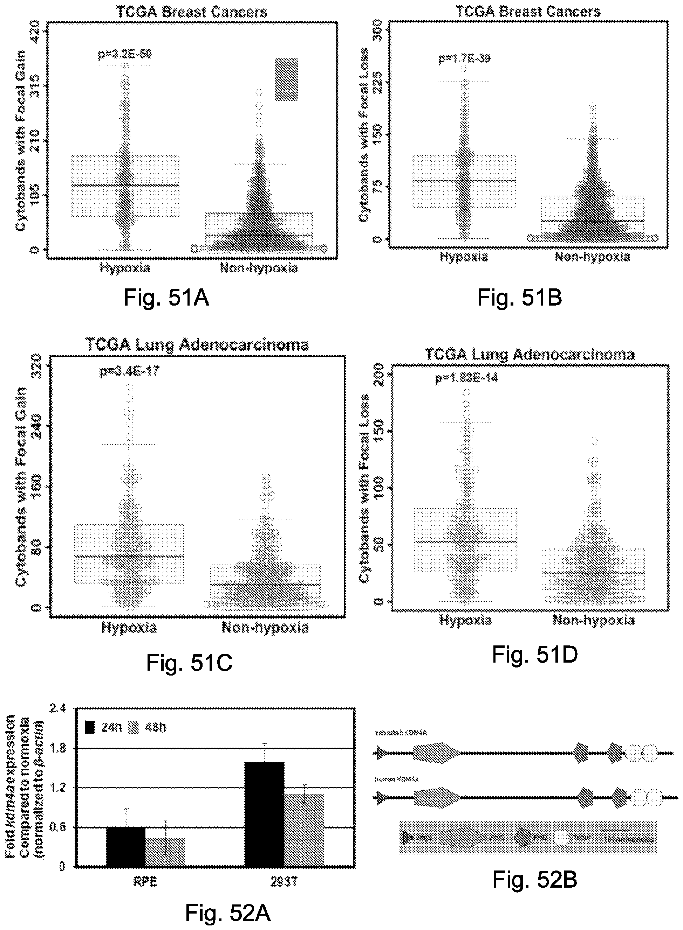

Figs. 51A-51D demonstrate that brca and luad primary tumors have increased focal amplications and focal deletions. Fig. 51A demonstrates that TCGA Breast Cancer samples with a hypoxic gene signature have increased focal copy number gain. Fig. 51B demonstrates that TCGA Breast Cancer samples with a hypoxic gene signature have increased focal copy number loss. Fig. 51C demonstrates that TCGA lung adenocarcinoma samples with a hypoxic gene signature have increased focal copy number gain. Fig. 51D demonstrates that TCGA lung adenocarcinoma samples with a hypoxic gene signature have increased focal copy number loss.

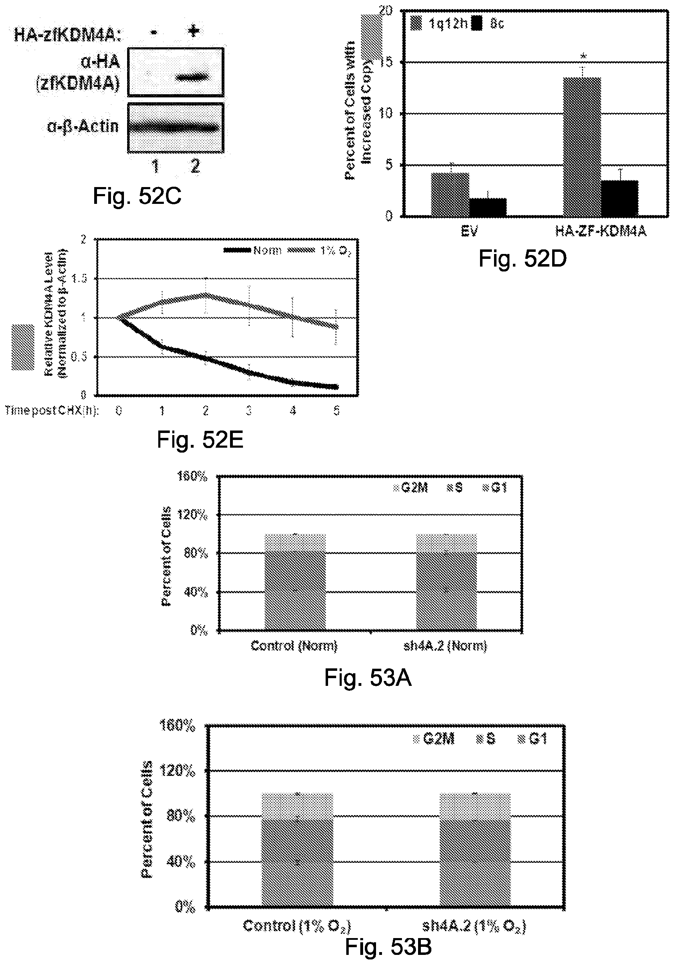

Figs. 52A-52E demonstrate that hypoxia stabilizes kdm4a protein levels and zebrafish kdm4a promotes copy gain. Fig. 52A demonstrates that KDM4A transcript levels do not correlate with increased protein observed in hypoxia. KDM4A mRNA levels were analyzed by qPCR and normalized to β-actin. Fig. 52B depicts a schematic depicting the structural homology between the zebrafish and human KDM4A proteins. Figs. 52C-52D demonstrate that Zebrafish KDM4A retains a degree of demethylase activity under hypoxic conditions. RPE cells were transfected with HA-zfKDM4A for 24 hours and subsequently maintained in 1% O₂ for 24 hours. Fig. 52E demonstrates that hypoxia increases the half-life of KDM4A in 293T cells. Quantification of half-life indicates a half-life of 1hr 51min ± 28min in normoxia, and 6hr 13min ± 10min in hypoxia. Error bars represent the S.E.M. * indicates significant difference from control samples (P<0.05) by two-tailed students t-test.

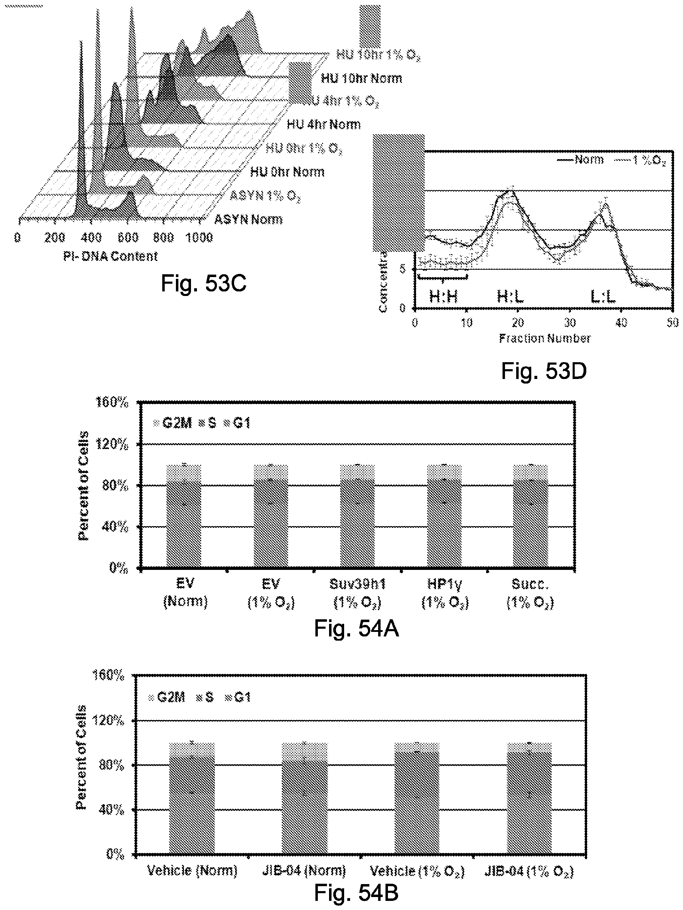

Figs. 53A-53D demonstrate that cell cycle and cscl density profiles for normoxic and hypoxic cells. Figs. 53A-53B depict cell cycle profile following depletion of KDM4A in normoxia (Fig. 53A) and in hypoxia (fig. 53B). Fig. 53C depicts a representative FACS analysis demonstrating cell cycle progression through HU release in normoxia and hypoxia. Cell cycle profiles are provided for asynchronous (Asyn), HU arrested (0hr), and released (4hr and 10hr) cells at 21% O₂ (norm) and 1% O₂. Fig. 53D depicts aCsCl density gradient profile from normoxia and hypoxia samples used in the rereplication experiment. Positions of the light:light (L:L; no replication) heavy:light (H:L; normal replication) and heavy:heavy (H:H; rereplicated) are indicated. Error bars represent the S.E.M. of the 3 biological replicates used.
Figs. 54A-54B demonstrate that inhibition of hypoxia-dependent copy gains are not associated with cell cycle arrest. Fig. 54A depicts a cell cycle profile following overexpression of SUV39h1, HP1γ or 2mM succinate treatment. Fig. 54B depicts cell Cycle profile following JIB-04 treatment. Error bars represent the S.E.M.

### DETAILED DESCRIPTION

It is demonstrated herein that KDM4A, a H3K9/36me3 lysine demethylase, is amplified and overexpressed in several tumor types, leading to copy gain of specific chromosomal domains. The level of activity of KDM4A has important effects on the behavior of cancer cells, influencing whether a cell will be sensitive or resistant to a number of chemotherapies and whether a tumor is likely to be abnormally regressive and/or resistant to therapy. Accordingly, described herein are methods of more effectively and safely administering chemotherapies to subjects, as well as methods of treatment that comprise modulating the level of KDM4A activity.

As described herein, "KDM4A," "Lysine-specific demethylase 4A," or "JMJD2A" refers to a H3K9/36me3 lysine demethylase of the Jumonji domain 2 (JMJD2) family which converts specific trimethylated histone residues to the dimethylated form. KDM4A encodes a polypeptide having a JmjN domain, JmjC domain, two TUDOR domains, and two PHD-type zinc fingers. The sequence of KDM4A for a number of species is well known in the art, e.g., human KDM4A (e.g. NCBI Gene ID: 9682; (mRNA: SEQ ID NO: 1, NCBI Ref Seq: NM_014663)(polypeptide: SEQ ID NO: 2, NCBI Ref Seq:NP_055478).

KDM4A activity refers to the removal of a methyl from a trimethylated target histone to produce a dimethylated histone. Assays for measuring the activity of KDM4A are known in the art. Non-limiting examples of assays for KDM4A activity can include, MALDI-TOF spectrometry, and immunblotting or immunofluorescence microscopy with antibodies specific for tri and dimethylated histone targets, e.g. as described in Whetstine et al. Cell 2006 3:467-481.

The inventor has determined that cells with an increased level of KDM4A activity and/or expression (including cells having KDM4A gene amplification) are resistant to S-hase chemotherapeutics. Accordingly, subjects with increased levels of KDM4A (e.g. higher than normal KDM4A gene expression and/or KDM4A gene amplification) are not likely to benefit from administration of an S-phase chemotherapeutic, while subjects not having such increased levels can advantageously be administered an S-phase chemotherapeutic.

Accordingly, in one aspect, described herein is a method of treating cancer, the method comprising administering an S-phase chemotherapeutic to a subject determined to have a level of KDM4A gene expression which is not higher than a reference level or determined not to have KDM4A gene amplification and not administering an S-phase chemotherapeutic to a subject determined to have a level of KDM4A gene expression which is higher than a reference level or determined to have KDM4A gene amplification. As used herein, an "S-phase chemotherapeutic" refers to a chemotherapeutic agent that inhibits a cell from successfully progressing through S-phase, e.g. inhibits DNA replication. In some embodiments, S-phase chemotherapeutics can prevent the production and/or transport of metabolites needed for DNA replication and/or synthesis or inhibit the activity of enzymes involved in the production or transport of such metabolites and/or enzymes involved in the replication and/or synthesis of DNA or RNA. Non-limiting examples of S-phase chemotherapeutics can include cisplatin; 5-flurouracil; 6-mercaptopurine; capecitabine; cladribine; clorfarabine; cytarabine; doxorubicin; fludarabine; floxuridine; gemcitabine; hydroxyurea; methotrexate; pemetrexed; pentostatin; prednisone; procarbazine; and thioguanine.

The inventor has also determined the effects of KDM4A dampening mutations on the behavior of cancer cells and the response to therapeutic agents. As used herein, "KDM4A dampening mutations" refers to a mutation, either in KDM4A or elsewhere in the genome, transcriptome, or proteome, which results in a decreased level of KDM4A activity and/or expression. In some embodiments, a decreased level of KDM4A activity and/or expression is a level which is statistically significantly lower than a reference level. In some embodiments, a decreased level of KDM4A activity and/or expression is a level which is 50% or less of a reference level, e.g. 50% or less, 40% or less, 30% or less, 20% or less, or 10% or less than a reference level.

Non-limiting examples of KDM4A dampening mutations can include a mutation that decreases KDM4A enzymatic activity; a mutation that increases the proportion or level of KDM4A that is located in the cytoplasm as opposed to the nucleus; or a mutation that increases the turnover rate of KDM4A polypeptide. In some embodiments, a KDM4A dampening mutation is a mutation that increases the proportion or level of KDM4A that is located in the cytoplasm as opposed to the nucleus. Methods of measuring the location and/or turnover of KDM4A polypeptide are known in the art, e.g. immunochemical methods described elsewhere herein, e.g. immunohistochemistry.

In some embodiments, the KDM4A dampening mutation can be a mutation of KDM4A which alters the sequence of the KDM4A polypeptide, e.g. a mutation that alters the sequence of the KDM4A polypeptide relative to SEQ ID NO: 2 and/or that alters the sequence of the KDM4A polypeptide relative to the KDM4A polypeptide encoded by healthy, non-tumor cells of the same subject. In some embodiments, the KDM4A dampening mutation can be a polymorphism. In some embodiments, the KDM4A dampening mutation can be a mutation of KDM4 selected from the group consisting of E23K; S28N; I87V; E113K; K123I; N128S; R152W; R218W; G225C; A235V; R239H; G278S; T289I; V319M; P326T; P348L; E368K; G376V; R400Q; E426K; E482A; V490M; R498H; D524V; E558Q; R597H; A662S; S713L; V743I; R765Q; G783FS; L803GS; R825C; R825H; V919M; L941F; S948T; V1003A; D1023Y; R1025C; and E1032K. In some embodiments, the KDM4A dampening mutation can be, e.g. E482A (rs586339) or a SNP selected from Table 7.

In some embodiments, the KDM4A dampening mutation can comprise the loss of the kdm4a allele.

In some embodiments, the subject can be determined to be homozygous for the KDM4A dampening mutation. In some embodiments, the subject can be determined to have two alleles of KDM4A comprising and/or affected by an KDM4A dampening mutation - e.g., the subject is homozygous for one or more KDM4A dampening mutations or each allele comprises separate KDM4A dampening mutations.

A KDM4A dampening mutation can be detected, e.g. the presence of a KDM4A dampening mutation can be determined using any assay known in the art, including, but not limited to hybridization; sequencing; exome capture; PCR; RFLP; high-throughput sequencing; and KDM4A immunochemical detection methods. In some embodiments, the KDM4A dampening mutation can be present, or can be detected, in the genomic DNA of a tumor cell. In some embodiments, the KDM4A dampening mutation can be present, or can be detected, in the mRNA transcripts of a tumor cell. In some embodiments, the KDM4A dampening mutation can be present, or can be detected, in the polypeptides of a tumor cell. In some embodiments, the cell and/or subject can be homozygous for a given KDM4A dampening mutation or heterozygous for a given KDM4A dampening mutation.

In some embodiments, a KDM4A dampening mutation can comprise a mutation of IDH that results in increased 2-HG production. Such IDH mutations are known in the art and are discussed, e.g., in Dang et al. Nature 2010 465:966; Lu et al. Nature 2012 483:474-8; Ward et al. 2010 Cancer Cell 17:225-234; Yen et al. 2010 29:6409-6417; Chowdhury et al. 2011 EMBO Reports 12:463-9; and US Patent Publications US2012/0121515; US2011/0229479; US2012/0202207; and US2013/0035329; and International Patent Applications PCT/US2010/053623; PCT/US2011/05615; and PCT/US2012/054145.

As described herein, "IDH" or "isocitrate dehydrogenase" refers to an enzyme that catalyzes the decarboxylation of isocitrate to yield alpha-ketoglutarate and carbon dioxide. Three isoforms of IDH exist in humans, IDH1, IDH2, and IDH. As used herein, "IDH" can refer to any of the isoforms of IDH. The sequence of IDH for a number of species is well known in the art, e.g., human IDH1 (e.g. NCBI Gene ID: 3417; (mRNA: SEQ ID NO: 3, NCBI Ref Seq: NM_NM_005896)(polypeptide: SEQ ID NO: 4, NCBI Ref Seq:NP_005887).

In some embodiments, a mutation of IDH that results in increased 2-HG production can be present in a cancer selected from the group consisting of chondrosarcoma; glioblastoma, glioblastoma multiforme (GBM); leukemia, and acute myeloid leukemia (AML).

In some embodiments, a KDM4A dampening mutation can comprise a mutation that reduces the level and/or activity of succinate dehydrogenase, e.g. in increased levels of succinate. Such SDH mutations are known in the art and are discussed, e.g., Brandon et al. Oncogene 2006 25:4647-4662; Bayley et al. BMC Medical Genetics 2005 6:39; and Bardella et al. BBA-Bioenergetics 2011 1807:1432-1443.

As described herein, "SDH" or "succienate dehydrogenase" refers to an enzyme complex that catalyzes the oxidation of succinate to fumarate. SDH is comprised of four subunits, SdhA, SdhB, SdhC, and SdhD. A mutation of SDH can be a mutation in any one of or a combination of the four subunits. The sequence of SDH subunits for a number of species is well known in the art, e.g., human SdhA (e.g. NCBI Gene ID: 6389; (mRNA: SEQ ID NO: 13, NCBI Ref Seq: NM_004168)(polypeptide: SEQ ID NO: 14, NCBI Ref Seq:NP_004159), human SdhB (e.g. NCBI Gene ID: 6390; (mRNA: SEQ ID NO: 15, NCBI Ref Seq: NM_003000)(polypeptide: SEQ ID NO: 16, NCBI Ref Seq:NP_002991), human SdhC (e.g. NCBI Gene ID: 6391; (mRNA: SEQ ID NO: 17, NCBI Ref Seq: NM_003001)(polypeptide: SEQ ID NO: 18, NCBI Ref Seq:NP_002992), and human SdhD (e.g. NCBI Gene ID: 6392; (mRNA: SEQ ID NO: 19, NCBI Ref Seq: NM_003002)(polypeptide: SEQ ID NO: 20, NCBI Ref Seq:NP_002993).

In some embodiments, a KDM4A dampening mutation can be a mutation in a histone that prevents KDM4A from binding to a substrate, e.g. H3.1, H3.3, G34V, G34R, and/or G35 mutations. In some embodiments, a KDM4A dampening mutation can be a mutation that alters KDM4A recruitment through the PHD or Tudor domains, e.g. H3K4 or H4K20 mutations.

The presence of KDM4A dampening mutations in a cell can increase or decrease the sensitivity of the cell to certain chemotherapeutic agents. In one aspect, provided herein is a method of treating cancer, the method comprising administering a chemotherapeutic selected from the group consisting of mTOR inhibitors; protein synthesis inhibitors; Braf inhibitors; PI3K inhibitors; Cdk inhibitors; Aurora B inhibitors; FLT3 inhibitors; PLK1/2/3 inhibitors; Eg5 inhibitors; β-tubulin inhibitors; BMP inhibitors; HDAC inhibitors; Akt inhibitors; IGF1R inhibitors; p53 inhibitors; hdm2 inhibitors; STAT3 inhibitors; and VEGFR inhibitors and not administering a chemotherapeutic selected from the group consisting of EGFR inhibitors; ErbB2 inhibitors; transcription inhibitors; and MEK1/2 inhibitors to a subject determined to have a KDM4A dampening mutation. In some embodiments, the KDM4A dampening mutation is present in the tumor, but not the non-tumor cells of the subject.

In some embodiments, subjects can have KDM4A dampening mutations in the germline and/or non-cancerous somatic cells. Such mutations in non-cancerous cells can cause a subject to be more likely to exhibit side effects from certain treatments, i.e. treatments involving administration of agents which cells with a KDM4A dampening mutation are more sensitive to. In some embodiments of any of the aspects of methods of treatment described herein, the subject having a KDM4A dampening mutation has more KDM4A dampening mutations in the cancer cells than in the non-cancerous cells, e.g. the cancer cells have acquired somatic KDM4A dampening mutations. In some embodiments of any of the aspects of methods of treatment described herein, the subject having a KDM4A dampening mutation has more KDM4A dampening mutations in the cancer cells than in the non-cancerous cells, e.g. the cancer cells have acquired somatic KDM4A dampening mutations, and the subject further has one or more germline KDM4A dampening mutations, e.g. a subset of the KDM4A dampening mutations present in the cancer cells are also present in non-cancerous cells of the subject.

In one aspect, described herein is a method of treating cancer, the method comprising administering a reduced dose of a chemotherapeutic agent selected from the group consisting of mTOR inhibitors; protein synthesis inhibitors; Braf inhibitors; PI3K inhibitors; Cdk inhibitors; Aurora B inhibitors; FLT3 inhibitors; PLK1/2/3 inhibitors; Eg5 inhibitors; β-tubulin inhibitors; BMP inhibitors; HDAC inhibitors; Akt inhibitors; IGF1R inhibitors; p53 inhibitors; hdm2 inhibitors; STAT3 inhibitors; and VEGFR inhibitors, to a subject determined to have a KDM4A dampening mutation in non-tumor cells. In one aspect, described herein is a method comprising not administering a chemotherapeutic agent selected from the group consisting of mTOR inhibitors; protein synthesis inhibitors; Braf inhibitors; PI3K inhibitors; Cdk inhibitors; Aurora B inhibitors; FLT3 inhibitors; PLK1/2/3 inhibitors; Eg5 inhibitors; β-tubulin inhibitors; BMP inhibitors; HDAC inhibitors; Akt inhibitors; IGF1R inhibitors; p53 inhibitors; hdm2 inhibitors; STAT3 inhibitors; and VEGFR inhibitors, to a subject determined to have a KDM4A dampening mutation in non-tumor cells. In one aspect, described herein is a method of treating cancer comprising administering a chemotherapeutic agent selected from the group consisting of mTOR inhibitors; protein synthesis inhibitors; Braf inhibitors; PI3K inhibitors; Cdk inhibitors; Aurora B inhibitors; FLT3 inhibitors; PLK1/2/3 inhibitors; Eg5 inhibitors; β-tubulin inhibitors; BMP inhibitors; HDAC inhibitors; Akt inhibitors; IGF1R inhibitors; p53 inhibitors; hdm2 inhibitors; STAT3 inhibitors; and VEGFR inhibitors, to a subject determined not to have a KDM4A dampening mutation in non-tumor cells. In one aspect, described herein is a method of treating cancer, the method comprising administering a chemotherapeutic agent selected from the group consisting of mTOR inhibitors; protein synthesis inhibitors; Braf inhibitors; PI3K inhibitors; Cdk inhibitors; Aurora B inhibitors; FLT3 inhibitors; PLK1/2/3 inhibitors; Eg5 inhibitors; β-tubulin inhibitors; BMP inhibitors; HDAC inhibitors; Akt inhibitors; IGF1R inhibitors; p53 inhibitors; hdm2 inhibitors; STAT3 inhibitors; and VEGFR inhibitors, to a subject determined to have a KDM4A dampening mutation in non-tumor cells and monitoring the subject for side effects, e.g. monitoring the subject more closely than normal for side effects (e.g. testing for markers of side effects more frequently, keeping the subject under direct observation, etc).

Protein synthesis inhibitors include agents that can reduce or inhibit the synthesis of one more polypeptides. Non-limiting examples of protein synthesis inhibitors can include cycloheximide, phyllanthoside, nagilactone D, bactobolin, SUN2071, undulatone, SUN0237, septacidin, holacanthone, acivicin, bisantrene, chromomycin A3, deoxydoxorubicin, daunorubicin, doxorubicin, mithramycin, enchinomycin, dactinomycin, olivomycin, oxanthrazole, ellipticine, etoposide, teniposide, vincristine, vinblastine, paclitaxel, maytansine, rhizoxin, macbecin II, fluorpan, tetrocarcin A, and carmustin. Non-limiting examples of mTOR inhibitors can include rapamycin, everolimus, temsirolimus, AZD8055, AP-23573, AP-23481, LY294002, wortmannin, BEZ-235, BKM-120, BGT-226, and the like. Non-limiting examples of Braf inhibitors can include PLX-4032, GSK21 18436, PLX-3603. Non-limiting examples of PI3K inhibitors can include LY294002, CUDC-907, wortmannin, BEZ-235, BKM-120, and BGT-226. Non-limiting examples of Cdk inhibitors can include dinacinib, AT7519, falvopirodol, roscovitine, SNS-032, JNJ-7706621, BS-181, SCH 900776, PHA-793887, AZD5438, PHA-767491, R547, BMS-265246, and palbociclib. Non-limiting examples of Aurora B inhibitors can include ENMD-2076, TAK 901, AMG 900, SNS-314, PHA-680632 and barasertib. Non-limiting examples of FLT3 inhibitors can include quizartinib, dovitinib, TG101209, KW 2449, amuvatinib, tandutinib, and TCS 359. Non-limiting examples of PLK1/2/3 inhibitors can include BI 2536, volasertib, rigosertib, GSK461364, HMN-214, and MLN0905. Non-limiting examples of Eg5 inhibitors can include monastrol, s-trityl-1-cysteine, HR22C16, and CK0106023. Non-limiting examples of β-tubulin inhibitors can include ABT-751, vinblastine, vincristine, vinorelbine, vinflunine, cryptophycin 52, halichondrins, dolastatins, hemiasterlins, colchicines, combrestatin, 2-methoxy-estradiol, E7010, paclitaxel, docetaxel, epothilon, and doscodermolide. Non-limiting examples of BMP inhibitors can include LDN-193189 and DMH1. Non-limiting examples of HDAC inhibitors can include sodium butyrate, suberanoyl hydroxamic acid (SAHA, vorinostat), CUDC-907, LBH-589, valproic acid, and MS-275. Non-limiting examples of Akt inhibitors can include AT7867, KRX-0401, and MK-2206. Non-limiting examples of IGF1R inhibitors can include picropodophyllin, AG538, AG1024, NVP-AEW541, figitumumab, linsitinib, and GSK1904529. Non-limiting examples of p53 inhibitors can include pifithrin-alpha. Non-limiting examples of hdm2 inhibitors can include CAS 414905-09-02 (cat No. Sc-221707 from Santa Cruz Biotechnology; Dallas TX). Non-limiting examples of STAT3 inhibitors can include S3I-M2001, cucurbitacin, niclosamide, cyrptotanshinone, DS 1008, WP1066, Stattic, S31-201, andkahweol. Non-limiting examples of VEGFR or VEGF inhibitors can include bevacizumab, sunitinib, or sorafenib.

As described herein, KDM4A dampening mutations, e.g. mutations which decrease the activity of KDM4A sensitize cells to the effects of certain chemotherapeutic agents. KDM4A levels and/or activity can also be lowered by administering a KDM4A inhibitor. Accordingly, in one aspect, described herein is a method of treating cancer, the method comprising administering an inhibitor of KDM4A; and administering a chemotherapeutic agent selected from the group consisting of S-phase chemotherapeutics; mTOR inhibitors; protein synthesis inhibitors; Braf inhibitors; PI3K inhibitors; Cdk inhibitors; Aurora B inhibitors; FLT3 inhibitors; PLK1/2/3 inhibitors; Eg5 inhibitors; β-tubulin inhibitors; BMP inhibitors; HDAC inhibitors; Akt inhibitors; IGF1R inhibitors; p53 inhibitors; hdm2 inhibitors; STAT3 inhibitors; and VEGFR inhibitors.

As used herein, the term "inhibitor" refers to an agent which can decrease the expression and/or activity of the targeted expression product (e.g. mRNA encoding the target or a target polypeptide), e.g. by at least 10% or more, e.g. by 10% or more, 50% or more, 70% or more, 80% or more, 90% or more, 95% or more, or 98 % or more. The efficacy of an inhibitor of, for example, KDM4A, e.g. its ability to decrease the level and/or activity of KDM4A can be determined, e.g. by measuring the level of an expression product of KDM4A and/or the activity of KDM4A. Methods for measuring the level of a given mRNA and/or polypeptide are known to one of skill in the art, e.g. RTPCR with primers can be used to determine the level of RNA and Western blotting with an antibody (e.g. an anti-KDM4A antibody, e.g. Cat No. ab105953; Abcam; Cambridge, MA) can be used to determine the level of a polypeptide. The activity of, e.g. KDM4A can be determined using methods known in the art and described above herein. In some embodiments, the inhibitor of KDM4A can be an inhibitory nucleic acid; an aptamer; an antibody reagent; an antibody; or a small molecule.

Non-limiting examples of inhibitors of KDM4A can include an inhibitory nucleic acid; an aptamer; a miRNA; Suv39H1; HP1; increased oxygen levels; and succinate. In some embodiments, the inhibitor can be an allosteric or enzymatic inhibitor, e.g., succinate. miRNAs can include, e.g. miR23a, miR23b, miR200a, miR200b, miR200c, and miR137a or variants thereof. In some embodiments, the miRNA can be selected from the group consisting of miR23a (e.g. NCBI Gene ID: 407010; SEQ ID NO: 21), miR23b (e.g. NCBI Gene ID: 407011; SEQ ID NO: 22), miR200a (e.g. NCBI Gene ID: 406983; SEQ ID NO: 23), miR200b (e.g. NCBI Gene ID: 406984; SEQ ID NO: 24), miR200c (e.g. NCBI Gene ID: 406985; SEQ ID NO: 25), miR137a (e.g. NCBI Gene ID: 406928; SEQ ID NO: 26) or variants thereof. In some embodiments, the miRNA can be selected from the group consisting of miR23a, miR23b, miR200b, miR200c, miR137a or variants thereof. In some embodiments, the KDM4A inhibitor can be the small molecule JIB-04 or derivatives thereof (for further details, see, e.g. Wang et al. Nature Communciations 2013 4).

In some embodiments, a KDM4A inhibitor can inhibit KDM4A; KDM5; and/or KDM6. For example, JIB-04 can inhibit all three of KDM4A; KDM5; and KDM6.

As described herein, inhibitors of KDM4A sensitize cells to the action of certain compounds, and thus inhibitors of KDM4A can be administered to subjects being treated with those compounds to increase efficacy of the compounds in treating conditions other than cancer. In some embodiments, provided herein is a method comprising administering an inhibitor of KDM4A to a subject being treated with a compound selected from the group consisting of mTOR inhibitors; protein synthesis inhibitors; Braf inhibitors; PI3K inhibitors; Cdk inhibitors; Aurora B inhibitors; FLT3 inhibitors; PLK1/2/3 inhibitors; Eg5 inhibitors; β-tubulin inhibitors; BMP inhibitors; HDAC inhibitors; Akt inhibitors; IGF1R inhibitors; p53 inhibitors; hdm2 inhibitors; STAT3 inhibitors; and/or VEGFR inhibitors. In some embodiments, the compound is rapamyacin.

In some embodiments, provided herein is a method of treating graft versus host disease, the method comprising administering an inhibitor of KDM4A; and administering an mTOR inhibitor. In some embodiments, the mTOR inhibitor is rapamycin. As used herein, "graft versus host" refers to a condition arising as a complication of a transplantation, in which functional immune cells in the transplanted material recognize the recipient as "foreign" and mount an immunologic attack. Symptoms can include damage to the liver, skin, muscosa, lungs, bone marrow, thymus, connective tissue, and gastrointestinal tract. Graft versus host disease can occur after bone marrow transplantation or transfusion. In some embodiments, a subject in need of treatment for graft versus host disease can be a subject exhibiting symptoms of, having, or diagnosed as having graft versus host disease. In some embodiments, a subject in need of treatment for graft versus host disease can be a subject who has received a transplant but is not yet exhibiting symptoms of graft versus host disease, e.g. the subject can be treated prophylactically.

In some embodiments, described herein is a method of treating graft versus host disease in a subject in need of treatment therof, the method comprising administering an inhibitor of KDM4A to a subject determined to have a KDM4A dampening mutation. In some embodiments, described herein is a method of treating graft versus host disease in a subject in need of treatment therof, the method comprising selecting a subject with a KDM4A dampening mutation; and administering an inhibitor of KDM4A.

As demonstrated herein, inhibitors of ubiquitination and/or inhibitors of the proteasome can counteract reduced KDM4A expression and/or activity. Accordingly, in some embodiments of any of the aspects described herein, a subject administered an inhibitor of KMD4A can be further administered a ubiquitination inhibitor and/or a proteasomal inhibitor. In some embodiments, the KDM4A inhibitor and ubiquitin and/or proteasomal inhibitor can provide a synergistic therapeutic effect. Inhibitors of ubiquitin and the proteasome are well know in the art (see, e.g. Guedat and Colland. 2007 BMC Biochemistry 2007 8:S14) and can include, by way of non-limiting example, bortezomib, celastrol, ginkgolic acid, epoxomicin, MG-132, lactacystin, MEL23, SMER3, MG-115, R0106-9920, tyropeptin A, NSC697923, PYR-41, WP1130, salinosporamide A, (-)-epigallocatechin 3-gallate ((-)-EGCG), PR-171, ER-805751, ritonavir.

In some embodiments of any of the foregoing aspects, the method can further comprise a step of generating a report based on the detection of a KDM4A gene amplification, the level of KDM4A activity, the level of KDM4A expression, and/or a KDM4A dampening mutation.

In some embodiments, provided herein is a method of treating cancer and/or graft v. host disease, the method compirising administerin an inhibitor of a PhD-Tudor polypeptide, e.g. a polypeptide comprising at least one PhD domain and one Tudor domain. In some embodiments, the PhD-Tudor polypeptide can be a selected from the group consisting of: KDM4A; KDM4C; KDM4D; KDM4E; or KDM4B. In some embodiments, the inhibitor of a PhD-Tudor polypeptide can be administered in combination with a chemotherapeutic selected from the group consisting of: S-phase chemotherapeutics; mTOR inhibitors; protein synthesis inhibitors; Braf inhibitors; PI3K inhibitors; Cdk inhibitors; Aurora B inhibitors; FLT3 inhibitors; PLK1/2/3 inhibitors; Eg5 inhibitors; β-tubulin inhibitors; BMP inhibitors; HDAC inhibitors; Akt inhibitors; IGF1R inhibitors; p53 inhibitors; hdm2 inhibitors; STAT3 inhibitors; and VEGFR inhibitors. In some embodiments, the inhibitor of a PhD-Tudor polypeptide can be administered in combination with an mTOR inhibitor.

In one aspect, described herein is an assay comprising detecting the presence or absence of a KDM4A dampening mutation in a test sample obtained from a subject; wherein the presence of a KDM4A dampening mutation indicates the subject has a higher risk of having or developing autism or schizophrenia. In one aspect, described herein is a method of identifying a subject in need of treatment for autism or schizophrenia, the method comprising detecting the presence or absence of a KDM4A dampening mutation in a test sample obtained from a subject; and identifying the subject as being in need of treatment for autism or schizophrenia when a KDM4A dampening mutation is detected. In one aspect, described herein is a method of determining if a subject is at risk for autism or schizophrenia, the method comprising detecting the presence or absence of a KDM4A dampening mutation in a test sample obtained from a subject;determining that the subject is at risk for autism or schizophrenia when the presence of a KDM4A dampening mutation is detected; and determining that the subject is not at risk for autism or schizophrenia when the presence of a KDM4A dampening mutation is not detected. In some embodiments, described herein is a method of treating autism or schizophrenia, the method comprising administering an agonist of KDM4A to a subject in need of treatment for autism or schizophrenia. In some embodiments, described herein is a method of treating autism or schizophrenia in a subject in need of treatment therof, the method comprising administering an agonist of KDM4A to a subject determined to have a KDM4A dampening mutation. In some embodiments, described herein is a method of treating autism or schizophrenia in a subject in need of treatment therof, the method comprising selecting a subject with a KDM4A dampening mutation; and administering an agonist of KDM4A.

In some embodiments, diseases associated with 1q21 duplication and deletion can be diagnosed, prognosed, and/or treated in accordance with the aspects described herein.

Particularly high levels of KDM4A levels and/or activity can increase DNA damage, leading to toxicity in cells with such levels and/or activity. Accordingly, in one aspect, described herein is a method of treating cancer, the method comprising administering an agonist of KDM4A to a subject determined to have a level of KDM4A gene expression which is higher than a reference level or determined to have KDM4A gene amplification. As used herein, the term "agonist of KDM4A" refers to any agent that increases the level and/or activity of KDM4A. As used herein, the term "agonist" refers to an agent which increases the expression and/or activity of the target by at least 10% or more, e.g. by 10% or more, 50% or more, 100% or more, 200% or more, 500% or more, or 1000 % or more. Non-limiting examples of agonists of KDM4A can include KDM4A polypeptides or fragments thereof and nucleic acids encoding a KDM4A polypeptide, e.g. a polypeptide comprising the sequence SEQ ID NO: 1 or a nucleic acid comprising the sequence of SEQ ID NO: 2 or variants thereof.

As described herein, hypoxic conditions in tumor cells can lead to an increase in the level of KDM4A, which promotes gene amplification. Accordingly, in some embodiments of any of the aspects described herein, the methods and uses applicable to the treatment, diagnosis, and/or prognosis of subjects determined to have increased levels of KDM4A and/or a KDM4A dampening mutation can be similarly applied to subjects determined to have hypoxic tumors. Methods of determining whether a tumor is hypoxic are known in the art and include, by way of non-limiting example:
a. determining that a cell of the tumor comprises amplification at 1q21.2, 1q12h, or Xq13.1;
b. determining that a cell of the tumor comprises amplification at 1q21.2, 1q12h, or Xq13.1 but does not comprise amplification at 1q23.3, 8C, or the 1 q telomere;
c. determining that the cell lysate can stabilize HIF1α (hypoxia inducible factor 1, alpha subunit; NCBI Gene ID: 3091) (e.g. determining an increased level of HI1α polypeptide is present in the cell relative to a reference level); or
d. determining that the cell expresses increased levels of CAIX (carbonic anhydrase IX; NCBI Gene ID: 768) relative to a reference level (e.g. increased levels of CAIX polypeptide and/or mRNA);
e. determining that the cell expresses increased levels of MCL-1 and/or CKS1B
f. determining that the cell expresses an increased level of one or more genes selected from Table 8 (e.g. one gene, two genes, three genes, four genes, five genes, six genes, seven genes, or more genes selected from Table 8); and/or
g. determining that the cell expresses a decreased level of one or more genes selected from Table 9 (e.g. one gene, two genes, three genes, four genes, five genes, six genes, seven genes, or more genes selected from Table 9); and/or
h. determining that there is co-amplification of regions identified by peaks in Figs. 44E, 44G, or 44H, or by using similar analyses on other tumor types.

Amplification at specific loci can be determined by the methods described herein, e.g., by use of probes specific for a particular locus, quantitative PCR, FISH, and the like. Methods for detecting, e.g., the level of mRNA and/or polypeptide expression products are described elsewhere herein.

As described herein, the presence of certain mutations and/or alterations in KDM proteins can indicate the likelihood of a subject experiencing a positive outcome following treatment for cancer. Accordingly, described herein is an assay for determining the likelihood of a subject experiencing a positive outcome following treatment for cancer, the assay comprising determining the level or mutational status of KDM4A; KDM4C; KDM4B; KDM4E; and/or KDM4D in a tumor cell sample obtained from the subject; wherein the subject has a decreased likelihood of experiencing a positive outcome following treatment for cancer if:
a. the subject is determined to have a deletion or decreased level of expression of KDM4C as compared to a reference level;
b. the subject is determined to have a deletion, amplification, or increased or decreased level of KDM4D, KDM4E, KDM4C, or KDM4B as compared to a reference level;
c. the subject is determined to have an amplification or increased level of expression of KDM4A as compared to a reference level;
d. the subject is determined to have a KDM4A dampening mutation;
e. the subject is determined to have a mutation of a KDM4 family member selected from any of Tables 2-6.

Methods of determining levels of expression and/or determining the presence or absence of a given mutation are described elsewhere herein. Where the sequences of the KDM family members are known, one of skill in the art can readily design detection reagents, e.g. antibodies and/or nucleic acid probes. Further, such detection reagents are commercially available, e.g. anti-KDM4C antibody reagents (e.g. Cat No. ab27531, AbCam; Cambridge, MA). The sequences of KDM family members are known in the art, e.g. human KDM4B (NCBI Gene ID: 23030 (polypeptide, NCBI Ref Seq: NP_055830, SEQ ID NO: 6)(mRNA, NCBI Ref Seq: NM_015015, SEQ ID NO: 5), human KDM4C (NCBI Gene ID: 23081 (polypeptide, NCBI Ref Seq: NP_055876, SEQ ID NO: 8)(mRNA, NCBI Ref Seq: NM_015061, SEQ ID NO: 7), human KDM4D (NCBI Gene ID: 55693 (polypeptide, NCBI Ref Seq: NP_060509, SEQ ID NO: 10)(mRNA, NCBI Ref Seq: NM_018039, SEQ ID NO: 9), and human KDM4E (NCBI Gene ID: 390245 (polypeptide, NCBI Ref Seq: NP_001155102, SEQ ID NO: 12)(mRNA, NCBI Ref Seq: NM_001161630, SEQ ID NO: 11).

In some embodiments, the subject determined to have an amplification or increased level of expression of KDM4A as compared to a reference level is a subject having ovarian cancer. In some embodiments, the subject determined to have a KDM4A dampening mutation is a subject having non-small cell lung cancer.

In some embodiments, a positive outcome can refer to, e.g. a reduction in tumor growth or size, remission of the cancer, reduced side effects from treatment, increased lifespan and/or decreased mortality.

In some embodiments of any of the aspects described herein the cancer can be selected from the group consisting of ovarian cancer; non-small cell lung cancer; multiple myeloma; breast cancer; pancreatic cancer; head and neck cancer; lung cancer; adenocarcinoma; lung adenocarcinoma; lung squamous cell carcinoma; renal cancer; stomach cancer; melanoma; colorectal cancer; AML; and uterine and endometrial cancer.

In some embodiments, the assays and methods can relate to detecting the presence of a mutation, e.g. a KDM4A dampening mutation or a gene amplification of KDM4A in a sample obtained from a subject. In some embodiments, the presence of the mutation can be determined using an assay selected from the group consisting of: hybridization; sequencing; exome capture; PCR; high-throughput sequencing; allele-specific probe hybridization; allele-specific primer extension, allele-specific amplification; 5' nuclease digestion; molecular beacon assay; oligonucleotide ligation assay; size analysis; single-stranded conformation polymorphism; real-time quantitative PCR, and any combinations thereof.

In some embodiments, the presence of a mutation can be detected by measuring the catalytic activity of KDM4A, e.g. by measuring the effect of a mutation on the activity of KDM4A. Assays for KDM4A activity are known in the art.

In some embodiments, the presence and/or absence of a mutation can be detected by determining the sequence of a genomic locus and/or an mRNA transcript. Such molecules can be isolated, derived, or amplified from a biological sample, such as a tumor sample. Nucleic acid (e.g. DNA) and ribonucleic acid (RNA) molecules can be isolated from a particular biological sample using any of a number of procedures, which are well-known in the art, the particular isolation procedure chosen being appropriate for the particular biological sample. For example, freeze-thaw and alkaline lysis procedures can be useful for obtaining nucleic acid molecules from solid materials; and proteinase K extraction can be used to obtain nucleic acid from blood (Roiff, A et al. PCR: Clinical Diagnostics and Research, Springer (1994)).

In some embodiments, the nucleic acid sequence of a target gene (e.g. KDM4A) in a sample obtained from a subject can be determined and compared to a reference sequence to determine if a sensitizing mutation is present in the subject. In some embodiments, the reference sequence can be, e.g. SEQ ID NOs: 1 or 2. In some embodiments, the sequence of the target gene can be determined by sequencing the target gene (e.g. the genomic sequence and/or the mRNA transcript thereof). Methods of sequencing a nucleic acid sequence are well known in the art. Briefly, a sample obtained from a subject can be contacted with one or more primers which specifically hybridize to a single-strand nucleic acid sequence flanking the target gene sequence and a complementary strand is synthesized. In some next-generation technologies, an adaptor (double or single-stranded) is ligated to nucleic acid molecules in the sample and synthesis proceeds from the adaptor or adaptor compatible primers. In some third-generation technologies, the sequence can be determined, e.g. by determining the location and pattern of the hybridization of probes, or measuring one or more characteristics of a single molecule as it passes through a sensor (e.g. the modulation of an electrical field as a nucleic acid molecule passes through a nanopore). Exemplary methods of sequencing include, but are not limited to, Sanger sequencing, dideoxy chain termination, high-throughput sequencing, next generation sequencing, 454 sequencing, SOLiD sequencing, polony sequencing, Illumina sequencing, Ion Torrent sequencing, sequencing by hybridization, nanopore sequencing, Helioscope sequencing, single molecule real time sequencing, RNAP sequencing, and the like. Methods and protocols for performing these sequencing methods are known in the art, see, e.g. "Next Generation Genome Sequencing" Ed. Michal Janitz, Wiley-VCH; "High-Throughput Next Generation Sequencing" Eds. Kwon and Ricke, Humanna Press, 2011; and Sambrook et al., Molecular Cloning: A Laboratory Manual (3 ed.), Cold Spring Harbor Laboratory Press, Cold Spring Harbor, N.Y., USA (2001).

In some embodiments, sequencing can comprise exome sequencing (i.e. targeted exome capture). Exome sequencing comprises enriching for an exome(s) of interest and then sequencing the nucleic acids comprised by the enriched sample. Sequencing can be according to any method known in the art, e.g. those described above herein. Methods of enrichment can include, e.g. PCR, molecular inversion probes, hybrid capture, and in solution capture. Exome capture methodologies are well known in the art, see, e.g. Sulonen et la. Genome Biology 2011 12:R94; and Teer and Mullikin. Hum Mol Genet 2010 19:R2. Kits for performing exome capture are available commercially, e.g. the TRUSEQ™ Exome Enrichment Kit (Cat. No. FC-121-1008; Illumnia, San Diego, CA). Exome capture methods can also readily be adapted by one of skill in the art to enrich specific exomes of interest.

In some embodiments, the presence of a mutation can be determined using a probe that is specific for the sensitizing mutation. In some embodiments, the probe can be detectably labeled. In some embodiments, a detectable signal can be generated by the probe when a sensitizing mutation is present.

In some embodiments, the probe specific for the mutation can be a probe in a hybridization assay, i.e. the probe can specifically hybridize to a nucleic acid comprising a mutation (as opposed to a wild-type nucleic acid sequence) and the hybridization can be detected, e.g. by having the probe and or the target nucleic acid be detectably labeled. Hybridization assays are well known in the art and include, e.g. northern blots and Southern blots.

In some embodiments, the probe specific for the mutation can be a probe in a PCR assay, i.e. a primer. In general, the PCR procedure describes a method of gene amplification which is comprised of (i) sequence-specific hybridization of primers to specific genes within a nucleic acid sample or library, (ii) subsequent amplification involving multiple rounds of annealing, elongation, and denaturation using a thermostable DNA polymerase, and optionally, (iii) screening the PCR products for a band or product of the correct size. The primers used are oligonucleotides of sufficient length and appropriate sequence to provide initiation of polymerization, i.e. each primer is specifically designed to be complementary to a strand of the genomic locus to be amplified. In an alternative embodiment, the presence of a sensitizing mutation in an mRNA tramscript can be determined by reverse-transcription (RT) PCR and by quantitative RT-PCR (QRT-PCR) or real-time PCR methods. Methods of RT-PCR and QRT-PCR are well known in the art. In some embodiments, the PCR product can be labeled, e.g. the primers can comprise a detectable label, or a label can be incorporated and/or bound to the PCR product, e.g. EtBr detection methods. Other non-limiting detection methods can include the detection of a product by mass spectroscopy or MALDI-TOF.

The term "label" refers to a composition capable of producing a detectable signal indicative of the presence of a reagent (e.g. a bound antibody reagent). Suitable labels include radioisotopes, nucleotide chromophores, enzymes, substrates, fluorescent molecules, chemiluminescent moieties, magnetic particles, bioluminescent moieties, and the like. As such, a label is any composition detectable by spectroscopic, photochemical, biochemical, immunochemical, electrical, optical or chemical means.

The nucleic acid sequences of, e.g. KDM4A have been assigned NCBI accession numbers for different species such as human, mouse and rat. In particular, the NCBI accession numbers for the nucleic acid sequences of the human expression products are included herein (SEQ ID NOs: 1 and 2). Accordingly, a skilled artisan can design appropriate primers based on the known sequence for determining the mRNA level of the target gene.

A mutation will typically be present in the genomic DNA of a tumor (e.g. cancerous) cell. Accordingly, the mutation can be detected in either or both of the genomic DNA or the mRNA transcripts of a cell. In some embodiments, the mutation can occur within a DNA and/or RNA sequence that is translated. Accordingly, in some embodiments, the mutation can be detected in the polypeptide of a cell.

Detection of polypeptides comprising a mutation can be according to any method known in the art (e.g. mass spectroscopy, flow cytometry, and/or immunological-based methods). Immunological methods to detect polypeptides comprising a sensitizing mutation in accordance with the present technology include, but are not limited to antibody techniques such as immunohistochemistry, immunocytochemistry, flow cytometry, fluorescent-activated cell sorting (FACS), immunoblotting, radioimmunoassays, western blotting, immunoprecipitation, enzyme-linked immunosorbant assays (ELISA), and derivative techniques that make use of antibody reagents as described herein.

Immunochemical methods require the use of an antibody reagent specific for the target molecule (e.g. the antigen or in the embodiments described herein, a polypeptide or fragment thereof comprising a sensitizing mutation). In some embodiments, an antibody reagent for determining the presence of a mutation in a sample can be an antibody reagent specific for a polypeptide comprising a mutation, e.g. a mutantion of KDM4A.

In some embodiments, the assays, methods, and/or systems described herein can comprise: an antibody reagent, e.g., an antibody reagent specific for a KDM4A polypeptide and/or an antibody reagent specific for a KDM4A polypeptide describing a mutation described herein. In some embodiments, the antibody reagnets and methods of using them described herein can comprise detecting the localization of KDM4A polypeptide, e.g. the relative concentrations of KDM4A in the cytoplasm or nucleus. In some embodiments, the antibody reagent can be detectably labeled. In some embodiments, the antibody reagent can be attached to a solid support (e.g. bound to a solid support). In some embodiments, the solid support can comprise a particle (including, but not limited to an agarose or latex bead or particle or a magnetic particle), a bead, a nanoparticle, a polymer, a substrate, a slide, a coverslip, a plate, a dish, a well, a membrane, and/or a grating. The solid support can include many different materials including, but not limited to, polymers, plastics, resins, polysaccharides, silicon or silica based materials, carbon, metals, inorganic glasses, and membranes.

In one embodiment, an assay, method, and/or system as described herein can comprise an ELISA. In an exemplary embodiment, a first antibody reagent can be immobilized on a solid support (usually a polystyrene micro titer plate). The solid support can be contacted with a sample obtained from a subject, and the antibody reagent will bind ("capture") antigens for which it is specific (e.g. a polypeptide comprising a sensitizing mutation). The solid support can then be contacted with a second labeled antibody reagent (e.g. a detection antibody reagent). The detection antibody reagent can, e.g. comprise a detectable signal, be covalently linked to an enzyme, or can itself be detected by a secondary antibody, which is linked to an enzyme through bio-conjugation. The presence of a signal indicates that both the first antibody reagent immobilized on the support and the second "detection" antibody reagent have bound to an antigen, i.e. the presence of a signal indicated the presence of polypeptide comprising a sensitizing mutation. Between each step the plate is typically washed with a mild detergent solution to remove any proteins or antibodies that are not specifically bound. After the final wash step the plate is developed by adding an enzymatic substrate to produce a visible signal, which indicates the presence of a sensitizing mutation in the sample. Older ELISAs utilize chromogenic substrates, though newer assays employ fluorogenic substrates with much higher sensitivity. There are other different forms of ELISA, which are well known to those skilled in the art. The standard techniques known in the art for ELISA are described in "Methods in Immunodiagnosis", 2nd Edition, Rose and Bigazzi, eds. John Wiley & Sons, 1980; Campbell et al., "Methods and Immunology", W. A. Benjamin, Inc., 1964; and Oellerich, M. 1984, J. Clin. Chem. Clin. Biochem. 22:895-904.

In one embodiment, the assays, systems, and methods described herein can comprise a lateral flow immunoassay test (LFIA), also known as the immunochromatographic assay, or strip test to measure or determine the presence of a polypeptide comprising a sensitizing mutation. LFIAs are a simple device intended to detect the presence (or absence) of a target in a sample. There are currently many LFIA tests are used for medical diagnostics either for home testing, point of care testing, or laboratory use. LFIA tests are a form of immunoassay in which the test sample flows along a solid substrate via capillary action. After the sample is applied to the test it encounters a colored antibody reagent, which mixes with the sample, and if bound to a portion of the sample, transits the substrate encountering lines or zones which have been pretreated with a second antibody reagent. Depending upon the presence or absence of the target in the sample the colored antibody reagent can become bound at the test line or zone. LFIAs are essentially immunoassays adapted to operate along a single axis to suit the test strip format or a dipstick format. Strip tests are extremely versatile and can be easily modified by one skilled in the art for detecting an enormous range of antigens from fluid samples such as urine, blood, tumor cell lysates etc. Strip tests are also known as dip stick test, the name bearing from the literal action of "dipping" the test strip into a fluid sample to be tested. LFIA strip test are easy to use, require minimum training and can easily be included as components of point-of-care test (POCT) diagnostics to be use on site in the field. LFIA tests can be operated as either competitive or sandwich assays. Sandwich LFIAs are similar to sandwich ELISA. The sample first encounters colored particles, which are labeled with antibody reagents specific for a target. The test line will also contain antibody reagents. The test line will show as a colored band in positive samples. In some embodiments, the lateral flow immunoassay can be a double antibody sandwich assay, a competitive assay, a quantitative assay or variations thereof. There are a number of variations on lateral flow technology. It is also possible to apply multiple capture zones to create a multiplex test.

A typical test strip consists of the following components: (1) sample application area comprising an absorbent pad (i. e. the matrix or material) onto which the test sample is applied; (2) conjugate or reagent pad- this contains antibody reagent(s) specific to the target which can be conjugated to colored particles (usually colloidal gold particles, or latex microspheres); (3) test results area comprising a reaction membrane - typically a hydrophobic nitrocellulose or cellulose acetate membrane onto which antibody reagents are immobilized in a line across the membrane as a capture zone or test line (a control zone may also be present, containing antibodies specific for the antibody reagents conjugated to the particles or microspheres); and (4) optional wick or waste reservoir - a further absorbent pad designed to draw the sample across the reaction membrane by capillary action and collect it. The components of the strip are usually fixed to an inert backing material and may be presented in a simple dipstick format or within a plastic casing with a sample port and reaction window showing the capture and control zones. While not strictly necessary, most tests will incorporate a second line, which contains an antibody that picks up free latex/gold in order to confirm the test has operated correctly.

The use of "dip sticks" or LFIA test strips and other solid supports have been described in the art in the context of an immunoassay for a number of antigen biomarkers. U.S. Pat. Nos. 4,943,522; 6,485,982; 6,187,598; 5,770,460; 5,622,871; 6,565,808, U. S. patent applications Ser. No. 10/278,676; U.S. Ser. No. 09/579,673 and U.S. Ser. No. 10/717,082, are non-limiting examples of such lateral flow test devices. Three U.S. patents (U.S. Pat. No. 4,444,880, issued to H. Tom; U.S. Pat. No. 4,305,924, issued to R. N. Piasio; and U.S. Pat. No. 4,135,884, issued to J. T. Shen) describe the use of "dip stick" technology to detect soluble antigens via immunochemical assays. The apparatuses and methods of these three patents broadly describe a first component fixed to a solid surface on a "dip stick" which is exposed to a solution containing a soluble antigen that binds to the component fixed upon the "dip stick," prior to detection of the component-antigen complex upon the stick. It is within the skill of one in the art to modify the teaching of these "dip stick" technology for the detection of a sensitizing mutation.

Immunochemistry is a family of techniques based on the use of a specific antibody, wherein antibodies are used to specifically target molecules inside or on the surface of cells. In some embodiments, immunohistochemistry ("IHC") and immunocytochemistry ("ICC") techniques can be used to detect the presence of a sensitizing mutation. IHC is the application of immunochemistry to tissue sections, whereas ICC is the application of immunochemistry to cells or tissue imprints after they have undergone specific cytological preparations such as, for example, liquid-based preparations. In some instances, signal amplification may be integrated into the particular protocol, wherein a secondary antibody, that includes a label, follows the application of an antibody reagent specific for a polypeptide comprising a sensitizing mutation. Typically, for immunohistochemistry, tissue obtained from a subject and fixed by a suitable fixing agent such as alcohol, acetone, and paraformaldehyde, is sectioned and reacted with an antibody. Conventional methods for immunohistochemistry are described in Buchwalow and Bocker (Eds) "Immunohistochemistry: Basics and Methods" Springer (2010): Lin and Prichard "Handbook of Practical Immunohistochemistry" Springer (2011). In some embodiments, immunocytochemistry may be utilized where, in general, tissue or cells are obtained from a subject are fixed by a suitable fixing agent such as alcohol, acetone, and paraformaldehyde, to which is reacted an antibody. Methods of immunocytological staining of human samples is known to those of skill in the art and described, for example, in Burry. "Immunocytochemistry: A Practical Guide for Biomedical Research" Springer (2009).

In some embodiments, one or more of the antibody reagents described herein can comprise a detectable label and/or comprise the ability to generate a detectable signal (e.g. by catalyzing reaction converting a compound to a detectable product). Detectable labels can comprise, for example, a light-absorbing dye, a fluorescent dye, or a radioactive label. Detectable labels, methods of detecting them, and methods of incorporating them into an antibody reagent are well known in the art.

In some embodiments, detectable labels can include labels that can be detected by spectroscopic, photochemical, biochemical, immunochemical, electromagnetic, radiochemical, or chemical means, such as fluorescence, chemifluoresence, or chemiluminescence, or any other appropriate means. The detectable labels used in the methods described herein can be primary labels (where the label comprises a moiety that is directly detectable or that produces a directly detectable moiety) or secondary labels (where the detectable label binds to another moiety to produce a detectable signal, e.g., as is common in immunological labeling using secondary and tertiary antibodies). The detectable label can be linked by covalent or non-covalent means to the antibody reagent. Alternatively, a detectable label can be linked such as by directly labeling a molecule that achieves binding to the antibody reagent via a ligand-receptor binding pair arrangement or other such specific recognition molecules. Detectable labels can include, but are not limited to radioisotopes, bioluminescent compounds, chromophores, antibodies, chemiluminescent compounds, fluorescent compounds, metal chelates, and enzymes.

In other embodiments, the detection antibody is label with a fluorescent compound. When the fluorescently labeled antibody is exposed to light of the proper wavelength, its presence can then be detected due to fluorescence. In some embodiments, a detectable label can be a fluorescent dye molecule, or fluorophore including, but not limited to fluorescein, phycoerythrin, phycocyanin, o-phthaldehyde, fluorescamine, Cy3™, Cy5™, allophycocyanine, Texas Red, peridenin chlorophyll, cyanine, tandem conjugates such as phycoerythrin-Cy5™, green fluorescent protein, rhodamine, fluorescein isothiocyanate (FITC) and Oregon Green™, rhodamine and derivatives (e.g., Texas red and tetrarhodimine isothiocynate (TRITC)), biotin, phycoerythrin, AMCA, CyDyes™, 6-carboxyfhiorescein (commonly known by the abbreviations FAM and F), 6-carboxy-2',4',7',4,7-hexachlorofiuorescein (HEX), 6-carboxy-4',5'-dichloro-2',7'-dimethoxyfiuorescein (JOE or J), N,N,N',N'-tetramethyl-6carboxyrhodamine (TAMRA or T), 6-carboxy-X-rhodamine (ROX or R), 5-carboxyrhodamine-6G (R6G5 or G5), 6-carboxyrhodamine-6G (R6G6 or G6), and rhodamine 110; cyanine dyes, e.g. Cy3, Cy5 and Cy7 dyes; coumarins, e.g umbelliferone; benzimide dyes, e.g. Hoechst 33258; phenanthridine dyes, e.g. Texas Red; ethidium dyes; acridine dyes; carbazole dyes; phenoxazine dyes; porphyrin dyes; polymethine dyes, e.g. cyanine dyes such as Cy3, Cy5, etc; BODIPY dyes and quinoline dyes. In some embodiments, a detectable label can be a radiolabel including, but not limited to ³H, ¹²⁵I, ³⁵S, ¹⁴C, ³²P, and ³³P. In some embodiments, a detectable label can be an enzyme including, but not limited to horseradish peroxidase and alkaline phosphatase. An enzymatic label can produce, for example, a chemiluminescent signal, a color signal, or a fluorescent signal. Enzymes contemplated for use to detectably label an antibody reagent include, but are not limited to, malate dehydrogenase, staphylococcal nuclease, delta-V-steroid isomerase, yeast alcohol dehydrogenase, alpha-glycerophosphate dehydrogenase, triose phosphate isomerase, horseradish peroxidase, alkaline phosphatase, asparaginase, glucose oxidase, beta-galactosidase, ribonuclease, urease, catalase, glucose-VI-phosphate dehydrogenase, glucoamylase and acetylcholinesterase. In some embodiments, a detectable label is a chemiluminescent label, including, but not limited to lucigenin, luminol, luciferin, isoluminol, theromatic acridinium ester, imidazole, acridinium salt and oxalate ester. In some embodiments, a detectable label can be a spectral colorimetric label including, but not limited to colloidal gold or colored glass or plastic (e.g., polystyrene, polypropylene, and latex) beads.

In some embodiments, antibodies can also be labeled with a detectable tag, such as c-Myc, HA, VSV-G, HSV, FLAG, V5, HIS, or biotin. Other detection systems can also be used, for example, a biotin-streptavidin system. In this system, the antibodies immunoreactive (i. e. specific for) with the biomarker of interest is biotinylated. Quantity of biotinylated antibody bound to the biomarker is determined using a streptavidin-peroxidase conjugate and a chromagenic substrate. Such streptavidin peroxidase detection kits are commercially available, e. g. from DAKO; Carpinteria, CA. An antibody reagent can also be detectably labeled using fluorescence emitting metals such as ¹⁵²Eu, or others of the lanthanide series. These metals can be attached to the antibody reagent using such metal chelating groups as diethylenetriaminepentaacetic acid (DTPA) or ethylenediaminetetraacetic acid (EDTA).

In some embodiments, the sequence, level, activity, and/or localization of, e.g. KDM4A can be compared to a reference sample or level. In some embodiments, the reference level can be the level in a healthy subject not diagnosed as having or not having cancer. In some embodiments, the reference level can be the level in a healthy, non-cancerous cell from the same subject.

The term "sample" or "test sample" as used herein denotes a sample taken or isolated from a biological organism, e.g., a tumor sample from a subject. Exemplary biological samples include, but are not limited to, a biofluid sample; serum; plasma; urine; saliva; a tumor sample; a tumor biopsy and/or tissue sample etc. The term also includes a mixture of the above-mentioned samples. The term "test sample" also includes untreated or pretreated (or pre-processed) biological samples. In some embodiments, a test sample can comprise cells from subject. In some embodiments, a test sample can be a tumor cell test sample, e.g. the sample can comprise cancerous cells, cells from a tumor, and/or a tumor biopsy.

The test sample can be obtained by removing a sample of cells from a subject, but can also be accomplished by using previously isolated cells (e.g. isolated at a prior timepoint and isolated by the same or another person). In addition, the test sample can be freshly collected or a previously collected sample.

In some embodiments, the test sample can be an untreated test sample. As used herein, the phrase "untreated test sample" refers to a test sample that has not had any prior sample pre-treatment except for dilution and/or suspension in a solution. Exemplary methods for treating a test sample include, but are not limited to, centrifugation, filtration, sonication, homogenization, heating, freezing and thawing, and combinations thereof. In some embodiments, the test sample can be a frozen test sample, e.g., a frozen tissue. The frozen sample can be thawed before employing methods, assays and systems described herein. After thawing, a frozen sample can be centrifuged before being subjected to methods, assays and systems described herein. In some embodiments, the test sample is a clarified test sample, for example, by centrifugation and collection of a supernatant comprising the clarified test sample. In some embodiments, a test sample can be a pre-processed test sample, for example, supernatant or filtrate resulting from a treatment selected from the group consisting of centrifugation, filtration, thawing, purification, and any combinations thereof. In some embodiments, the test sample can be treated with a chemical and/or biological reagent. Chemical and/or biological reagents can be employed to protect and/or maintain the stability of the sample, including biomolecules (e.g., nucleic acid and protein) therein, during processing. One exemplary reagent is a protease inhibitor, which is generally used to protect or maintain the stability of protein during processing. The skilled artisan is well aware of methods and processes appropriate for pre-processing of biological samples required for determination of the presence of a sensitizing mutation as described herein.

In some embodiments, the methods, assays, and systems described herein can further comprise a step of obtaining a test sample from a subject. In some embodiments, the subject can be a human subject.

In some embodiments, the methods described herein relate to treating a subject having or diagnosed as having cancer with a composition or treatment described herein. Subjects having cancer can be identified by a physician using current methods of diagnosing cancer. Symptoms and/or complications of cancer which characterize these conditions and aid in diagnosis are well known in the art and include but are not limited to, growth of a tumor, impaired function of the organ or tissue harboring cancer cells, etc. Tests that may aid in a diagnosis of, e.g. cancer include, but are not limited to, tissue biopsies and histological examination. A family history of cancer, or exposure to risk factors for cancer (e.g. tobacco products, radiation, etc.) can also aid in determining if a subject is likely to have cancer or in making a diagnosis of cancer.

The compositions and methods described herein can be administered to a subject having or diagnosed as having cancer. In some embodiments, the methods described herein comprise administering an effective amount of compositions described herein to a subject in order to alleviate a symptom of a cancer. As used herein, "alleviating a symptom of a cancer" is ameliorating any condition or symptom associated with the cancer. As compared with an equivalent untreated control, such reduction is by at least 5%, 10%, 20%, 40%, 50%, 60%, 80%, 90%, 95%, 99% or more as measured by any standard technique. A variety of means for administering the compositions described herein to subjects are known to those of skill in the art. Such methods can include, but are not limited to oral, parenteral, intravenous, intramuscular, subcutaneous, transdermal, airway (aerosol), pulmonary, cutaneous, topical, injection, or intratumoral administration. Administration can be local or systemic.

The term "effective amount" as used herein refers to the amount needed to alleviate at least one or more symptom of the disease or disorder, and relates to a sufficient amount of pharmacological composition to provide the desired effect. The term "therapeutically effective amount" therefore refers to an amount of an agent that is sufficient to provide a particular anti-tumor effect when administered to a typical subject. An effective amount as used herein, in various contexts, would also include an amount sufficient to delay the development of a symptom of the disease, alter the course of a symptom disease (for example but not limited to, slowing the progression of a symptom of the disease), or reverse a symptom of the disease. Thus, it is not generally practicable to specify an exact "effective amount". However, for any given case, an appropriate "effective amount" can be determined by one of ordinary skill in the art using only routine experimentation.

Effective amounts, toxicity, and therapeutic efficacy can be determined by standard pharmaceutical procedures in cell cultures or experimental animals, e.g., for determining the LD50 (the dose lethal to 50% of the population) and the ED50 (the dose therapeutically effective in 50% of the population). The dosage can vary depending upon the dosage form employed and the route of administration utilized. The dose ratio between toxic and therapeutic effects is the therapeutic index and can be expressed as the ratio LD50/ED50. Compositions and methods that exhibit large therapeutic indices are preferred. A therapeutically effective dose can be estimated initially from cell culture assays. Also, a dose can be formulated in animal models to achieve a circulating plasma concentration range that includes the IC50 (*i.e.,* the concentration of the active agent which achieves a half-maximal inhibition of symptoms) as determined in cell culture, or in an appropriate animal model. Levels in plasma can be measured, for example, by high performance liquid chromatography. The effects of any particular dosage can be monitored by a suitable bioassay, e.g., assay for tumor growth, among others. The dosage can be determined by a physician and adjusted, as necessary, to suit observed effects of the treatment.

In some embodiments, the technology described herein relates to a pharmaceutical composition, and optionally a pharmaceutically acceptable carrier. Pharmaceutically acceptable carriers and diluents include saline, aqueous buffer solutions, solvents and/or dispersion media. The use of such carriers and diluents is well known in the art. Some non-limiting examples of materials which can serve as pharmaceutically-acceptable carriers include: (1) sugars, such as lactose, glucose and sucrose; (2) starches, such as corn starch and potato starch; (3) cellulose, and its derivatives, such as sodium carboxymethyl cellulose, methylcellulose, ethyl cellulose, microcrystalline cellulose and cellulose acetate; (4) powdered tragacanth; (5) malt; (6) gelatin; (7) lubricating agents, such as magnesium stearate, sodium lauryl sulfate and talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol (PEG); (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) pH buffered solutions; (21) polyesters, polycarbonates and/or polyanhydrides; (22) bulking agents, such as polypeptides and amino acids (23) serum component, such as serum albumin, HDL and LDL; (22) C₂-C₁₂ alcohols, such as ethanol; and (23) other non-toxic compatible substances employed in pharmaceutical formulations. Wetting agents, coloring agents, release agents, coating agents, sweetening agents, flavoring agents, perfuming agents, preservative and antioxidants can also be present in the formulation. The terms such as "excipient", "carrier", "pharmaceutically acceptable carrier" or the like are used interchangeably herein. In some embodiments, the carrier inhibits the degradation of the active agent, as described herein.

In some embodiments, the pharmaceutical composition as described herein can be a parenteral dose form. Since administration of parenteral dosage forms typically bypasses the patient's natural defenses against contaminants, parenteral dosage forms are preferably sterile or capable of being sterilized prior to administration to a patient. Examples of parenteral dosage forms include, but are not limited to, solutions ready for injection, dry products ready to be dissolved or suspended in a pharmaceutically acceptable vehicle for injection, suspensions ready for injection, and emulsions. In addition, controlled-release parenteral dosage forms can be prepared for administration of a patient, including, but not limited to, DUROS®-type dosage forms and dose-dumping.

Suitable vehicles that can be used to provide parenteral dosage forms as disclosed within are well known to those skilled in the art. Examples include, without limitation: sterile water; water for injection USP; saline solution; glucose solution; aqueous vehicles such as but not limited to, sodium chloride injection, Ringer's injection, dextrose Injection, dextrose and sodium chloride injection, and lactated Ringer's injection; water-miscible vehicles such as, but not limited to, ethyl alcohol, polyethylene glycol, and propylene glycol; and non-aqueous vehicles such as, but not limited to, corn oil, cottonseed oil, peanut oil, sesame oil, ethyl oleate, isopropyl myristate, and benzyl benzoate. Compounds that alter or modify the solubility of a pharmaceutically acceptable salt of a composition as disclosed herein can also be incorporated into the parenteral dosage forms of the disclosure, including conventional and controlled-release parenteral dosage forms.

Pharmaceutical compositions can also be formulated to be suitable for oral administration, for example as discrete dosage forms, such as, but not limited to, tablets (including without limitation scored or coated tablets), pills, caplets, capsules, chewable tablets, powder packets, cachets, troches, wafers, aerosol sprays, or liquids, such as but not limited to, syrups, elixirs, solutions or suspensions in an aqueous liquid, a non-aqueous liquid, an oil-in-water emulsion, or a water-in-oil emulsion. Such compositions contain a predetermined amount of the pharmaceutically acceptable salt of the disclosed compounds, and may be prepared by methods of pharmacy well known to those skilled in the art. See generally, Remington: The Science and Practice of Pharmacy, 21st Ed., Lippincott, Williams, and Wilkins, Philadelphia PA. (2005).

Conventional dosage forms generally provide rapid or immediate drug release from the formulation. Depending on the pharmacology and pharmacokinetics of the drug, use of conventional dosage forms can lead to wide fluctuations in the concentrations of the drug in a patient's blood and other tissues. These fluctuations can impact a number of parameters, such as dose frequency, onset of action, duration of efficacy, maintenance of therapeutic blood levels, toxicity, side effects, and the like. Advantageously, controlled-release formulations can be used to control a drug's onset of action, duration of action, plasma levels within the therapeutic window, and peak blood levels. In particular, controlled- or extended-release dosage forms or formulations can be used to ensure that the maximum effectiveness of a drug is achieved while minimizing potential adverse effects and safety concerns, which can occur both from under-dosing a drug (i.e., going below the minimum therapeutic levels) as well as exceeding the toxicity level for the drug. In some embodiments, the composition can be administered in a sustained release formulation.

Controlled-release pharmaceutical products have a common goal of improving drug therapy over that achieved by their non-controlled release counterparts. Ideally, the use of an optimally designed controlled-release preparation in medical treatment is characterized by a minimum of drug substance being employed to cure or control the condition in a minimum amount of time. Advantages of controlled-release formulations include: 1) extended activity of the drug; 2) reduced dosage frequency; 3) increased patient compliance; 4) usage of less total drug; 5) reduction in local or systemic side effects; 6) minimization of drug accumulation; 7) reduction in blood level fluctuations; 8) improvement in efficacy of treatment; 9) reduction of potentiation or loss of drug activity; and 10) improvement in speed of control of diseases or conditions. Kim, Cherng-ju, Controlled Release Dosage Form Design, 2 (Technomic Publishing, Lancaster, Pa.: 2000).

Most controlled-release formulations are designed to initially release an amount of drug (active ingredient) that promptly produces the desired therapeutic effect, and gradually and continually release other amounts of drug to maintain this level of therapeutic or prophylactic effect over an extended period of time. In order to maintain this constant level of drug in the body, the drug must be released from the dosage form at a rate that will replace the amount of drug being metabolized and excreted from the body. Controlled-release of an active ingredient can be stimulated by various conditions including, but not limited to, pH, ionic strength, osmotic pressure, temperature, enzymes, water, and other physiological conditions or compounds.

A variety of known controlled- or extended-release dosage forms, formulations, and devices can be adapted for use with the salts and compositions of the disclosure. Examples include, but are not limited to, those described in U.S. Pat. Nos.: 3,845,770; 3,916,899; 3,536,809; 3,598,123; 4,008,719; 5674,533; 5,059,595; 5,591 ,767; 5,120,548; 5,073,543; 5,639,476; 5,354,556; 5,733,566; and 6,365,185 B1. These dosage forms can be used to provide slow or controlled-release of one or more active ingredients using, for example, hydroxypropylmethyl cellulose, other polymer matrices, gels, permeable membranes, osmotic systems (such as OROS® (Alza Corporation, Mountain View, Calif. USA)), or a combination thereof to provide the desired release profile in varying proportions.

The methods described herein can further comprise administering a second agent and/or treatment to the subject, e.g. as part of a combinatorial therapy. In some embodiments, a second agent and/or treatment can comprise dietary succinate supplementation. Non-limiting examples of a second agent and/or treatment can include radiation therapy, surgery, gemcitabine, cisplastin, paclitaxel, carboplatin, bortezomib, AMG479, vorinostat, rituximab, temozolomide, rapamycin, ABT-737, PI-103; alkylating agents such as thiotepa and CYTOXAN® cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethiylenethiophosphoramide and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (*e.g*., calicheamicin, especially calicheamicin gamma1I and calicheamicin omegaI1 (see, *e.g.,* Agnew, Chem. Intl. Ed. Engl., 33: 183-186 (1994)); dynemicin, including dynemicin A; bisphosphonates, such as clodronate; an esperamicin; as well as neocarzinostatin chromophore and related chromoprotein enediyne antiobiotic chromophores), aclacinomysins, actinomycin, authramycin, azaserine, bleomycins, cactinomycin, carabicin, caminomycin, carzinophilin, chromomycinis, dactinomycin, daunorubicin, detorubicin, 6-diazo-5-oxo-L-norleucine, ADRIAMYCIN® doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elformithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2-ethylhydrazide; procarbazine; PSK® polysaccharide complex (JHS Natural Products, Eugene, Oreg.); razoxane; rhizoxin; sizofuran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g., TAXOL® paclitaxel (Bristol-Myers Squibb Oncology, Princeton, N.J.), ABRAXANE® Cremophor-free, albumin-engineered nanoparticle formulation of paclitaxel (American Pharmaceutical Partners, Schaumberg, Ill.), and TAXOTERE® doxetaxel (Rhone-Poulenc Rorer, Antony, France); chloranbucil; GEMZAR® gemcitabine; 6-thioguanine; mercaptopurine; methotrexate; platinum analogs such as cisplatin, oxaliplatin and carboplatin; vinblastine; platinum; etoposide (VP-16); ifosfamide; mitoxantrone; vincristine; NAVELBINE.RTM. vinorelbine; novantrone; teniposide; edatrexate; daunomycin; aminopterin; xeloda; ibandronate; irinotecan (Camptosar, CPT-11) (including the treatment regimen of irinotecan with 5-FU and leucovorin); topoisomerase inhibitor RFS 2000; difluoromethylornithine (DMFO); retinoids such as retinoic acid; capecitabine; combretastatin; leucovorin (LV); oxaliplatin, including the oxaliplatin treatment regimen (FOLFOX); lapatinib (Tykerb.RTM.); inhibitors of PKC-alpha, Raf, H-Ras, EGFR (*e.g*., erlotinib (Tarceva®)) and VEGF-A that reduce cell proliferation and pharmaceutically acceptable salts, acids or derivatives of any of the above.

In addition, the methods of treatment can further include the use of radiation or radiation therapy. Further, the methods of treatment can further include the use of surgical treatments.

In certain embodiments, an effective dose of a composition as described herein can be administered to a patient once. In certain embodiments, an effective dose of a composition can be administered to a patient repeatedly. For systemic administration, subjects can be administered a therapeutic amount of a composition, such as, e.g. 0.1 mg/kg, 0.5 mg/kg, 1.0 mg/kg, 2.0 mg/kg, 2.5 mg/kg, 5 mg/kg, 10 mg/kg, 15 mg/kg, 20 mg/kg, 25 mg/kg, 30 mg/kg, 40 mg/kg, 50 mg/kg, or more.

In some embodiments, after an initial treatment regimen, the treatments can be administered on a less frequent basis. For example, after treatment biweekly for three months, treatment can be repeated once per month, for six months or a year or longer. Treatment according to the methods described herein can reduce levels of a marker or symptom of a condition, e.g. cancer by at least 10%, at least 15%, at least 20%, at least 25%, at least 30%, at least 40%, at least 50%, at least 60%, at least 70%, at least 80 % or at least 90% or more.

The dosage of a composition as described herein can be determined by a physician and adjusted, as necessary, to suit observed effects of the treatment. With respect to duration and frequency of treatment, it is typical for skilled clinicians to monitor subjects in order to determine when the treatment is providing therapeutic benefit, and to determine whether to increase or decrease dosage, increase or decrease administration frequency, discontinue treatment, resume treatment, or make other alterations to the treatment regimen. The dosing schedule can vary from once a week to daily depending on a number of clinical factors, such as the subject's sensitivity to the composition. The desired dose or amount of activation can be administered at one time or divided into subdoses, e.g., 2-4 subdoses and administered over a period of time, e.g., at appropriate intervals through the day or other appropriate schedule. In some embodiments, administration can be chronic, e.g., one or more doses and/or treatments daily over a period of weeks or months. Examples of dosing and/or treatment schedules are administration daily, twice daily, three times daily or four or more times daily over a period of 1 week, 2 weeks, 3 weeks, 4 weeks, 1 month, 2 months, 3 months, 4 months, 5 months, or 6 months, or more. A composition can be administered over a period of time, such as over a 5 minute, 10 minute, 15 minute, 20 minute, or 25 minute period.

The dosage ranges for the administration, according to the methods described herein depend upon, for example, the form of the composition, its potency, and the extent to which symptoms, markers, or indicators of a condition described herein are desired to be reduced, for example the percentage reduction desired for tumor size or growth. The dosage should not be so large as to cause adverse side effects, such as toxicity in healthy tissue. Generally, the dosage will vary with the age, condition, and sex of the patient and can be determined by one of skill in the art. The dosage can also be adjusted by the individual physician in the event of any complication.

The efficacy of a composition in, e.g. the treatment of a condition described herein, or to induce a response as described herein (e.g. reduced growth of cancer cells) can be determined by the skilled clinician. However, a treatment is considered "effective treatment," as the term is used herein, if one or more of the signs or symptoms of a condition described herein are altered in a beneficial manner, other clinically accepted symptoms are improved, or even ameliorated, or a desired response is induced e.g., by at least 10% following treatment according to the methods described herein. Efficacy can be assessed, for example, by measuring a marker, indicator, symptom, and/or the incidence of a condition treated according to the methods described herein or any other measurable parameter appropriate, e.g. tumor size. Efficacy can also be measured by a failure of an individual to worsen as assessed by hospitalization, or need for medical interventions (i.e., progression of the disease is halted). Methods of measuring these indicators are known to those of skill in the art and/or are described herein. Treatment includes any treatment of a disease in an individual or an animal (some non-limiting examples include a human or an animal) and includes: (1) inhibiting the disease, e.g., preventing a worsening of symptoms (e.g. pain or inflammation); or (2) relieving the severity of the disease, e.g., causing regression of symptoms. An effective amount for the treatment of a disease means that amount which, when administered to a subject in need thereof, is sufficient to result in effective treatment as that term is defined herein, for that disease. Efficacy of an agent can be determined by assessing physical indicators of a condition or desired response, (e.g. a reduction in tumor growth). It is well within the ability of one skilled in the art to monitor efficacy of administration and/or treatment by measuring any one of such parameters, or any combination of parameters. Efficacy can be assessed in animal models of a condition described herein, for example treatment of cancer. When using an experimental animal model, efficacy of treatment is evidenced when a statistically significant change in a marker is observed, e.g. tumor growth.

*In vitro* and animal model assays are provided herein which allow the assessment of a given dose of a composition. By way of non-limiting example, the effects of a dose can be assessed by contacting a tumor cell line grown in vitro with a composition described herein and/or treating it in accordance with the methods described herein. The efficacy of a given dosage combination can also be assessed in an animal model, e.g. a mouse model of any of the cancer described herein.

For convenience, the meaning of some terms and phrases used in the specification, examples, and appended claims, are provided below. Unless stated otherwise, or implicit from context, the following terms and phrases include the meanings provided below. The definitions are provided to aid in describing particular embodiments, and are not intended to limit the claimed invention, because the scope of the invention is limited only by the claims. Unless otherwise defined, all technical and scientific terms used herein have the same meaning as commonly understood by one of ordinary skill in the art to which this invention belongs. If there is an apparent discrepancy between the usage of a term in the art and its definition provided herein, the definition provided within the specification shall prevail.

For convenience, certain terms employed herein, in the specification, examples and appended claims are collected here.

The terms "decrease", "reduced", "reduction", or "inhibit" are all used herein to mean a decrease by a statistically significant amount. In some embodiments, "reduce," "reduction" or "decrease" or "inhibit" typically means a decrease by at least 10% as compared to a reference level (e.g. the absence of a given treatment) and can include, for example, a decrease by at least about 10%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 98%, at least about 99% , or more. As used herein, "reduction" or "inhibition" does not encompass a complete inhibition or reduction as compared to a reference level. "Complete inhibition" is a 100% inhibition as compared to a reference level. A decrease can be preferably down to a level accepted as within the range of normal for an individual without a given disorder.

The terms "increased", "increase", "enhance", or "activate" are all used herein to mean an increase by a statically significant amount. In some embodiments, the terms "increased", "increase", "enhance", or "activate" can mean an increase of at least 10% as compared to a reference level, for example an increase of at least about 20%, or at least about 30%, or at least about 40%, or at least about 50%, or at least about 60%, or at least about 70%, or at least about 80%, or at least about 90% or up to and including a 100% increase or any increase between 10-100% as compared to a reference level, or at least about a 2-fold, or at least about a 3-fold, or at least about a 4-fold, or at least about a 5-fold or at least about a 10-fold increase, or any increase between 2-fold and 10-fold or greater as compared to a reference level. In the context of a marker or symptom, a "increase" is a statistically significant increase in such level.

As used herein, a "subject" means a human or animal. Usually the animal is a vertebrate such as a primate, rodent, domestic animal or game animal. Primates include chimpanzees, cynomologous monkeys, spider monkeys, and macaques, e.g., Rhesus. Rodents include mice, rats, woodchucks, ferrets, rabbits and hamsters. Domestic and game animals include cows, horses, pigs, deer, bison, buffalo, feline species, e.g., domestic cat, canine species, e.g., dog, fox, wolf, avian species, e.g., chicken, emu, ostrich, and fish, e.g., trout, catfish and salmon. In some embodiments, the subject is a mammal, e.g., a primate, e.g., a human. The terms, "individual," "patient" and "subject" are used interchangeably herein.

Preferably, the subject is a mammal. The mammal can be a human, non-human primate, mouse, rat, dog, cat, horse, or cow, but is not limited to these examples. Mammals other than humans can be advantageously used as subjects that represent animal models of cancer. A subject can be male or female.

A subject can be one who has been previously diagnosed with or identified as suffering from or having a condition in need of treatment (e.g. cancer) or one or more complications related to such a condition, and optionally, have already undergone treatment for cancer or the one or more complications related to cancer. Alternatively, a subject can also be one who has not been previously diagnosed as having cancer or one or more complications related to cancer. For example, a subject can be one who exhibits one or more risk factors for cancer or one or more complications related to cancer or a subject who does not exhibit risk factors.

A "subject in need" of treatment for a particular condition can be a subject having that condition, diagnosed as having that condition, or at risk of developing that condition.

As used herein "an increased likelihood" refers to at least a 1.5-fold greater likelihood of a particular scenario, e.g. a 1.5 fold, or 2-fold, or 2.5-fold, or 3-fold, or 4-fold, or greater risk. As used herein "a decreased likelihood" refers to at least a 80% lower likelihood of a particular scenario, e.g. a 80% lower, a 50% lower, 40% lower, 30% lower, 20% lower, 10% lower, or lower risk.

As used herein, the term "cancer"or "tumor" refers to an uncontrolled growth of cells which interferes with the normal functioning of the bodily organs and systems. A subject that has a cancer or a tumor is a subject having objectively measurable cancer cells present in the subject's body. Included in this definition are benign and malignant cancers, as well as dormant tumors or micrometastases. Cancers which migrate from their original location and seed vital organs can eventually lead to the death of the subject through the functional deterioration of the affected organs.

As used herein "gene copy number" refers to the number of copies of a given gene that occur in the genome. As used herein, "gene amplification" refers to the presence of a greater than normal gene copy number within the cell. In some embodiments, the copies are located on the same chromosome. In some embodiments, the copies are located on more than one chromosome. In some embodiments, gene copy number can include partial copies of a gene, e.g. less than the full coding sequence.

As used herein, "expression level" refers to the number of mRNA molecules and/or polypeptide molecules encoded by a given gene that are present in a cell or sample. Expression levels can be increased or decreased relative to a reference level.

The term "agent" refers generally to any entity which is normally not present or not present at the levels being administered to a cell, tissue or subject. An agent can be selected from a group including but not limited to: polynucleotides; polypeptides; small molecules; and antibodies or antigen-binding fragments thereof. A polynucleotide can be RNA or DNA, and can be single or double stranded, and can be selected from a group including, for example, nucleic acids and nucleic acid analogues that encode a polypeptide. A polypeptide can be, but is not limited to, a naturally-occurring polypeptide, a mutated polypeptide or a fragment thereof that retains the function of interest. Further examples of agents include, but are not limited to a nucleic acid aptamer, peptide-nucleic acid (PNA), locked nucleic acid (LNA), small organic or inorganic molecules; saccharide; oligosaccharides; polysaccharides; biological macromolecules, peptidomimetics; nucleic acid analogs and derivatives; extracts made from biological materials such as bacteria, plants, fungi, or mammalian cells or tissues and naturally occurring or synthetic compositions. An agent can be applied to the media, where it contacts the cell and induces its effects. Alternatively, an agent can be intracellular as a result of introduction of a nucleic acid sequence encoding the agent into the cell and its transcription resulting in the production of the nucleic acid and/or protein environmental stimuli within the cell. In some embodiments, the agent is any chemical, entity or moiety, including without limitation synthetic and naturally-occurring non-proteinaceous entities. In certain embodiments the agent is a small molecule having a chemical moiety selected, for example, from unsubstituted or substituted alkyl, aromatic, or heterocyclyl moieties including macrolides, leptomycins and related natural products or analogues thereof. Agents can be known to have a desired activity and/or property, or can be selected from a library of diverse compounds. As used herein, the term "small molecule" can refer to compounds that are "natural product-like," however, the term "small molecule" is not limited to "natural product-like" compounds. Rather, a small molecule is typically characterized in that it contains several carbon-carbon bonds, and has a molecular weight more than about 50, but less than about 5000 Daltons (5 kD). Preferably the small molecule has a molecular weight of less than 3 kD, still more preferably less than 2 kD, and most preferably less than 1 kD. In some cases it is preferred that a small molecule have a molecular mass equal to or less than 700 Daltons.

As used herein the term "chemotherapeutic agent" refers to any chemical or biological agent with therapeutic usefulness in the treatment of diseases characterized by abnormal cell growth. Such diseases include tumors, neoplasms and cancer as well as diseases characterized by hyperplastic growth. These agents can function to inhibit a cellular activity upon which the cancer cell depends for continued proliferation. In some aspect of all the embodiments, a chemotherapeutic agent is a cell cycle inhibitor or a cell division inhibitor. Categories of chemotherapeutic agents that are useful in the methods disclosed herein include alkylating/alkaloid agents, antimetabolites, hormones or hormone analogs, and miscellaneous antineoplastic drugs. Most of these agents are directly or indirectly toxic to cancer cells. In one embodiment, a chemotherapeutic agent is a radioactive molecule. One of skill in the art can readily identify a chemotherapeutic agent of use (e.g. see Slapak and Kufe, Principles of Cancer Therapy, Chapter 86 in Harrison's Principles of Internal Medicine, 14th edition; Perry et al. , Chemotherapy, Ch. 17 in Abeloff, Clinical Oncology 2nd ed. 2000 Churchill Livingstone, Inc; Baltzer L, Berkery R (eds): Oncology Pocket Guide to Chemotherapy, 2nd ed. St. Louis, Mosby-Year Book, 1995; Fischer D S, Knobf M F, Durivage H J (eds): The Cancer Chemotherapy Handbook, 4th ed. St. Louis, Mosby-Year Book, 1993). In some embodiments, the chemotherapeutic agent can be a cytotoxic chemotherapeutic. The term "cytotoxic agent" as used herein refers to a substance that inhibits or prevents the function of cells and/or causes destruction of cells. The term is intended to include radioactive isotopes (e.g. At211, 1131, 1125, Y90, Re186, Re188, Sm153, Bi212, P32 and radioactive isotopes of Lu), chemotherapeutic agents, and toxins, such as small molecule toxins or enzymatically active toxins of bacterial, fungal, plant or animal origin, including fragments and/or variants thereof.

As used herein, the terms "protein" and "polypeptide" are used interchangeably herein to designate a series of amino acid residues, connected to each other by peptide bonds between the alpha-amino and carboxy groups of adjacent residues. The terms "protein", and "polypeptide" refer to a polymer of amino acids, including modified amino acids (e.g., phosphorylated, glycated, glycosylated, etc.) and amino acid analogs, regardless of its size or function. "Protein" and "polypeptide" are often used in reference to relatively large polypeptides, whereas the term "peptide" is often used in reference to small polypeptides, but usage of these terms in the art overlaps. The terms "protein" and "polypeptide" are used interchangeably herein when referring to a gene product and fragments thereof. Thus, exemplary polypeptides or proteins include gene products, naturally occurring proteins, homologs, orthologs, paralogs, fragments and other equivalents, variants, fragments, and analogs of the foregoing.

As used herein an "antibody" refers to IgG, IgM, IgA, IgD or IgE molecules or antigen-specific antibody fragments thereof (including, but not limited to, a Fab, F(ab')₂, Fv, disulphide linked Fv, scFv, single domain antibody, closed conformation multispecific antibody, disulphide-linked scfv, diabody), whether derived from any species that naturally produces an antibody, or created by recombinant DNA technology; whether isolated from serum, B-cells, hybridomas, transfectomas, yeast or bacteria.

As described herein, an "antigen" is a molecule that is bound by a binding site on an antibody agent. Typically, antigens are bound by antibody ligands and are capable of raising an antibody response *in vivo.* An antigen can be a polypeptide, protein, nucleic acid or other molecule or portion thereof. The term "antigenic determinant" refers to an epitope on the antigen recognized by an antigen-binding molecule, and more particularly, by the antigen-binding site of said molecule.

As used herein, the term "antibody reagent" refers to a polypeptide that includes at least one immunoglobulin variable domain or immunoglobulin variable domain sequence and which specifically binds a given antigen. An antibody reagent can comprise an antibody or a polypeptide comprising an antigen-binding domain of an antibody. In some embodiments, an antibody reagent can comprise a monoclonal antibody or a polypeptide comprising an antigen-binding domain of a monoclonal antibody. For example, an antibody can include a heavy (H) chain variable region (abbreviated herein as VH), and a light (L) chain variable region (abbreviated herein as VL). In another example, an antibody includes two heavy (H) chain variable regions and two light (L) chain variable regions. The term "antibody reagent" encompasses antigen-binding fragments of antibodies (e.g., single chain antibodies, Fab and sFab fragments, F(ab')2, Fd fragments, Fv fragments, scFv, and domain antibodies (dAb) fragments (see, e.g. de Wildt et al., Eur J. Immunol. 1996; 26(3):629-39as well as complete antibodies. An antibody can have the structural features of IgA, IgG, IgE, IgD, IgM (as well as subtypes and combinations thereof). Antibodies can be from any source, including mouse, rabbit, pig, rat, and primate (human and non-human primate) and primatized antibodies. Antibodies also include midibodies, humanized antibodies, chimeric antibodies, and the like.

The VH and VL regions can be further subdivided into regions of hypervariability, termed "complementarity determining regions" ("CDR"), interspersed with regions that are more conserved, termed "framework regions" ("FR"). The extent of the framework region and CDRs has been precisely defined (see, Kabat, E. A., et al. (1991) Sequences of Proteins of Immunological Interest, Fifth Edition, U.S. Department of Health and Human Services, NIH Publication No. 91-3242, and Chothia, C. et al. (1987) J. Mol. Biol. 196:901-917). Each VH and VL is typically composed of three CDRs and four FRs, arranged from amino-terminus to carboxy-terminus in the following order: FR1, CDR1, FR2, CDR2, FR3, CDR3, FR4.

The terms "antigen-binding fragment" or "antigen-binding domain", which are used interchangeably herein are used to refer to one or more fragments of a full length antibody that retain the ability to specifically bind to a target of interest. Examples of binding fragments encompassed within the term "antigen-binding fragment" of a full length antibody include (i) a Fab fragment, a monovalent fragment consisting of the VL, VH, CL and CH1 domains; (ii) a F(ab')2 fragment, a bivalent fragment including two Fab fragments linked by a disulfide bridge at the hinge region; (iii) an Fd fragment consisting of the VH and CH1 domains; (iv) an Fv fragment consisting of the VL and VH domains of a single arm of an antibody, (v) a dAb fragment (Ward et al., (1989) Nature 341:544-546), which consists of a VH or VL domain; and (vi) an isolated complementarity determining region (CDR) that retains specific antigen-binding functionality. As used herein, the term "specific binding" refers to a chemical interaction between two molecules, compounds, cells and/or particles wherein the first entity binds to the second, target entity with greater specificity and affinity than it binds to a third entity which is a non-target. In some embodiments, specific binding can refer to an affinity of the first entity for the second target entity which is at least 10 times, at least 50 times, at least 100 times, at least 500 times, at least 1000 times or greater than the affinity for the third nontarget entity.

Additionally, and as described herein, a recombinant humanized antibody can be further optimized to decrease potential immunogenicity, while maintaining functional activity, for therapy in humans. In this regard, functional activity means a polypeptide capable of displaying one or more known functional activities associated with a recombinant antibody or antibody reagent thereof as described herein. Such functional activities include, e.g. the ability to bind to KDM4A.

As used herein, the term "nucleic acid" or "nucleic acid sequence" refers to any molecule, preferably a polymeric molecule, incorporating units of ribonucleic acid, deoxyribonucleic acid or an analog thereof. The nucleic acid can be either single-stranded or double-stranded. A single-stranded nucleic acid can be one nucleic acid strand of a denatured double- stranded DNA. Alternatively, it can be a single-stranded nucleic acid not derived from any double-stranded DNA. In one aspect, the nucleic acid can be DNA. In another aspect, the nucleic acid can be RNA. Suitable nucleic acid molecules are DNA, including genomic DNA or cDNA. Other suitable nucleic acid molecules are RNA, including mRNA.

Aptamers are short synthetic single-stranded oligonucleotides that specifically bind to various molecular targets such as small molecules, proteins, nucleic acids, and even cells and tissues. These small nucleic acid molecules can form secondary and tertiary structures capable of specifically binding proteins or other cellular targets, and are essentially a chemical equivalent of antibodies. Aptamers are highly specific, relatively small in size, and non-immunogenic. Aptamers are generally selected from a biopanning method known as SELEX (Systematic Evolution of Ligands by Exponential enrichment) (Ellington et al. Nature. 1990;346(6287):818-822; Tuerk et al., Science. 1990;249(4968):505-510; Ni et al., Curr Med Chem. 2011;18(27):4206-14). Methods of generating an apatmer for any given target are well known in the art. Preclinical studies using, e.g. aptamer-siRNA chimeras
and aptamer targeted nanoparticle therapeutics have been very successful in mouse models of cancer and HIV (Ni et al., Curr Med Chem. 2011;18(27):4206-14).

Inhibitors of the expression of a given gene can be an inhibitory nucleic acid. In some embodiments, the inhibitory nucleic acid is an inhibitory RNA (iRNA). Double-stranded RNA molecules (dsRNA) have been shown to block gene expression in a highly conserved regulatory mechanism known as RNA interference (RNAi). The inhibitory nucleic acids described herein can include an RNA strand (the antisense strand) having a region which is 30 nucleotides or less in length, i.e., 15-30 nucleotides in length, generally 19-24 nucleotides in length, which region is substantially complementary to at least part the targeted mRNA transcript. The use of these iRNAs enables the targeted degradation of mRNA transcripts, resulting in decreased expression and/or activity of the target.

As used herein, the term "iRNA" refers to an agent that contains RNA as that term is defined herein, and which mediates the targeted cleavage of an RNA transcript via an RNA-induced silencing complex (RISC) pathway. In one embodiment, an iRNA as described herein effects inhibition of the expression and/or activity of KDM4A. In certain embodiments, contacting a cell with the inhibitor (e.g. an iRNA) results in a decrease in the target mRNA level in a cell by at least about 5%, about 10%, about 20%, about 30%, about 40%, about 50%, about 60%, about 70%, about 80%, about 90%, about 95%, about 99%, up to and including 100% of the target mRNA level found in the cell without the presence of the iRNA.

In some embodiments, the iRNA can be a dsRNA. A dsRNA includes two RNA strands that are sufficiently complementary to hybridize to form a duplex structure under conditions in which the dsRNA will be used. One strand of a dsRNA (the antisense strand) includes a region of complementarity that is substantially complementary, and generally fully complementary, to a target sequence. The target sequence can be derived from the sequence of an mRNA formed during the expression of the target. The other strand (the sense strand) includes a region that is complementary to the antisense strand, such that the two strands hybridize and form a duplex structure when combined under suitable conditions. Generally, the duplex structure is between 15 and 30 inclusive, more generally between 18 and 25 inclusive, yet more generally between 19 and 24 inclusive, and most generally between 19 and 21 base pairs in length, inclusive. Similarly, the region of complementarity to the target sequence is between 15 and 30 inclusive, more generally between 18 and 25 inclusive, yet more generally between 19 and 24 inclusive, and most generally between 19 and 21 nucleotides in length, inclusive. In some embodiments, the dsRNA is between 15 and 20 nucleotides in length, inclusive, and in other embodiments, the dsRNA is between 25 and 30 nucleotides in length, inclusive. As the ordinarily skilled person will recognize, the targeted region of an RNA targeted for cleavage will most often be part of a larger RNA molecule, often an mRNA molecule. Where relevant, a "part" of an mRNA target is a contiguous sequence of an mRNA target of sufficient length to be a substrate for RNAi-directed cleavage (i.e., cleavage through a RISC pathway). dsRNAs having duplexes as short as 9 base pairs can, under some circumstances, mediate RNAi-directed RNA cleavage. Most often a target will be at least 15 nucleotides in length, preferably 15-30 nucleotides in length.

In yet another embodiment, the RNA of an iRNA, e.g., a dsRNA, is chemically modified to enhance stability or other beneficial characteristics. The nucleic acids featured in the invention may be synthesized and/or modified by methods well established in the art, such as those described in "Current protocols in nucleic acid chemistry," Beaucage, S.L. et al. (Edrs.), John Wiley & Sons, Inc., New York, NY, USA. Modifications include, for example, (a) end modifications, e.g., 5' end modifications (phosphorylation, conjugation, inverted linkages, etc.) 3' end modifications (conjugation, DNA nucleotides, inverted linkages, etc.), (b) base modifications, e.g., replacement with stabilizing bases, destabilizing bases, or bases that base pair with an expanded repertoire of partners, removal of bases (abasic nucleotides), or conjugated bases, (c) sugar modifications (e.g., at the 2' position or 4' position) or replacement of the sugar, as well as (d) backbone modifications, including modification or replacement of the phosphodiester linkages. Specific examples of RNA compounds useful in the embodiments described herein include, but are not limited to RNAs containing modified backbones or no natural internucleoside linkages. RNAs having modified backbones include, among others, those that do not have a phosphorus atom in the backbone. For the purposes of this specification, and as sometimes referenced in the art, modified RNAs that do not have a phosphorus atom in their internucleoside backbone can also be considered to be oligonucleosides. In particular embodiments, the modified RNA will have a phosphorus atom in its internucleoside backbone.

Modified RNA backbones can include, for example, phosphorothioates, chiral phosphorothioates, phosphorodithioates, phosphotriesters, aminoalkylphosphotriesters, methyl and other alkyl phosphonates including 3'-alkylene phosphonates and chiral phosphonates, phosphinates, phosphoramidates including 3'-amino phosphoramidate and aminoalkylphosphoramidates, thionophosphoramidates, thionoalkylphosphonates, thionoalkylphosphotriesters, and boranophosphates having normal 3'-5' linkages, 2'-5' linked analogs of these, and those) having inverted polarity wherein the adjacent pairs of nucleoside units are linked 3'-5' to 5'-3' or 2'-5' to 5'-2'. Various salts, mixed salts and free acid forms are also included. Representative U.S. patents that teach the preparation of the above phosphorus-containing linkages include, but are not limited to, U.S. Pat. Nos. 3,687,808; 4,469,863; 4,476,301; 5,023,243; 5,177,195; 5,188,897; 5,264,423; 5,276,019; 5,278,302; 5,286,717; 5,321,131; 5,399,676; 5,405,939; 5,453,496; 5,455,233; 5,466,677; 5,476,925; 5,519,126; 5,536,821; 5,541,316; 5,550,111; 5,563,253; 5,571,799; 5,587,361; 5,625,050; 6,028,188; 6,124,445; 6,160,109; 6,169,170; 6,172,209; 6, 239,265; 6,277,603; 6,326,199; 6,346,614; 6,444,423; 6,531,590; 6,534,639; 6,608,035; 6,683,167; 6,858,715; 6,867,294; 6,878,805; 7,015,315; 7,041,816; 7,273,933; 7,321,029; and US Pat RE39464

Modified RNA backbones that do not include a phosphorus atom therein have backbones that are formed by short chain alkyl or cycloalkyl internucleoside linkages, mixed heteroatoms and alkyl or cycloalkyl internucleoside linkages, or one or more short chain heteroatomic or heterocyclic internucleoside linkages. These include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; amide backbones; and others having mixed N, O, S and CH₂ component parts. Representative U.S. patents that teach the preparation of the above oligonucleosides include, but are not limited to, U.S. Pat. Nos. 5,034,506; 5,166,315; 5,185,444; 5,214,134; 5,216,141; 5,235,033; 5,64,562; 5,264,564; 5,405,938; 5,434,257; 5,466,677; 5,470,967; 5,489,677; 5,541,307; 5,561,225; 5,596,086; 5,602,240; 5,608,046; 5,610,289; 5,618,704; 5,623,070; 5,663,312; 5,633,360; 5,677,437; and, 5,677,439.

In other RNA mimetics suitable or contemplated for use in iRNAs, both the sugar and the internucleoside linkage, *i.e.,* the backbone, of the nucleotide units are replaced with novel groups. The base units are maintained for hybridization with an appropriate nucleic acid target compound. One such oligomeric compound, an RNA mimetic that has been shown to have excellent hybridization properties, is referred to as a peptide nucleic acid (PNA). In PNA compounds, the sugar backbone of an RNA is replaced with an amide containing backbone, in particular an aminoethylglycine backbone. The nucleobases are retained and are bound directly or indirectly to aza nitrogen atoms of the amide portion of the backbone. Representative U.S. patents that teach the preparation of PNA compounds include, but are not limited to, U.S. Pat. Nos. 5,539,082; 5,714,331; and 5,719,262. Further teaching of PNA compounds can be found, for example, in Nielsen et al., Science, 1991, 254, 1497-1500.

Some embodiments featured in the invention include RNAs with phosphorothioate backbones and oligonucleosides with heteroatom backbones, and in particular --CH₂--NH--CH₂--, --CH₂--N(CH₃)--O--CH₂-[known as a methylene (methylimino) or MMI backbone], --CH₂--O--N(CH₃)--CH₂--, --CH₂--N(CH₃)-N(CH₃)--CH₂-- and --N(CH₃)--CH₂--CH₂--[wherein the native phosphodiester backbone is represented as --O- -P--O--CH₂--] of the above-referenced U.S. Pat. No. 5,489,677, and the amide backbones of the above-referenced U.S. Pat. No. 5,602,240. In some embodiments, the RNAs featured herein have morpholino backbone structures of the above-referenced U.S. Pat. No. 5,034,506.

Modified RNAs can also contain one or more substituted sugar moieties. The iRNAs, e.g., dsRNAs, featured herein can include one of the following at the 2' position: OH; F; O-, S-, or N-alkyl; O-, S-, or N-alkenyl; O-, S- or N-alkynyl; or O-alkyl-O-alkyl, wherein the alkyl, alkenyl and alkynyl may be substituted or unsubstituted C₁ to C₁₀ alkyl or C₂ to C₁₀ alkenyl and alkynyl. Exemplary suitable modifications include O[(CH₂)ₙO]ₘCH₃, O(CH₂)ₙOCH₃, O(CH₂)ₙNH₂, O(CH₂)ₙCH₃, O(CH₂)ₙONH₂, and O(CH₂)ₙON[(CH₂)ₙCH₃)]₂, where n and m are from 1 to about 10. In other embodiments, dsRNAs include one of the following at the 2' position: C₁ to C₁₀ lower alkyl, substituted lower alkyl, alkaryl, aralkyl, O-alkaryl or O-aralkyl, SH, SCH₃, OCN, Cl, Br, CN, CF₃, OCF₃, SOCH₃, SO₂CH₃, ONO₂, NO₂, N₃, NH₂, heterocycloalkyl, heterocycloalkaryl, aminoalkylamino, polyalkylamino, substituted silyl, an RNA cleaving group, a reporter group, an intercalator, a group for improving the pharmacokinetic properties of an iRNA, or a group for improving the pharmacodynamic properties of an iRNA, and other substituents having similar properties. In some embodiments, the modification includes a 2'-methoxyethoxy (2'-O--CH₂CH₂OCH₃, also known as 2'-O-(2-methoxyethyl) or 2'-MOE) (Martin et al., Helv. Chim. Acta, 1995, 78:486-504) *i.e.,* an alkoxy-alkoxy group. Another exemplary modification is 2'-dimethylaminooxyethoxy, *i.e.,* a O(CH₂)₂ON(CH₃)₂ group, also known as 2'-DMAOE, as described in examples herein below, and 2'-dimethylaminoethoxyethoxy (also known in the art as 2'-O-dimethylaminoethoxyethyl or 2'-DMAEOE), *i.e.,* 2'-O--CH₂--O--CH₂--N(CH₂)₂, also described in examples herein below.

Other modifications include 2'-methoxy (2'-OCH₃), 2'-aminopropoxy (2'-OCH₂CH₂CH₂NH₂) and 2'-fluoro (2'-F). Similar modifications can also be made at other positions on the RNA of an iRNA, particularly the 3' position of the sugar on the 3' terminal nucleotide or in 2'-5' linked dsRNAs and the 5' position of 5' terminal nucleotide. iRNAs may also have sugar mimetics such as cyclobutyl moieties in place of the pentofuranosyl sugar. Representative U.S. patents that teach the preparation of such modified sugar structures include, but are not limited to, U.S. Pat. Nos. 4,981,957; 5,118,800; 5,319,080; 5,359,044; 5,393,878; 5,446,137; 5,466,786; 5,514,785; 5,519,134; 5,567,811; 5,576,427; 5,591,722; 5,597,909; 5,610,300; 5,627,053; 5,639,873; 5,646,265; 5,658,873; 5,670,633; and 5,700,920, certain of which are commonly owned with the instant application.

An iRNA can also include nucleobase (often referred to in the art simply as "base") modifications or substitutions. As used herein, "unmodified" or "natural" nucleobases include the purine bases adenine (A) and guanine (G), and the pyrimidine bases thymine (T), cytosine (C) and uracil (U). Modified nucleobases include other synthetic and natural nucleobases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl uracil and cytosine, 6-azo uracil, cytosine and thymine, 5-uracil (pseudouracil), 4-thiouracil, 8-halo, 8-amino, 8-thiol, 8-thioalkyl, 8-hydroxyl anal other 8-substituted adenines and guanines, 5-halo, particularly 5-bromo, 5-trifluoromethyl and other 5-substituted uracils and cytosines, 7-methylguanine and 7-methyladenine, 8-azaguanine and 8-azaadenine, 7-deazaguanine and 7-daazaadenine and 3-deazaguanine and 3-deazaadenine. Further nucleobases include those disclosed in U.S. Pat. No. 3,687,808, those disclosed in Modified Nucleosides in Biochemistry, Biotechnology and Medicine, Herdewijn, P. ed. Wiley-VCH, 2008; those disclosed in The Concise Encyclopedia Of Polymer Science And Engineering, pages 858-859, Kroschwitz, J. L, ed. John Wiley & Sons, 1990, these disclosed by Englisch et al., Angewandte Chemie, International Edition, 1991, 30, 613, and those disclosed by Sanghvi, Y S., Chapter 15, dsRNA Research and Applications, pages 289-302, Crooke, S. T. and Lebleu, B., Ed., CRC Press, 1993. Certain of these nucleobases are particularly useful for increasing the binding affinity of the oligomeric compounds featured in the invention. These include 5-substituted pyrimidines, 6-azapyrimidines and N-2, N-6 and 0-6 substituted purines, including 2-aminopropyladenine, 5-propynyluracil and 5-propynylcytosine. 5-methylcytosine substitutions have been shown to increase nucleic acid duplex stability by 0.6-1.2°C (Sanghvi, Y. S., Crooke, S. T. and Lebleu, B., Eds., dsRNA Research and Applications, CRC Press, Boca Raton, 1993, pp. 276-278) and are exemplary base substitutions, even more particularly when combined with 2'-O-methoxyethyl sugar modifications.

Representative U.S. patents that teach the preparation of certain of the above noted modified nucleobases as well as other modified nucleobases include, but are not limited to, the above noted U.S. Pat. No. 3,687,808, as well as U.S. Pat. Nos. 4,845,205; 5,130,30; 5,134,066; 5,175,273; 5,367,066; 5,432,272; 5,457,187; 5,459,255; 5,484,908; 5,502,177; 5,525,711; 5,552,540; 5,587,469; 5,594,121, 5,596,091; 5,614,617; 5,681,941; 6,015,886; 6,147,200; 6,166,197; 6,222,025; 6,235,887; 6,380,368; 6,528,640; 6,639,062; 6,617,438; 7,045,610; 7,427,672; and 7,495,088, and U.S. Pat. No. 5,750,692.

The RNA of an iRNA can also be modified to include one or more locked nucleic acids (LNA). A locked nucleic acid is a nucleotide having a modified ribose moiety in which the ribose moiety comprises an extra bridge connecting the 2' and 4' carbons. This structure effectively "locks" the ribose in the 3'-endo structural conformation. The addition of locked nucleic acids to siRNAs has been shown to increase siRNA stability in serum, and to reduce off-target effects (Elmen, J. et al., (2005) Nucleic Acids Research 33(1):439-447; Mook, OR. et al., (2007) Mol Canc Ther 6(3):833-843; Grunweller, A. et al., (2003) Nucleic Acids Research 31(12):3185-3193). Representative U.S. Patents that teach the preparation of locked nucleic acid nucleotides include, but are not limited to, the following: U.S. Pat. Nos. 6,268,490; 6,670,461; 6,794,499; 6,998,484; 7,053,207; 7,084,125; and 7,399,845.

Another modification of the RNA of an iRNA featured in the invention involves chemically linking to the RNA one or more ligands, moieties or conjugates that enhance the activity, cellular distribution, pharmacokinetic properties, or cellular uptake of the iRNA. Such moieties include but are not limited to lipid moieties such as a cholesterol moiety (Letsinger et al., Proc. Natl. Acid. Sci. USA, 1989, 86: 6553-6556), cholic acid (Manoharan et al., Biorg. Med. Chem. Let., 1994, 4:1053-1060), a thioether, *e.g.,* beryl-S-tritylthiol (Manoharan et al., Ann. N.Y. Acad. Sci., 1992, 660:306-309; Manoharan et al., Biorg. Med. Chem. Let., 1993, 3:2765-2770), a thiocholesterol (Oberhauser et al., Nucl. Acids Res., 1992, 20:533-538), an aliphatic chain, *e.g.,* dodecandiol or undecyl residues (Saison-Behmoaras et al., EMBO J, 1991, 10:1111-1118; Kabanov et al., FEBS Lett., 1990, 259:327-330; Svinarchuk et al., Biochimie, 1993, 75:49-54), a phospholipid, e.g., di-hexadecyl-rac-glycerol or triethyl-ammonium 1,2-di-O-hexadecyl-rac-glycero-3-phosphonate (Manoharan et al., Tetrahedron Lett., 1995, 36:3651-3654; Shea et al., Nucl. Acids Res., 1990, 18:3777-3783), a polyamine or a polyethylene glycol chain (Manoharan et al., Nucleosides & Nucleotides, 1995, 14:969-973), or adamantane acetic acid (Manoharan et al., Tetrahedron Lett., 1995, 36:3651-3654), a palmityl moiety (Mishra et al., Biochim. Biophys. Acta, 1995, 1264:229-237), or an octadecylamine or hexylamino-carbonyloxycholesterol moiety (Crooke et al., J. Pharmacol. Exp. Ther., 1996, 277:923-937).

As used herein, the terms "treat," "treatment," "treating," or "amelioration" refer to therapeutic treatments, wherein the object is to reverse, alleviate, ameliorate, inhibit, slow down or stop the progression or severity of a condition associated with a disease or disorder, e.g. cancer. The term "treating" includes reducing or alleviating at least one adverse effect or symptom of a condition, disease or disorder associated with a cancer. Treatment is generally "effective" if one or more symptoms or clinical markers are reduced. Alternatively, treatment is "effective" if the progression of a disease is reduced or halted. That is, "treatment" includes not just the improvement of symptoms or markers, but also a cessation of, or at least slowing of, progress or worsening of symptoms compared to what would be expected in the absence of treatment. Beneficial or desired clinical results include, but are not limited to, alleviation of one or more symptom(s), diminishment of extent of disease, stabilized (*i.e.,* not worsening) state of disease, delay or slowing of disease progression, amelioration or palliation of the disease state, remission (whether partial or total), and/or decreased mortality, whether detectable or undetectable. The term "treatment" of a disease also includes providing relief from the symptoms or side-effects of the disease (including palliative treatment).

As used herein, the term "pharmaceutical composition" refers to the active agent in combination with a pharmaceutically acceptable carrier e.g. a carrier commonly used in the pharmaceutical industry. The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

As used herein, the term "administering," refers to the placement of a compound as disclosed herein into a subject by a method or route which results in at least partial delivery of the agent at a desired site. Pharmaceutical compositions comprising the compounds disclosed herein can be administered by any appropriate route which results in an effective treatment in the subject.

The term "statistically significant" or "significantly" refers to statistical significance and generally means a two standard deviation (2SD) or greater difference. In some embodiments, statistically significant can refer to P ≤ to 0.05 by two-tailed student's T-test, e.g. in the experimental data presented in the Examples herein.

Other than in the operating examples, or where otherwise indicated, all numbers expressing quantities of ingredients or reaction conditions used herein should be understood as modified in all instances by the term "about." The term "about" when used in connection with percentages can mean ±1%.

As used herein the term "comprising" or "comprises" is used in reference to compositions, methods, and respective component(s) thereof, that are essential to the method or composition, yet open to the inclusion of unspecified elements, whether essential or not.

The term "consisting of' refers to compositions, methods, and respective components thereof as described herein, which are exclusive of any element not recited in that description of the embodiment.

As used herein the term "consisting essentially of' refers to those elements required for a given embodiment. The term permits the presence of elements that do not materially affect the basic and novel or functional characteristic(s) of that embodiment.

The singular terms "a," "an," and "the" include plural referents unless context clearly indicates otherwise. Similarly, the word "or" is intended to include "and" unless the context clearly indicates otherwise. Although methods and materials similar or equivalent to those described herein can be used in the practice or testing of this disclosure, suitable methods and materials are described below. The abbreviation, "e.g." is derived from the Latin exempli gratia, and is used herein to indicate a non-limiting example. Thus, the abbreviation "e.g." is synonymous with the term "for example."

Definitions of common terms in cell biology and molecular biology can be found in "The Merck Manual of Diagnosis and Therapy", 19th Edition, published by Merck Research Laboratories, 2006 (ISBN 0-911910-19-0); Robert S. Porter et al. (eds.), The Encyclopedia of Molecular Biology, published by Blackwell Science Ltd., 1994 (ISBN 0-632-02182-9); Immunology by Werner Luttmann, published by Elsevier, 2006; Benjamin Lewin, Genes X, published by Jones & Bartlett Publishing, 2009 (ISBN-10: 0763766321); Kendrew et al. (eds.), , Molecular Biology and Biotechnology: a Comprehensive Desk Reference, published by VCH Publishers, Inc., 1995 (ISBN 1-56081-569-8) and Current Protocols in Protein Sciences 2009, Wiley Intersciences, Coligan et al., eds.

Unless otherwise stated, the present invention was performed using standard procedures, as described, for example in Sambrook et al., Molecular Cloning: A Laboratory Manual (3 ed.), Cold Spring Harbor Laboratory Press, Cold Spring Harbor, N.Y., USA (2001); Davis et al., Basic Methods in Molecular Biology, Elsevier Science Publishing, Inc., New York, USA (1995); Current Protocols in Protein Science (CPPS) (John E. Coligan, et. al., ed., John Wiley and Sons, Inc.), Current Protocols in Cell Biology (CPCB) (Juan S. Bonifacino et. al. ed., John Wiley and Sons, Inc.), and Culture of Animal Cells: A Manual of Basic Technique by R. Ian Freshney, Publisher: Wiley-Liss; 5th edition (2005), Animal Cell Culture Methods (Methods in Cell Biology, Vol. 57, Jennie P. Mather and David Barnes editors, Academic Press, 1st edition, 1998).

Other terms are defined herein within the description of the various aspects of the invention.

All patents and other publications; including literature references, issued patents, published patent applications, and co-pending patent applications; cited throughout this application are provided solely for their disclosure prior to the filing date of the present application. Nothing in this regard should be construed as an admission that the inventors are not entitled to antedate such disclosure by virtue of prior invention or for any other reason. All statements as to the date or representation as to the contents of these documents is based on the information available to the applicants and does not constitute any admission as to the correctness of the dates or contents of these documents.

The description of embodiments of the disclosure is not intended to be exhaustive or to limit the disclosure to the precise form disclosed. While specific embodiments of, and examples for, the disclosure are described herein for illustrative purposes, various equivalent modifications are possible within the scope of the disclosure, as those skilled in the relevant art will recognize. For example, while method steps or functions are presented in a given order, alternative embodiments may perform functions in a different order, or functions may be performed substantially concurrently. The teachings of the disclosure provided herein can be applied to other procedures or methods as appropriate. The various embodiments described herein can be combined to provide further embodiments. Aspects of the disclosure can be modified, if necessary, to employ the compositions, functions and concepts of the above references and application to provide yet further embodiments of the disclosure. Moreover, due to biological functional equivalency considerations, some changes can be made in protein structure without affecting the biological or chemical action in kind or amount. These and other changes can be made to the disclosure in light of the detailed description. All such modifications are intended to be included within the scope of the appended claims.

Specific elements of any of the foregoing embodiments can be combined or substituted for elements in other embodiments. Furthermore, while advantages associated with certain embodiments of the disclosure have been described in the context of these embodiments, other embodiments may also exhibit such advantages, and not all embodiments need necessarily exhibit such advantages to fall within the scope of the disclosure.

The technology described herein is further illustrated by the following examples which in no way should be construed as being further limiting.

### EXAMPLES

### EXAMPLE 1: H3K9/36me3 Demethylase KDM4A Promotes Site-Specific Copy Gain and Rereplication of Regions Amplified in Tumors

Acquired chromosomal instability and copy number alterations are hallmarks of cancer. Enzymes capable of promoting site-specific copy number changes have yet to be identified. It is demonstrated herein that H3K9/36me3 lysine demethylase KDM4A/JMJD2A overexpression leads to localized copy gain of 1q12, 1q21, and Xq13.1 without global chromosome instability. KDM4A amplified tumors have increased copy gains for these same regions. The local copy gain of 1q12h occurs within a single cell cycle, requires S phase and is not stable but regenerated each cell division. Sites with increased copy number are re-replicated and have increased KDM4A, MCMs and DNA polymerase localization. HP1γ or KMT1A/Suv39h1 overexpression suppresses the copy gain, while H3K9/K36 methylation interference promotes gain. These results demonstrate that overexpression of a chromatin modifier results in site-specific copy gains. This indicates how copy number changes can originate during tumorigenesis and demonstrates that transient overexpression of specific chromatin modulators can promote these events.

### INTRODUCTION

Genomic instability is a major contributing factor to the development and onset of age-related diseases such as cancer (Maslov and Vijg, 2009; Negrini et al., 2010). Cancer cells are often characterized by copy number alterations: copy gains or losses of chromosome arms and/or whole chromosomes as well as amplifications of smaller genomic fragments (Beroukhim et al., 2010; Hook et al., 2007; Stratton et al., 2009). Genome wide analysis of copy number changes in cancer has identified chromosomal regions with higher frequencies of amplification, which often contain putative oncogenes (Beroukhim et al., 2010). In some cases, the oncogenes have been shown to impact cellular behavior (e.g., cMyc and Mcl1), while other genes within these regions do not have clear connections with tumorigenesis. The lack of obvious connection does not preclude the gene's involvement. For example, cellular stresses (e.g., environmental and chemotherapeutic) can select for gene amplification that will promote cancer cell survival, as exemplified by the amplification of dihydrofolate reductase (DHFR) when cells are treated with methotrexate (MTX) (Schimke, 1984). Even though cancer genomes frequently have altered chromosomal regions, there is little knowledge about the regulatory mechanisms or factors that are involved in promoting copy number alterations at specific regions of the genome.

Several mechanisms have been proposed for generating copy number variation (CNV). The mis-regulation of DNA replication is a major contributing factor to copy gain and DNA amplification (Hook et al., 2007). For example, many models for DNA amplification incorporate stalled replication forks and DNA double-strand breaks that are generated during replication. It is proposed that these stalled/collapsed replication forks are associated with, and can cause, tandem duplications. A second mechanism that has been proposed to contribute to CNV involves the use of breaks or repair intermediates as primers for re-replication of specific stretches of DNA. These re-replicated regions can re-incorporate into the genome, resulting in gene duplications or deletions. Alternatively, it is also possible that these events will not integrate in the genome (Hastings et al., 2009). A third mechanism which could generate re-replicated fragments and copy number alteration is the head to tail collision of elongating DNA polymerases (Davidson et al., 2006; Hook et al., 2007). Since chromatin structure impacts replication initiation and elongation efficiency as well as DNA damage response and repair (Alabert and Groth, 2012; Papamichos-Chronakis and Peterson, 2013), the chromatin state or modifying enzyme(s) could have a significant impact on each of these possible mechanisms.

Recently, Kiang and colleagues demonstrated that local DNA fragment amplification occurs during S phase (Kiang et al., 2010) and that the chromatin context or chromosome microenvironments play a major role in this process. Consistent with an important role for the chromatin context, mis-regulation of the histone 4 lysine 20 mono-methyltransferase KMT5A (H4K20me1, PR-Set7/Set8) promotes re-replication, at least in part, by increasing H4K20me2/3 levels and promoting ORC recruitment through binding of H4K20me3 (Beck et al., 2012; Tardat et al., 2010). This study highlights a role for histone methylation in regulating the recruitment of replication machinery. However, the role of methylation in modulating replication is not limited to the direct recruitment of DNA replication factors. For example, the maintenance and establishment of histone 3 lysine 9 tri-methylation (H3K9me3) and heterochromatin protein 1 (HP1) recruitment have significant roles in modulating proper replication timing, DNA damage response and repair of heterochromatic regions (Black and Whetstine, 2011; Hayashi et al., 2009; Schwaiger et al., 2010; Wu et al., 2006; Wu et al., 2005). Typically, these heterochromatic regions have less accessibility, fewer origins and are late replicating, unless they are made more accessible (Black and Whetstine, 2011). For example, the inventors have previously demonstrated that the H3K9me3 demethylase KDM4A/JMJD2A was able to increase accessibility and alter the replication timing at specific heterochromatic regions (Black et al., 2010). The regulation of KDM4A protein levels was also important in modulating its chromatin occupancy, replication initiation and S phase progression (Van Rechem et al., 2011). Furthermore, Mallette and colleagues demonstrate that increased KDM4A expression abrogates 53BP1 recruitment to DNA damage sites, suggesting a role for KDM4A in DNA damage response (Mallette et al., 2012).

Investigated herein was the possibility the effect of overexpression of catalytically active KDM4A. The results described herein provide an enzymatic link to the proposed methods for generating copy number alterations through re-replication, which can underlie site-specific copy number changes in cancer. Consistent with this notion, KDM4A was shown to be overexpressed in both breast and lung cancer where it correlates with the promotion of tumorigenesis (Berry et al., 2012; Mallette and Richard, 2012). These studies did not explore the basis for increased expression or whether KDM4A levels were associated with genome instability.

Described herein is analysis of The Cancer Genome Atlas (TCGA) data, which revealed that KDM4A is amplified and overexpressed in several tumor types. Experimentally, it was observed that KDM4A overexpression in transgenic cells was sufficient to promote copy gain of specific chromosomal domains (e.g., 1q12). KDM4A-dependent copy gains were induced in less than 24 hours and required replication. Interestingly, the KDM4A-dependent copy gains were not stably inherited, but generated transiently in each subsequent S phase and cleared by late G2. These copy gains required KDM4A catalytic activity and were not generated by other KDM4 family members. Furthermore, it is demonstrated herein that KDM4A-dependent copy gains could be antagonized by co-expression of the H3K9me3 methyltransferase Suv39h1 or HP1γ. Consistent with these observations, interference with H3K9/36 methylation using H3.3 methionine variants (Lewis et al., 2013) resulted in increased copy gain of 1q12. These results emphasize the impact mis-regulating chromatin structure has on site-specific copy gains. Overexpression of KDM4A was sufficient to promote re-replication of regions with copy gain. Consistent with this observation, mass spectrometry analysis demonstrated that KDM4A associates with replication machinery (e.g., licensing factors and DNA polymerases). KDM4A overexpression resulted in increased KDM4A association, decreased H3K9me3, decreased HP1γ and increased association of replication machinery at regions that undergo KDM4A-dependent re-replication. Consistent with KDM4A regulating copy number gains and re-replication at specific loci in the genome, tumors with copy gains in KDM4A were significantly enriched for copy gains of 1q21 and Xq13.1. When KDM4A was overexpressed in different cell types, the copy gain observed in these tumors could be recapitulated. These findings demonstrate that overexpression of the KDM4A lysine demethylase results in copy gain of specific genomic regions that are frequently observed in human tumors. Finally, these results highlight the impact that transient mis-regulation or overexpression of a single chromatin regulator can have on copy number changes during tumorigenesis.

### RESULTS

*KDM4A is Amplified and Overexpressed in Cancer.* KDM4A has previously been demonstrated to be overexpressed in breast and lung cancer (Berry et al., 2012; Mallette and Richard, 2012). However, a comprehensive profile of primary tumors for alterations in KDM4A expression levels has yet to be established. In addition, there are few molecular insights into the mechanisms that promote increased expression in tumors. Therefore, a comprehensive analysis of KDM4A copy number and expression level in 1,770 primary tumor samples from 8 different cancer types (Figs. 8A-8L) represented in The Cancer Genome Atlas [TCGA, (Beroukhim et al., 2010)] was conducted. The analysis found evidence of increased KDM4A copy number (GISTIC annotation of +1 or +2; (Mermel et al., 2011)) in 18.9% of tumors (335 out of 1,770 samples, Fig. 1A) and copy loss in 22.1% of tumors (392 out of 1,770 samples, Fig. 1A). To ensure that changes in KDM4A copy number resulted in changes in transcript levels, KDM4A expression from RNA-sequencing data from the same 1,770 samples was correlated with the KDM4A copy number annotation (Fig. 1B, Fig. 8A). Amplification or deletion of KDM4A resulted in increased or decreased KDM4A expression, respectively. Amplification and deletion of KDM4B-D in cancer was also observed (Fig. 8B-8D). Similar to KDM4A, expression of KDM4B-D also correlated with amplification or deletion (Fig. 8B-8D). These data demonstrate that amplification of KDM4A in cancer results in increased expression of KDM4A, providing a molecular basis for the elevated KDM4A levels observed in different tumor samples.
To determine whether the correlation of KDM4A copy number and expression exists in each of the tumor types, they were also analyzed independently (Fig. 8E-8L). KDM4A was both amplified and deleted across many disparate cancer types and KDM4A expression correlated with copy number in these samples (Fig. 8E-8L). Interestingly, ovarian cancer was significantly enriched in amplification of KDM4A (P=1.4x10-21 for Gain vs No change or Loss by Fisher's exact test), which was amplified in 46% (94 out of 204) of the tumors, with relatively few examples of deletion (9.8%; 20 out of 204 samples) (Fig. 1C and Fig. 8I). Consistent with the data compiled from the different tumor types, amplification of KDM4A in ovarian cancer also correlated with increased expression (Fig. 1D and Fig. 8I).

Since ovarian tumors displayed a large percentage of KDM4A amplification, it was tested whether KDM4A focal amplification (GISTIC +2) significantly associated with the time to death in the ovarian cancer patient data set. In fact, KDM4A focal amplification modestly correlated with a faster time to death in ovarian cancer with a median time to death of 691 days compared to 1052 days without KDM4A amplification (Fig. 1E, P=0.02); however, KDM4A loss (GISTIC -2 or -1) was not significantly different from patients without changes in KDM4A copy number (P=0.85). In sharp contrast to KDM4A, few cases of focal amplifications were observed for KDM4B-D and no statistical significance was associated with their focal amplifications and time to death (Fig. 1F-1H). However, focal deletion of KDM4C (GISTIC -2) modestly associated with worse outcome (P=0.014), while broad loss or gain (GISTIC -1 or +1) of KDM4D modestly associated with poor outcome (P=0.013 and P=0.018, respectively). These data highlight the differences between the KDM4 family and cancer outcome, which suggests non-overlapping functions in certain cancer types. These results also emphasize the importance of understanding the biological processes that are mis-regulated upon increased expression of KDM4A in cancer. Finally, these data indicate that KDM4A levels can function as a biomarker in ovarian cancer.

*KDM4A Overexpression Promotes Copy Gain of 1q12.* The inventors previously demonstrated that KDM4A overexpression in 293T cells promoted faster S phase progression, increased chromatin accessibility and altered replication timing (Black et al., 2010). KDM4A was also shown to impact the DNA damage response through 53BP1 (Mallette et al., 2012). In order to address whether KDM4A overexpression can promote genomic instability, which is a hallmark of cancer (Luo et al., 2009), KDM4A was overexpressed in the karyotypically stable, immortalized, but not transformed, RPE1-hTERT (RPE) cell line (Jiang et al., 1999). Stable GFP (referred to as control or CTRL) and GFP-KDM4A (referred to as KDM4A) expressing RPE cells were generated. Similar to the previously reported 293T stable cells (Fig. 9A), RPE stable cell lines expressed KDM4A about 2-3 fold over endogenous level (Fig. 9B). To determine if overexpression of KDM4A led to wide scale chromosome instability, the RPE GFP-CTRL and GFP-KDM4A cell lines were analyzed by spectral karyotyping (SKY; Fig. 2A and 2B). SKY was conducted on two independent polyclonal populations of RPE cells at two different times in culture. In one GFP-CTRL population a Robertsonian translocation (T13;21) was observed, while in one CTRL and both KDM4A overexpressing line a non-clonal extra copy of chromosome 3 was observed (data not shown).

Since both KDM4A and CTRL cell lines exhibited this amplification of chromosome 3 it is likely a KDM4A-independent abnormality. There were no additional major genomic events consistent between the two independent KDM4A cell lines that were not observed in the CTRL cell lines. We also analyzed our 293T KDM4A overexpressing stable cell line by G-band analysis. We did not observe amplification of chromosome 3 in 293T cells or any amplification, or translocation specific to GFP-KDM4A cells (data not shown). Thus, overexpression of KDM4A did not lead to consistent large scale genomic changes: chromosome number changes, detectable translocations, deletions or inversions. These data support the notion that modest KDM4A overexpression does not promote large scale genomic instability.

Since KDM4A modulated replication timing and regulated the highly repetitive Chrl sat2 locus (Black et al., 2010), it was reasoned that KDM4A might promote instability at specific genomic loci that could be below the detection threshold of SKY. In order to identify these candidate regions, the previously performed chromatin immunoprecipitation (ChIP) on chip analysis of KDM4A binding in 293T cells that either expressed GFP (CTRL) or GFP-KDM4A (KDM4A) was reanalyzed (Fig. 9C) (Van Rechem et al., 2011). The original analysis identified numerous regions on chromosomes 1-4 with altered BrdU incorporation in KDM4A overexpressing cells (Van Rechem et al., 2011). Since this altered BrdU incorporation occurred over large tracts, KDM4A enrichment over large sections of the genome was analyzed. Specifically, KDM4A occupancy was analyzed by determining the average probe intensities for each cytogenetic band and a Z-score was computed for each band. Of the top 10 cytogenetic bands with enrichment for KDM4A occupancy, only 1q12 was specifically enriched in KDM4A overexpressing cells when compared to control cells (Fig. 9C).

1q12/21 is a region with frequent copy number variation (CNV) in lung cancer and multiple myeloma (Brunet et al., 2009; Brzustowicz et al., 2000; Inoue et al., 2004; Yakut et al., 2006). To determine if the copy number of 1q12 was altered following manipulation of KDM4A protein levels, fluorescent in situ hybridization (FISH) was performed in CTRL and KDM4A overexpressing 293T cells to determine if the copy number of 1q12 was altered (Fig. 2C, Fig. 9D). A commercial 1q12h chromosome 1 enumeration probe (CYTOCELL™ through Rainbow Scientific) was utilized to score the number of foci in each cell. CTRL 293T cells typically exhibited 3-4 copies of 1q12h, which was consistent with the G-band analysis (data not shown). Cells were required to have in excess of 4 copies of 1q12h to score positive for copy gain. This same scoring criterion was also used for all additional chromosome FISH probes (e.g., Chr8 and Chr X; Fig. 2C, Fig. 9D). It was observed that KDM4A overexpression resulted in increased copy number of 1q12h in 14% of cells (Fig. 2C). However, this was not an increase in the entire 1q chromosome arm as the 1q telomere did not have increased copy number in KDM4A cells (Fig. 2C; 1qTel). Furthermore, no change was observed at additional pericentric regions on chromosomes 8 and X (Fig. 2C).

Since 293T cells have an aberrant, hypertriploid karyotype, these results were validated in the karyotypically stable, near diploid, RPE stable cell lines that were used in the SKY analysis (Fig. 2A, 2B and Fig. 9B). In order to score as an increased copy number in RPE cells, all cells with greater than two copies of the given chromosome were included. Similar to the 293T cells, it was observed that 17% of KDM4A overexpressing RPE cells showed an increase in copy of 1q12h (Fig. 2D). Consistent with the results in 293T cells, significant changes in copy number were not observed for the 1q telomere, or the centromeres of chromosome 2, 6, 8, or X (Fig. 2D). Inclusion of all copy number alterations did not alter the conclusion as KDM4A overexpression promoted primarily a copy number increase of 1q12h (Fig. 9E).

It was previously demonstrated that KDM4A overexpression increased chromatin accessibility (Black et al., 2010). To examine the possibility that altered chromatin accessibility resulted in the increased detection of 1q12h in KDM4A cells, FISH analysis was performed on control and KDM4A overexpressing cells depleted of Condensin 1 (CapD2) or Condensin 2 (CapD3) (Fig. 9F). Depletion of either condensin did not increase detection of 1q12h amplification in CTRL or KDM4A cells (Fig. 9G). Therefore, the increased copy number of 1q12h in KDM4A cells is most likely not an artifact of increased chromatin accessibility.

KDM4A has been reported to interact with p53 (Kim et al., 2012) and could potentially lead to altered p53 functionality, and in turn, alter genome stability. RPE cells contain wild type p53 (Jiang et al., 1999). Therefore, p53 functionality was tested in the RPE control and KDM4A overexpressing cells. p53 stabilization and activation was tested by treating cells with the DNA damaging agent doxorubicin. Treatment with doxorubicin resulted in stabilization of p53 and activation of p53 target genes, supporting the notion that the p53 pathway is functional in RPE cells that overexpress KDM4A (Fig. 9H and 9I). These data are consistent with KDM4A overexpression promoting localized changes in copy number without substantial changes in chromosome stability.

*KDM4A-dependent Copy Gain of 1Q12 is Dose-dependent, ReQuires Catalytic Activity and Tudor Domains.* In order to determine if the expression level and catalytic activity of KDM4A are required for 1q12h copy gain, catalytically active and inactive (H188A; (Whetstine et al., 2006)) KDM4A was expressed in 293T cells with and without KDM4A depletion by shRNA treatment (Fig. 3A, 10A). Transient overexpression of KDM4A was sufficient to promote 1q12h copy gain, but not alter copy number of chromosome 8 (Chr 8). However, concomitant depletion of KDM4A suppressed 1q12h gain, which emphasizes the importance of increasing KDM4A levels in order to observe the increased 1q12h (Fig. 3A, 10A). Importantly, overexpression of catalytically dead KDM4A (Fig. 3A, 3B; H188A) was unable to promote 1q12h copy gain, and the copy gain was not observed with KDM4A depletion, which emphasizes that the 1q12h gain is not a dominant negative affect due to KDM4A overexpression.

The timeframe in which KDM4A overexpression is sufficient to alter copy number of 1q12h was investigated. Surprisingly, transient transfection of KDM4A into RPE cells led to altered copy number of 1q12h in less than 24 hours of expression (Fig. 3C, 3D, Fig. 10B, 10C). In agreement with the data obtained from stable RPE cell lines, altered copy number was not observed at the centromeres of chromosome 6, 8 or X (Fig. 3D). The occurrence of copy gains within twenty-four hours following increased KDM4A levels suggests that other genetic alterations within the cellular background are not necessary for KDM4A-dependent regulation of 1q12 copy gain.

It was confirmed that the copy gain required the catalytic activity (H188A) and enzymatic domains (JmjC and JmjN; referred to as ΔNC) in RPE cells (Fig. 3B, 3C and Fig. 10B). However, the KDM4A catalytic domain alone was insufficient to generate 1q12h gain (Fig. 3B, 3C; referred to as NC). The ability of the chromatin binding domains (PHDs and Tudors) within KDM4A to impact the copy gain were then investigated. The loss of the Tudor domains alone was sufficient to block the 1q12h gain observed with KDM4A overexpression (Fig. 3B, 3C; referred to as NCMP). Taken together, these data emphasize that transient exposure to increased KDM4A levels is sufficient to promote 1q12h copy gain, but this can only occur with a catalytically active enzyme and functional Tudor domains.

*Not All KDM4 Members Promote 1Q12h Copy Gain.* It was next sought to determine if the regulation of 1q12 copy number was specific to KDM4A or was potentiated by the other demethylases within the KDM4 family. KDM4 member specificity was addressed by conducting 1q12h FISH in parental RPE cells transiently transfected for 24 hours to analyze copy number gain. GFP-tagged KDM4A, 4B and 4C were similarly expressed, while KDM4D expression was significantly higher (Fig. 10C). As expected, KDM4A overexpression led to increased 1q12h copy number (Fig. 3E). However, overexpression of KDM4B, KDM4C, or KDM4D for 24 hours did not alter 1q12h copy number (Fig. 3E). These data suggest that the regulation of 1q12 copy number is unique to KDM4A and raises the possibility that either the other family members may not be involved in modulating copy number gain or that they may regulate copy number of distinct genomic regions yet to be identified.

*Interfering with H3K9 or H3K36 Methylation Promotes 1Q12 Copy Gain.* The requirement for KDM4A catalytic activity to promote 1q12 copy gain suggests that methylation of chromatin or a non-histone target is important for proper regulation of 1q12h ploidy. Recently, Lewis and colleagues demonstrated that H3.3 variants with a methionine in place of the lysine (i.e., H3K27M, H3K9M and H3K36M) can inhibit EZH2 (K27M), G9a (K9M), Suv39h1 (K9M) as well as reduce H3K36me3 levels (K36M) (Lewis et al., 2013). These H3.3 variants were used to ascertain if interfering with methylation at any one of these sites could promote 1q12h copy gain. RPE cells were transduced with lentivirus that express H3.3 wild-type (WT), H3.3K9M (K9M), H3.3K36M (K36M), H3.3K27M (K27M) and H3.3G34V (G34V). The cells were collected 24 hours post infection for analysis of 1q12h copy number by FISH. Each variant was expressed and successfully incorporated into chromatin and reduced the corresponding tri-methylation (Fig. 10D, 10E). Expression of H3.3 WT, G34V and H3K27M failed to promote 1q12h copy gain; however, expression of either H3.3K9M or H3.3K36M was sufficient to promote low level gain of 1q12h (Fig. 3F; P=0.026 for K9M and P=0.006 for K36M). Furthermore, the increased copy of 1q12h was not caused by a gain of all of chromosome 1 because an increase in 1q telomere foci (1q Tel) or in the 1q23.3 cytogenetic band midway down the 1q arm (Fig. 3F) was not observed. Since H3.3K9M promoted copy gain at 1q12h and inhibits Suv39h1 (Lewis et al., 2013), it was reasoned that overexpression of the H3K9me3 methyltransferase Suv39h1 may suppress KDM4A-dependent copy gain. Consistent with this prediction, co-expression of Halo-Suv39h1 was sufficient to abrogate KDM4A-dependent 1q12h copy gain (P=.0003 for KDM4A and P=0.47 for KDM4A+Suv39h1) (Fig. 3G, 10F). These results highlight the importance of methylation in modulating site-specific copy gain, especially the lysines that are substrates for KDM4A.

*Co-expression of HP1γ Antagonizes KDM4A-dependent Increased 1q12 Copy Number.* It was previously demonstrated that the KDM4A-dependent changes in cell cycle progression and replication of Chrl sat2 could be antagonized by HPly overexpression, but not by HP1α or HP1I3 overexpression (Black et al., 2010). Therefore, it was investigated whether HPly overexpression could antagonize the increased copy number of 1q12 in KDM4A overexpressing cells. To test this hypothesis, parental RPE cells were con-transfected with RFP-HP1y and GFP-KDM4A for 24 hours (Fig. 10G). Co-transfection of HPly significantly reduced the number of KDM4A overexpressing cells with altered copy number of 1q12h (P=0.034) to levels comparable to that seen in control transfected cells (P=0.29) (Fig. 3H). A slight increase in the number of control cells with abnormal 1q12h with HPly overexpression was observed, however the slight increase was not significant (P=0.08) when compared to control cells co-transfected with vector alone. These data emphasize the antagonistic relationship between KDM4A and HPly. Surprisingly, transfection of HPly into RPE cells stably overexpressing KDM4A or stably co-overexpressing both HPly and KDM4A did not reverse the increased copy number of 1q12h (data not shown). These results indicate that once an altered chromatin conformation is established, HPly is insufficient to restore proper regulation of 1q12 copy number in KDM4A overexpressing cells.

*KDM4A-dependent* 1q12 *Copy Gain Is Not Stably Inherited and Requires Replication.* The ability of stable or transient KDM4A overexpression to promote increased 1q12 copy number raised the question of whether the increased copy number was stably inherited in a subset of the population or was regenerated during subsequent cell cycles. First, it was sought to determine if the copy gain of 1q12 was stably inherited. To address this question, single cell clones of the stably overexpressing KDM4A RPE cell line were established and used to perform 1q12h FISH (Fig. 4A). If the copy number of 1q12h was stably inherited, one would expect to observe some clones with 100% of cells with 1q12h copy gain. Instead, the 1q12h FISH analysis of 27 independent clones revealed a distribution of copy gain between 1.5-37%, which supports the model that the increased 1q12 copy number is most likely not stably inherited (Fig. 4A, Fig. 11A). The clones lacking increased copy of 1q12h (below black dashed line) no longer overexpressed KDM4A (Fig. 11A), emphasizing the need for overexpression as seen with the transient transfections and shRNA depletions described above (Fig. 3A). Furthermore, the average of all clones assayed was 17.0%, which was in agreement with the analysis of the starting stable population (Fig. 4B, 2D, respectively).

It was next investigated whether stably overexpressing KDM4A RPE cells gain extra copies of 1q12h during each subsequent cell division. Since DNA fragment amplification requires S phase and CNV is associated with aberrant DNA replication and recombination (Hook et al., 2007; Kiang et al., 2010), it was tested whether S phase was required for the gain of 1q12h in KDM4A overexpressing cells. The stable RPE cell lines were treated with hydroxyurea (HU), which arrests cells in the G1/S phase (Fig. 10B), and subsequently subjected to FISH analysis with probes specific for 1q12h. Arresting the KDM4A overexpressing RPE cells with HU was sufficient to eliminate the increased copy number of 1q12h in the stable KDM4A overexpressing cells (Fig. 4C). To address the possibility that replication stress could have promoted apoptosis, and in turn, resulted in fewer cells containing increased copies of 1q12h, apoptosis was evaluated by analyzing annexin V staining in the HU arrested cells used for FISH. There was no observed increased apoptosis in vehicle treated KDM4A overexpressing cells or in HU treated KDM4A overexpressing cells (Fig. 10C). However, doxorubicin treatment could induce apoptosis in both CTRL and KDM4A cells, which was significantly reduced in KDM4A overexpressing cells (Fig. 10C). These data are consistent with the notion that copy gain of 1q12h in KDM4A overexpressing cells requires replication to be generated.

Since the additional copies of 1q12h do not appear to be stably inherited, it was hypothesized that cells with 1q12h copy gain must be eliminated prior to mitotic cell division. To address this possibility, the stable KDM4A overexpressing lines were treated with the CDK1 inhibitor R03306, which arrests cells in late G2 [Fig. 10B; (Vassilev, 2006)]. After arresting the stable RPE cells, copy gain of 1q12h was analyzed by FISH. KDM4A cells arrested in late G2 failed to display increased copy number of 1q12h (Fig. 4D). Taken together, these data support a model whereby KDM4A promotes copy gain of specific chromosomal regions during S phase, which are then eliminated by the end of the G2 phase of cell cycle.

Given that KDM4A-dependent 1q12 copy gain did not occur during G1/S arrest or in G2/M arrested cells, it was hypothesized that KDM4A promotes copy gain of 1q12 during S phase. Therefore, GFP-KDM4A or GFP-CTRL RPE cells were arrested in HU for 20 hours and subsequently released into S phase. 1q12h copy number was analyzed by FISH during S phase progression. In agreement with this hypothesis, GFP-KDM4A cells, but not GFP-CTRL cells, were able to promote additional copies of 1q12h, but not Chr 8 centromere following HU release (Fig. 4E, 4F). The additional copies occurred between 2 and 6 hours post HU release and were lost between 8 and 10 hours following HU release. Taken together, these observations demonstrate that overexpression of KDM4A promotes generation of additional copies of 1q12 during S phase, which are subsequently lost later in the same cell cycle.

*KDM4A Associates with Replication Machinery and Promotes Re-replication of 1Q12.* In order to gain some molecular insight into how KDM4A is involved in generating 1q12h copy gain, KDM4A interacting proteins were identified by performing mass spectrometry analysis of exogenously expressed KDM4A. Specifically, a Halo-tagged KDM4A expression construct was transiently overexpressed in 293T cells and the interacting proteins were isolated through affinity purification with HALOLINK™ resin from DNAse treated lysates. The associated proteins were then identified by LC-MS/MS from two independent experiments. Upon analyzing the KDM4A interacting proteins with the IPA software from Ingenuity, a significant enrichment of proteins involved in replication was observed (Fig. 5A; P=0.00000795). These associated interactions were then validated, particularly the replication enrichment since many of these proteins are required for re-replication [e.g., MCMs and DNA polymerases; (Arias and Walter, 2007; Snaith and Forsburg, 1999)].

A previous study demonstrated that KDM4A interacted with cullin 1 (Van Rechem et al., 2011), while others demonstrated that KDM4A interacts with p53 (Kim et al., 2012). Additional interactions identified in the LC-MS/MS analysis were validated by conducting endogenous KDM4A co-immunoprecipitation experiments. To eliminate potential interference arising from interactions associated with DNA fragments or chromatin, the immunoprecipitations were conducted in the presence of ethidium bromide and/or benzonase. Consistent with the mass spectrometry analysis, it was possible to co-immunoprecipitate the replication licensing factors. Interactions between MCM2, MCM3, and MCM7 and endogenous KDM4A were detected in 293T (Fig. 12A) and RPE cells (Fig. 5B). While MCM2 was not identified in our mass spectrometry analysis, it co-immunoprecipitated with KDM4A, suggesting that the entire MCM complex interacts with KDM4A. It was further demonstrated that endogenous KDM4A associated with Halo-tagged DNA polymerase subunits in RPE and 293T cells (Fig. 5B, Fig. 12A). These results support a model whereby KDM4A interacts with the replication machinery to promote copy gain of specific genomic loci.

Since KDM4A overexpression both promoted copy gain in a replication-dependent manner and interacts with DNA polymerases and the replication licensing machinery, it was hypothesized that KDM4A overexpression was promoting re-replication within 1q12. To test this hypothesis, cesium chloride density gradient centrifugation was used. Asynchronously growing control and KDM4A overexpressing stable RPE cell lines were labeled for 14 hours with bromo-deoxyuridine (BrdU) so that replicated DNA would have a higher density than un-replicated DNA. The isolated genomic DNA was purified, fragmented and separated on a cesium chloride gradient by ultracentrifugation (Karnani et al., 2009). Fractions were collected from the bottom of the gradient and analyzed for DNA concentration using a nanodrop (Fig. 12B). The labeling procedure was performed for less than one complete cell cycle, producing an enrichment in heavy-light (H:L) replicated DNA, while still maintaining an un-replicated light-light fraction (L:L). This indicates that the labeling did not proceed long enough for cells to undergo a second cell cycle and thus generate heavy-heavy (H:H) re-replicated DNA. A peak of enrichment of H:H DNA was not detected, indicating that KDM4A overexpression does not promote widespread re-replication as seen with other chromatin regulators [KMT5A;(Tardat et al., 2010)]. This observation was consistent with the SKY and FISH studies demonstrating no large scale genomic anomalies.

In order to determine whether 1q12 was in fact re-replicated, the fractions where the H:H DNA should separate were pooled and purified and the re-replicated DNA assayed for specific regions by quantitative PCR. The inventors have previously demonstrated that Chrl sat2 is bound by KDM4A and that KDM4A regulates Chrl sat2 accessibility and replication timing. Therefore, it examined whether Chrl sat 2, which resides in 1q12 (Wong et al., 2001) would be a target for KDM4A-dependent re-replication. A seven fold enrichment of Chrl sat2 was observed in the re-replicated fraction in KDM4A overexpressing cells, while the 13-actin locus and a region near the X centromere, which was previously reported as a KDM4A target (Black et al., 2010), were not enriched (Fig. 5C, 12C). The enrichment in Chrl sat2 re-replication represented a small amount of the input DNA and was consistent with a subpopulation of cells generating additional copies of 1q12h and the disappearance of the copy gain prior to completion of cell cycle (Fig. 12C). Taken together, these data demonstrate that KDM4A associates with replication proteins (i.e., replication licensing factors and DNA polymerases) and that KDM4A overexpression promotes re-replication at a specific locus that exhibits copy gains.

*Overexpression of KDM4A Promotes Chromatin State Changes and Recruitment of Replication Machinery.* The data described herein support a model whereby overexpression of KDM4A promotes methylation changes, displacement of HPly and recruitment of replication machinery to specific genomic regions resulting in re-replication. To test this model, chromatin immunoprecipitation experiments were performed to evaluate methylation levels, HPly enrichment and replication machinery occupancy at the re-replicated Chrl sat2 region. As a negative control, an intergenic region on chromosome 10 (Chr10) that is acetylated at H3K9 in numerous cell types (according to UCSC browser; data not shown) that should not be enriched for H3K9me3 or KDM4A was used. Overexpression of KDM4A results in increased recruitment of KDM4A to Chrl sat2 (Fig. 5D) but not at Chr10. The inventors previously reported that overexpression of KDM4A promoted loss of H3K9me3 and HPly at Chrl sat2 in 293T cells (Black et al., 2010). Consistent with these results, the increased recruitment of KDM4A to Chrl sat2 in RPE cells promotes loss of H3K9me3 and eviction of HPly from Chrl sat2 (Fig. 5E, 5F). No change in H3K36me3 at Chrl sat2 was observed, which was consistent with findings in 293T cells (Black et al., 2010). Finally, MCM7 and DNA polymerase α (Polα) ChIPs were conducted to assess whether there was an increase in their recruitment upon KDM4A overexpression. Both MCM7 and Polα were enriched at Chrl sat2, but not at Chr10, upon KDM4A overexpression (Fig. 5G, 5H, respectively). Taken together, these data demonstrate that overexpression of KDM4A promotes H3K9me3 and HP1γ loss, increased replication machinery recruitment and re-replication of 1q12.

*Identification of Regions Co-Amplified with KDM4A in Cancer.* It was next determined what additional regions would have increased copy gain in the presence of KDM4A overexpression. Since KDM4A is amplified and overexpressed in tumors and KDM4A overexpression in cell lines results in increased copy gain of 1q12, it was determined whether KDM4A amplification in primary tumors was correlated with increased copy number of 1q12 and whether additional chromosomal regions had copy gains when KDM4A amplification occurred. This analysis was done by identifying the most significantly correlated focal copy gains across each of the 807 cytogenetic bands in 4,420 tumor samples (representing 19 tumor types including the 8 analyzed in Figs. 8A-8L) when compared with KDM4A amplification located at 1p34.2. This analysis was restricted to focal cytogenetic band amplifications (less than arm level; Fig. 6A). Copy gains from 1p11.2 through 1q21.3 on chromosome 1 were observed in two independent statistical tests (Fig. 6A and 13A, shading; See Experimental Procedures). Due to the minimal sequence annotation and repetitive nature of 1q12, a correlation was not calculated for this cytogenetic band. However, the cytogenetic bands immediately flanking 1q12, 1p11.2 and 1q21.1, both exhibited co-gain with KDM4A amplification, which suggests that 1q12 is most likely co-amplified in these tumors. Furthermore, this co-amplification was not due to enrichment for amplification of entire chromosome 1 or complete amplification of the 1q arm as the correlation decreases approaching the 1q telomere. Interestingly, this co-amplified region, as well as others, exhibited specificity for KDM4A co-amplification because there was not a strong correlation when the identical analysis was performed with respect to co-amplification of KDM4B (Fig. 6B and 13B).

The correlation between co-amplified cytogenetic bands and KDM4A in the ovarian cancer data sets was analyzed (Fig. 6C and 13C). As observed with the complete cancer data set, KDM4A was co-amplified with 1p11.2-1q21.3. However, it was also observed that some KDM4A co-amplified regions were lost (e.g., the region on 17q- position 17q24.2 to 17q25.3) and others were enhanced (e.g., the region on X chromosome- position Xp11.2 to Xq13.2) in the ovarian cancer profile (Fig. 6C and 13C). These results suggest that individual co-amplified regions could universally be observed, while others could be differentially regulated in a tumor and/or tissue-specific manner.

Next, it was tested whether gains in 1q21.1-1q21.3 were affected by the amplification level of KDM4A (Fig 6D-6F). Indeed, when KDM4A had a high-level focal amplification (GISTIC +2), a significantly greater fraction of samples were amplified in 1q21.1-1q21.3 compared to cases in which KDM4A was not amplified (GISTIC 0) (Fisher's exact test; P= 2^{∗}10-9 in 1q21.1, 1.9^{∗}10-9 in 1q21.2, and 1.02^{∗}10-10 in 1q21.3) (See Experimental Procedures; Fig. 13D-13I). When comparing lower-level amplification of KDM4A (GISTIC +1) to the KDM4A-unamplified cases, a reduced sample fraction was observed, but still highly significant amplification of 1q21.1-1q21.3 (P= 2.04^{∗}10-25 in 1q21.1, 6.28^{∗}10-22 in 1q21.2, and 3^{∗}10-24 in 1q21.3). In contrast, when stratifying the samples based on KDM4B amplification status (Fig 6G-6I), the differences are not significant for +2 vs 0 (all P-values > 0.1 by Fisher's exact test), although a minimal trend in the same direction is observed. These results demonstrate that amplification of KDM4A in tumors is correlated with co-amplification of specific cytogenetic bands, which indicates that KDM4A can promote copy number alterations of these regions in vivo.

*KDM4A Overexpression Promotes Copy Gain and Re-replication of Regions Co-Amplified in Cancer.* The observation that amplification of 1q21.1 through 1q21.3 correlated with amplification of KDM4A raised the possibility that KDM4A may promote copy gain and re-replication of these regions. To test this hypothesis, FISH analysis was performed in the stable KDM4A overexpressing RPE cell lines for 1q12/1q21.1 and 1q21.2 (Fig. 7A, 7B). Increased copy number of 1q12 through 1q21.2 was observed in KDM4A overexpressing cells, but not in control cells (Fig. 7B). The co-amplification analysis of all tumors indicated that the correlation with KDM4A copy number diminished at 1q23, suggesting that 1q23 is not amplified in KDM4A overexpressing cells. This observation would allow the definition of a boundary for KDM4A-dependent copy gains in the RPE stable cells. Consistent with this hypothesis, increased copy number of 1q23.3 was not observed by FISH in the KDM4A overexpressing RPE cells (Fig. 7B).

To test the predictive value from tumor co-amplified regions, it was next asked whether KDM4A could promote copy gain of other chromosomal domains identified through the co-amplification analysis described herein. The small focal peak on the X-chromosome (Xp11.2-Xq13.2), which was specific to co-amplification with KDM4A across all cancers and even more enriched in ovarian cancer was examined (Fig. 6A, 6C, respectively). Two additional FISH probes specific for Xq13.1 and Xq13.2 (Fig. 7C) were used to assay for possible copy gain in KDM4A overexpressing RPE cells. Consistent with the tumor co-amplification data, it was found that KDM4A overexpressing RPE cells exhibit increased copy gains of Xq13.1. However, it was also observed that this increase in copy number is restricted to a defined region as probes specific for the X centromere and Xq13.2 do not display a similar increase in copy number (Fig. 7D). The ability of KDM4A to promote increased copy of 1q12/21, 1q21.2, and Xq13.1 was not restricted to RPE cells as increased copies of these regions were also observed in KDM4A-overexpressing 293T cells (Fig. 13J). These data demonstrate that KDM4A overexpression promotes copy gain of specific chromosome regions, which are co-amplified with KDM4A in primary tumors.

Based on the co-amplification analysis from TCGA tumor data sets, it was not clear whether the entire intervening sequence between 1q12h and 1q21.3 was amplified in the same tumor cells. In order to address this question, the 1q12/21 or 1q21.2 FISH probes were scored with the 1q12h probe in the same KDM4A overexpressing cells (Fig. 7E). When the FISH analysis was conducted with the 1q12h and 1q12/21.1 probes that are located at either end of the 1q12 cytogenetic band (Fig. 7A), it was observed that approximately 2/3 of the KDM4A overexpressing cells with 1q12h amplification also had 1q12/21.1 amplification, while approximately 1/3 had only 1q12/21.1 copy gain but not a gain in 1q12h (Fig. 7E). In contrast, when comparing 1q12h with 1q21.2, it was observed that the majority of cells with an amplification of 1q12h or 1q21.2 did not have amplification of the opposite cytogenetic band (Fig. 7E). Taken together, these data demonstrate that the entire region between 1q12h and 1q21.3 is not contiguously amplified, but that KDM4A is directing copy gain within these cytogenetic bands. Furthermore, these results are consistent with KDM4A promoting copy gain of different regions in different cells and that the total population of cells affected by KDM4A overexpression is greater than the 17% predicted from 1q12h amplification alone. The ability to detect non-overlapping foci also suggests that the additional copies may exist as extrachromosomal pieces. In agreement with this, analysis of combined FISH for 1q12/21 and chromosome 1 paint demonstrated that the additional 1q12/21 copy could exist adjacent to chromosome 1 paint territories or physically distinct from the chromosome 1 paint territory (data not shown).

It was next addressed if the additional regions of KDM4A-dependent copy gain in RPE cells were created through re-replication. To address this, regions under the FISH probes in the indicated cytogenetic bands were assayed in the CsCl gradient H:H fraction (Fig. 7F). In agreement with the FISH analysis, re-replication was observed at 1q12h (indicated by Chr1 sat2), 1q12/21.1, 1q21.2 and 1q21.3, but not in 1q23.3 or on Chr10. Detection of re-replication inside Xq13.1 was possible, but not near the X centromere. In addition, it was confirmed that the re-replicated regions were in fact bound by KDM4A, which was further enriched upon overexpression of KDM4A (Fig. 7G, Fig. 13K). As with Chr1 sat2, KDM4A overexpression promoted loss of HP1γ (Fig. 7H) and recruitment of MCM7 and Polα to 1q21.2 and Xq13.1 (Fig. 7I, 7J, respectively). Taken together, these data are consistent with the model that KDM4A overexpression promotes copy gain and re-replication at specific sites within the genome in vivo (tumors) and in vitro (transgenic cell lines).

### DISCUSSION

Genomic instability is a major contributing factor to the development and onset of age-related diseases. Cancer cells are often characterized by alterations in copy number of genes, specific genomic regions, chromosome arms, and entire chromosomes. However, the underlying molecular mechanisms that lead to these copy number alterations are poorly understood. Described herein is that transient overexpression of a single chromatin modifying enzyme, KDM4A, is sufficient to promote re-replication and copy gain of specific chromosomal domains. Furthermore, KDM4A-dependent amplified regions are found co-amplified with KDM4A in primary tumors. Described herein is a mechanism whereby improper regulation of a single chromatin modifier contributes to copy number variation of specific genomic regions.

*KDM4 Members and Cancer.* Previous studies have surveyed KDM4A expression levels in breast and lung cancer on a small number of primary tumors (Berry et al., 2012; Mallette and Richard, 2012). Described herein is a comprehensive analysis of KDM4A copy number alterations in 8 different tumor types from 1,770 primary tumors. These results demonstrate that KDM4A is amplified in different tumor types, and these amplifications correlate with increased expression of KDM4A. It is therefore critical to evaluate the effect KDM4A overexpression has on cellular processes. Intriguingly, when compared to other tumor types ovarian cancer is enriched for amplifications of KDM4A, which correlate with poor outcome in these cases. Levels of KDM4A may therefore represent a good biomarker for ovarian cancer, especially with respect to novel therapies that target this lysine demethylase. In addition, ovarian patients with increased levels of KDM4A may respond more poorly to S phase chemotherapeutics since KDM4A overexpression resulted in better recovery from hydroxyurea treatment (Black et al., 2010). Consistent with this possibility, ovarian cell lines with 1q12-21 amplification (which is the region KDM4A promotes copy gain of in transgenic cell lines and tumors) are often more resistant to cisplatin treatment (Kudoh et al., 1999; Takano et al., 2001). Interestingly, drug resistance in multiple myeloma is also associated with 1q12-21 amplification (Inoue et al., 2004).

Because the KDM4 enzyme family appears to have similar enzymatic characteristics, one might assume they would have similar distributions of genomic anomalies across the cancer landscape or have similar effects on patient outcome. In order to evaluate this question, the same tumor datasets were analyzed for copy gain and loss versus expression. Interestingly, focal deletions were observed in KDM4B-D, while none were observed in KDM4A across the 8 different tumor types (Fig. 8A-8D). It was also found that KDM4A was the only family member that had a correlation between focal amplification and poor outcome for ovarian cancer patients. Overall, this data highlights the idea that these family members are not created equal.

Consistent with KDM4 family members having independent roles, copy gain of 1q12 was only observed upon overexpression of KDM4A. These results were consistent with the in vivo observation that regions which co-amplify with focal KDM4A amplification in tumors predict regions where KDM4A will promote copy gain and re-replication in transgenic cell models (Figs. 6A-6I and 7A-7J). Furthermore, the distribution of co-amplified regions in KDM4A amplified tumors was distinct from KDM4B focally amplified tumors. These data demonstrate a remarkable level of specificity for individual KDM4 family members and specific genomic regions. It will be interesting to determine if other KDM4 family members also promote re-replication and copy number alterations of specific chromosomal regions. Interestingly, the distribution of co-amplified regions was subtly different between ovarian cancer and the distribution across all cancers. This suggests that KDM4A promotes site-specific copy gain and re-replication depending on tissue or tumor type, which could be a reflection of the different nuclear architecture or chromatin landscape between cancer types.

*Chromatin Environment and KDM4A-dependent Copy Gain.* Work in yeast has demonstrated that specific genomic regions can generate extra DNA fragments in S phase, which appears to depend on chromosomal or chromatin environment (Kiang et al., 2010). These observations are consistent with the KDM4A-dependent re-replication of specific chromosomal regions demonstrated herein. These regions are enriched for KDM4A binding upon overexpression of KDM4A, re-replicate, and have increased copy number during S phase. Interestingly, not all KDM4A occupied sites are re-replicating in the cell lines that have been tested (see Xcen, (Black et al., 2010), Fig. 7F). However, this could reflect the chromatin environment in these particular cells, or distinct chromatin states influenced by other chromatin modifiers. This would be consistent with Xq13.1 and X centromere copy gain in KDM4A focally amplified tumors, while only seeing the copy gain and re-replication of Xq13.1 in the transgenic cell lines described herein. Thus, different genomic regions can be susceptible to KDM4A-dependent re-replication in other cell lines and tissue types.

Consistent with the model that chromatin state impacts copy gain, it is demonstrated herein that interfering with H3K9 or K36 methylation resulted in the site-specific gain of 1q12h in 24 hours; while, overexpression of the H3K9me3 methyltransferase Suv39h1 or the H3K9me3 reader HP1γ was able to suppress the 1q12h gain. Surprisingly, KDM4A-dependent copy gains would not be reversed in cells stably overexpressing KDM4A by overexpressing HP1γ, even though these copy gains are regenerated each cell cycle. Without wishing to be bound by theory, this observation supports a model whereby KDM4A establishes a chromatin state that promotes re-replication and increased copy number of specific chromosomal regions. Formation of this chromatin state could be antagonized by HP1y, but not reversed once established. Taken together, these data indicate that the reader, the lysine and the KDM/KMT balance are required to maintain regulation at these regions. Therefore, local chromatin environment can be an important determinant in designating whether certain regions are more susceptible to copy gains and re-replication (see Fig. 14).

The results described herein indicate that KDM4A overexpression promotes heterochromatin displacement, recruitment of the replication licensing machinery and DNA polymerases so that inappropriate re-initiation of replication occurs at specific sites (Fig. 14). This inappropriate initiation can be the result of the association with and recruitment by KDM4A. Consistent with this idea, KDM4A-dpeendent copy gain requires the Tudor domains and KDM4A overexpression promotes increased occupancy of KDM4A, MCM7 and DNA polymerase at re-replicated regions. Furthermore, increased chromatin accessibility can promote KDM4A-dependent loading or the maintenance of the MCM complex on chromatin in the absence of CDT1, CDC6 and ORC. Consistent with this idea, CDT1, CDC6 and ORC are not necessary for replication after MCMs have been successfully loaded (Arias and Walter, 2007). Thus, KDM4A can be a mechanism for nucleating replication (Fig. 14). Alternatively, KDM4A can promote a more accessible chromatin environment, which is more permissive for inappropriate initiation within a replicated region. The later model would be consistent with the observation that interfering with H3K9 or K36 methylation can promote the site-specific gain in cells with wild type KDM4A levels. These two mechanisms can function cooperatively as increased KDM4A levels displace HP1γ at regions that are copy gained, while overexpression of HPIγ or Suv39h1 are able to suppress the phenotype. Regardless of the exact details, the data supports the model that alterations of heterochromatin and methylation at specific regions are more prone to re-replication, and in turn, copy gain.

*KDM4A and Extrachromosomal DNA.* It is demonstrated herein that KDM4A overexpression promotes increased copy number in an S phase-dependent manner. It was further demonstrated that each region with copy gain is not necessarily contiguous and may not exist in the same cell (Fig. 7E). These observations indicate that the the number of cells that have site-specific copy gains as well as the number of additional copies present per cell may be underestimated.

Since the re-replicated regions examined here are not inherited and are instead removed prior to completion of G2, it is likely that such regions exist as extrachromosomal DNA (Fig. 14). As such, it is possible that KDM4A is promoting re-replication at regions that promote head-to-tail collision of one replication fork chasing another. This model fits a previous study that showed deregulation of replication licensing promotes DNA fragmentation that was consistent with fork collision (Davidson et al., 2006). This model is consistent with the requirement of S phase for focal copy gains and the increased number of replication forks in KDM4A cells (Black et al., 2010). The presence of these fragments could also explain why KDM4A overexpressing cells exhibit an increased stabilization of p53 (Fig. 9H); however, this would be below the threshold of copy gain or fragmentation required to elicit the p53 checkpoint (Kracikova et al., 2013). It is also possible that site-specific re-replication may not be restricted to cancer cells. Transiently up regulating enzymes that direct site-specific re-replication to increase copy number of specific genes may be a general mechanism to allow cells the plasticity to respond to developmental, environmental, or stress conditions without altering their genetic makeup.

*KDM4A-dependent Transient Copy Gain.* Without wishing to be bound by theory, since transient KDM4A overexpression was sufficient to promote localized changes in copy number in a single cell cycle, the amplification of specific regions most likely precedes genetic changes in tumors. For instance, transient misregulation of chromatin regulators by altered environmental factors, metabolic changes, hypoxia or miRNAs could lead to temporary changes in copy number of small genomic regions. If these regions contain oncogenes, this could create a feedback loop that promotes tumorigenesis while masking the originating event (e.g., transient up-regulation of KDM4A). Of note, several putative oncogenes reside in the 1q12 and 1q21 cytogenetic bands, including Bcl9 and Mcl1. In fact, KDM4A binds the Bcl9 locus and causes both copy gain and re-replication of this site (Fig. 7G, 7H, respectively). However, increased expression of Bcl9 is not observed, which likely reflects the lack of additional stimulus, transcription factors or the low percentage of cells with this particular copy gain (Fig. 7H; 1q21.2, data not shown).

It remains unclear how the re-replicated regions in KDM4A overexpressing cells are removed as cells exit S phase and enter G2/M. Since KDM4A-dependent copy gains are generated during S phase and lost by the end of G2 phase, it is possible that cells possess an active method for degradation or removal of these regions. Without wishing to be bound by theory, other events could promote incorporation of these transiently amplified regions, and in turn, influence tumorigenesis. Interestingly, KDM4A overexpression by itself did not lead to inheritance of the re-replicated and amplified regions. If the amplified regions can be incorporated into the genome, it will be interesting to determine what factors influence inheritance of KDM4A-dependent amplified regions.

It is clear that the chromatin context influences replication timing and initiation choices. However, chromatin may additionally play an important role in ensuring replication fidelity and preventing re-replication. Distinct chromatin domains may have increased propensity for re-replication under different circumstances and cell types. This is supported by work in Drosophila that demonstrates that heterochromatic regions re-replicate upon loss of geminin (Ding and MacAlpine, 2010) and the fact that interference with H3K9 and H3K36 methylation promoted site-specific gain. Additionally, some chromatin modifiers regulate replication on a more global scale as inappropriate regulation of KMT5A results in widespread re-replication (Tardat et al., 2010). Interestingly, this re-replication is also dependent on KMT5B/C (Suv420h1/2) (Beck et al., 2012). Taken together, these results suggest that proper regulation of chromatin state is critical for suppressing re-replication. Therefore, a "chromatin checkpoint" may be intimately associated with the timing of replication and the propensity to undergo re-replication. This type of checkpoint could then be modulated by a series of chromatin modifiers or input signals. Thus, mis-regulation of chromatin modulators could promote aberrant replication following disruption of the chromatin checkpoint. Furthermore, mis-regulation of additional modifiers may aberrantly regulate different genomic regions and contribute to the heterogeneity observed in tumors. Importantly, this study emphasizes that a transient mis-regulation could also be sufficient to initiate the copy gains.

### EXPERIMENTAL PROCEDURES

*Cell Culture.* HEK293T (called 293T throughout) and hTERT-RPE-1 (called RPE throughout) cells were maintained in DMEM with 10% fetal bovine serum, 1% penicillin/streptomycin, and L-glutamine. Stable 293T cell lines were generated as in (Black et al., 2010). Stable RPE cell lines were generated by retroviral transduction of MSCV-GFP or MSCV-GFP-KDM4A. Cells were selected for 96 hours with puromycin. All experiments on stable cells were performed after two months of culture post infection with KDM4A. Transient transfection experiments were performed using Roche X-tremeGENE 9 DNA transfection reagent in OPTI-MEM I media (Gibco) for four hours. Media was then replaced with standard DMEM. No selection was used in transient transfection experiments.

*Expression Plasmids.* The pFN21A clone expressing an N-Terminal HALOTAG™ fusion of human full-length KDM4A or Suv39h1 (NM_014663) was obtained from Kazusa DNA Research Institute (Kisarazu, Japan). HALOTAG™ (ADN27525.1) control vector (Promega) was used for expression of the HALOTAG™ protein alone. MSCV-GFP-KDM4A, MSCV-GFP-H188A, MSCV-RFP-HP1, pSuper and pSuper sh2A4C constructs were made as described (Black et al., 2010). N-terminal HA-FLAG (NHF) deletion constructs were generated using primers at the indicated amino acid residues and cloned into pEntry using Gateway technology (Invitrogen). Fragments were transferred to N-terminal HA-FLAG (NHF) destination vector following the manufacturer's instructions. H188A contains two point mutations to eliminate catalytic activity H188A and W208R (Black et al., 2010). All clones were sequence verified.

*Western Blots.* Western blots were performed as in (Black et al., 2010). Antibodies used were: KDM4A (Neuro mAB, 75-189), β-actin (Millipore), GFP (Neuro mAB, 73-131), RFP (Abcam, ab62341), p53 (Santa Cruz, sc-126), MCM2 (Cell Signaling, #3619S), MCM3 (Cell Signaling, #4012S), MCM7 (Cell Signaling, #3735S), Halo (Promega), Actinin (Santa Cruz, sc-17829), HA 12CA5 (Roche), FLAG M2 (Sigma), DNA Pol α (Abcam, ab31777).

*G-Band and Spectral Karyotyping.* G-Band analysis was performed and analyzed by WiCell Cytogenetics Institute (Wisconsin). SKY and corresponding analysis was performed by Cristina Montagna in the Molecular Cytogenetic Core at Albert Einstein College of Medicine. The RPE stable cell lines were analyzed by SKY two independent times: once after six months in culture and a second time after an additional three months of culture.

*Subcellular localization and Catalytic Activity of KDM4A deletion fragments.* The indicated NHF-tagged KDM4A deletion constructs were transfected into RPE cells grown on coverslips in 10 cm dishes using X-tremeGENE 9™ DNA transfection reagent (Roche). H3K36me3 and subcellular localization were assayed by examining transfected cells (positive for HA staining; HA.11 Covance) following fixation in 3.7% PFA in PBS (Whetstine et al., 2006).

*Immunoprecipitation and Chromatin Immunoprecipitation.* Immunoprecipitations were performed essentially as described in (Van Rechem et al., 2011). Briefly, cells were lysed in cellular lysis buffer (5mM PIPES, 85mM KCl, 0.5% NP40) and nuclei were collected following centrifugation. Nuclei were lysed in IPH buffer with sonication, lysates were cleared and immunoprecipitations were performed in the presence of 100ug/ml ethidium bromide and digested with 0.25ul benzonase to eliminate protein-nucleic acid interactions. Immunoprecipitation from 293T cells were carried out in IPH with 150mM NaCl and from RPE cells in 300mM NaCl. Immunoprecipitation of HaloTag polymerase subunits with endogenous KDM4A was performed according to the manufacturers protocol (Promega) with minor modification. Whole cell lysates were prepared using sonication of cells in Halo lysis buffer. Immunoprecipitations were performed with ethidium bromide overnight at 4ûC. Elution was performed using SDS sample loading buffer.

Chromatin immunoprecipitations were performed as in (Black et al., 2010) with some minor changes. Sonication was performed using a Qsonica Q800R™ system with a constant chiller. For ChIP, RPE cells were arrested in 2mM HU for 20 hours prior to crosslinking to assess enrichment in KDM4A, HP1y, H3K9me3, H3K36me3, and DNA polymerase α at G1/S transition. For release from HU, HU containing media was removed by aspiration, and cells were rinsed twice with fresh DMEM prior to addition of fresh DMEM for release for the indicated times. 1 to 10ug of chromatin was used for each IP, which was dependent on the antibody used. For HP1y, chromatin was prepared and sonicated in TE with 1%SDS, diluted to 0.2% SDS prior to dilution for the immunoprecipitation. Chromatin for histone ChIP was prepared and sonicated in TE with 1% SDS. Chromatin for KDM4A, Polα, and MCM7 was prepared and sonicated in TE with 0.2% SDS. Prior to ChIP, RPE chromatin was precleared for one hour with protein A agarose, and for one hour with magnetic protein A or G beads (Invitrogen; to match antibody type). Immunoprecipitated DNA was purified using either PCR Purification Columns (Promega) or AMPureXP™. Data presented are averages from the two independently prepared polyclonal RPE cell lines from at least two independent chromatin preparations per cell line. Antibodies used for ChIP are as follows: DNA Polα (Abcam, ab31777 lot 290601), MCM7 (Cell Signaling, #3735S lots 02/2013 and 03/2013), KDM4A (Black et al., 2010), HPly (Millipore, 05-690 lot DAM1501782), H3K9me3 (Abcam, ab8898 lot GR30928-1), H3K36me3 (Abcam, ab9050 lot GR10860-1), H3 (Abcam, ab1791 lot GR63387-1).

*Fluorescent In Situ Hybridization (FISH).* FISH was performed as described in (Manning et al., 2010). Probes for 1q12h, 1q telomere, and chromosome 2, 6, 8, and X alpha satellite were purchased from Rainbow Scientific. Probes for 1q12 (RP11-80L2), Xq13.2 (RP11-451A22), Xq13.1 (RP11-177A4) were purchased as BAC clones from Children's Hospital Oakland Research Institute (CHORI BacPac) FISH verified clone repository. Oligonucleotide probes for 1q21.2 (BCL9) and 1q23.3 were purchased from Agilent (SureFISH). BACS were prepared utilizing PureLink HiPure Plasmid Filter Maxiprep kit (Life Technologies) using the recommended modified wash buffer. Probes were nick translated (Abbot Molecular Kit) in the presence of fluorescently labeled dTTP (Enzo Life Science). Images of multiple planes of fields of nuclei were acquired on an Olympus IX81 Spinning Disk Microscope and analyzed using Slidebook 5.0 software. We used a conservative scoring metric for copy gain. Any foci that were touching were scored as a single foci to prevent increased numbers due to normally replicated foci. For RPE cells, copy gain was scored as any cell with 3 or more distinct foci. For 293T cells, copy gain was scored for any cell with 5 or more distinct foci. For RPE cells each experiment includes at least one replicate from the two different polyclonal stable cell preparations. At least 100 cells for each replicate were scored for all experiments except analysis of single cell clones.

*Infection with Histone H3.3 variants.* Lentiviral stocks provided from the Allis lab were used to infect RPE cells in the presence of 8pg/ml polybrene for 8 hours (Lewis et al., 2013). Cells were washed 2 times with DMEM. Cells were collected 24 hours later for analysis by FISH and western blot. Incorporation of histone variants was confirmed by subcellular fractionation and western blotting.

*Fluorescent In Situ Hybridization (FISH) coupled to Chromosome 1 Paint.* FISH/Paint was performed using the 1q12/21 probe labeled in Spectrum Orange combined with a chromosome 1 painting probe labeled in Spectrum Green™. The chromosome paint probe was generated using standard protocols (Montagna et al., 2002). Equal volumes of locus specific and paint probe resuspended in hybridization solution (50% dextran sulfate/2× SSC) were combined and denatured at 85°C for 5 min, applied to the slides and incubated overnight at 37°C in a humidified chamber. Before hybridization the slides with interphase nuclei were denatured with 50% formamide/2× SSC at 80°C for 1.5 min and then dehydrated with serial ethanol washing steps (ice cold 70%, 90% and 100% for 3 min each). After hybridization the slides were washed three times for 5 min with 50% formamide/2× SSC, 1× SSC and 4× SSC/0.1%Tween. Slides were dehydrated with serial ethanol washing steps (as above) and mounted with ProLong Gold™ antifade reagent with DAPI (Invitrogen) for imaging. FISH/Paint images were acquired with a manual inverted fluorescence microscope (Axiovert 200, Zeiss) with fine focusing oil immersion lens (×40, NA 1.3 oil and ×60, NA 1.35 oil). Multiple focal planes were acquired for each channel to ensure that signals on different focal planes were included. The resulting fluorescence emissions were collected using 425-475 nm (for DAPI), 546-600 nm (for spectrum orange), and 500-550 nm (for AlexaFluor488) filters. The microscope was equipped with a Camera Hall 100 and the Applied Spectral Imaging software.

*Cesium Chloride Gradient Centrifugation.* RPE cells were treated with 100pM BrdU 14 hours prior to harvest. Cells were lysed in RIPA supplemented with 100µg of RNAse A (Fisher) for 2 hours at 37 degrees Celsius. Buffer was supplemented to 1% SDS and 20 pg of proteinase K was added and digested overnight at 55 degrees Celsius. DNA was extracted three times with phenol:chloroform:isoamyl alcohol and ethanol precipitated. Precipitated DNA (approximately 300 pg) was resuspended in NEB buffer 2 supplemented with RNAse A and digested with 200U of EcoRI and BamHI (NEB) overnight at 37 degrees Celsius. Digests were supplemented to 1% SDS and digested with 10 pg of proteinase K for 1 hour at 55 degrees Celsius. DNA was extracted with phenol:chloroform:isoamyl alcohol and ethanol precipitated Precipitated DNA was resuspended in TE and concentration determined by Nanodrop. 100 pg of DNA was mixed with 1g/ml CsCl in TE (refractive index of 1.4015-1.4031) in Quick-Seal ultracentrifuge tubes (Beckman). The CsCl gradient was centrifuged at 44,400 RPM in a VTi-65 rotor for 72 hours at 25 degrees Celsius. Fractions were collected from the bottom of the gradient in ∼200 pl aliquots. DNA concentration of each fraction was measured by Nanodrop. Appropriate fractions were pooled, dialyzed against 0.1X TE and concentrated by dialysis against 0.1XTE with 40% glycerol. Concentrated pools were ethanol precipitated and resuspended in ddH2O prior to analysis by quantitative PCR (qPCR). Each re-replicated fraction was diluted to 15ng/ul stock and 7.5ng of re-replicated DNA pool was analyzed by qPCR on a Roche LC480 using FastStart Universal SYBR Green Master Mix (Roche) following the manufacturer's instructions. 7.5ng of input DNA was analyzed by qPCR at the same time. Each sample was normalized to its own input prior to determination of fold-change in re-replication.

*HaloTag Mammalian Pull-Down Assay for Mass Spectrometry.* HEK293T cells (12 x 106 cells) were plated in a 150 mm dish and grown to 70-80% confluence (approximately 18 hours). The cells were then transfected with 30µg of plasmid DNA using FuGENE HD Transfection Reagent (Promega) for 24 hours, according to manufacturer's protocol. Cells expressing Halo-KDM4A or Halo-CTRL were incubated in mammalian lysis buffer (50mM Tris-HCl, pH 7.5, 150mM NaCl, 1% Triton X-100, and 0.1% sodium deoxycholate) supplemented with Protease Inhibitor cocktail (Promega) and RQ1 RNAse-Free DNAse (Promega) for 10 minutes on ice. Lysate was then homogenized with a syringe and centrifuged at 14.000 x g for 5 minutes to pellet cellular debris. Clarified lysate was incubated with HaloLink Resin (Promega) that had been pre-equilibrated in resin wash buffer (TBS and 0.05% IGEPAL CA-640 (Sigma)) for 15 minutes at 22oC with rotation. Resin was then washed 5 times with wash buffer, and protein interactors were eluted with SDS elution buffer (50mM Tris-HCl, pH 7.5, and 1% SDS).

*Mass Spectrometry Analysis.* HaloTag™ pulldown or co-immunoprecipitation purified complexes were analyzed and processed by MS Bioworks, LLC (Ann Arbor, Michigan). The samples were separated on a SDS-PAGE gel, which was then Coomassie stained and cut into 10 fragments. Each gel piece was processed with the Progest™ Protein Digestion Station (Digilab). Briefly, gel slices were washed using 25 mM ammonium bicarbonate and acetonitrile, followed by reduction with 10 mM dithiothreitol, and alkylation with 50 mM iodoacetamide. Proteins were digested with trypsin (Promega) for 4 hours and digestion was quenched with formic acid. Gel digests were analyzed directly by nano LC/MS/MS with a NanoAcquity™ HPLC (Waters) interfaced with an Orbitrap Velos Pro™ (Thermo Scientific) tandem mass spectrometer. Digested peptides were loaded on a trapping column and eluted over a 75 µm analytical column packed with Jupiter Proteo Resin™ (Phenomenex) at 350nl/min. The mass spectrometer was operated in data-dependent mode, with MS performed in the Orbitrap at 60.000 full width at half maximum (FWHM) resolution, and MS/MS performed in the LTQ. The 15 most abundant ions were selected for MS/MS. The data were searched with Mascot™ (Matrix Science) against the concatenated forward/decoy UniProt Human Database, and Mascot DAT files were visualized and filtered by Scaffold (Proteome Software). Data were filtered using a minimum protein value of 90%, a minimum peptide value of 50% (Protein and Peptide Prophet scores), and required at least two unique peptides per protein. Spectral counting was performed and normalized spectral abundance factors determined. Data were reported at less than 1% false discovery rate (FDR) at the protein level based on counting the number of forward and decoy matches.

*KDM4A Copy Number Determination in TCGA Data Set.* The somatic copy number alterations (SCNAs) for 24,176 genes of the pan-cancer data set including 4,420 samples across multiple tumor types are annotated by GISTIC2.0™ (Beroukhim et al., 2007; Beroukhim et al., 2010; Network, 2008). The copy number change in each gene is defined as possessing deep deletion (-2), shallow deletions (-1), neutral copy number (0), low gain (+1), and high gain (+2) in each sample using sample-specific thresholds. High gains are segments with copy number that exceed the maximum median chromosomal arm copy number for that sample by at least 0.1; low gains are segments with copy numbers from 2.1 to the high gain threshold; neutral segments have copy numbers between 1.9 and 2.1; shallow losses have copy numbers between 1.9 and the deep deletion threshold; and deep deletion have copy numbers that are below the minimum median chromosomal arm copy number for that sample by at least 0.1.

*KDM4A mRNA expression from RNA-seq in TCGA Data Set.* Reads per kilobase of exon model per million mapped reads (RPKM) were annotated for 16,407 genes in 1953 samples across multiple tumor types. 1770 samples having both copy number and RPKM data were used to quantify an association between copy number alterations and the mRNA expression levels in KDM4A in Fig. 1B and 1D. The "Gain" group corresponds to the sample set with KDM4A GISTIC annotation = +1 or +2, the "No change" group corresponds to the samples set with KDM4A GISTIC annotation = 0, and the "Loss" group is associated to the sample set with KDM4A GISTIC annotation = -1 or -2.

*Clinical Data in TCGA Data Set.* Overall survival in 541 Ovarian Cancer samples (256 alive and 285 deceased) was defined as the interval from the date of initial surgical resection to the date of last known contact or death. The association of the KDM4A copy number status, Del (-2), Loss (-1), None (0), Gain (+1), Amp (+2) to the clinical outcome in Fig. 1E through 1H was tested for 285 deceased patients by Student's t-test (one-tailed) and statistical significance was considered when P < 0.05.

*Determination of Cytoband Copy Number and Correlation with KDM4A in TCGA Data Set.* In addition to the copy number annotation for each gene, the mean focal copy number for 807 cytobands including X-chromosome were annotated in each sample by taking an average of focal copy numbers of every genes within the same cytoband. Arm-level SCNA contributions to the mean focal copy number in each cytoband were removed by only considering GISTIC annotated focal copy numbers much smaller than a chromosome arm or entire chromosome. Detecting chromosomal regions significantly co-amplified with KDM4A copy gains or amplifications was first performed by the one-tailed Student's t-test for the mean focal copy number changes between KDM4A copy-gained samples (GISTIC annotation = +1 or +2) and KDM4A copy-neutral samples (GISTIC annotation = 0) across 807 cytobands. We also calculated the significance using the gene-specific copy-number for KDM4A and KDM4B as positive controls (Fig. 6A-C). The second independent test was performed by approximating a null distribution of mean cytoband copy differences by a normal function N(µ12 - µ0,σ0 2 / n0 + σ12 2 / n12) where µ0 and µ12 are samples means across all cytobands, σ0 2 and σ12 2 are mean sample-specific variances within each group, and n0 and n12 are the number of samples in KDM4A copy-neutral and KDM4A copy-gained groups, respectively. This test is based on comparing the means of the two sets while permuting values within each of the samples (and using a Gaussian approximation). The p-values across 807 cytobands were annotated by computing the probability of more extreme differences than the corresponding cytoband copy difference in the null distribution. The empirical cumulative distribution functions (the fraction of samples below the given mean focal copy) were determined by enumerating samples having the mean focal copy number less than or equal to the value on the x-axis for KDM4A and KDM4B amplified (+2), copy-gained (+1), and copy-neutral samples (0).

### REFERENCES

Alabert, C., and Groth, A. (2012). Chromatin replication and epigenome maintenance. Nat Rev Mol Cell Biol 13, 153-167.
Arias, E.E., and Walter, J.C. (2007). Strength in numbers: preventing rereplication via multiple mechanisms in eukaryotic cells. Genes Dev 21, 497-518.
Beck, D.B., Burton, A., Oda, H., Ziegler-Birling, C., Torres-Padilla, M.E., and Reinberg, D. (2012). The role of PR-Set7 in replication licensing depends on Suv4-20h. Genes Dev 26, 2580-2589.
Beroukhim, R., Getz, G., Nghiemphu, L., Barretina, J., Hsueh, T., Linhart, D., Vivanco, I., Lee, J.C., Huang, J.H., Alexander, S., et al. (2007). Assessing the significance of chromosomal aberrations in cancer: methodology and application to glioma. Proc Natl Acad Sci U S A 104, 20007-20012.
Beroukhim, R., Mermel, C.H., Porter, D., Wei, G., Raychaudhuri, S., Donovan, J., Barretina, J., Boehm, J.S., Dobson, J., Urashima, M., et al. (2010). The landscape of somatic copy-number alteration across human cancers. Nature 463, 899-905.
Berry, W.L., Shin, S., Lightfoot, S.A., and Janknecht, R. (2012). Oncogenic features of the JMJD2A histone demethylase in breast cancer. Int J Oncol 41, 1701-1706.
Black, J.C., Allen, A., Van Rechem, C., Forbes, E., Longworth, M., Tschop, K., Rinehart, C., Quiton, J., Walsh, R., Smallwood, A., et al. (2010). Conserved antagonism between JMJD2A/KDM4A and HP1gamma during cell cycle progression. Mol Cell 40, 736-748.
Black, J.C., and Whetstine, J.R. (2011). Chromatin landscape: methylation beyond transcription. Epigenetics 6, 9-15.
Brunet, A., Armengol, L., Heine, D., Rosell, J., Garcia-Aragones, M., Gabau, E., Estivill, X., and Guitart, M. (2009). BAC array CGH in patients with Velocardiofacial syndrome-like features reveals genomic aberrations on chromosome region 1q21.1. BMC Med Genet 10, 144.
Davidson, I.F., Li, A., and Blow, J.J. (2006). Deregulated replication licensing causes DNA fragmentation consistent with head-to-tail fork collision. Mol Cell 24, 433-443.
Ding, Q., and MacAlpine, D.M. (2010). Preferential re-replication of Drosophila heterochromatin in the absence of geminin. PLoS Genet 6.
Hastings, P.J., Lupski, J.R., Rosenberg, S.M., and Ira, G. (2009). Mechanisms of change in gene copy number. Nat Rev Genet 10, 551-564.
Hayashi, M.T., Takahashi, T.S., Nakagawa, T., Nakayama, J., and Masukata, H. (2009). The heterochromatin protein Swi6/HP1 activates replication origins at the pericentromeric region and silent mating-type locus. Nat Cell Biol 11, 357-362.
Hook, S.S., Lin, J.J., and Dutta, A. (2007). Mechanisms to control rereplication and implications for cancer. Curr Opin Cell Biol 19, 663-671.
Inoue, J., Otsuki, T., Hirasawa, A., Imoto, I., Matsuo, Y., Shimizu, S., Taniwaki, M., and Inazawa, J. (2004). Overexpression of PDZKI within the 1q12-q22 amplicon is likely to be associated with drug-resistance phenotype in multiple myeloma. Am J Pathol 165, 71-81.
Jiang, X.R., Jimenez, G., Chang, E., Frolkis, M., Kusler, B., Sage, M., Beeche, M., Bodnar, A.G., Wahl, G.M., Tlsty, T.D., and Chiu, C.P. (1999). Telomerase expression in human somatic cells does not induce changes associated with a transformed phenotype. Nat Genet 21, 111-114.
Karnani, N., Taylor, C.M., and Dutta, A. (2009). Microarray analysis of DNA replication timing. Methods Mol Biol 556, 191-203.
Kiang, L., Heichinger, C., Watt, S., Bahler, J., and Nurse, P. (2010). Specific replication origins promote DNA amplification in fission yeast. J Cell Sci 123, 3047-3051.
Kim, T.D., Shin, S., Berry, W.L., Oh, S., and Janknecht, R. (2012). The JMJD2A demethylase regulates apoptosis and proliferation in colon cancer cells. J Cell Biochem 113, 1368-1376.
Kracikova, M., Akiri, G., George, A., Sachidanandam, R., and Aaronson, S.A. (2013). A threshold mechanism mediates p53 cell fate decision between growth arrest and apoptosis. Cell Death Differ. Kudoh, K., Takano, M., Koshikawa, T., Hirai, M., Yoshida, S., Mano, Y., Yamamoto, K., Ishii, K., Kita, T., Kikuchi, Y., et al. (1999). Gains of Iq21-q22 and 13q12-q14 are potential indicators for resistance to cisplatin-based chemotherapy in ovarian cancer patients. Clin Cancer Res 5, 2526-2531.
Lewis, P.W., Muller, M.M., Koletsky, M.S., Cordero, F., Lin, S., Banaszynski, L.A., Garcia, B.A., Muir, T.W., Becher, O.J., and Allis, C.D. (2013). Inhibition of PRC2 Activity by a Gain-of-Function H3 Mutation Found in Pediatric Glioblastoma. Science.
Luo, J., Solimini, N.L., and Elledge, S.J. (2009). Principles of cancer therapy: oncogene and non-oncogene addiction. Cell 136, 823-837.
Mallette, F.A., Mattiroli, F., Cui, G., Young, L.C., Hendzel, M.J., Mer, G., Sixma, T.K., and Richard, S. (2012). RNF8- and RNF168-dependent degradation of KDM4A/JMJD2A triggers 53BP1 recruitment to DNA damage sites. Embo J.
Mallette, F.A., and Richard, S. (2012). JMJD2A promotes cellular transformation by blocking cellular senescence through transcriptional repression of the tumor suppressor CHD5. Cell Rep 2, 1233-1243.
Manning, A.L., Longworth, M.S., and Dyson, N.J. (2010). Loss of pRB causes centromere dysfunction and chromosomal instability. Genes Dev 24, 1364-1376.
Maslov, A.Y., and Vijg, J. (2009). Genome instability, cancer and aging. Biochim Biophys Acta 1790, 963-969.
Mermel, C.H., Schumacher, S.E., Hill, B., Meyerson, M.L., Beroukhim, R., and Getz, G. (2011). GISTIC2.0 facilitates sensitive and confident localization of the targets of focal somatic copy-number alteration in human cancers. Genome Biol 12, R41.
Montagna, C., Andrechek, E.R., Padilla-Nash, H., Muller, W.J., and Ried, T. (2002). Centrosome abnormalities, recurring deletions of chromosome 4, and genomic amplification of HER2/neu define mouse mammary gland adenocarcinomas induced by mutant HER2/neu. Oncogene 21, 890-898.
Negrini, S., Gorgoulis, V.G., and Halazonetis, T.D. (2010). Genomic instability--an evolving hallmark of cancer. Nat Rev Mol Cell Biol 11, 220-228.
Network, C.G.A.R. (2008). Comprehensive genomic characterization defines human glioblastoma genes and core pathways. Nature 455, 1061-1068.
Papamichos-Chronakis, M., and Peterson, C.L. (2013). Chromatin and the genome integrity network. Nat Rev Genet 14, 62-75.
Schimke, R.T. (1984). Gene amplification, drug resistance, and cancer. Cancer Res 44, 1735-1742. Schwaiger, M., Kohler, H., Oakeley, E.J., Stadler, M.B., and Schubeler, D. (2010). Heterochromatin protein 1 (HP1) modulates replication timing of the Drosophila genome. Genome Res 20, 771-780. Snaith, H.A., and Forsburg, S.L. (1999). Rereplication phenomenon in fission yeast requires MCM proteins and other S phase genes. Genetics 152, 839-851.
Stratton, M.R., Campbell, P.J., and Futreal, P.A. (2009). The cancer genome. Nature 458, 719-724. Takano, M., Kudo, K., Goto, T., Yamamoto, K., Kita, T., and Kikuchi, Y. (2001). Analyses by comparative genomic hybridization of genes relating with cisplatin-resistance in ovarian cancer. Hum Cell 14, 267-271.
Tardat, M., Brustel, J., Kirsh, O., Lefevbre, C., Callanan, M., Sardet, C., and Julien, E. (2010). The histone H4 Lys 20 methyltransferase PR-Set7 regulates replication origins in mammalian cells. Nat Cell Biol 12, 1086-1093.
Van Rechem, C., Black, J.C., Abbas, T., Allen, A., Rinehart, C.A., Yuan, G.C., Dutta, A., and Whetstine, J.R. (2011). The SKP1-Cul1-F-box and leucine-rich repeat protein 4 (SCF-FbxL4) ubiquitin ligase regulates lysine demethylase 4A (KDM4A)/Jumonji domain-containing 2A (JMJD2A) protein. J Biol Chem 286, 30462-30470.
Vassilev, L.T. (2006). Cell cycle synchronization at the G2/M phase border by reversible inhibition of CDK1. Cell Cycle 5, 2555-2556.
Whetstine, J.R., Nottke, A., Lan, F., Huarte, M., Smolikov, S., Chen, Z., Spooner, E., Li, E., Zhang, G., Colaiacovo, M., and Shi, Y. (2006). Reversal of histone lysine trimethylation by the JMJD2 family of histone demethylases. Cell 125, 467-481.
Wong, N., Lam, W.C., Lai, P.B., Pang, E., Lau, W.Y., and Johnson, P.J. (2001). Hypomethylation of chromosome 1 heterochromatin DNA correlates with q-arm copy gain in human hepatocellular carcinoma. Am J Pathol 159, 465-471.
Wu, R., Singh, P.B., and Gilbert, D.M. (2006). Uncoupling global and fine-tuning replication timing determinants for mouse pericentric heterochromatin. J Cell Biol 174, 185-194.
Wu, R., Terry, A.V., Singh, P.B., and Gilbert, D.M. (2005). Differential subnuclear localization and replication timing of histone H3 lysine 9 methylation states. Mol Biol Cell 16, 2872-2881.
Yakut, T., Schulten, H.J., Demir, A., Frank, D., Danner, B., Egeli, U., Gebitekin, C., Kahler, E., Gunawan, B., Urer, N., et al. (2006). Assessment of molecular events in squamous and non-squamous cell lung carcinoma. Lung Cancer 54, 293-301.

### EXAMPLE 2

As described herein, KDM4A is a contributor to cancer pathophysiology. Single nucleotide changes within this enzyme is not limited to somatic mutations because germline single nucleotide polymorphisms (SNPs) can occur. Identification and characterization of such mutations can permit the classification of patients and provide diagnostic and prognostic information to guide treatment.

Described herein and in Figs. 15A-15E, 16A-16D, 17A-17F, 18, 19A-19E, 20A-20E, 21A-21B, and 22A-22B is the importance of a germline variant and somatic mutations within the histone fri-demethylase KDM4A/JMJD2A to chemotherapeutic response. The KDM4A SNP associates with worse outcome for non-small cell lung cancer (NSCLC) patients, impacts KDM4A regulation (cellular localization, ubiquitination and turnover) and associates with increased mTOR inhibitor sensitivity.

Also identified are somatic mutations throughout the KDM4A gene. It is demonstrated herein that somatic mutations that impair KDM4A function or levels have increased mTOR inhibitor sensitivity. These findings have direct clinical implications because the SNP, protein levels mis-localization and mutations altering KDM4A function are able to provide a mechanism for sensitizing cells to clinically relevant chemotherapeutics.

These findings highlight the use of this germline variant and somatic mutations as biomarkers for stratifying NSCLC as well as other cancer patients when being treated with certain classes of chemotherapeutics such as mTOR inhibitors or protein translation inhibitors.

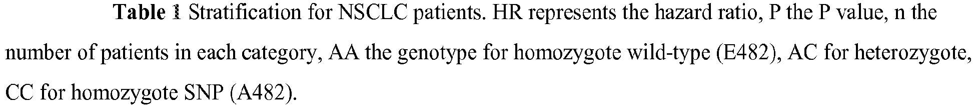

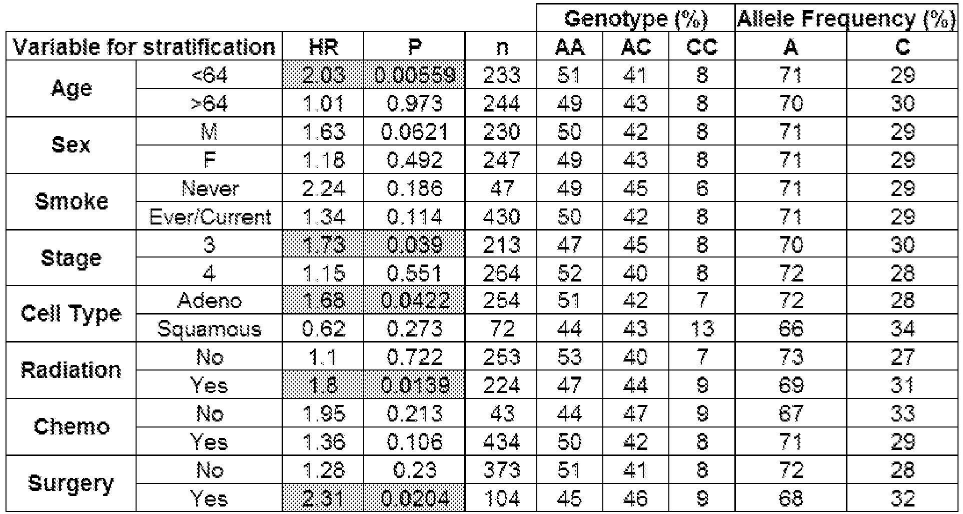

**Table 2: Mutations of KDM4A and their effects on localization and demethylase activity. For localization: +++ indicates > 80% nuclear, ++ indicates 60-79% nuclear, + indicates 40-59% nuclear, - indicates 20-39% nuclear, -- indicates 0 to 19% nuclear. For Activity: + indicates active, - indicates inactive, +/- indicates partial acitivty. NT = not tested.**

| Variant | Localization | H3k36 Demethylase Activity | H3k9 Demethylase Activity |
|---|---|---|---|
| 2A-E23K | - | + | + |
| 2A-S28N | +++ | + | + |
| 2A-I87V | +++ | + | + |
| 2A-E113K | +++ | + | +/- |
| 2A-K123I | + | + | NT |
| 2A-N128S | NT | NT | NT |
| 2A-R152W* | +++ | + | + |
| 2A-R218W | ++ | - | + |
| 2A-G225C | ++ | +/- | + |
| 2A-A236V | +++ | + | + |
| 2A-R239H | +++ | +/- | +/- |
| 2A-G278S | +++ | - | - |
| 2A-T289I | +++ | - | +/- |
| 2A-V319M | ++ | + | + |
| 2A-P326T | ++ | + | + |
| 2A-P348L | ++ | + | + |
| 2A-E368K | ++ | + | + |
| 2A-G376V | ++ | + | + |
| 2A-R400Q | + | + | + |
| 2A-E426K | +++ | + | + |
| 2A-V490M | ++ | + | + |
| 2A-R498H | ++ | + | + |
| 2A-D524V | +++ | + | + |
| 2A-E558Q | + | + | + |
| 2A-R597H | NT | NT | NT |
| 2A-A662S | NT | NT | NT |
| 2A-S713L | NT | NT | NT |
| 2A-V743I* | +++ | + | + |
| 2A-R765Q | -- | - | - |
| 2A-G783FS | -- | - | NT |
| 2A-L803FS | -- | + | NT |
| 2A-R825C* | +++ | + | + |
| 2A-R825H* | ++ | + | + |
| 2A-V919M | + | + | NT |
| 2A-L941F | NT | NT | NT |
| 2A-S948T | NT | NT | NT |
| 2A-V1003A | ++ | + | + |
| 2A-D1023Y | ++ | + | + |
| 2A-R1025C* | ++ | + | + |
| 2A-E1032K | ++ | + | + |

**Table 3: Mutations of KDM4B and their effects on localization and demethylase activity.**

| Variant | Localization | H3k36 Demethylase Activity | H3k9 Demethylase Activity |
|---|---|---|---|
| **I35K** | NT | NT | NT |
| **A43V** | NT | NT | NT |
| **R120H** | NT | NT | NT |
| **N150S** | NT | NT | NT |
| **E191K** | NT | NT | NT |
| **Y196H** | NT | NT | NT |
| **S197N** | NT | NT | NT |
| **R222Q** | NT | NT | NT |
| **G230V** | NT | NT | NT |
| **A237T** | NT | NT | NT |
| **M243I** | NT | NT | NT |
| **R296L** | NT | NT | NT |
| **V320M** | NT | NT | NT |
| **F321L** | NT | NT | NT |
| **T340M** | NT | NT | NT |
| **R346W** | NT | NT | NT |
| **P380S** | NT | NT | NT |
| **Q542L** | NT | NT | NT |
| **Q542H** | NT | NT | NT |
| **G572V** | NT | NT | NT |
| **P623T** | NT | NT | NT |
| **S747C** | NT | NT | NT |
| **R775H** | NT | NT | NT |
| **A792T** | NT | NT | NT |
| **T811S** | NT | NT | NT |
| **D894N** | NT | NT | NT |
| **S978F** | NT | NT | NT |
| **Frameshift** | | | |
| **P463** | NT | NT | NT |

**Table 4: Mutations of KDM4C and their effects on localization and demethylase activity.**

| Variant | Localization | H3k36 Demethylase Activity | H3k9 Demethylase Activity |
|---|---|---|---|
| **R58I** | NT | NT | NT |
| **A71S** | NT | NT | NT |
| **R121H** | NT | NT | NT |
| **K125N** | NT | NT | NT |
| **V130A** | NT | NT | NT |
| **V158I** | NT | NT | NT |
| **R220Q** | NT | NT | NT |
| **E268K** | NT | NT | NT |
| **T379I** | NT | NT | NT |
| **D396N** | NT | NT | NT |
| **D408E** | NT | NT | NT |
| **S434F** | NT | NT | NT |
| **E498D** | NT | NT | NT |
| **E519D** | NT | NT | NT |
| **S534R** | NT | NT | NT |
| **R572H** | NT | NT | NT |
| **A614V** | NT | NT | NT |
| **T623M** | NT | NT | NT |
| **A630S** | NT | NT | NT |
| **A646S** | NT | NT | NT |
| **T677A** | NT | NT | NT |
| **S713R** | NT | NT | NT |
| **C718R** | NT | NT | NT |
| **A719G** | NT | NT | NT |
| **G740E** | NT | NT | NT |
| **A750V** | NT | NT | NT |
| **W751C** | NT | NT | NT |
| **L765F** | NT | NT | NT |
| **V782L** | NT | NT | NT |
| **T794A** | NT | NT | NT |
| **V870M** | NT | NT | NT |
| **S872F** | NT | NT | NT |
| **S928C** | NT | NT | NT |
| **I962T** | NT | NT | NT |
| **T1042A** | NT | NT | NT |
| **nonsense** | | | |
| **p.E695** | NT | NT | NT |
| **p.R786** | NT | NT | NT |
| **p.R997** | NT | NT | NT |

**Table 5: Mutations of KDM4D and their effects on localization and demethylase activity.**

| Variant | Localization | H3k9 Demethylase Activity |
|---|---|---|
| **G47D** | - | - |
| **D64N** | - | - |
| **R82W** | - | - |
| **Q88R** | +++ | +++ |
| **R102C** | +++ | +++ |
| **R123P** | - | - |
| **K210N** | - | - |
| **V214L*** | - | - |
| **R243W** | - | - |
| **A247D** | - | - |
| **P260H** | +++ | +++ |
| **R263H** | +++ | +++ |
| **E270D** | - | - |
| **P276L** | - | - |
| **R316W** | - | - |
| **R332H** | +++ | +++ |
| **K337R** | +++ | +++ |
| **R338C** | +++ | ++ |
| **V345M** | +++ | +++ |
| **P350S** | +++ | +++ |
| **G373C** | +++ | +++ |
| **R375I** | +++ | +++ |
| **R392H** | +++ | +++ |
| **S393I** | +++ | +++ |
| **G394E** | +++ | +++ |
| **R419Q** | - | - |
| **S484L** | +++ | +++ |
| **E487K** | +++ | +++ |
| **L508R** | +++ | +++ |
| **Frameshift** | | |
| **E256** | NT | NT |
| **K91** | NT | NT |
| **S107** | NT | NT |
| **nonsense** | | |
| **E27** | NT | NT |
| **R123** | NT | NT |
| **K124** | NT | NT |

**Table 6: Mutations of KDM4E and their effects on localization and demethylase activity.**

| Variant | Localization | H3k9 Demethylase Activity |
|---|---|---|
| **I15T** | NT | NT |
| **E64K** | NT | NT |
| **E162Q** | NT | NT |
| **S247L** | NT | NT |
| **L251I** | NT | NT |
| **G370V** | NT | NT |
| **G465E** | NT | NT |
| **R491G** | NT | NT |
| **D499Y** | NT | NT |
| **Nonsense** | | |
| **Q85** | NT | NT |
| **L480** | NT | NT |

### EXAMPLE 3

Hypoxia induces 1q12h copy number gains which can be antagonized by Suv39H1, HP1 gamma or succinate. RPE cells were transfected with RFP,
Suv39h1 or HP1G or treated with 2mM succinate for 24 hours. Cells were moved to 4% oxygenor kept at nor moxic conditions and copy number was determined (Fig. 24). Hypoxia increases the protein level of KDM4A in different cell lines (Fig. 25) in multiple cellular compartments (Fig. 26). The hypoxia-induced changes in KDM4A levels and/or localization can be reduced by NEM, which stabilizes SUMOylation and ubiquitination. NEM also alters the chromatin association (Fig. 26).

### EXAMPLE 4

miRNAs regulate the expression level of KDM4A (Fig. 27A) and transfection with certain miRNAs induces sensitization of U2OS cells to rapamycin and PLX4720 (Figs. 27B-27C).

### EXAMPLE 5

The effect of KMD4A mutations on drug sensitivity was examined by depleting cells of KDM4A (shA10) and then transfecting with wild-type or mutant KDM4A. The sensitivity of the cells to rapamycin was then determined. Expression of widltype KDM4A reduced the senstitivity of the cells as compared to the shA10 depletion alone (Fig. 28). A catalytic mutant of KDM4A (H188A) did not reduce drug sensitivity as compared to transfection with wildtype KDM4A (Fig. 30). A SNV mutation that causes a R1025C mutation was equally as sensitive as KDM4A/JMJD2A depletion (shA10) when treated with Rapamycin (Fig. 29). A mutation that causes a frameshift G783fs was equally as sensitive as KDM4A/JMJD2A depletion (shA10) when treated with Rapamycin (Fig. 31). This differed from WT expression in the depletion background, which was less sensitive.

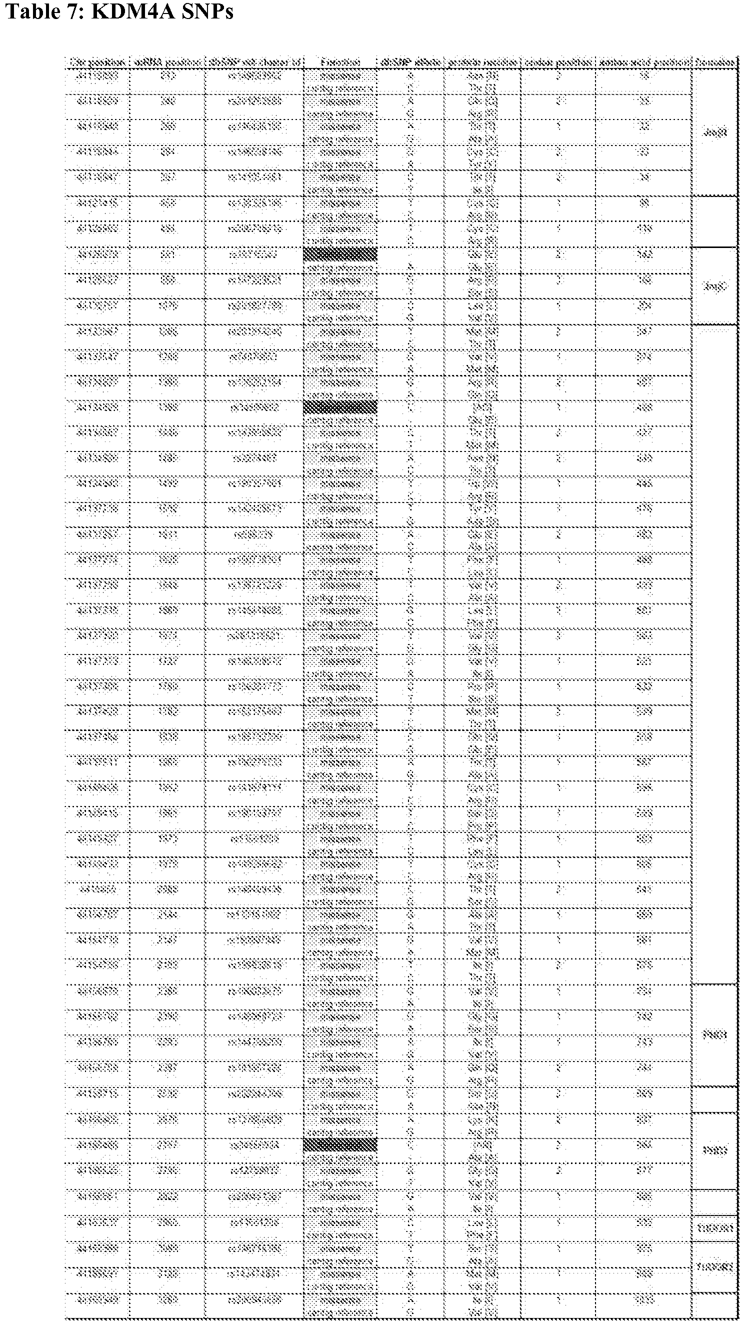

### EXAMPLE 6: Histone Demethylase KDM4A Contains a Coding Polymorphism that Associates with Outcome and Alters Drug Response

Single nucleotide polymorphisms (SNPs) occur in chromatin-modulating factors; however, little is known about their impact on cancer progression or treatment. Therefore, we need to establish the biochemical and/or molecular contribution of the coding variants, how they can be used to sub-classify patients and how they can impact therapeutic response. In this report, we demonstrate that a germline SNP within the histone tri-demethylase KDM4A/JMJD2A promotes KDM4A turnover and predicts non-small cell lung cancer (NSCLC) patient outcome. We further demonstrate that SNP status, reduced KDM4A protein levels and its chemical inhibition enhance mTOR inhibitor sensitivity. The reduction in KDM4A protein levels decrease protein translation, which enhance the reduced protein synthesis caused by mTOR inhibition. Taken together, our data emphasize the importance of using KDM4A SNP status and protein levels as a candidate biomarker for mTOR inhibitor therapy and highlight the use of combined inhibition of KDM4A and mTOR in treating lung cancer.

It is demonstrated herein that:
a. KDM4A SNP-A482 is associated with NSCLC patients' outcome.
b. KDM4A SNP-A482 promotes ubiquitination and turnover.
c. SNP-A482 status, KDM4A levels and chemical inhibition increase mTOR inhibitor chemotherapeutic sensitivity.
d. Reduced KDM4A protein levels decrease translation and enhance the reduced protein synthesis caused by mTOR inhibition.

The nucleosome, composed of 147 base pairs (bp) of DNA wrapped around an octamer of histones, is the basic unit of chromatin. In general, chromatin can be classified into two states, open euchromatin, which typically replicates early and contains transcriptionally active genes, and compact and late-replicated heterochromatin that contains inactive genes and DNA repeat elements (Black and Whetstine, 2011). RNA and proteins are necessary to help and/or prevent the compaction of chromatin. Histones are heavily post-translationally modified by proteins called chromatin-modifying enzymes. At least 16 different classes of modifications are responsible for the regulation of chromatin state and biological processes such as transcription and DNA repair (Dawson and Kouzarides, 2012).

Described herein is the function of a SNP in the coding region of KDM4A, resulting in the conversion of the glutamic acid amino acid in position 482 to alanine (E482A), with alanine encoded by the minor allele. It is demonstrated that this SNP is linked to outcome in non-small cell lung cancer (NSCLC) patients. Biochemically, this SNP regulates the stability of KDM4A through its interaction with the SCF complex, leading to its increased ubiquitination, subsequent degradation, and decreased half-life. Furthermore, drug screen analyses uncover a function for this SNP in cell sensitivity to chemotherapeutic drugs targeting the mTOR pathway. It is further demonstrated that this increased sensitivity is due to reduced KDM4A levels. The reduction in KDM4A protein levels decrease protein translation, which enhance the reduced protein synthesis caused by mTOR inhibition. Together, the findings in this report reveal a new role for KDM4A in overall translation and highlight the importance of germline variation in response to chemotherapies. These data reveal a biomarker and predictor for targeted therapy for NSCLC patients, while permitting combination therapies targeting mTOR and KDM4A.

### RESULTS

**SNP-A482 alters KDM4A regulation.** It was investigated whether SNPs in KDM4A could impact enzyme regulation, function and/or cancer cell drug response. Because non-synonymous coding SNPs are more likely to directly alter protein functions due to a change in an amino acid sequence (Cargill et al., 1999; Halushka et al., 1999), the dbSNP database was evaluated and the only coding SNP for KDM4A with a reported frequency was identified. KDM4A SNP rs586339A>C presents a minor allele frequency (MAF) of 0.238. The rs586339 SNP results in a single base substitution that leads to an amino acid substitution: E482 (GAA) to A482 (GCA). Therefore, this germline variant is referred to herein as SNP-A482 (Fig. 19A). Adenine "A" encoding E482 was identified to be the major allele [referred to as wild type (WT) throughout Example 6] for two main reasons: 1) this amino acid is conserved across species (Fig. 32 and 19B); and 2) both dbSNP database and HapMap analysis reported "A" as the major allele. Upon evaluating the HapMap project, we observed different allelic frequencies across various ethnic populations (Fig. 19 D) (2005; Altshuler et al., 2010). These data highlight the ethnic diversity observed for this SNP, suggesting some level of selective pressure may alter the distribution of this allele in the human population. The average HapMap allelic frequency across all evaluated populations is 64.6% for homozygote for the major allele (WT), 29.9% for heterozygote, and 5.5% for homozygote for the minor allele (SNP-A482) (Fig. 19D). The presence of the SNP in cell lines was confirmed using Sanger sequencing (Figu. 19C) and restriction fragment length polymorphism (RFLP) (not shown).

It was sought to determine if there was a biochemical difference between KDM4A WT and KDM4A SNP-A482. To achieve this goal, it was first evaluated whether SNP-A482 impacted KDM4A localization by transiently transfecting cells with GFP-tagged KDM4A. GFP-KDM4A WT was exclusively nuclear in 85% of the cells and localized in both the nucleus and the cytoplasm in the remaining 15%, whereas GFP-KDM4A SNP-A482 was exclusively nuclear in 51% of the cells and both nuclear and cytoplasmic in 49% (Fig. 15E, p=0.018). These data highlight a difference in these two KDM4A variants.

Because KDM4A SNP-A482 was more cytoplasmic and the change in localization pattern is associated with KDM4A ubiquitination (Butler et al., 2012; Mallette et al., 2012), it was evaluated whether SNP-A482 impacted KDM4A protein regulation. When KDM4A SNP-A482 was expressed in HEK 293T cells, an increase in higher molecular weight products was observed (Fig. 37), which was then demonstrated to be ubiquitination by immunoblotting for ubiquitin after conducting denaturing GFP immunoprecipitation (IP) (Fig. 15B)). Multiple experiments indicated that there was a two-fold increase in KDM4A SNP-A482 ubiquitination when the normalized IP was compared to wild type (Fig. 15B). KDM4A can be ubiquitinated by the SCF complex containing the E3 ligase Cullin1 (Tan et al., 2011; Van Rechem et al., 2011). Consistent with KDM4A SNP-A482 being more ubiquinated, KDM4A SNP-A482 coimmunoprecipitated more MYC-Cullin1, which reflects an increased association with the SCF complex (Fig. 15D). In addition, KDM4A SNP-A482 exhibited a shorter half-life than KDM4A WT (1h38min versus 2h58min, respectively; Fig. 15C). Taken together, these data demonstrate that SNP-A482 results in altered KDM4A ubiquitination and contributes to changes in KDM4A stability.

KDM4A SNP-A482 is associated with NSCLC patients' outcome. Since KDM4A SNP-A482 affected KDM4A protein stability, it was hypothesized that KDM4A SNP-482 may impact clinical parameters in lung cancer patients. Therefore, a well characterized cohort of non-small cell lung cancer (NSCLC) patients was evaluated (Heist et al., 2008; Huang et al., 2009; Huang et al., 2011a; Huang et al., 2011b) and it was determined whether homozygous KDM4A SNP-A482 NSCLC patients were associated with differential outcome based on various clinical parameters. Interestingly, NSCLC patients and non-NSCLC patients presented comparable allelic frequency, suggesting that there is no selection against the A482 allele in NSCLC patients (Fig. 19E). However, patients that were homozygous for KDM4A SNP-A482 were associated with worse outcome for certain late stage patient parameters (Fig. 38A). For example, stage 3 patients had a 1.73-fold worse outcome (Hazard Ratio HR=1.73, p=0.039; Fig. 33A) when compared to heterozygote and wild type patients. KDM4A SNP-A482 homozygous status was also associated with worse outcome with radiation (HR=1.8, p=0.014; Fig. Fig. 33B), when patients were less than 64 years of age (HR=2.03, p=0.006), when the NSCLC patients had an adenocarcinoma (HR=1.68, p=0.042; Fig. 33D), or when patients received surgical intervention (HR=2.31, p=0.02; Fig. 33E). However, not all types of stratification presented a link to outcome (Fig. 33F). These data suggest that there is an important association between KDM4A SNP-A482 status and lung cancer outcome.

**KDM4A SNP-A482 alters chemotherapeutic sensitivity in lung cancer cell lines but does not affect other biological processes.** Since some patients with KDM4A SNP-A482 had a worse outcome, it was sought to establish whether chemotherapeutics to target homozygous KDM4A SNP-A482 lung cancer cells could be identified. By using this approach, novel therapeutic opportunities based on the *kdm4a* germline status in NSCLC patients could be identifed. An unbiased association study was conducted between 88 lung cancer cell lines that were genotyped (Fig. 19E) and screened for drug sensitivity to 87 preclinical and clinical compounds at three different drug concentrations. Similar to the analysis of the NSCLC patients, the cell lines homozygous for the minor allele were compared to the cell lines heterozygous and homozygous for the major allele. The results of the analysis are summarized as a volcano plot representing the statistical significance (inverted Y axis) versus the effect of KDM4A SNP-A482 on drug sensitivity. Each dot represents a drug, with only the drugs above the *X* axis considered as significantly associated with KDM4A SNP-A482 (p<0.05). The right quadrant represents an increased sensitivity to the drugs for the cells homozygous KDM4A SNP-A482, whereas the left quadrant represents an increased resistance for these cells compared to heterozygous and homozygous WT cells (Fig. 17A). KDM4A SNP-A482 was significantly associated with altered drug response to 20 compounds; seventeen compounds were associated with an increased drug sensitivity and three compounds were associated with an increased drug resistance (Figs. 17A-17B). These compounds were then classified based on reported literature and known targets (Fig. 17B). The most striking enrichment was observed for mTOR inhibitors. Indeed, lung cancer cells homozygous for KDM4A SNP-A482 had increased sensitivity to five different mTOR/PI3K inhibitors (p=0.002; Figs. 17A, 17B). Importantly, the majority of the compounds that resulted in increased sensitivity in homozygous KDM4A SNP-A482 cell lines were related to the mTOR/PI3K pathway (10 out of 17, Fig. 20A), emphasizing an important and unappreciated link between KDM4A and this pathway.

In order to further establish the relationship between KDM4A SNP-A482 and mTOR inhibition, the relationship between drug sensitivity and other SNPs within KDM4A was examined (Figs. 38A-38B and 39A-39B). To do this, the lung cancer cell lines used in the initial screen were genotyped for two additional KDM4A SNPs. rs517191, which is not linked to outcome in the NSCLC patients, and rs6429632, which is linked to overall poor outcome in homozygous late stage patients and for which the outcome associations were different than KDM4A SNP-A482 were studied (Fig. 38A-38B and 39A). Interestingly, only cells homozygous for KDM4A SNP-A482 and not rs517191 or rs6429632 exhibited increased sensitivity to the majority of mTOR inhibitors compared to the cells heterozygous and homozygous for the major alleles (Fig. 39B and data not shown). These data suggest that KDM4A SNP-A482 status might constitute a biomarker of drug response to specific chemotherapeutic compounds such as mTOR and PI3K inhibitors, which has important clinical implications since these drugs are being used to treat NSCLC (Cagle et al., 2013; Gridelli et al., 2008).

Because SNP-A482 affected KDM4A stability and altered cellular drug sensitivity, it was assessed whether additional biological processes regulated by KDM4A were also affected by SNP-A482. Since KDM4A is a histone demethylase (Black et al., 2012), it was determined whether SNP-A482 affects KDM4A catalytic activity *in vivo.* Immunofluorescence experiments in U2OS cells transfected with GFP-KDM4A revealed that H3K36me3 and H3K9me3 catalytic activities did not appear to be altered between KDM4A WT and SNP-A482 (data not shown). KDM4A overexpression leads to a faster progression through S phase (Black et al., 2010) and results in site-specific copy gain (1q12h) (Black et al., 2013). Consistent with these previous reports, stable cells overexpressing KDM4A SNP-A482 presented the same cell cycle progression profile as KDM4A WT cells (Fig. 40A) and generated site-specific copy gains like KDM4A WT (Fig. 40B). Taken together, these results demonstrate that the presence of SNP-A482 does not affect known KDM4A-related functions but has an important role in generating increased sensitivity to mTOR/PI3K inhibitors.

**mTOR inhibition reduces KDM4A protein levels.** Because there was enrichment for mTOR inhibitor sensitivity in relation to KDM4A SNP-A482, the biochemical relationship between KDM4A SNP-A482 and this class of compounds was further evaluated. Based on the observation that KDM4A SNP-A482 increases KDM4A ubiquitination and turnover (Figs. 15B-15D), the impact that mTOR inhibition had on endogenous KDM4A levels was determined (Fig. 20E and Figs 34A-34B). Consistent with mTOR inhibition affecting mRNA translation (Bjornsti and Houghton, 2004; Populo et al., 2012), endogenous KDM4A protein levels were reduced during Rapamycin treatment (Fig. 20E and Fig. 34A), whereas RNA levels were unchanged (Fig. 34B). Since KDM4A SNP-A482 has a faster half-life compare to KDM4A WT (Fig. 15C), it was hypothesized that KDM4A SNP-A482 protein levels would be lower than KDM4A WT protein levels in the presence of mTOR inhibitors. To test this hypothesis, two cell lines were treated with different KDM4A genotypes (*i.e.,* homozygote KDM4A WT or SNP-A482) with Rapamycin and endogenous KDM4A levels analyzed over time (Fig. 34C). Indeed, H290 cells, which are homozygous for KDM4A SNP-A482, exhibited reduced endogenous KDM4A protein levels upon mTOR inhibition, whereas LU99B cells, which are homozygous for KDM4A WT, did not exhibit this phenotype (Fig. 34C, compare lines; p=0.034, significance for overall difference). In order to rule out the contribution of different genetic backgrounds in these cancer cell lines, the same experiments were performed within a single cell line that transiently overexpressed GFP-KDM4A WT or GFP-KDM4A SNP-A482. The same phenotype is observed in these cells (Fig. 34D, compare lines; p=0.003, significance for overall difference). Taken together, these data indicate that mTOR inhibition leads to a more pronounced decrease in KDM4A SNP-A482 protein levels.

**KDM4A protein levels alter cell sensitivity to rapalogs.** Based on the above observations, it was hypothesized that the increased mTOR inhibitor sensitivity observed for KDM4A SNP-A482 cell lines was due to the stronger reduction in KDM4A levels. To test this hypothesis, KDM4A was depleted from cells using shRNAs, and cell proliferation was monitored during Rapamycin treatment. Indeed, shRNAs directed against KDM4A further extended the already increased doubling time observed after Rapamycin treatment (Figs. 35A and 20D). In order to confirm this result, cells were depleted of KDM4A before being treated with Rapamycin or the ATP-competitive mTOR inhibitor AZD8055 for 48h before cell proliferation and viability was assessed using MTT assays. Independent experiments with two different shRNAs resulted in increased sensitivity to mTOR inhibition upon KDM4A knock down (Figs. 35B and 41A-41B). These data indicate that KDM4A levels play an important role in sensitizing cells to mTOR inhibitors. They also emphasize the impact that KDM4A SNP-A482 or KDM4A inhibition can have on the response to chemotherapy.

In order to determine if the inhibition of KDM4A alters cell sensitivity to mTOR inhibitors, HEK 293T cells were co-treated for 48h with a KDM4A inhibitor, JIB-04 (Wang et al., 2013), and Rapamycin or AZD8055 before assessing proliferation and doubling time as described above (Figs. 35C, 21B, and 41D). JIB-04 treatment enhanced the effect of both Rapamycin and AZD8055 (Figs. 35C, and 41C-41D), demonstrating that KDM4A activity can be disrupted or decreased to sensitize cells to mTOR inhibition.

**KDM4A protein levels impact overall translation.** A major mTOR function is to modulate protein translation (Bjornsti and Houghton, 2004; Populo et al., 2012). Therefore, it was hypothesized that decreased KDM4A protein levels increase the cellular toxicity of mTOR inhibitors by also impacting protein translation. To test this hypothesis, KDM4A was depleted with two different shRNAs and overall translation assessed over two hours by measuring the incorporation of the methionine analog AHA (L-Azidohomoalanine) (Figs. 35D and 21B). Interestingly, the depletion of KDM4A reduced overall translation (Figs. 35D and 21B). It was next asked whether KDM4A depletion could enhance the reduced translation observed upon mTOR inhibition with the same assay. Upon KDM4A knock-down and treatment with three separate doses of Rapamycin, a further decrease of translation was observed in cells treated with all doses of Rapamycin (Figs. 35E and 41E-41F). Interestingly, the decreased translation observed upon KDM4A knock-down alone is equivalent to the effect of low doses of Rapamycin for which no significant effect on cell proliferation was observed (Fig. 35E, compare the ratio at the bottom to the effect on proliferation observed in Figs. 35B and 41B; the biotin/**-actin ratio reported in Fig. 35E represent the average of two independent experiments).

In order to determine if the inhibition of KDM4A alters overall translation, HEK 293T cells were treated for 24h with JIB-04 and Rapamycin before assessing methionine incorporation as described above (Figs. 41G-41H). JIB-04 treatment enhanced the alteration of translation observed after Rapamycin treatment (Figs. 41G-41H).

**KDM4A is implicated in the initiation of translation.** In order to determine if KDM4A could have a direct role in translation, endogenous KDM4A was immunoprecipitated from HEK 293T cells and the binding partners analyzed by mass spectrometry (Fig. 35F). The IPA analysis of the data resulted in the "translation" category being highly significantly represented (Fig. 35F). Some of these interactions have been confirmed by endogenous coimmunoprecipitation. Interestingly, an antibody directed against the Tudor domains does not pull down any of the translation factors (Fig. 35G). Because most of the translation factors identified by mass spectrometry are involved in the initiation of translation, fractions were purified from polysome profiles in order to determine the potential presence of KDM4A (Fig. 35H). Interestingly, KDM4A was present in the 40S fraction, confirming a potential role in the initiation of translation. In order to confirm a potential translation initiation defect upon KDM4A inhibition the polysome profiles from HEK 293T cells treated with JIB-04 were studied (Fig. 35I). Interestingly, JIB-04 leads to a translation initiation defect (Fig. 35I). Additionally, JIB-04 can sensitize cells to Rapamycin by increasing their translation initiation defect (Fig. 35J). JIB-04 can target KDM4 members as well as KDM5A and KDM6B. In order to determine if these other targets can be involved in the decreased translation observed upon JIB-04 treatment, KDM4A, KDM5A and KDM6B were depleted from HEK 293T cells with siRNAs and overall translation examined. Interestingly, KDM5A and KDM6B knock-down affects translation to the same extent than KDM4A knock-down (Fig. 35K). This data supports the role of KDM4A in modulating translation and the observation of KDM5A and KDM6B depletion causing a protein synthesis effect indicates that they are involved and targeting them in conjunction with or separately from KDM4 can provide a clinical advantage. This possibility is highlighted by the affects observed with JIB-04 since this compound hits all three to different degrees and gives an enhanced affect. Finally, in order to determine if the absence of KDM4A could alter the stability of factors directly involved in initiation of translation, HEK 293T lysates were analyzed upon KDM4A knock-down and no obvious variation in the levels of the proteins analyzed ws observed (Figs. 41I-41J). Without wishing to be bound by theory, these data indicate that the enhanced mTOR inhibitor sensitivity that was observed with KDM4A SNP-A482, chemical inhibition of KDM4A, or KDM4A knock-down was the result of an enhanced change in overall translation, which could be due to defect in the initiation of translation.

### DISCUSSION

Personalized therapy is becoming more common in cancer treatment (Cagle et al., 2013). The ultimate goal of this approach is to target an Achilles' heel based on the genetic/biochemical properties of a target in certain cancer types and/or individuals. In order to achieve this goal, genetic and biochemical properties need to be established for genetic alterations linked to diseases. For example, SNP status can be used to assess risk of disease or predict outcome and/or response to treatment and may also have a place in the sub-classification of patients for optimal treatment delivery (Lee et al., 2013; Zeron-Medina et al., 2013). In the present study, the biochemical and biological effects of a coding SNP within the H3K9/36 histone tri-demethylase KDM4A were assessed. It was demonstrated that KDM4A SNP-A482 is more ubiquitinated and exhibits a faster turnover rate than the protein encoded by the major allele (E482, referred to as WT). Moreover, stratified late stage NSCLC patients homozygous for KDM4A SNP-A482 present a worse outcome than patients heterozygous or homozygous for KDM4A WT. Interestingly, it was discovered that cancer cells homozygous for the minor allele are more sensitive to specific drugs, including compounds targeting the mTOR pathway. It was further demonstrated that the sensitization to such drugs is actually the result of decreased KDM4A protein levels and enhanced translation suppression. Lastly, it was established that the combined chemical inhibition of KDM4A and mTOR results in a stronger suppression of cell proliferation, highlighting the potential for targeting these two enzymes to better treat cancers such as NSCLC (Fig. 36).

**KDM4A SNP-A482 as a biomarker.** This is the first report that identifies a coding SNP within a chromatin modifier with links to NSCLC outcome and drug response. These data are particularly important given that 85% of lung cancers are NSCLC, with 70% presenting as advanced disease, *i.e.* locally advanced IIIB or metastatic disease IV, and are not considered curable. The five year survival rates for these advanced NSCLC stages are 7% and 2%, respectively. Testing for multiple biomarkers in order to apply more targeted therapies is already considered to be the standard of care for lung cancer (Cagle et al., 2013). Therefore, the present study provides another option to stratify and treat sub-groups of NSCLC patients.

Because KDM4A can be depleted or non-lung cancer cell lines treated with a KDM4A inhibitor and increased sensitivity to mTOR inhibitors observed, this genetic or chemical based targeting strategy can be applied to other cancers. For example, mTOR inhibitor Temsirolimus (CCI-779) is used for advanced refractory renal cell carcinoma (RCC) (Atkins et al., 2004). A phase II clinical trial presented an objective response rate for only 7% of RCC patients (Atkins et al., 2004). Interestingly, this proportion is similar to that observed for individual homozygous for the rs586339 minor allele (KDM4A SNP-A482) (Figs. 19D and 19E).

**KDM4A levels impact translation and provide a novel therapeutic strategy.** Previous reports demonstrated that the regulation of KDM4A protein levels was key to regulating S phase progression, DNA replication, transcription and DNA repair (Black et al., 2010; Mallette et al., 2012; Tan et al., 2011; Van Rechem et al., 2011). In the current study, an unexpected role for KDM4A in translation is described, which is also greatly influenced by KDM4A protein levels and regulation. Interestingly, KDM4A appears being directly implicated in translation because of its presence in the 40S fraction of the ribosome and its interaction with proteins involved in the initiation of translation.

The data presented herein indicate that increased KDM4A turnover or reduced KDM4A levels are able to reduce overall translation without changing cell proliferation. However, the importance of KDM4A in protein translation becomes enhanced with chemotherapeutics targeting this process. In fact, the enhanced effect on translation correlates with stronger cell proliferation phenotypes. Taken together, these data indicate that certain combination of chemotherapies (*e.g*., KDM4A inhibition plus mTOR inhibition) permit strategies to treat various cancer types. In addition, a genetic feature in KDM4A (SNP-A482) is described that permits patient stratification and more effective treatment options for these individuals. Since the *kdm4a* allele is lost in certain tumors (Black et al., 2013), these tumors can also be prime candidates for mTOR-related chemotherapies in the future.

The data presented in the current study indicate that KDM4A SNP-A482 status or KDM4A levels of expression should be considered in patients receiving mTOR inhibitors or other compounds that could be impinging on the mTOR pathway. The identification of KDM4A SNP-A482 has highlighted the importance of KDM4A in treatments involving mTOR inhibitors and identifies a therapeutic pathway for treating cancer patients that are homozygous for KDM4A SNP-A482. Another therapeutic strategy is combined therapy using KDM4A and mTOR inhibitors. This type of approach can prevent development of resistant clones either by hitting the targeted pathway twice or by directly targeting modifiers known to be involved in the development of resistance. Indeed, KDM5A has been involved in the development of resistance to EGFR treatment in NSCLC (Sharma et al., 2010). In addition, it is described herein that the inhibition of KDM4A sensitizes cancer cells to mTOR inhibition; therefore, KDM4 inhibitors could serve as a tool to sensitize cancer cells to certain chemotherapies. For example, KDM4A inhibition can be coupled to genotoxic therapies affecting S phase since KDM4A depletion increased sensitivity to replication stress drugs from worm to man (Black et al., 2010; Black et al., 2013). This study presents strategies for personalized targeted therapy in cancer, particularly NSCLC, based on KDM4A SNP-A482 status and/or KDM4A protein levels.

### EXPERIMENTAL PROCEDURES

**Cell culture and drug treatments.** For tissue culture and generation of stable cell lines see (Black et al., 2010). Rapamycin (LC Laboratories) and AZD8055 (Selleckchem) were used at indicated concentrations. Cycloheximide and MG132 were used as described in (Van Rechem et al., 2011). JIB-04 (Xcessbio) was used at a final concentration of 250nM. For the translation assays, DMEM depleted of Methionine and Cysteine (Life Technologies 21013-024) was used.

**Plasmids and transfections.** Plasmid transfections were done using X-tremeGENE 9 DNA transfection reagent (Roche) on 6x10⁵ HEK 293T cells plated in 10 cm dishes 20h prior to transfection. The complexes were incubated with the cells in OptiMEM for 4h before being replaced by fresh media. Cells were harvested 48 to 72h after transfection. The transfected plasmids were: pMSCV-GFP (Black et al., 2010), pMSCV-GFP-KDM4A (Black et al., 2010), pMSCV-GFP-KDM4A-E482A, pcDNA3-3xMYC-Cullin1 (Van Rechem et al., 2011), pSUPER (Black et al., 2010), pSUPER-4C (referred as 4A.2 throughout the Figs.) (Black et al., 2010), pLKO, pLKO-A06 (referred as 4A.6 throughout the figures), pLKO-A10 (referred as 4A.10 throughout the figures).

**Western blot analysis.** Western blot analyses were performed according to (Black et al., 2010).

**Antibodies.** The antibodies used were Actinin (Santa Cruz, sc-17829), Streptavidin-HRP (Cell Signaling 3999S). Ubiquitin, KDM4A, Cullin 1 and β-Actin antibodies were described in (Van Rechem et al., 2011).

**HapMap frequencies.** The HapMap frequencies are from the HapMap Public Release #28 (available on the world wide web at hapmap.ncbi.nlm.nih.gov/cgi-perl/snp_details_phase3?name=rs586339&source=hapmap28_B36&tmpl=snp_details_phase3)(2005; Altshuler et al., 2010).

Coimmunoprecipitation. The coimmunoprecipitations experiments were performed as described in (Van Rechem et al., 2011).

**Subcellular localization and catalytic activity of KDM4A WT and SNP-A482.** All immunofluorescence experiments were performed as in (Black et al., 2013). For the localization experiment a minimum of 50 cells were blinded and scored. For the catalytic activity, cells overexpressing a high level of KDM4A WT or SNP-A482 were analyzed in a non quantitative manner.

**Flow Cytometry.** Cell synchronization and FACS experiments were performed as described in (Black et al., 2010).

**Fluorescent In Situ Hybridization (FISH).** FISH experiments were performed as described in (Black et al., 2013).

**Patient outcome.** For complete methods see (Heist et al., 2008). P values have been calculated based on a recessive model with unadjusted covariates.

**Drug screen.** A panel of 88 cell lines were used in a high-throughput viability screen as described before (McDermott et al., 2008).

Cells were seeded in either 96-well at ∼15% confluency in medium with 5% FBS and penicillin/streptavidin. The optimal cell number for each cell line was determined to ensure that each was in growth phase at the end of the assay. After overnight incubation cells were treated with three concentrations of each compound in triplicate (10-fold dilutions series) using liquid handling robotics, and then returned to the incubator for assay at a 72h time point. Cells were fixed in 4% formaldehyde for 30 minutes and then stained with 1µM of the fluorescent nucleic acid stain Syto60 (Invitrogen) for 1h. Quantitation of fluorescent signal intensity was performed using a fluorescent plate reader at excitation and emission wavelengths of 630/695nM. The mean of triplicate values for each drug concentration was compared with untreated wells, and a viability ratio was calculated.

The statistical association between drug sensitivity and KDM4A SNP-A482 status was tested using a series of Fisher exact tests designated to capture the differential drug effect across the full range of viability observed: for these tests the cell lines were designated as KDM4A SNP-A482 positive if they are homozygous for the minor alleles and compared to heterozygous and homozygous major alleles cell lines grouped together. For each drug and concentration used the cell lines were ranked ordered from most to least sensitive and a contingency table was built by designating the top 5% sensitive cell lines as "sensitive" and the rest as "resistant". The result of the Fisher exact test for this threshold of sensitivity was recorded and the procedure was repeated using as a sensitivity threshold the 10^{th} percentile to the 75^{th} percentile by five percentiles increment. The minimum p value for a given drug across the three concentrations tested is reported. The drug effect is the difference between the mean viability of KDM4A SNP-A482 homozygous cell lines and the mean viability of the other cell lines tested at the concentration matching the reported Fisher exact test p value. The statistical significance of the enrichment for mTOR inhibitors in the group of drugs associated with the presence of the minor allele KDM4A SNP-A482 was tested using a Fisher exact test with five mTOR inhibitors and 15 other drugs statistically linked to KDM4A SNP-A482 status versus one mTOR inhibitor and 66 other drugs not statistically linked to KDM4A SNP-A482 status.

**Monitored cell proliferation assay.** Seventy two hours post transfection 1x10⁴ HEK 293T cells were seeded per well of a 96 well plate, and then treated after 24h. Cell proliferation was monitored with an xCELLigence system (Roche) (Vistejnova et al., 2009).

**MTT assays.** MTT assays were performed following supplier's instructions from the Cell Proliferation Kit I™ (MTT) from Roche. Briefly, 1x10⁴ cells were seeded per well of a 96 well plate and grown for 24h before treatment. Forty-eight hours later, cells were assayed.

**Translation assays.** Translation assays were performed following supplier's instructions from Click-IT™ Metabolic Labeling Reagents for Proteins (Life Technologies). Cells were incubated in presence of DMEM without Cysteine and Methionine for 1h, then grown in presence of 50µM AHA (L-azidohomoalanine, Life Technologies C10102) for 2h, harvested and washed extensively with PBS. Cells were lysed in 1% SDS in 50mM Tris pH8 with 10% Glycerol and sonicated using a bath sonicator (QSonica™ Q800R) for 30min. The Click-IT reactions were performed following the supplier's instructions from Click-IT™ Protein Reaction Buffer Kit (Life Technologies). Briefly, 50 µg to 100 µg of lysates were used per reaction with 40 µM Alkyne-Biotin (Life Technologies B10185), and 10 µg were assayed by western blot using a Streptavidin antibody conjugated to HRP.

**Statistics.** All errors bars represent SEM. Half lives were calculated using a polynomial trend line (Van Rechem et al., 2011). P values were determined by a two-tailed student's t test or a two way ANOVA when annotated; * represents p<0.05. For the patient study frequency comparisons were tested using the chi-square test, and Hazard Ratios were calculated using a Cox model. For the drug screen p values were calculated with Fisher exact test.

### REFERENCES

(2005). A haplotype map of the human genome. Nature 437, 1299-1320.
Altshuler, D.M., Gibbs, R.A., Peltonen, L., Dermitzakis, E., Schaffner, S.F., Yu, F., Bonnen, P.E., de Bakker, P.I., Deloukas, P., Gabriel, S.B., et al. (2010). Integrating common and rare genetic variation in diverse human populations. Nature 467, 52-58.
Atkins, M.B., Hidalgo, M., Stadler, W.M., Logan, T.F., Dutcher, J.P., Hudes, G.R., Park, Y., Liou, S.H., Marshall, B., Boni, J.P., et al. (2004). Randomized phase II study of multiple dose levels of CCI-779, a novel mammalian target of rapamycin kinase inhibitor, in patients with advanced refractory renal cell carcinoma. J Clin Oncol 22, 909-918.
Barreiro, L.B., Laval, G., Quach, H., Patin, E., and Quintana-Murci, L. (2008). Natural selection has driven population differentiation in modern humans. Nat Genet 40, 340-345.
Bjornsti, M.A., and Houghton, P.J. (2004). The TOR pathway: a target for cancer therapy. Nat Rev Cancer 4, 335-348.
Black, J.C., Allen, A., Van Rechem, C., Forbes, E., Longworth, M., Tschop, K., Rinehart, C., Quiton, J., Walsh, R., Smallwood, A., et al. (2010). Conserved antagonism between JMJD2A/KDM4A and HP1gamma during cell cycle progression. Mol Cell 40, 736-748.
Black, J.C., Manning, A.L., Van Rechem, C., Kim, J., Ladd, B., Cho, J., Pineda, C.M., Murphy, N., Daniels, D.L., Montagna, C., et al. (2013). KDM4A lysine demethylase induces site-specific copy gain and rereplication of regions amplified in tumors. Cell 154, 541-555.
Black, J.C., Van Rechem, C., and Whetstine, J.R. (2012). Histone lysine methylation dynamics: establishment, regulation, and biological impact. Mol Cell 48, 491-507.
Black, J.C., and Whetstine, J.R. (2011). Chromatin landscape: methylation beyond transcription. Epigenetics 6, 9-15.
Brookes, A.J. (1999). The essence of SNPs. Gene 234, 177-186.
Butler, L.R., Densham, R.M., Jia, J., Garvin, A.J., Stone, H.R., Shah, V., Weekes, D., Festy, F., Beesley, J., and Morris, J.R. (2012). The proteasomal de-ubiquitinating enzyme POH1 promotes the double-strand DNA break response. Embo J 31, 3918-3934.
Cagle, P.T., Allen, T.C., and Olsen, R.J. (2013). Lung cancer biomarkers: present status and future developments. Arch Pathol Lab Med 137, 1191-1198.
Cargill, M., Altshuler, D., Ireland, J., Sklar, P., Ardlie, K., Patil, N., Shaw, N., Lane, C.R., Lim, E.P., Kalyanaraman, N., et al. (1999). Characterization of single-nucleotide polymorphisms in coding regions of human genes. Nat Genet 22, 231-238.
Dawson, M.A., and Kouzarides, T. (2012). Cancer epigenetics: from mechanism to therapy. Cell 150, 12-27. Fujimoto, A., Totoki, Y., Abe, T., Boroevich, K.A., Hosoda, F., Nguyen, H.H., Aoki, M., Hosono, N., Kubo, M., Miya, F., et al. (2012). Whole-genome sequencing of liver cancers identifies etiological influences on mutation patterns and recurrent mutations in chromatin regulators. Nat Genet 44, 760-764.
Gridelli, C., Maione, P., and Rossi, A. (2008). The potential role of mTOR inhibitors in non-small cell lung cancer. Oncologist 13, 139-147.
Halushka, M.K., Fan, J.B., Bentley, K., Hsie, L., Shen, N., Weder, A., Cooper, R., Lipshutz, R., and Chakravarti, A. (1999). Patterns of single-nucleotide polymorphisms in candidate genes for blood-pressure homeostasis. Nat Genet 22, 239-247.
Hanahan, D., and Weinberg, R.A. (2011). Hallmarks of cancer: the next generation. Cell 144, 646-674.
Heist, R.S., Zhai, R., Liu, G., Zhou, W., Lin, X., Su, L., Asomaning, K., Lynch, T.J., Wain, J.C., and Christiani, D.C. (2008). VEGF polymorphisms and survival in early-stage non-small-cell lung cancer. J Clin Oncol 26, 856-862.
Huang, Y.T., Heist, R.S., Chirieac, L.R., Lin, X., Skaug, V., Zienolddiny, S., Haugen, A., Wu, M.C., Wang, Z., Su, L., et al. (2009). Genome-wide analysis of survival in early-stage non-small-cell lung cancer. J Clin Oncol 27, 2660-2667.
Huang, Y.T., Lin, X., Chirieac, L.R., McGovern, R., Wain, J.C., Heist, R.S., Skaug, V., Zienolddiny, S., Haugen, A., Su, L., et al. (2011a). Impact on disease development, genomic location and biological function of copy number alterations in non-small cell lung cancer. PLoS One 6, e22961.
Huang, Y.T., Lin, X., Liu, Y., Chirieac, L.R., McGovern, R., Wain, J., Heist, R., Skaug, V., Zienolddiny, S., Haugen, A., et al. (2011b). Cigarette smoking increases copy number alterations in nonsmall-cell lung cancer. Proc Natl Acad Sci U S A 108, 16345-16350.
Kandoth, C., McLellan, M.D., Vandin, F., Ye, K., Niu, B., Lu, C., Xie, M., Zhang, Q., McMichael, J.F., Wyczalkowski, M.A., et al. (2013). Mutational landscape and significance across 12 major cancer types. Nature 502, 333-339.
Kogure, M., Takawa, M., Cho, H.S., Toyokawa, G., Hayashi, K., Tsunoda, T., Kobayashi, T., Daigo, Y., Sugiyama, M., Atomi, Y., et al. (2013). Deregulation of the histone demethylase JMJD2A is involved in human carcinogenesis through regulation of the G(1)/S transition. Cancer Lett 336, 76-84.
Lee, J.C., Espeli, M., Anderson, C.A., Linterman, M.A., Pocock, J.M., Williams, N.J., Roberts, R., Viatte, S., Fu, B., Peshu, N., et al. (2013). Human SNP links differential outcomes in inflammatory and infectious disease to a FOXO3-regulated pathway. Cell 155, 57-69.
Liu, J., Lee, W., Jiang, Z., Chen, Z., Jhunjhunwala, S., Haverty, P.M., Gnad, F., Guan, Y., Gilbert, H.N., Stinson, J., et al. (2012). Genome and transcriptome sequencing of lung cancers reveal diverse mutational and splicing events. Genome Res 22, 2315-2327.
Mallette, F.A., Mattiroli, F., Cui, G., Young, L.C., Hendzel, M.J., Mer, G., Sixma, T.K., and Richard, S. (2012). RNF8- and RNF168-dependent degradation of KDM4A/JMJD2A triggers 53BP1 recruitment to DNA damage sites. Embo J 31, 1865-1878.
Mallette, F.A., and Richard, S. (2012). JMJD2A promotes cellular transformation by blocking cellular senescence through transcriptional repression of the tumor suppressor CHD5. Cell Rep 2, 1233-1243.
McDermott, U., Sharma, S.V., and Settleman, J. (2008). High-throughput lung cancer cell line screening for genotype-correlated sensitivity to an EGFR kinase inhibitor. Methods Enzymol 438, 331-341.
Pasqualucci, L., Trifonov, V., Fabbri, G., Ma, J., Rossi, D., Chiarenza, A., Wells, V.A., Grunn, A., Messina, M., Elliot, O., et al. (2011). Analysis of the coding genome of diffuse large B-cell lymphoma. Nat Genet 43, 830-837.
Populo, H., Lopes, J.M., and Soares, P. (2012). The mTOR Signalling Pathway in Human Cancer. Int J Mol Sci 13, 1886-1918.
Schork, N.J., Murray, S.S., Frazer, K.A., and Topol, E.J. (2009). Common vs. rare allele hypotheses for complex diseases. Curr Opin Genet Dev 19, 212-219.
Sharma, S.V., Lee, D.Y., Li, B., Quinlan, M.P., Takahashi, F., Maheswaran, S., McDermott, U., Azizian, N., Zou, L., Fischbach, M.A., et al. (2010). A chromatin-mediated reversible drug-tolerant state in cancer cell subpopulations. Cell 141, 69-80.
Tan, M.K., Lim, H.J., and Harper, J.W. (2011). SCFFBXO22 Regulates Histone H3 Lysine 9 and 36 Methylation Levels by Targeting Histone Demethylase KDM4A for Ubiquitin-Mediated Proteasomal Degradation. Mol Cell Biol 31, 3687-3699.
Van Rechem, C., Black, J.C., Abbas, T., Allen, A., Rinehart, C.A., Yuan, G.C., Dutta, A., and Whetstine, J.R. (2011). The SKP1-Cull-F-box and Leucine-rich Repeat Protein 4 (SCF-FbxL4) Ubiquitin Ligase Regulates Lysine Demethylase 4A (KDM4A)/Jumonji Domain-containing 2A (JMJD2A) Protein. J Biol Chem 286, 30462-30470.
Vistejnova, L., Dvorakova, J., Hasova, M., Muthny, T., Velebny, V., Soucek, K., and Kubala, L. (2009). The comparison of impedance-based method of cell proliferation monitoring with commonly used metabolic-based techniques. Neuro Endocrinol Lett 30 Suppl 1, 121-127.
Wang, L., Chang, J., Varghese, D., Dellinger, M., Kumar, S., Best, A.M., Ruiz, J., Bruick, R., Pena-Llopis, S., Xu, J., et al. (2013). A small molecule modulates Jumonji histone demethylase activity and selectively inhibits cancer growth. Nat Commun 4, 2035.
Wang, Z., and Moult, J. (2001). SNPs, protein structure, and disease. Hum Mutat 17, 263-270.
Zeron-Medina, J., Wang, X., Repapi, E., Campbell, M.R., Su, D., Castro-Giner, F., Davies, B., Peterse, E.F., Sacilotto, N., Walker, G.J., et al. (2013). A polymorphic p53 response element in KIT ligand influences cancer risk and has undergone natural selection. Cell 155, 410-422.

### EXAMPLE 7: A Conserved Hypoxic Response Generates Site-specific Copy Gains of Chromosomal Regions

Somatic copy number alterations are a hallmark of cancer. There is emerging evidence that copy number heterogeneity is a feature within tumors. The molecular bases for this heterogeneity are not fully understood. It is demonstrated herein that hypoxia induces transient site-specific copy gains in primary, non-transformed and transformed human cell lines. This response is conserved at a syntenic region in zebrafish cells. Regions with site-specific copy-gain are enriched in primary tumors with a hypoxic gene signature. Hypoxia-driven copy gains are dependent upon the KDM4A histone demethylase, which is stabilized under hypoxic conditions. Hypoxia-dependent copy gains are blocked by overexpression of chromatin modulators Suv39h1 or HP1γ, by inhibition of KDM4A with a small molecule or a natural catabolite succinate. The results described herein elucidate a mechanism for generating copy number heterogeneity in tumors and establish this as a biological response that can be pharmacologically regulated.

It is demonstrated herein that:
Hypoxia promotes site-specific copy gains in human primary and cancer cells
Hypoxia induces conserved site-specific copy gain in a syntenic region in zebrafish
Hypoxia generates site-specific copy gains through stabilization of KDM4A
Chemical inhibition of KDM4A abrogates hypoxia-dependent site-specific gains

Copy gains or losses of chromosome arms and/or whole chromosomes, as well as amplifications of smaller genomic fragments are frequently observed in cancer (Beroukhim et al., 2010; Hook et al., 2007; Stratton et al., 2009). These genomic events are also observed in other diseases such as autism and schizophrenia (Brunetti-Pierri et al., 2008; Levinson et al., 2011; Stefansson et al., 2008). Interestingly, genome wide analyses of copy number changes in cancer and neurological diseases have identified chromosomal regions with higher frequencies of gains and/or amplification (Beroukhim et al., 2010). The consistent observation of specific genomic regions suggests that there are underlying driver events that have yet to be fully appreciated.

Next generation sequencing has provided evidence for heterogeneity both within and between primary tumors. While it has long been understood that tumors within the same pathological subtype have different mutational and copy number profiles (Burrell et al., 2013), it has recently become appreciated that intra-tumoral heterogeneity likely plays an important role in tumor development, metastatic potential and acquired drug resistance (Burrell et al., 2013; Gerlinger et al., 2012; Junttila and de Sauvage, 2013; Nathanson et al., 2014). Traditionally, copy number variations (CNV) have been thought of as heritable genetic events that emerge through an adaptive advantage, however, recent work suggests that at least some copy gains may be transient and could arise given the correct genetic, therapeutic or environmental conditions (Black et al., 2013; Nathanson et al., 2014). Understanding how and where copy gains arise is a key question for understanding tumor development, treatment and efficacy of personalized medicine.

In the case of cancer, copy gained regions often contain oncogenes or pro-survival genes that are thought to impact cellular behavior (e.g., cMyc and Mcl1). These copy gained regions often contain other genes that lack clear connections to tumorigenesis; however, this lack of connection does not preclude the gene's involvement in cellular responses to stresses (e.g., environmental and chemotherapeutic). In fact, copy number alterations have been observed from bacteria to man. For example, in the case of cancer cell survival, the amplification of dihydrofolate reductase (DHFR) emerges when cells are treated with methotrexate (MTX) (Schimke, 1984); while in the case of antibiotic selection, bacteria have the ability to amplify regions for drug resistance and survival (Slager et al., 2014). Since these mechanisms of adaptive copy number exist, there are likely biological triggers and specific enzymes that are responsible for facilitating such events at distinct regions in the genome. However, their identify remains elusive. It is important to note that the acquisition of regions does not necessarily mean that the genes within the regions are contributing to or causing a response. Regardless of the consequence of copy gains, there is little knowledge about the regulatory mechanisms or factors that are involved in promoting copy number alterations at specific regions of the genome.

The mis-regulation of DNA replication is a major contributing factor to copy gain and DNA amplification (Hastings et al., 2009; Hook et al., 2007). Chromatin structure impacts replication initiation and elongation efficiency as well as DNA damage response and repair (Alabert and Groth, 2012; Papamichos-Chronakis and Peterson, 2013). Therefore, the chromatin state or modifying enzyme(s) could have a significant impact on mechanisms promoting CNV, especially copy gain. Consistent with this possibility Kiang and colleagues demonstrated in yeast that chromatin context or chromosome microenvironments play a major role in local DNA fragment amplification during S phase (Kiang et al., 2010). Most recently, the first enzyme capable of generating site-specific copy gains was described. The histone 3 lysine 9/36 (H3K9/36) tridemethylase KDM4A was shown to generate site-specific copy gain in the human genome through modulation of heterochromatin (Black et al., 2013). KDM4A was amplified in a subset of human tumors and was co-amplified with regions frequently observed in cancer and diseases such as autism and schizophrenia. KDM4A was amplified in a subset of human tumors and was co-amplified with regions frequently observed in cancer. Overexpression of KDM4A in human cells promoted rereplication and copy gain of the same regions co-amplified with KDM4A in tumors (Black et al., 2013). KDM4A-dependent copy gains were generated in a catalytically-dependent manner upon transient or constant exposure to KDM4A, which raises the possibility that transient mis-expression or stabilization of KDM4A could impact cellular copy number. This work established that copy gains are in fact directly regulated by an enzyme and through chromatin alterations. These studies highlighted a mechanism for copy alteration and indicated that amplification of KDM4A was a pathological event that can cause copy gains in human tumors.

These initial observations highlighted a pathological state that could promote copy gains. However, a major question remained: "are there physiological signals or cues that cells encounter, and in turn, directly cause copy gains within defined regions of the genome?" It was reasoned that tumor cells encounter various stresses that could promote copy gains, which could ultimately contribute to the copy number heterogeneity observed in tumors. This same notion could also explain why these copy gained regions (e.g., 1q21) emerge in other diseases such as autism and schizophrenia. Therefore, site-specific copy gains wre systematically screened after cells were treated with a panel of cellular stresses that occur during development and tumorigenesis. Surprisingly, only one condition, hypoxia promoted site-specific copy gain of regions frequently observed in tumors. This observation occurred with physiological levels of hypoxia (1% O₂) and occurred in diverse cancer cell lines and in primary T cells in culture for only 48 hours. Most importantly, it is demonstrated herein that copy gain following hypoxia is conserved at a syntenic region in zebrafish cells, while a non-syntenic region was not gained. It is further demonstrated herein that breast and lung tumors with a defined hypoxia signature were associated with gains in the regions generated in human and zebrafish cell culture. The tumors harboring the hypoxic signature were also associated with a faster time to death in both the breast and lung cancer patients.

Since copy gained regions are associated with several drug resistant tumors, it was sought to identify the reason they were gained in response to hypoxia and establish whether these events were preventable. Unexpectedly, it is demonstrated herein that hypoxia resulted in stabilization of KDM4A, which generated site-specific copy gains in an S phase-dependent manner. It is further demonstrated that rereplication occurred at these gained regions upon hypoxia exposure. Finally, it is demonstrated that pretreatment of cells with succinate (a naturally occurring catabolite that inactivates α-ketoglutarate-dependent enzymes) as well as the use of a KDM4A chemical inhibitor blocked hypoxia induced gains. Taken together, this study demonstrates that copy gains occur under distinct cellular stresses from fish to man. These observations also highlight the dynamics associated with copy gain and suggest that enzyme levels, S phase status, cellular stresses and metabolic state could be contributing to the copy number heterogeneity observed in human tumors. These studies also establish the ability to therapeutically target copy gain, which has major implications in drug resistant tumors that present the 1q12-1q21 copy gains (Dimova et al., 2009; Diskin et al., 2009; Fonseca et al., 2006; Giulino-Roth et al., 2012; Inoue et al., 2004; Lestini et al., 2009; Vrana et al., 2002).

### RESULTS

**Hypoxia, but not other cellular stresses promotes site-specific copy gain.** It was sought to identify cellular stress conditions that might promote site-specific copy gain. The screen was performed in the nearly diploid, immortalized, but non-transformed RPE cell line (Black et al., 2013; Jiang et al., 1999). Deveral environmental conditions that cells may be exposed to during development and tumorigenesis were screened, including: reactive oxygen species (ROS, H₂O₂), ER stress (Tunicamycin, TU), temperature stress (heat shock, 43 °C), metabolic stress (low serum, 0.1% FBS; No glucose), hypoxia (1% O₂), and radiation stress (2Gy gamma irradiation) (Fig. 42A). Cells were exposed to the indicated stresses (see Experimental Procedures) and assayed for copy gain by fluorescent in situ hybridization (FISH) after 24 hours (Figs. 42B-42H). The screen was conducted using regions previously determined to undergo site-specific copy gain (1q12h and 1q21.2) as well as regions that did not previously exhibit copy gain (1q23.3 and chromosome 8 centromere; 8C). In order for a condition to score as positive for site-specific copy gain, it was required that both 1q12h and 1q21.2 exhibit copy gain, while 1q23.3 and 8C did not. Gamma irradiation, known to induce chromosome instability (Holmberg et al., 1998; Little, 1998), promoted gain of all regions tested on chromosome 1 and served as a positive control for the ability to detect copy gains. Therefore, gamma radiation did not meet the criteria for inducing site-specific gains. ROS, heat shock, the metabolic stresses and TU did not promote site-specific copy gain (Figs. 42B-42H). However, growth in 1% O₂ for 24 hours (also referred to as hypoxia; Fig. 49A) was sufficient to promote copy gain of 1q12h, 1q21.2 and Xq13.1 without increasing copy of 1q23.3 and 8C or the 1 q telomere (Figs. 42B and 49B). Hypoxic conditions were confirmed by stabilization of HIF1α or expression of carbonic anhydrase IX (CAIX) in cell lysates prepared from the same cells as those used in the DNA FISH analyses (Fig. 49A) (Wykoff et al., 2000). Interestingly, copy gain of 1q12h and 1q21.2 were predominantly mutually exclusive (Fig. 49C), which further underscored the nature of the site-specific gains. Cell cycle analysis of stress conditions by flow cytometry demonstrated that hypoxia, ROS and heat shock stress did not significantly alter cell cycle; however, metabolic stress, TU and gamma irradiation resulted in altered cell cycle profiles (Fig. 49D-49J). These data demonstrate that hypoxia promotes low-level site-specific copy gains within 24 hours without dramatic changes in cell cycle profile.

**Hypoxia promotes site-specific copy gain in primary cells.** It was next sought to determine if hypoxia could promote site-specific copy gain in primary cells. To accomplish this goal, CD4+ T cells were isolated by fluorescence assisted cell sorting (FACS) from buffy coat and peripheral blood of healthy individuals (Fig. 43A). Following isolation, T cells were allowed to recover in normoxia for 24 hours in the presence of IL2 with or without stimulation with anti-human CD3 and CD28 antibodies. Following recovery, T cells were maintained in normoxia or transferred to hypoxia for an additional 24 hours and analyzed by FISH for site-specific copy gain. Only stimulated cells grown in 1% O₂ for 24 hours exhibited copy gain of 1q12h and 1q21.2, but not 1q23.3 or 8C (Fig. 43B). These results indicate that primary cells subjected to hypoxic conditions promote site-specific copy gain in a proliferation-dependent manner.

**Hypoxia-dependent copy gain occurs in diverse cancer cell types.** To address whether copy gain was a prevalent response to hypoxia, a diverse panel of cell lines was analyzed, including: breast cancer lines (MDA-MB 231, MDA-MB 468), a neuroblastoma line (SK-N-AS), and kidney lines (293T and UMRC2) for copy gain of 1q12h by FISH following growth in hypoxia. Growth under hypoxic conditions was sufficient to induce copy gain in a variety of cancer cell lines. In each cell line, copy gain of 1q12h was observed under hypoxic conditions, but not 8c. Furthermore, UMRC2 cells, which lack VHL [lost by deletion; (Gameiro et al., 2013)], have stabilized HIF1α and CAIX expression under normoxic conditions and were unable to generate copy gain without hypoxia. These data indicate that HIF1α stabilization is not sufficient to promote site-specific copy gain.

**Hypoxia-dependent copy gain is evolutionarily conserved.** The ability of RPE cells and primary CD4+ T cells to respond to hypoxia with site-specific copy gain led to the hypothesis that copy gains could be an evolutionarily conserved response to hypoxia. To test this hypothesis, the zebrafish cell line AB.9 (Paw and Zon, 1999) was utilized. Analysis of genome sequencing data indicated that the 1q21.2 region analyzed in human cells (FISH probe encompasses entire BCL9 gene, bar top of schematic) is syntenic with the zebrafish BCL9 gene (Fig. 43C). A FISH probe was generated using a BAC to the syntenic region on zebrafish chromosome 1 (bar at bottom of schematic) and this was used to analyze zebrafish cells grown in 1% O₂ for copy gain (Fig. 43D). The syntenic region of zebrafish chromosome 1 to BCL9 (1q21.2) was gained in AB.9 cells exposed to hypoxia. A second homologous, but non-syntenic region to the human IGBP1 gene (Fig. 43E; region covered by the Xq13.1 probe in human cells, bar top of schematic) was also analyzed by FISH and was not copy gained in response to growth in hypoxia (Fig. 43F).

**Hypoxia-dependent copy gain occurs in diverse cancer cell types.** Growth under hypoxic conditions was also sufficient to induce copy gain in a variety of cancer cell lines. Breast cancer lines (MDA-MB 231, MDA-MB 468), neuroblastoma (SK-N-AS), and kidney lines (293T and UMRC2) were analyzed for copy gain of 1q12h by FISH following growth in hypoxia (Fig. 50A-50H). In each cell line, copy gain of 1q12h was observed under hypoxic conditions, but not 8C. Furthermore, UMRC2 cells, which lack VHL (lost by deletion; (Gameiro et al., 2013)), have stabilized HIF1α and CAIX expression under normoxic conditions and were unable to generate copy gain without hypoxia. These data strongly indicate that HIF1α stabilization is not sufficient to promote site-specific copy gain (Figs. 50I-50K).

**Site-specific copy gain in primary human tumors correlates with hypoxia.** The ability of both primary cells and cultured cancer lines to promote site-specific gain in response to hypoxia suggests that hypoxic conditions in primary tumors may contribute to CNV and tumor heterogeneity. To address this possibility, primary breast tumors and primary lung tumors in the TCGA data set were analyzed for CNV changes in hypoxic compared to non-hypoxic tumors. To identify hypoxic tumors, the hypoxia metagene derived by Winter and colleagues, which consists of 92 upregulated and 52 downregulated genes was utilized. The signature included the hypoxia marker CAIX (Tables 8 and 9) (Winter et al., 2007). An unbiased consensus hierarchical clustering approach was applied to stratify breast cancer (BRCA) and lung adenocarcinoma (LUAD) TCGA samples using this gene set (data not shown). This strategy yielded a stable cluster of tumors with a hypoxic gene signature (data not shown). As validation of this gene set and clustering approach, 65 out of 88 basal BRCA samples reside in the hypoxic cluster. Basal breast cancer has been previously demonstrated to be more hypoxic than other sub-classifications of breast cancer (Perou, 2010).

Previous reports have suggested that hypoxia is a negative prognostic marker in multiple tumor types (Eschmann et al., 2005; Hockel et al., 1996; Wang et al., 2014). For this reason, the association between deceased patients (time to death) with tumors that did or did not have the hypoxic signature was anlayzed. The number of deceased patients (time to death) was analyzed because the following variables presented a significant challenge in evaluating the association of hypoxia with clinical outcome: 1) there was a short follow-up time for survival in both breast and lung cancer patients (median survival time - 21.1 months in BRCA and 12.4 months in LUAD); and 2) there was a high fraction of censored samples (820 out of 920 samples in BRCA and 313 out of 430 samples in LUAD). These analyses identified a significantly higher risk in the hypoxic samples in both BRCA (Fig. 44A, P= 0.00011, one-tailed Wilcoxon rank-sum test) and LUAD (Figure 3B, P=0.0097, one-tailed Wilcoxon rank-sum test). These data are consistent with previous reports highlighting the negative prognostic association of hypoxia (Eschmann et al., 2005; Wang et al., 2014).

After validating the associations to poor outcome, it was tested whether hypoxic tumor samples were more likely to have focal CNV than non-hypoxic samples. The number of cytogenetic bands harboring focal copy gains or focal copy losses in each of the hypoxic and non-hypoxic samples was calculated (Figs. 44C-44D). A significant enrichment of focal events (gain plus loss) in hypoxia samples (P= 7.2x10⁻⁵⁰ for BRCA and P=6.7x10⁻¹⁸ for LUAD by the one-tailed Wilcoxon rank-sum test) was observed. Hypoxia samples were also enriched when considering only the number of cytobands with focal gains (Figs. 44E, 44G) or with focal losses (Figs. 44F, 44H). These results indicate that hypoxia contributes to CNV in human tumors.

It was next asked if specific cytogenetic bands are gained in hypoxic BRCA and LUAD samples. To address this question, it was determined whether focal amplifications were enriched in hypoxic versus non-hypoxic BRCA and LUAD samples. A p value was calculated (See Experimental Procedures) for enrichment of focal amplification for each of 807 cytogenetic bands as well as the mean copy number in hypoxic and non-hypoxic BRCA (Figs. 44-44G) and LUAD samples (Figs. 44H-44J). A strong enrichment of copy gain of 1p11.2 through 1q23.3 was observed in hypoxic BRCA (Fig. 44E) and LUAD (Fig. 44H) that was not present in non-hypoxic samples (Figs. 44F, 44I). This copy gain enrichment was also reflected in the mean-copy number distribution for these regions (Figs. 44E, 44H) as the hypoxic samples exhibited a higher mean copy number than non-hypoxic samples. The enriched region encompasses both 1q12h and 1q21.2 that were demonstrated are site-specifically gained in cell lines in response to growth in hypoxia (Figs. 42B, 44B, and 50A-50K). Taken together, these data highlight that hypoxic conditions are associated with worse outcome and contribute to focal CNV in tumors and that regions with hypoxia-dependent copy gain in cell culture are also focally gained in hypoxic primary tumors in two different cancer types.

### Hypoxia stabilizes KDM4A protein levels

It was recently demonstrated that disruption of H3K9 or K36 methylation promoted site-specific copy gains (Black et al., 2013). Therefore, it was assessed whether enzymes targeting the demethylation of these sites were involved. Hypoxia has been proposed to inactive the JmjC-containing demethylases; however, KDM3A (an H3K9me2 demethylase) was shown to be upregulated upon hypoxic exposure and retain enzyme activity during hypoxia (Krieg et al., 2010; Lee et al., 2013). Therefore, it was first tested whether this H3K9 lysine demethylase was able to promote site-specific copy gains. When KDM3A was overexpressed site-specific copy gains observed with hypoxia were not generated (Figs. 45A-45B; *e.g*., 1q12h). Additional reports suggested that the H3K9/36 demethylases KDM4B and KDM4C were upregulated during hypoxia; however, it was previously demonstrated that these enzymes were unable to promote gains of 1q12h as well (Black et al., 2013; Krieg et al., 2010; Lee et al., 2013). Therefore, it was tested whether the H3K9/36 KDM4A enzyme that was previously reported to promote site-specific copy gain was differentially expressed during hypoxia and whether enzymatic activity was retained during hypoxia (Black et al., 2013). As previously demonstrated, KDM4A RNA levels were not dramatically changed (Fig. 52A)(Beyer et al., 2008); however, KDM4A protein levels were increased with as little as 24 hours of exposure to hypoxia. In fact, KDM4A levels were increased in all human cancer cell lines tested (Fig. 52C-52D). An increase in KDM4A expression was also observed in primary CD4+ T cells treated with hypoxia (Fig. 45E). The induction of KDM4A protein levels does not appear to be a general response to stress as only hypoxia and TU treatment significantly increased KDM4A levels. No changes in KDM4A protein levels were consistently observed with heat shock, ROS or metabolic stress (Fig. 45F). In the case of TU, cells are enriched in the G1/S phase of cell cycle (Fig. 49H), which is the point when KDM4A levels are at their highest (Black et al., 2010). Therefore, the increase in KDM4A levels is most likely associated with the cell cycle arrest.

The observation that hypoxia drives copy gain of a syntenic region in zebrafish led to the hypothesis that since KDM4A had similar structure from fish to man (Fig. 52B) the zebrafish protein would be increased upon exposure to hypoxia. Consistent with this hypothesis, HA-tagged zebrafish KDM4A (zfKDM4A) that was expressed in RPE cells treated with hypoxia had an increase in protein levels (Fig. 45G). Similar results were obtained with exogenously expressed human KDM4A (huKDM4A; data not shown). Since these expression vectors did not contain the 5'- or 3'-untranslated regions for KDM4A, these data indicate both the zebrafish and human KDM4A are being stabilized in response to hypoxia. It was also demonstrated that overexpression of zebrafish KDM4A (Fig. 52C) was sufficient to induce site-specific copy gain in human RPE cells, which showed a conserved role for this enzyme in generating site-specific copy gains (Fig. 52D; *e*.*g*., 1q12h).

KDM4A is regulated by proteasomal degradation (Mallette et al., 2012; Tan et al., 2011; Van Rechem et al., 2011). Therefore, it was hypothesized that KDM4A levels were elevated during hypoxia because of increased protein stability. This possibility was tested by evaluating the half life of KDM4A in normoxic and hypoxic conditions. Both normoxic and hypoxic cells were treated with cycloheximide, which blocks protein synthesis, and assayed for KDM4A levels by western blotting (Figs. 45H, 45I, and 52E). In both RPE and 293T cells, the KDM4A half life increased upon hypoxic exposure. Cells under normoxic conditions had a half life of 1 hr 49 min and 1 hr 51min in RPE and 293T cells, respectively. In the case of hypoxic exposure, the KDM4A half life was extended to 4 hrs 56 min and 6 hrs 13 min in RPE and 293T cells, respectively. Taken together, these data demonstrate a conserved role for hypoxia in stabilizing KDM4A.

**KDM4A retains catalytic activity under hypoxic conditions.** Jumonji domain containing demethylases require oxygen as a molecular cofactor (Shi and Whetstine, 2007) so it is possible that they may be inactive under hypoxic conditions. This is true for certain members of this protein family, while others retain activity in hypoxia such as KDM3A (Lee et al., 2013). However, extensive analyses have yet to be done for all demethylases (Chervona and Costa, 2012). Using a standard cellular demethylase assay (Cloos et al., 2006; Klose et al., 2006; Whetstine et al., 2006), it was determined whether human and zebrafish KDM4A retained activity upon 1% O₂ exposure. Cells that were transfected with HA-tagged human KDM4A (huKDM4A) or zebrafish KDM4A (zfKDM4A) and treated with hypoxia for 24 hours and then immunostained for H3K36me3 (data not shown). It was observed that both huKDM4A and zfKDM4A demethylate H3K36me3 under normoxic conditions (data not shown; (Cloos et al., 2006; Klose et al., 2006; Whetstine et al., 2006)). Under hypoxic conditions, huKDM4A (data not shown) and zfKDM4A (data not shown)) demethylated H3K36me3. As previously reported, a reduced, but still catalytically active huKDM4A on H3K9me3 was observed under hypoxic conditions (data not shown; (Lee et al., 2013)).

**Hypoxia-dependent copy gains are transient and reversible.** Oxygen levels change during development and tumorigenesis (Dunwoodie, 2009; Vaupel, 2004); therefore, it was assessed whether site-specific copy gains are reversible upon return to normal oxygen levels. RPE cells were grown in 1% O₂ for 24, 48 and 72 hours, as well as 48 hours followed by a return to normoxia for 24 hours 9Fig. 46A). FISH analysis for 1q12h copy gain revealed an increased percentage of cells with copy gain at 24, 48, and 72 hours of growth in hypoxia (*, p<0.05); however, upon return to normoxia, the number of cells with extra copies of 1q12h returned to baseline (Fig. 46A, †, p<0.05). Importantly, KDM4A levels remained elevated throughout the time in hypoxia and returned to baseline upon return to normoxia (Fig. 46B). These data indicate that both copy gains and KDM4A protein regulation are dynamic and reversible.

In order to further evaluate the kinetics of copy gain and KDM4A regulation during hypoxia, a refined time course was performed following return to normoxia. RPE cells were grown in hypoxia for 48 hours (0 hr release) prior to a return to normoxia for the indicated times (Fig. 46C). FISH analysis demonstrates that copy gain of 1q12h persists for the first 2 hours following release from hypoxia but is lost by four hours after return to normoxia. Consistent with this observation, KDM4A levels begin returning to baseline levels within 2 hours of return to normoxia and reach a baseline following 4 hours of return to normoxia (Fig. 46D). These data demonstrate that hypoxia-dependent copy gains are transient and that they can be rapidly lost following a return to normoxia.

Hypoxia-dependent copy gains require KDM4A. The kinetic properties of hypoxia-dependent copy gain and concomitant stabilization of KDM4A suggest that KDM4A mediates site-specific copy gain in hypoxic conditions. To test this hypothesis, KDM4A was depleted in 293T cells grown in normoxia or hypoxia (Fig. 46E). KDM4A depletion in hypoxia resulted in KDM4A levels that were similar to the levels observed in normoxic conditions. This reduction in KDM4A levels was sufficient to abrogate hypoxia-dependent copy gain of 1q12h and 1q21.2 (Fig. 46F). The abrogation of hypoxia-dependent copy gain was not due to cell cycle arrest, as depletion of KDM4A did not significantly alter cell cycle profiles when compared to cells grown in hypoxia without KDM4A depletion (Figs. 53A-53B).

**Hypoxia-dependent copy gains require S phase.** In Fig. 43B, it was demonstrated that primary human CD4+ T cells require stimulation prior to undergoing hypoxia-dependent copy gain, which suggested that proliferation is required. Consistent with this observation, it was previously demonstrated that KDM4A-dependent copy gains were transiently generated during S phase (Black et al., 2013). Therefore, it was tested whether hypoxia-dependent gains exhibited similar requirements for S phase. RPE cells in either normoxia or hypoxia were synchronized into G1/S phase by treating with hydroxyurea (HU). Cells were released from HU and monitored for copy gains by FISH (Fig. 47A) and for cell cycle progression by flow cytometry (Fig. 53C). A strong difference in release from HU in normoxia or hypoxia was not observed; however, hypoxic cells were slightly slower in their progression through S phase as seen by the increased S phase population 10 hours post-release (Fig. 53C). FISH analysis of the arrested and released cells revealed that 1q12h and 1q21.2 copy gains were present in asynchronously dividing hypoxic cells, but not in G1/S arrested hypoxic cells (Fig. 47A). These data demonstrate the requirement for replication in order to generate hypoxia-dependent copy gains. Upon release from HU arrest, hypoxic cells exhibited 1q12h copy gain within two hours and continued to exhibit copy gain until eight hours following release. Interestingly, 1q21.2 began to return to baseline earlier than 1q12h (eight hours versus ten hours, respectively) (Fig. 47A). Ten hours post-HU release, during which cells were predominantly in late S to G2/M (Fig. 53C), hypoxic cells no longer exhibited 1q12h or 1q21.2 copy gains, which emphasizes an active process is involved in removing these gains during late S phase. Consistent with preceding observations, KDM4A protein levels remained elevated during S phase progression upon hypoxia treatment; while KDM4A levels decreased as cells progressed through S phase under normoxia (Fig. 47B, compare normoxic and hypoxic samples). Taken together, these data demonstrate the importance of S phase for copy gains and also highlight the association with KDM4A levels. However, these data also reiterate that cells harbor a mechanism for removing these acquired gains.

**Hypoxia promotes chromatin association of KDM4A and rereplication.** Ectopic expression of KDM4A results in increased chromatin association throughout the genome and is associated with altered BrdU incorporation (Black et al., 2013; Van Rechem et al., 2011). Therefore, it was sought to determine if hypoxic exposure would increase KDM4A expression levels, and in turn, the association of KDM4A with chromatin. Indeed, fractionation of cells grown in either normoxia or hypoxia revealed that KDM4A levels are increased in the chromatin fraction upon hypoxic exposure (Fig. 47C). KDM4A overexpressing cells had increased chromatin enrichment, less heterochromatin at regions that ultimately underwent rereplication and copy gain (Black et al., 2013). Therefore, it was assessed whether the site-specific copy gains were undergoing rereplication during hypoxia by coupling quantitative PCR with CsCl density gradient centrifugation. RPE cells grown in normoxia or hypoxia were labeled with BrdU and the replicated DNA was isolated and separated by density in the CsCl gradient (Fig. 53D). A heavy:heavy peak was not observed in normoxic or hypoxic cells, indicating that the BrdU labeling minimized the amount of rereplication caused by cells proceeding through another cell cycle. Fractions from where the heavy:heavy peak would occur were isolated, purified and assayed for DNA content by quantitative PCR. Eenrichment of regions representing 1q12h, 1q12/21 and 1q21.2, but not 1q23.3 or the β-actin locus were observed. These data support the model that hypoxia induces site-specific copy gain by promoting KDM4A association with chromatin and facilitating rereplication of specific regions.

**Hypoxia-dependent copy gains can be prevented.** It was previously demonstrated that KDM4A-dependent site-specific copy gain could be antagonized by the H3K9me3 methyltransferase SUV39h1 or the H3K9me3 binding protein HP1γ (Black et al., 2013). The fact that hypoxia-dependent copy gains correlate with changes in expression of KDM4A, are transient and require S phase for generation led us to ask whether hypoxia-dependent copy gains could be abrogated by expression of either SUV39h1 or HP1γ. Overexpression of SUV39h1 or HP1γ (Fig. 48A) did not alter either the KDM4A protein levels under hypoxic conditions or the cell cycle distribution of RPE cells (Fig. 54A). However, overexpression of either SUV39h1 or HP1γ was able to abrogate hypoxic induction of site-specific copy gain (Fig. 48B).

It was next asked whether KDM4A inhibition could prevent the copy gains generated during hypoxia. Since JmjC-containing proteins can be inhibited by the natural catabolite succinate (Black et al., 2012), RPE cells were treated with 2mM succinate prior to growth in hypoxia. Like SUV39h1 or HP1γ, succinate treatment did not alter KDM4A stabilization or cell cycle progression (Fig. 48A and Fig. 54A) but was sufficient to abrogate hypoxia-dependent copy gain of 1q12h (Fig. 48B). These data highlight the impact catabolites could have on copy number within cells.

The ability of succinate to inhibit hypoxia-dependent copy gains suggests that inhibitors of KDM4A could also function to block induction of copy gains during hypoxia. To test this hypothesis, the KDM4, KDM5A, KDM6B inhibitor JIB-04 was utilized (Wang et al., 2013). Treatment with JIB-04 did not substantially alter KDM4A protein levels in hypoxia (Fig. 48C). However, treatment with JIB-04 did significantly reduce hypoxia-dependent copy gain of 1q12h in RPE cells (Fig. 48D; †, p<0.05). Treatment with JIB-04 did not block hypoxia-dependent copy gain through cell cycle arrest, as the dose used did not result in significant differences in cell cycle between normoxic and hypoxic cells. Taken together, these results demonstrate that at least some hypoxia-dependent copy gains can be blocked by antagonizing KDM4A or by inhibiting KDM4A activity.

### DISCUSSION

Described herein is a mechanism through which cells respond to stress through generation of site-specific copy gains that does not require genetic manipulation or drug treatment. Cells exposed to a panel of cellular stresses that occur during development and tumorigenesis were systematically screened for site-specific copy gain. It was determined that cells exposed to physiological hypoxia (1% O₂), but not other physiological stresses, exhibited copy gain in as little as 24 hours. Hypoxia promoted site-specific gains not only in transformed cancer cells, but also primary human T cells. The generation of site-specific copy gains was conserved across species, as a syntenic region in zebrafish cells is also gained when exposed to hypoxia. Analysis of primary human tumors from TCGA demonstrated that breast and lung tumors that exhibit a hypoxic gene signature were associated with copy gains in the regions generated in human and zebrafish cell culture. Surprisingly, site-specific copy gains in response to hypoxia were mediated through stabilization of KDM4A. KDM4A protein levels strongly correlate with hypoxic induction and return to baseline when cells are returned to normoxia. Return of cells to normoxia, also leads to loss of the copy gained regions within 4 hours demonstrating that these gains are reversible. Finally, it was demonstrated that hypoxia-dependent copy gains are druggable, as pretreatment of cells with succinate or a KDM4A chemical inhibitor blocked hypoxia-induced copy gains. Described herein is a conserved response to hypoxia from zebrafish to man that generates site-specific copy gains. These results also highlight how hypoxia could contribute to tumor heterogeneity and suggest that KDM4A inhibitors may be useful co-therapeutics.

**Hypoxia and Genome Instability.** Hypoxia has long been associated with poor prognosis for cancer patients (Figs. 44A-44B)(Eschmann et al., 2005; Hockel et al., 1996; Wang et al., 2014). Hypoxia has been hypothesized to contribute to this poor prognosis through increasing metatstatic potential, increasing point mutation rates and increasing genomic instability, which includes gene amplifications and gene deletions (Rofstad, 2000). Increased genomic instability under hypoxia has been linked to induction of fragile sites resulting in gene amplification of neighboring regions (Coquelle et al., 1998). Hypoxia has been demonstrated to increase resistance to doxorubicin, methotrexate, and actinomycin D through gene amplification (Coquelle et al., 1998; Luk et al., 1990; Rice et al., 1986; Schimke et al., 1987; Young et al., 1988). These gene amplification events were often accompanied by overreplication of DNA and an increase in total cellular DNA content and it was not clear how large the DNA fragments containing gene amplifications were (Coquelle et al., 1998; Luk et al., 1990; Rice et al., 1986). In order to achieve effects on gene amplification rates, these studies utilized severe hypoxia, often less than 0.2% O₂ and required return of cells to normoxia (Coquelle et al., 1998; Luk et al., 1990; Rice et al., 1986; Schimke et al., 1987; Young et al., 1988). Most analyses of hypoxia-induced genome instability have been conducted in rodent cells, and the effects on gene amplification are not necessarily conserved in human cells (Sharma and Schimke, 1994).

In sharp contrast to previous reports, these results offer a conserved hypoxic response that highlights the reversibility of site-specific copy gains that are generated during hypoxic exposure. The results described herein also demonstrate that both primary cells and cancer cells are capable of generating this phenotype in a short timeline. It is demonstrated herein that regions undergo rereplication and that this is influenced by a specific enzyme. These observations emphasize the importance of chromatin enzymes in targeting rereplication and copy gain during hypoxia and establish a clear therapeutic target. Also described herein is an oxygen sensing mechanism for directly regulating copy gains, which has direct implications in understanding copy gain alterations in tumors and during development.

**Hypoxia and Tumor Heterogeneity.** Within a tumor, tumor subtypes and between metastases and primary there is a remarkable diversity in copy number alterations (Burrell et al., 2013). The mechanistic basis for how this heterogeneity develops remains an open question. Sequencing analyses have demonstrated the existence of subclones within a primary tumor that can be selected for based on treatment (Nathanson et al., 2014) or based on metastatic site (Campbell et al., 2010). Generally, these copy number events are believed to originate from aberrant DNA replication and repair events or aberrant mitoses so that heritable genetic alterations occur (Burrell et al., 2013; Hastings et al., 2009). However, even within the same small tumor biopsies, amplification of different receptor tyrosine kinases have been observed in adjacent cells (Snuderl et al., 2011). This suggests the existence of non-clonal, and perhaps non-genetic, events that contribute to heterogeneity. In agreement with this, analysis of EGFR mutations and amplifications in glioblastoma cancer patients revealed that treatment with EGFR inhibitors can select for a transient extrachromosomal amplification of a specific EGFR isoform (Nathanson et al., 2014). The data described herein suggests that tumors with hypoxic microenvironments will also contribute to intra-tumoral heterogeneity. It is demonstrated herein that hypoxic BRCA and LUAD samples have increased numbers of focal copy number changes when compared with non-hypoxic tumors (Figs. 44A-44B). While it is unclear if these focal changes are inherited or transient, amplification of 1q12h and 1q21.2 in tissue culture models in response to hypoxia are transient. Furthermore, the fact that 1q12h and 1q21.2 exist predominantly in exclusive cell populations implies that even within the same hypoxic region in the same tumor, different cells could have different amplified regions. Therefore, adjacent cells within a tumor would present "heterogeneity" even though the driving event (*i.e*., hypoxia) is the same. Since altered metabolism could enrich specific catabolites such as succinate, a natural inhibitor of demethylases, could also result in another characteristic influencing heterogeneity within a cell population. Taken together, the results described herein highlight the impact stress, metabolic state and proliferative capacity on copy number within cell populations.

**Conserved Hypoxic Copy Gains.** It is demonstrated herein that hypoxia induces copy gain of a syntenic region to human 1q21.2 (BCL9) in zebrafish cells. Importantly, this demonstrates that copy gains of related chromosomal domains are conserved across species in response to hypoxia. It is interesting to note that the surrounding gene position and chromosome architecture is conserved between human1q21.2 and zebrafish BCL9, indicating a conserved syntenic structure. In contrast, a zebrafish region homologous to human Xq13.1 IGBP1 locus, which was amplified in response to hypoxia (Fig. 49B), was not amplified in zebrafish cells. This region did not have a conserved genic or chromosomal architecture and was thus non-syntenic. This suggests that perhaps syntenic regions or chromosome domains might influence the ability for site-specific copy gains to occur.

**Stress and Copy Gain.** Identified herein are specific regions that are reversibly amplified in response to hypoxia. This observation suggests that other stress conditions may result in gains or losses of other regions. ROS, temperature, ER and metabolic stresses were tested (Figs. 42C-42H) and did site-specific copy gain of both 1q12h and 1q21.2 were not observed.

The results described herein indicate that cells utilize site-specific copy gained regions to help respond to stress, which can then be removed when cells return to favorable environmental conditions. The results described herein also suggest that different tumor microenvironments may induce copy gain of different, but specific genomic regions. Without wishing to be bound by theory, this would be one explanation for how tumors acquire intra-tumoral heterogeneity and how copy number could differ during tumor development. Furthermore, these results underscore how non-genetic alterations in the tumor microenvironment including the availability of oxygen or catabolites (i.e succinate) can contribute to or limit intra-tumoral heterogeneity (Junttila and de Sauvage, 2013).

Described herein are site-specific copy gains as a conserved response to hypoxia. While the present study identifies KDM4A as a key enzymatic regulator of this response.

### EXPERIMENTAL PROCEDURES

**Cell Culture and Transfections.** HEK293T (called 293T throughout), hTERT-RPE-1 (called RPE throughout), MDA-MB 231, MDA-MB 468, and UMRC2 cells were maintained in DMEM with 10% fetal bovine serum, 1% penicillin/streptomycin, and L-glutamine. SK-N-AS cells were maintained in DMEM/F12 (GIBCO) with 10% fetal bovine serum, 1% penicillin/streptomycin, and L-glutamine. Zebrafish AB.9 cells (Paw and Zon, 1999) were maintained in DMEM with 20% fetal bovine serum, 1% penicillin/streptomycin, and L-glutamine at 28°C. Transient transfection experiments were performed using Roche X-tremeGENE 9 DNA transfection reagent in OPTI-MEM™ I media (Gibco) for four hours or overnight. No selection was used in transient transfection experiments.

**Hypoxic Conditions.** Cells were plated onto culture dishes and allowed to adhere for 20-24hrs in normoxia (5% CO₂, 21% O₂, and 74% N₂). For hypoxic treatment, cells were maintained in a HERA Cell 150™ incubator (Thermo Scientific) flushed with 5% CO₂, 1% O₂ or 4% O₂, and balanced with N₂ for the duration of the experiment. Incubator calibrations were carried out by Bianchi Associates Calibrations/Verifications.

**Drug Treatments and Synchronization.** Cells were treated with the following chemical and metabolic stresses for 24hrs: 2µg/ml Tunicamycin (TU, Abcam), 60µM H₂O₂ (Thermo Fisher Scientific), reduced-serum DMEM (0.1% FBS), and Glucose-free DMEM (No Gluc, GIBCO). For heat shock (HS) treatment, cells were incubated at 43°C for 30min and returned to 37°C for 24hrs prior to collection. Irradiation was carried out using a Cesium-137 radioisotope and administered at a dose of 2Gy.

For G1/S synchronization, cells were treated with 2mM hydroxyurea (HU, Sigma) for 20hrs. To release, cells were washed twice with culture medium pre-conditioned in normoxia or hypoxia, and supplied with fresh pre-conditioned media. For JIB-04 treatment, normoxic cells were pre-treated with 62.5nM JIB-04 (Xcessbio) for 24hrs, then treated again with JIB-04 and either transferred to 1% O₂ or maintained in normoxia for an additional 24hrs. Succinate was administered at a final concentration of 2mM and cells were either maintained in normoxia for 72hrs or maintained in normoxia for 48hrs prior to being transferred to 1% O₂ for 24hrs.

**Fluorescent In Situ Hybridization (FISH).** FISH was performed as described in (Black et al., 2013; Manning et al., 2010). Probes for 1q12h, 1q telomere, chromosome 8 centromere (alpha satellite), and X centromere (alpha satellite) were purchased from Rainbow Scientific. Probes for Xq13.1 (RP11-177A4), Zebrafish BCL9 (CH73-15J19) and Zebrafish IGBP1 (CH73-223D24) were purchased as BAC clones from Children's Hospital Oakland Research Institute (CHORI BacPac) clone repository. Probes for 1q21.2 (BCL9) and 1q23.3 were purchased from Agilent (SureFISH).

**Human CD4+ T cell purification and *in vitro* culture.** Buffy coats (Sanguine Biosciences) or peripheral blood of healthy controls was diluted 1:2 in room-temperature PBS lacking Ca²⁺/Mg²⁺. Mononuclear cells were isolated by Ficoll-Paque Plus™ (GE Healthcare) density-gradient centrifugation following the manufacturer's protocol. PBMCs were resuspended at a density of 20x10⁶ cells/mL and reacted with Fc receptor blocking solution (Human TruStain FcX, Biolegend), followed by surface staining with APC anti-human CD4 antibody (Clone OKT4, Biolegend) for 45 minutes on ice. Antibody-stained cells were resuspended in HBSS (GIBCO) supplemented with 10mM glucose and sorted by flow cytometry. Sorted cells (including CD4+ T cells) were collected in 5mL tubes containing 1mL collection medium (DMEM supplemented with 30% FBS) and reanalyzed by flow cytometry to ensure ≥99% purity in defined gates. Sorted cells were allowed to recover in complete medium [RPMI (GIBCO) supplemented with 10% FBS] for 2 hours. For resting CD4+ T cell culture, cells were seeded onto 60mm dishes and maintained in complete medium supplemented with 10ng/mL recombinant human interleukin-2 (rhIL-2, R&D Systems). For stimulated CD4+ T cell culture, 60mm dishes were pre-coated with a cocktail containing 5µg/mL anti-human CD3 (Clone HIT3a, Biolegend) and 3µg/mL anti-human CD28 (Clone CD28.2, Biolegend) for 1 hour, after which cells were seeded onto the coated dish. Stimulated CD4+ T cells were maintained in complete medium supplemented with 10ng/mL rhIL-2, and anti-CD3/CD28 antibodies. Resting and stimulated CD4+ T cells were allowed to recover for 24 hours in normoxia (21% O₂), followed by an additional 24 hours in normoxia or in hypoxia (1% O₂) prior to being collected.

**Western Blots.** Western blots were performed as in (Black et al., 2010). Briefly, adherent cells were either scraped directly into PBS, or washed with PBS, trypsinized and collected by centrifuging at 2,000 RPM for 5 minutes. For preparation of whole-cell lysates, cell pellets were washed once in ice-cold PBS and resuspended in RIPA lysis buffer [50mM Tris pH 7.4, 150mM NaCl, 0.25% Sodium Deoxycholate, 1% NP40, 1mM EDTA, 10% Glycerol] supplemented with cOmplete protease inhibitor and PhosSTOP™ phosphatase inhibitor cocktails (Roche). Cells were lysed on ice for 15 minutes and immediately frozen at -80°C for 10min. Lysates were subsequently sonicated at 70% amplitude for 15 minutes in a QSonica Q800R™ and cleared of cell debris by centrifuging at 12,000RPM for 15 minutes, before being analyzed by western blotting. For HIF1α, expression, adherent cells were washed twice with ice-cold PBS and scraped directly in warmed 1x Laemmli buffer. Samples were sonicated at 70% amplitude for 15 minutes and boiled immediately prior to western blotting.

**Half-Life Determination.** Protein turnover was assessed as outlined in (Van Rechem et al., 2011). Briefly, cells maintained in normoxia and hypoxia were treated with 400µM Cycloheximide (Sigma) for the indicated time, after which lysates were prepared and analyzed by western blot.

**Cesium Chloride Gradient Centrifugation.** CsCl density gradient centrifugation was performed as in (Black et al., 2013). Briefly, RPE cells were grown in normoxia or 1% O₂ for 24 hours prior to addition of BrdU. Cells were labeled with BrdU for 12 hours and 45 minutes. Each rereplicated fraction was diluted to 15ng/ul stock and 7.5ng of rereplicated DNA pool was analyzed by qPCR on a Roche LC480™ using FastStart™ Universal SYBR Green™ Master Mix (Roche) following the manufacturer's instructions. 7.5ng of input DNA was analyzed by qPCR at the same time. Each sample was normalized to its own input prior to determination of fold-change in re-replication. Primers used in this study will be provided upon request.

**Flow Cytometry and Cell Cycle Analysis.** Asynchronously growing, or G1/S arrested cells were prepared and fixed as in (Black et al., 2010). Cells were stained with 10µM EdU for 1 hour prior to collection. Cell cycle was analyzed by PI staining or EdU incorporation using Click-IT EdU™ Flow Cytometry Assay Kit (Life Technologies). Flow cytometry of CD4+ T cells and cell cycle distribution were analyzed using a BD FACS ARIA II™.

**Data Processing for TCGA Breast Cancer and Lung Adenocarcinoma.** All genomic data of mutation, copy number, and mRNA expression for TCGA Breast Cancer (BRCA) and Lung Adenocarcinoma (LUAD) were downloaded from Broad GDAC (Genome Data Analysis Center) Firehose analysis run named "15 January 2014" (doi:10.7909/C1H41PXV).

**Time to Death versus Hypoxia.** A short follow-up time for survival (median survival time - 21.1 months in BRCA and 12.4 months in LUAD) and a high fraction of censored samples (820 out of 920 samples in BRCA and 313 out of 430 samples in LUAD) is a significant challenge in evaluating the association of hypoxia samples with clinical outcome. Instead, we examined the association with the number of deceased patients, illuminating a significant higher risk in the hypoxic samples in both BRCA (Fig. 44C, P= 0.00011) and LUAD (Figure 3D, P=0.0097) by the one-tailed Wilcoxon rank-sum test.

### REFERENCES

Alabert, C., and Groth, A. (2012). Chromatin replication and epigenome maintenance. Nat Rev Mol Cell Biol 13, 153-167.
Beroukhim, R., Mermel, C.H., Porter, D., Wei, G., Raychaudhuri, S., Donovan, J., Barretina, J., Boehm, J.S., Dobson, J., Urashima, M., et al. (2010). The landscape of somatic copy-number alteration across human cancers. Nature 463, 899-905.
Beyer, S., Kristensen, M.M., Jensen, K.S., Johansen, J.V., and Staller, P. (2008). The histone demethylases JMJD1A and JMJD2B are transcriptional targets of hypoxia-inducible factor HIF. J Biol Chem 283, 36542-36552.
Black, J.C., Allen, A., Van Rechem, C., Forbes, E., Longworth, M., Tschop, K., Rinehart, C., Quiton, J., Walsh, R., Smallwood, A., et al. (2010). Conserved antagonism between JMJD2A/KDM4A and HP1gamma during cell cycle progression. Mol Cell 40, 736-748.
Black, J.C., Manning, A.L., Van Rechem, C., Kim, J., Ladd, B., Cho, J., Pineda, C.M., Murphy, N., Daniels, D.L., Montagna, C., et al. (2013). KDM4A lysine demethylase induces site-specific copy gain and rereplication of regions amplified in tumors. Cell 154, 541-555.
Black, J.C., Van Rechem, C., and Whetstine, J.R. (2012). Histone lysine methylation dynamics: establishment, regulation, and biological impact. Mol Cell 48, 491-507.
Brunetti-Pierri, N., Berg, J.S., Scaglia, F., Belmont, J., Bacino, C.A., Sahoo, T., Lalani, S.R., Graham, B., Lee, B., Shinawi, M., et al. (2008). Recurrent reciprocal 1q21.1 deletions and duplications associated with microcephaly or macrocephaly and developmental and behavioral abnormalities. Nat Genet 40, 1466-1471. Burrell, R.A., McGranahan, N., Bartek, J., and Swanton, C. (2013). The causes and consequences of genetic heterogeneity in cancer evolution. Nature 501, 338-345.
Campbell, P.J., Yachida, S., Mudie, L.J., Stephens, P.J., Pleasance, E.D., Stebbings, L.A., Morsberger, L.A., Latimer, C., McLaren, S., Lin, M.L., et al. (2010). The patterns and dynamics of genomic instability in metastatic pancreatic cancer. Nature 467, 1109-1113.
Chervona, Y., and Costa, M. (2012). The control of histone methylation and gene expression by oxidative stress, hypoxia, and metals. Free Radic Biol Med 53, 1041-1047.
Cloos, P.A., Christensen, J., Agger, K., Maiolica, A., Rappsilber, J., Antal, T., Hansen, K.H., and Helin, K. (2006). The putative oncogene GASC1 demethylates tri- and dimethylated lysine 9 on histone H3. Nature 442, 307-311.
Coquelle, A., Toledo, F., Stern, S., Bieth, A., and Debatisse, M. (1998). A new role for hypoxia in tumor progression: induction of fragile site triggering genomic rearrangements and formation of complex DMs and HSRs. Mol Cell 2, 259-265.
Dimova, I., Orsetti, B., Theillet, C., Dimitrov, R., and Toncheva, D. (2009). Copy Number Changes in 1q21.3 and 1q23.3 have Different Clinical Relevance in Ovarian Tumors. Balkan Journal of Medical Genetics 12, 29-37.
Diskin, S.J., Hou, C., Glessner, J.T., Attiyeh, E.F., Laudenslager, M., Bosse, K., Cole, K., Mosse, Y.P., Wood, A., Lynch, J.E., et al. (2009). Copy number variation at 1q21.1 associated with neuroblastoma. Nature 459, 987-991.
Dunwoodie, S.L. (2009). The role of hypoxia in development of the Mammalian embryo. Dev Cell 17, 755-773.
Eschmann, S.M., Paulsen, F., Reimold, M., Dittmann, H., Welz, S., Reischl, G., Machulla, H.J., and Bares, R. (2005). Prognostic impact of hypoxia imaging with 18F-misonidazole PET in non-small cell lung cancer and head and neck cancer before radiotherapy. J Nucl Med 46, 253-260.
Fonseca, R., Van Wier, S.A., Chng, W.J., Ketterling, R., Lacy, M.Q., Dispenzieri, A., Bergsagel, P.L., Rajkumar, S.V., Greipp, P.R., Litzow, M.R., et al. (2006). Prognostic value of chromosome 1q21 gain by fluorescent in situ hybridization and increase CKS1B expression in myeloma. Leukemia 20, 2034-2040.
Gameiro, P.A., Yang, J., Metelo, A.M., Perez-Carro, R., Baker, R., Wang, Z., Arreola, A., Rathmell, W.K., Olumi, A., Lopez-Larrubia, P., et al. (2013). In vivo HIF-mediated reductive carboxylation is regulated by citrate levels and sensitizes VHL-deficient cells to glutamine deprivation. Cell Metab 17, 372-385.
Gerlinger, M., Rowan, A.J., Horswell, S., Larkin, J., Endesfelder, D., Gronroos, E., Martinez, P., Matthews, N., Stewart, A., Tarpey, P., et al. (2012). Intratumor heterogeneity and branched evolution revealed by multiregion sequencing. N Engl J Med 366, 883-892.
Giulino-Roth, L., Wang, K., MacDonald, T.Y., Mathew, S., Tam, Y., Cronin, M.T., Palmer, G., Lucena-Silva, N., Pedrosa, F., Pedrosa, M., et al. (2012). Targeted genomic sequencing of pediatric Burkitt lymphoma identifies recurrent alterations in antiapoptotic and chromatin-remodeling genes. Blood 120, 5181-5184.
Hastings, P.J., Lupski, J.R., Rosenberg, S.M., and Ira, G. (2009). Mechanisms of change in gene copy number. Nat Rev Genet 10, 551-564.
Hockel, M., Schlenger, K., Aral, B., Mitze, M., Schaffer, U., and Vaupel, P. (1996). Association between tumor hypoxia and malignant progression in advanced cancer of the uterine cervix. Cancer Res 56, 4509-4515.
Holmberg, K., Meijer, A.E., Harms-Ringdahl, M., and Lambert, B. (1998). Chromosomal instability in human lymphocytes after low dose rate gamma-irradiation and delayed mitogen stimulation. Int J Radiat Biol 73, 21-34.
Hook, S.S., Lin, J.J., and Dutta, A. (2007). Mechanisms to control rereplication and implications for cancer. Curr Opin Cell Biol 19, 663-671.
Inoue, J., Otsuki, T., Hirasawa, A., Imoto, I., Matsuo, Y., Shimizu, S., Taniwaki, M., and Inazawa, J. (2004). Overexpression of PDZK1 within the 1q12-q22 amplicon is likely to be associated with drug-resistance phenotype in multiple myeloma. Am J Pathol 165, 71-81.
Jiang, X.R., Jimenez, G., Chang, E., Frolkis, M., Kusler, B., Sage, M., Beeche, M., Bodnar, A.G., Wahl, G.M., Tlsty, T.D., and Chiu, C.P. (1999). Telomerase expression in human somatic cells does not induce changes associated with a transformed phenotype. Nat Genet 21, 111-114.
Junttila, M.R., and de Sauvage, F.J. (2013). Influence of tumour micro-environment heterogeneity on therapeutic response. Nature 501, 346-354.
Kiang, L., Heichinger, C., Watt, S., Bahler, J., and Nurse, P. (2010). Specific replication origins promote DNA amplification in fission yeast. J Cell Sci 123, 3047-3051.
Klose, R.J., Yamane, K., Bae, Y., Zhang, D., Erdjument-Bromage, H., Tempst, P., Wong, J., and Zhang, Y. (2006). The transcriptional repressor JHDM3A demethylates trimethyl histone H3 lysine 9 and lysine 36. Nature 442, 312-316.
Krieg, A.J., Rankin, E.B., Chan, D., Razorenova, O., Fernandez, S., and Giaccia, A.J. (2010). Regulation of the histone demethylase JMJD1A by hypoxia-inducible factor 1 alpha enhances hypoxic gene expression and tumor growth. Mol Cell Biol 30, 344-353.
Lee, H.Y., Yang, E.G., and Park, H. (2013). Hypoxia enhances the expression of prostate-specific antigen by modifying the quantity and catalytic activity of Jumonji C domain-containing histone demethylases. Carcinogenesis 34, 2706-2715.
Lestini, B.J., Goldsmith, K.C., Fluchel, M.N., Liu, X., Chen, N.L., Goyal, B., Pawel, B.R., and Hogarty, M.D. (2009). Mcl1 downregulation sensitizes neuroblastoma to cytotoxic chemotherapy and small molecule Bcl2-family antagonists. Cancer Biol Ther 8, 1587-1595.
Levinson, D.F., Duan, J., Oh, S., Wang, K., Sanders, A.R., Shi, J., Zhang, N., Mowry, B.J., Olincy, A., Little, J.B. (1998). Radiation-induced genomic instability. Int J Radiat Biol 74, 663-671.
Luk, C.K., Veinot-Drebot, L., Tjan, E., and Tannock, I.F. (1990). Effect of transient hypoxia on sensitivity to doxorubicin in human and murine cell lines. J Natl Cancer Inst 82, 684-692.
Mallette, F.A., Mattiroli, F., Cui, G., Young, L.C., Hendzel, M.J., Mer, G., Sixma, T.K., and Richard, S. (2012). RNF8- and RNF168-dependent degradation of KDM4A/JMJD2A triggers 53BP1 recruitment to DNA damage sites. Embo J.
Manning, A.L., Longworth, M.S., and Dyson, N.J. (2010). Loss of pRB causes centromere dysfunction and chromosomal instability. Genes Dev 24, 1364-1376.
Nathanson, D.A., Gini, B., Mottahedeh, J., Visnyei, K., Koga, T., Gomez, G., Eskin, A., Hwang, K., Wang, J., Masui, K., et al. (2014). Targeted therapy resistance mediated by dynamic regulation of extrachromosomal mutant EGFR DNA. Science 343, 72-76.
Papamichos-Chronakis, M., and Peterson, C.L. (2013). Chromatin and the genome integrity network. Nat Rev Genet 14, 62-75.
Paw, B.H., and Zon, L.I. (1999). Primary fibroblast cell culture. Methods Cell Biol 59, 39-43.
Perou, C.M. (2010). Molecular stratification of triple-negative breast cancers. Oncologist 15 Suppl 5, 39-48.
Rice, G.C., Hoy, C., and Schimke, R.T. (1986). Transient hypoxia enhances the frequency of dihydrofolate reductase gene amplification in Chinese hamster ovary cells. Proc Natl Acad Sci U S A 83, 5978-5982.
Rofstad, E.K. (2000). Microenvironment-induced cancer metastasis. Int J Radiat Biol 76, 589-605.
Schimke, R.T. (1984). Gene amplification, drug resistance, and cancer. Cancer Res 44, 1735-1742.
Schimke, R.T., Roos, D.S., and Brown, P.C. (1987). Amplification of genes in somatic mammalian cells. Methods Enzymol 151, 85-104.
Sharma, R.C., and Schimke, R.T. (1994). The propensity for gene amplification: a comparison of protocols, cell lines, and selection agents. Mutat Res 304, 243-260.
Shi, Y., and Whetstine, J.R. (2007). Dynamic regulation of histone lysine methylation by demethylases. Mol Cell 25, 1-14.
Slager, J., Kjos, M., Attaiech, L., and Veening, J.W. (2014). Antibiotic-induced replication stress triggers bacterial competence by increasing gene dosage near the origin. Cell 157, 395-406.
Snuderl, M., Fazlollahi, L., Le, L.P., Nitta, M., Zhelyazkova, B.H., Davidson, C.J., Akhavanfard, S., Cahill, D.P., Aldape, K.D., Betensky, R.A., et al. (2011). Mosaic amplification of multiple receptor tyrosine kinase genes in glioblastoma. Cancer Cell 20, 810-817.
Stefansson, H., Rujescu, D., Cichon, S., Pietilainen, O.P., Ingason, A., Steinberg, S., Fossdal, R.,
Stratton, M.R., Campbell, P.J., and Futreal, P.A. (2009). The cancer genome. Nature 458, 719-724.
Tan, M.K., Lim, H.J., and Harper, J.W. (2011). SCF(FBXO22) regulates histone H3 lysine 9 and 36 methylation levels by targeting histone demethylase KDM4A for ubiquitin-mediated proteasomal degradation. Mol Cell Biol 31, 3687-3699.
Van Rechem, C., Black, J.C., Abbas, T., Allen, A., Rinehart, C.A., Yuan, G.C., Dutta, A., and Whetstine, J.R. (2011). The SKP1-Cul1-F-box and leucine-rich repeat protein 4 (SCF-FbxL4) ubiquitin ligase regulates lysine demethylase 4A (KDM4A)/Jumonji domain-containing 2A (JMJD2A) protein. J Biol Chem 286, 30462-30470.
Vaupel, P. (2004). The role of hypoxia-induced factors in tumor progression. Oncologist 9 Suppl 5, 10-17.
Vrana, J.A., Bieszczad, C.K., Cleaveland, E.S., Ma, Y., Park, J.P., Mohandas, T.K., and Craig, R.W. (2002). An MCL1-overexpressing Burkitt lymphoma subline exhibits enhanced survival on exposure to serum deprivation, topoisomerase inhibitors, or staurosporine but remains sensitive to 1-beta-D-arabinofuranosylcytosine. Cancer Res 62, 892-900.
Wang, L., Chang, J., Varghese, D., Dellinger, M., Kumar, S., Best, A.M., Ruiz, J., Bruick, R., Pena-Llopis, S., Xu, J., et al. (2013). A small molecule modulates Jumonji histone demethylase activity and selectively inhibits cancer growth. Nat Commun 4, 2035.
Wang, W., He, Y.F., Sun, Q.K., Wang, Y., Han, X.H., Peng, D.F., Yao, Y.W., Ji, C.S., and Hu, B. (2014). Hypoxia-inducible factor 1alpha in breast cancer prognosis. Clin Chim Acta 428, 32-37.
Whetstine, J.R., Nottke, A., Lan, F., Huarte, M., Smolikov, S., Chen, Z., Spooner, E., Li, E., Zhang, G., Colaiacovo, M., and Shi, Y. (2006). Reversal of histone lysine trimethylation by the JMJD2 family of histone demethylases. Cell 125, 467-481.
Winter, S.C., Buffa, F.M., Silva, P., Miller, C., Valentine, H.R., Turley, H., Shah, K.A., Cox, G.J., Corbridge, R.J., Homer, J.J., et al. (2007). Relation of a hypoxia metagene derived from head and neck cancer to prognosis of multiple cancers. Cancer Res 67, 3441-3449.
Wykoff, C.C., Beasley, N.J., Watson, P.H., Turner, K.J., Pastorek, J., Sibtain, A., Wilson, G.D., Turley, H., Talks, K.L., Maxwell, P.H., et al. (2000). Hypoxia-inducible expression of tumor-associated carbonic anhydrases. Cancer Res 60, 7075-7083.
Young, S.D., Marshall, R.S., and Hill, R.P. (1988). Hypoxia induces DNA overreplication and enhances metastatic potential of murine tumor cells. Proc Natl Acad Sci U S A 85, 9533-9537.

### SUPPLEMENTAL EXPERIMENTAL PROCEDURES

**Fluorescent In Situ Hybridization (FISH).** FISH was performed as described in (Black et al., 2013; Manning et al., 2010). Probes for 1q12h, 1q telomere, chromosome 8 centromere (alpha satellite), and X centromere (alpha satellite) were purchased from Rainbow Scientific. Probes for Xq13.1 (RP11-177A4), Zebrafish BCL9 (CH73-15J19) and Zebrafish IGBP1 (CH73-223D24) were purchased as BAC clones from Children's Hospital Oakland Research Institute (CHORI BacPac) clone repository. Probes for 1q21.2 (BCL9) and 1q23.3 were purchased from Agilent (SureFISH). BACS were prepared utilizing PureLink HiPure™ Plasmid Filter Maxiprep kit (Life Technologies) using the recommended modified wash buffer. Probes were nick translated (Abbot Molecular Kit) in the presence of fluorescently labeled dTTP (Enzo Life Science). Images of multiple planes of fields of nuclei were acquired on an Olympus 1X81™ Spinning Disk Microscope and analyzed using Slidebook™ 5.0 software. A conservative scoring metric was used for copy gain. Any foci that were touching were scored as a single copy to prevent increased numbers due to normally replicated foci. For RPE cells, copy gain was scored as any cell with 3 or more distinct foci. For 293T cells, copy gain was scored for any cell with 5 or more distinct foci. For UMRC2 cells, copy gain was scored for any cell with 6 or more foci. For SK-N-AS cells, copy gain was scored for any cell with 5 or more foci. For MDA-MB-468 cells, copy gain was scored for any cell with 5 or more foci. For MDA-MB-231 cells, copy gain was scored for any cell with 6 or more foci. Approximately 100 cells for each replicate were scored for all experiments. All FISH experiments include at least 2 biological replicates. For each experiment at least one replicate includes FACS and western blot from the same cells used for FISH.

**Antibodies.** Antibodies used were: KDM4A (Neuro mAB, 75-189), β-actin (Millipore), RFP (Abcam, ab62341), Halo (Promega), Actinin (Santa Cruz, sc-17829), HA 12CA5 (Roche), HIF1α, (Santa Cruz, sc-10790), CAIX (Abcam, ab108351), LDH1 (Santa Cruz, sc-133123), Histone H3 (Abcam, ab1791), HA.11 (Covance).

**Expression Plasmids.** The pFN21A clone expressing an N-Terminal HaloTag™ fusion of human SUV39h1 (NM_014663) was obtained from Kazusa DNA Research Institute (Kisarazu, Japan). HaloTag™ (ADN27525.1) control vector (Promega) was used for expression of the HaloTag™ protein alone. MSCV-GFP-KDM4A, MSCV-RFP-HP1γ, pSuper and pSuper sh4A.2 constructs were made as described (Black et al., 2010). pCS2-3HA-KDM4A and pCS2-3HA-zebrafishKDM4A were prepared by gateway transfer into pCS2-3HA. All clones were sequence verified.

**Subcellular Localization and Catalytic Activity of KDM4A Deletion Fragments,** The indicated HA-tagged KDM4A constructs were transfected into RPE cells grown on coverslips in 6-well dishes using X-tremeGENE 9™ DNA transfection reagent (Roche). H3K36me3 and subcellular localization were assayed by examining transfected cells (positive for HA staining; HA.11 Covance) following fixation in 3% PFA in PBS (Whetstine et al., 2006).

**Data Processing for TCGA Breast Cancer and Lung Adenocarcinoma.** Copy Number Data: The segmented copy number data for 1007 BRCA samples and 493 LUAD samples was processed by GISITC2.0™ (Mermel et al., 2011) to annotate the somatic copy number alterations (SCNAs) for 24,174 genes. Copy-number data were dissociated to arm-level and focal copy-number alterations as described in the GISTIC2.0™ paper (Mermel et al., 2011). In addition to the copy number annotation for each gene, the mean focal copy number for 807 cytobands including X chromosome were calculated for each sample by taking the average of the focal SCNA values across all genes within a cytoband. The contribution of arm-level SCNAs to the mean cytoband focal copy was eliminated by only considering GISTIC annotated focal copy numbers spanning a much smaller region than a chromosome arm.

RNA-seq Data: The mRNA expression levels for 18264 genes in 1019 BRCA samples and 488 LUAD samples were annotated by the log₂-normalized RSEM (RNASeq by Expectation Maximization, (Li and Dewey, 2011)) values. RSEM values for 956 BRCA samples and 486 LUAD samples having copy number data were median-centered (by subtracting the median expression across tumor samples), yielding log₂ (Fold Changes) and utilized in the downstream analysis.

Somatic Mutation Data: The MAF (Mutation Annotation Format) file for 986 BRCA samples and 229 LUAD samples contained 73,729 and 92,133 somatic mutations, respectively.

BRCA subtype information: The subtype information for 504 BRCA samples based on PAM50 gene set was extracted from the supplemental data (BRCA.547.PAM50.SigClust.Subtypes.txt) of TCGA BRCA paper (2012).

**Hypoxia Signature Gene Set.** The hypoxia metagene (Winter et al., 2007), was downloaded from MSigDB (Subramanian et al., 2005) and used as a hypoxia signature gene set in a downstream analysis. The efficacy of this gene set was demonstrated as a significant prognostic factor for overall survivals in both HNSC and BRCA data set. The final hypoxia signature gene set (Tables 8 and 9) was comprised of 92 up-regulated (HS-up) and 52 down-regulated (HS-down) genes including well-known hypoxia biomarkers such as HIF1A, CA9, and VEGFA.

**Identifying Hypoxia Samples using Consensus Hierarchical Clustering.** Consensus hierarchical clustering was used to identify a cluster of samples that showed the most concordant expression pattern to the previously-defined hypoxia signature gene set (Winter et al., 2007). Using the mRNA expression data, the Spearman correlation coefficients were computed between pairs of samples using the median-centered log₂-normalized RSEM values. The consensus hierarchical clustering R package *ConsensusClusterPlus (Wilkerson and Hayes, 2010),* was applied with 1-Spearman correlation as a distance metric, and run over 1000 iterations of the "average linkage" method and 80% resampling rate. The number of clusters was varied from K=2 to 8. The hypoxia cluster was determined by examining the stability of the chosen cluster throughout K and the concordance of mRNA expression levels in each cluster to the known expression patterns of hypoxia up or down signatures. This process finally resulted in the choice of K=3 in BRCA (data not shown) and K=4 in LUAD (data not shown). Details described below:
(1) TCGA Breast Cancers. In the cluster membership of samples across K, the most hypoxia-related cluster at any given K (chosen based on the mean expression levels of the hypoxia-up genes) was highlighted in "black" and other colors represent different clusters (data not shown). At K=2 almost 60% samples belonged to the hypoxia cluster. Half of these samples were separated from the large black cluster and formed their own cluster (green) at K=3. The samples in the black cluster (35%) at K=3 had the most concordant expression pattern to both the up and down genes in the signature ("hypoxia-signature concordant cluster"), while the green cluster (42%) had an overall down-regulations regardless of hypoxia signatures ("hypoxia-signature neutral cluster"). On the other hand, the magenta cluster at K=3 (23%) had an opposite expression pattern to the known hypoxia signature ("hypoxia-signature discordant cluster"), which is also observed in the expression heatmap (data not shown). It was also observed that most samples in the black cluster at K=3 consistently remained in the hypoxic cluster up to K=8, indicating the strong stability of this cluster throughout K. Interestingly, the Basal (65 out of 88) and Her2 (31 out of 55) breast cancer subtypes were significantly enriched in the hypoxia cluster, while most Luminal A/B (322 out of 341) and eight Normal-like samples were in the non-hypoxia cluster.
(2) TCGA Lung Adenocarcinoma. Both clustering results at K=2 and 3 had a very similar stratification of samples except for two outlier samples (magenta) in K=3 (data not shown). Crossing from K=3 to 4 a small number of samples with a much weaker hypoxia-up signature were separated from the black hypoxia cluster, forming the green cluster at K=4. The majority of samples (42%) remained in the hypoxic cluster had the most concordant expression pattern to both up and down signatures ("hypoxia-signature concordant cluster"), while in the green cluster (11%) all hypoxia signature genes were down-regulated , hence called the "hypoxia-signature neutral cluster". On the contrary, the red cluster at K=4 (46%) had a largely discordant expression pattern with respect to the hypoxia signature (called the "hypoxia-signature discordant cluster"). Ignoring four outlier samples (magenta) at K=4 the stratification of samples into hypoxia-concordant, neutral, and discordant groups is analogous to the partitioning of BRCA at K=3. Most samples in the black cluster at K=4 remained in the hypoxic cluster up to K=8, demonstrating the strong stability of the chosen hypoxia cluster.

**Hypoxia Up and Down Scores.** In addition to the identification of hypoxia samples based on the clustering, hypoxic signature scores, HS-up and HS-down, were calculated based on the median of mRNA expression levels of the 92 up-regulated and 52 down-regulated genes, respectively. Samples were then ranked based on these scores. The HS-up (HS-down) scores were significantly higher (lower) in the determined hypoxic cluster in comparison to other clusters in both BRCA and LUAD.

**Detecting Chromosomal Regions Significantly Associated to Hypoxia.** Since the amplification of KDM4A can induce a site-specific copy gain at 1q21 (PMID: 23871696), samples with a focal copy gain of the 1p34.1 cytoband (where *KDM4A* resides) were excluded from downstream analysis (76 samples in BRCA and 46 samples in LUAD). Detecting chromosomal regions (*i.e.* cytobands) significantly associated with hypoxic samples was performed by the statistical test based on the normal approximation for the null distribution of mean cytoband copy difference between hypoxia and non-hypoxia samples. The null distribution was approximated by a normal density function with the population mean difference, m1 - m0, and the variances of S1/n1 + S0/n0. Here m1 and m0 are sample means, S1 and S0 are sample variances, and n1 and n0 are the number of samples in the hypoxia and the non-hypoxia group. The p-values for mean cytoband copy gains in hypoxia samples were computed by computing the probability of more extreme differences than the observed copy difference in the null distribution across 807 cytobands.

**Chromosomal Instability vs Hypoxia.** In order to examine whether the hypoxia samples had a significant enrichment of the chromosomal instability, the distributions of the number of cytobands harboring focal gains (mean cytoband focal copy > 0) or losses (mean cytoband focal copy < 0) per sample were compared between hypoxia and non-hypoxia samples in Fig. 44C for BRCA and Fig. 44D for LUAD.

### REFERENCES

(2012). Comprehensive molecular portraits of human breast tumours. Nature 490, 61-70.
Black, J.C., Allen, A., Van Rechem, C., Forbes, E., Longworth, M., Tschop, K., Rinehart, C., Quiton, J., Walsh, R., Smallwood, A., et al. (2010). Conserved antagonism between JMJD2A/KDM4A and HP1gamma during cell cycle progression. Mol Cell 40, 736-748.
Black, J.C., Manning, A.L., Van Rechem, C., Kim, J., Ladd, B., Cho, J., Pineda, C.M., Murphy, N., Daniels, D.L., Montagna, C., et al. (2013). KDM4A lysine demethylase induces site-specific copy gain and rereplication of regions amplified in tumors. Cell 154, 541-555.
Li, B., and Dewey, C.N. (2011). RSEM: accurate transcript quantification from RNA-Seq data with or without a reference genome. BMC Bioinformatics 12, 323.
Manning, A.L., Longworth, M.S., and Dyson, N.J. (2010). Loss of pRB causes centromere dysfunction and chromosomal instability. Genes Dev 24, 1364-1376.
Mermel, C.H., Schumacher, S.E., Hill, B., Meyerson, M.L., Beroukhim, R., and Getz, G. (2011). GISTIC2.0 facilitates sensitive and confident localization of the targets of focal somatic copy-number alteration in human cancers. Genome Biol 12, R41.
Subramanian, A., Tamayo, P., Mootha, V.K., Mukherjee, S., Ebert, B.L., Gillette, M.A., Paulovich, A., Pomeroy, S.L., Golub, T.R., Lander, E.S., and Mesirov, J.P. (2005). Gene set enrichment analysis: a knowledge-based approach for interpreting genome-wide expression profiles. Proc Natl Acad Sci U S A 102, 15545-15550.
Whetstine, J.R., Nottke, A., Lan, F., Huarte, M., Smolikov, S., Chen, Z., Spooner, E., Li, E., Zhang, G., Colaiacovo, M., and Shi, Y. (2006). Reversal of histone lysine trimethylation by the JMJD2 family of histone demethylases. Cell 125, 467-481.
Wilkerson, M.D., and Hayes, D.N. (2010). ConsensusClusterPlus: a class discovery tool with confidence assessments and item tracking. Bioinformatics 26, 1572-1573.
Winter, S.C., Buffa, F.M., Silva, P., Miller, C., Valentine, H.R., Turley, H., Shah, K.A., Cox, G.J., Corbridge, R.J., Homer, J.J., et al. (2007). Relation of a hypoxia metagene derived from head and neck cancer to prognosis of multiple cancers. Cancer Res 67, 3441-3449.

**Table 8: Hypoxia signature genes - upregulated by hypoxia**

| Signature Gene Name | NCBI Gene ID |
|---|---|
| ADORA2B | 136 |
| AK3L1 | 205 |
| ALDOA | 226 |
| ANGPTL4 | 51129 |
| ANKRD37 | 353322 |
| ANKRD9 | 122416 |
| ANLN | 54443 |
| B4GALT2 | 8704 |
| BCAR1 | 9564 |
| BMS1 | 9790 |
| BNIP3 | 664 |
| C16orf74 | 404550 |
| C20orf20 | 55257 |
| C7orf68 | 29923 |
| CA12 | 771 |
| CA9 | 768 |
| CDCA4 | 55038 |
| CNIH4 | 29097 |
| COL4A5 | 1287 |
| CORO1C | 23603 |
| DPM2 | 8818 |
| ECE2 | 9718 |
| EIF2S1 | 1965 |
| GAPDH | 2597 |
| SIP1 | 9839 |
| GMFB | 2764 |
| GPN3 | 51184 |
| GSS | 2937 |
| HAUS2 | 55142 |
| HES2 | 54626 |
| HOMER1 | 9456 |
| IGF2BP2 | 10644 |
| IL8 | 3576 |
| KCTD11 | 147040 |
| KRT17 | 3872 |
| LDHA | 3939 |
| LDLR | 3949 |
| METTL11A | 28989 |
| C16orf68 | 79091 |
| MIF | 4282 |
| MNAT1 | 4331 |
| MRPL14 | 64928 |
| MTX1 | 4580 |
| NDRG1 | 10397 |
| NDUFA4L2 | 56901 |
| NME1 | 4830 |
| NUDT15 | 55270 |
| P4HA1 | 5033 |
| PDZD11 | 51248 |
| PFKFB4 | 5210 |
| PGAM1 | 5223 |
| PGF | 5228 |
| PGK1 | 5230 |
| PLAU | 5328 |
| PLEKHG3 | 26030 |
| PPARD | 5467 |
| PPP4R1 | 9989 |
| PSMA7 | 5688 |
| PSMB7 | 5695 |
| PSMD2 | 5708 |
| PTGFRN | 5738 |
| PVR | 5817 |
| PYGL | 5836 |
| RAN | 5901 |
| RHOC | 389 |
| RNF24 | 11237 |
| RNPS1 | 10921 |
| RUVBL2 | 10856 |
| S100A10 | 6281 |
| S100A3 | 6274 |
| SLC16A1 | 6566 |
| SLC2A1 | 6513 |
| SLC6A8 | 6535 |
| SLCO1B3 | 28234 |
| C14orf156 | 81892 |
| SNX24 | 28966 |
| TANC2 | 26115 |
| TEAD4 | 7004 |
| TFAP2C | 7022 |
| TMEM189 | 387521 |
| TMEM30B | 161291 |
| TMTC3 | 160418 |
| TNS4 | 84951 |
| TPBG | 7162 |
| TPD52L2 | 7165 |
| TPI1 | 7167 |
| TRMT5 | 54570 |
| TUBB2C | 10383 |
| VAPB | 9217 |
| VEGFA | 7422 |
| VEZT | 55591 |
| XPO5 | 57510 |

**Table 9 - Hypoxia signature genes - downregulated by hypoxia**

| Signature Gene Name | NCBI Gene ID |
|---|---|
| ANKRD44 | 91526 |
| ARHGAP15 | 55843 |
| ARHGEF6 | 9459 |
| ARL6IP5 | 10550 |
| ATM | 472 |
| ATP8A1 | 10396 |
| BCL2 | 596 |
| C12orf35 | 55196 |
| CCL19 | 6363 |
| CD28 | 940 |
| CD48 | 962 |
| CD79A | 973 |
| CELF2 | 10659 |
| CHPT1 | 56994 |
| DOCK2 | 1794 |
| ENPP2 | 5168 |
| EVI2A | 2123 |
| EVI2B | 2124 |
| FAM65B | 9750 |
| FBLN5 | 10516 |
| FLI1 | 2313 |
| FRZB | 2487 |
| GIMAP1 | 170575 |
| GIMAP7 | 168537 |
| GYPC | 2995 |
| HLA-DOB | 3112 |
| HMHA1 | 23526 |
| ICAM2 | 3384 |
| IKZF1 | 10320 |
| IL16 | 3603 |
| INPP5D | 3635 |
| IRF8 | 3394 |
| ISCU | 23479 |
| ITM2A | 9452 |
| C13orf18 | 80183 |
| KLHDC1 | 122773 |
| LMO2 | 4005 |
| LOC100505549 | 100505549 |
| LOC100505746 | 100505746 |
| LRMP | 4033 |
| PARM1 | 25849 |
| PBXIP1 | 57326 |
| PTPRC | 5788 |
| RGS5 | 8490 |
| RHOH | 399 |
| SLC25A20 | 788 |
| SYNE1 | 23345 |
| SYNPO2 | 171024 |
| SYNRG | 11276 |
| TLR7 | 51284 |
| TRAPPC10 | 7109 |
| ZSCAN18 | 65982 |

## Claims

1. A composition comprising a therapeutically effective amount of an inhibitor of KDM4A and a chemotherapeutic agent for use in the treatment of cancer in a subject,
wherein the chemotherapeutic agent is an mTOR inhibitor selected from the group of rapamycin, everolimus, temsirolimus, AZD8055, AP-23573, AP-23481, LY294002, wortmannin, BEZ-235, BKM-120, BGT-226,
and wherein the inhibitor of KDM4A is a miRNA.

2. The composition for the use of claim 1, further comprising an ubiquitination inhibitor or proteasomal inhibitor.

3. The composition for the use of any of claims 1 or 2, wherein the cancer is selected from the group consisting of: ovarian cancer; non-small cell lung cancer; multiple myeloma; breast cancer; pancreatic cancer; head and neck cancer; lung cancer; adenocarcinoma; lung adenocarcinoma; lung squamous cell carcinoma; renal cancer; stomach cancer; melanoma; colorectal cancer; AML; and uterine and endometrial cancer.

4. The composition for the use of claim 1, wherein the cancer is non small cell lung cancer.

5. The composition for the use of claim 1, wherein the subject is determined to have a hypoxic tumor.

6. The composition for the use of claim 1, wherein the miRNA is miR23a (SEQ ID NO:21), miR23b (SEQ ID NO:22), miR200a (SEQ ID NO:23), miR200b (SEQ ID NO:24), miR200c (SEQ ID NO:25), or miR137a (SEQ ID NO:26).

## Patentansprüche

1. Zusammensetzung, umfassend eine therapeutisch wirksame Menge eines KDM4A-Inhibitorsund ein Chemotherapeutikum zur Verwendung bei der Behandlung von Krebs in einem Patienten, wobei das Chemotherapeutikum ein mTOR-Inhibitor ist, ausgewählt aus der Gruppe aus Rapamycin, Everolimus, Temsirolimus, AZD8055, AP-23573, AP-23481, LY294002, Wortmannin, BEZ-235, BKM-120, BGT-226,
und wobei der KDM4A-Inhibitor eine miRNA ist.

2. Zusammensetzung zur Verwendung nach Anspruch 1, weiter enthaltend einen Ubiquitinierungsinhibitor oder Proteasominhibitor.

3. Zusammensetzung zur Verwendung nach einem der Ansprüche 1 oder 2, wobei der Krebs ausgewählt ist aus der Gruppe bestehend aus: Eierstockkrebs; nichtkleinzelligem Lungenkrebs; multiplem Myelom; Brustkrebs; Bauchspeicheldrüsenkrebs; Kopf- und Halskrebs; Lungenkrebs; Adenokarzinom; Lungenadenokarzinom; Plattenepithelkarzinom der Lunge; Nierenkrebs; Magenkrebs; Melanom; Darmkrebs; AML; und Gebärmutter-und Gebärmutterschleimhautkrebs.

4. Zusammensetzung zur Verwendung nach Anspruch 1, wobei der Krebs nichtkleinzelliger Lungenkrebs ist.

5. Zusammensetzung zur Verwendung nach Anspruch 1, wobei bestimmt wird, dass der Patient einen hypoxischen Tumor hat.

6. Zusammensetzung zur Verwendung nach Anspruch 1, wobei die miRNA miR23a (SEQ-ID-NR: 21), miR23b(SEQ-ID-NR: 22), miR200a (SEQ-ID-NR: 23), miR200b(SEQ-ID-NR: 24), miR200c(SEQ-ID-NR: 25) oder miR137a (SEQ-ID-NR: 26) ist.

## Revendications

1. Composition comprenant une quantité thérapeutiquement efficace d'un inhibiteur de KDM4A et d'un agent chimiothérapeutique pour une utilisation dans le traitement du cancer chez un sujet,
dans laquelle l'agent chimiothérapeutique est un inhibiteur de mTOR choisi dans le groupe de la rapamycine, de l'évérolimus, du temsirolimus, de l'AZD8055, de l'AP-23573, de l'AP-23481, du LY294002, de la wortmannine, du BEZ-235, du BKM-120, du BGT-226,
et dans laquelle l'inhibiteur de KDM4A est un ARNmi.

2. Composition pour l'utilisation selon la revendication 1, comprenant en outre un inhibiteur d'ubiquitination ou un inhibiteur du protéasome.

3. Composition pour l'utilisation selon l'une quelconque des revendications 1 ou 2, dans laquelle le cancer est choisi dans le groupe constitué par : le cancer de l'ovaire ; le cancer du poumon non à petites cellules ; le myélome multiple ; le cancer du sein ; le cancer du pancréas; le cancer de la tête et du cou; le cancer du poumon; l'adénocarcinome ; l'adénocarcinome du poumon; le carcinome du poumon à cellules squameuses; le cancer du rein; le cancer de l'estomac ; le mélanome; le cancer colorectal ; la LAM ; et le cancer de l'utérus et de l'endomètre.

4. Composition pour l'utilisation selon la revendication 1, dans laquelle le cancer est un cancer du poumon non à petites cellules.

5. Composition pour l'utilisation selon la revendication 1, dans laquelle le sujet est déterminé avoir une tumeur hypoxique.

6. Composition pour l'utilisation selon la revendication 1, dans laquelle l'ARNmi est le miR23a (SEQ ID NO: 21), le miR23b (SEQ ID NO: 22), le miR200a (SEQ ID NO: 23), le miR200b (SEQ ID NO : 24), le miR200c (SEQ ID NO : 25), ou le miR137a (SEQ ID NO : 26).
